# EUROPEAN PATENT APPLICATION

(11) **EP 4 692 057 A1**
(43) Date of publication of application: **11.02.2026**
(21) Application number: 24784980.5
(22) Date of filing: 05.04.2024
(51) Int. Cl.: C07D 209/32, A61K 31/44, A61K 31/55, A61K 31/404, A61K 31/437, A61K 31/445, A61K 31/451, A61K 31/454, A61K 31/472, A61K 31/495, A61K 31/496, A61K 31/498, A61K 31/499, A61K 31/519, A61K 31/695, A61K 31/4035, A61K 31/4045, A61K 31/4365, A61K 31/4375, A61K 31/4725, A61K 31/4985

(54) **NOVEL B0AT1 INHIBITOR**

(30) Priority: 06.04.2023 JP 2023062369; 28.07.2023 JP 2023123729
(71) Applicant: Mitsubishi Tanabe Pharma Corporation, Osaka-shi, Osaka 541-8505 (JP)
(72) Inventor: IMAZU, Takuya, Osaka-shi, Osaka 541-8505 (JP); NAKAYAMA, Kazuki, Osaka-shi, Osaka 541-8505 (JP); SHIMOOKA, Kazunari, Osaka-shi, Osaka 541-8505 (JP)
(74) Representative: Ter Meer Steinmeister & Partner
(86) International application number: PCT/JP2024/014051
(87) International publication number: WO 2024/210198

(57) **Abstract**

The present invention aims to provide a novel B0AT1 inhibitor.

The present invention relates to a compound having a B0AT1 inhibitory action and represented by the following formula (I): 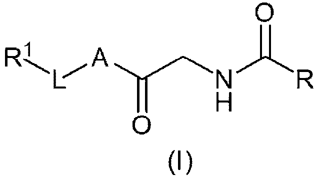 wherein each symbol is as defined in the specification, or a salt thereof. In addition, the present invention relates to a B0AT1 inhibitor containing the aforementioned compound and a pharmaceutical composition containing the aforementioned compound. One embodiment of the present invention is, for example, a medicament preferable for the prophylaxis and/or treatment of amino acid metabolism disorders such as phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, isovaleric acidemia and the like.

## Description

### [Technical Field]

The present invention relates to a novel compound having a neutral amino acid transporter B0AT1 inhibitory action.

### [Background Art]

SLC6A19, the causative gene for Hartnup disease, is localized to 5p15.33 and consists of 12 exons. Its gene product, the neutral amino acid transporter B0AT1, consists of 634 amino acids and has 12 transmembrane sites (Non-Patent Documents 1, 2). B0AT1 is a major transporter of neutral amino acids in the small intestine and kidney and is responsible for the absorption of glycine, leucine, phenylalanine, etc. in the small intestine and reabsorption thereof in the kidney (Non-Patent Documents 3 to 5). B0AT1 knockout mice are known to have elevated urinary amino acid levels (Non-Patent Document 6), and clinically, B0AT1 dysfunction also results in severe neutral amino aciduria, known as Hartnup disease (Non-Patent Document 7). Therefore, it has been thought that inhibition of B0AT1 might improve (alleviate) various diseases or conditions involving neutral amino acids, which are transport substrates.

In addition, specific examples of the therapeutic effects on diseases by inhibiting B0AT1 function have also been reported. For example, the pathology of model mouse of phenylketonuria is improved by causing congenital deficiency in B0AT1 or by inducing acquired inhibition of expression through administration of a nucleic acid compound that binds to mRNA (Non-Patent Document 4).

With this background, compounds that inhibit B0AT1 have attracted attention as drugs for the prophylaxis and/or treatment of various diseases or conditions involving neutral amino acids as transport substrates, specifically, amino acid metabolism disorders.

Examples of the amino acid metabolism disorder include designated intractable diseases such as phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, isovaleric acidemia and the like. As a treatment method therefor, continuous dietary therapy is mainly known. In addition to dietary therapy, administration of drugs that increase coenzymes and enzymes, and treatment methods based on injection of enzymes are also known, but none of these are fundamental treatments (e.g., Non-Patent Document 8, etc.).

Hence, there is a need to develop agents to prevent and/or treat diseases, specifically, amino acid metabolism disorders that can be alleviated by normalizing neutral amino acid metabolism through inhibition of B0AT1.

So far, as compounds with B0AT1 inhibitory activity are known, for example, nucleic acids having sequences that partially or completely match the RNA of B0AT1 (Non-Patent Document 4), nimesulide and its derivatives (Non-Patent Documents 9 and 10), which are existing marketed products, synromide (Non-Patent Document 11), which is a publicly known compound, and basic compounds found in library screening (Non-Patent Documents 12, 13), and the like have been known. In addition, cinnamic acid glycineamide compounds are also known (Patent Literature 1).

### [Citation List]

### [Patent Literature]

[Patent Literature 1]
   WO 2023/145804

### [Non Patent Literature]

[Non Patent Literature 1]
   Nat. Genet., 2004; 36: p.999-1002
[Non Patent Literature 2]
   Nat. Genet., 2004; 36: p.1003-1007
[Non Patent Literature 3]
   The Journal of Biological Chemistry 2004; 279: p.24467-24476
[Non Patent Literature 4]
   JCI Insight., 2018; 3(14): p.e121762
[Non Patent Literature 5]
   IUBMB Life, 2009; 61(6): p.591-599
[Non Patent Literature 6]
   MOLECULAR METABOLISM, 2015: p.406-417
[Non Patent Literature 7]
   Biochem. J., 2005; 389: p.745-751
[Non Patent Literature 8]
   Intractable Disease Information Center, Phenylketonuria (designated intractable disease 240) (http://www.nanbyou.or.jp/entry/4747)
[Non Patent Literature 9]
   Biochemical Phamracology, 2014; 89: p.422-430
[Non Patent Literature 10]
   Bioorganic and Medicinal chemistry letters, 2021; 53: p.128421
[Non Patent Literature 11]
   SLAS Discovery, 2019; 24(2): p.111-120
[Non Patent Literature 12]
   British Journal of Pharmacology, 2017; 174: p.468-482
[Non Patent Literature 13]
   Frontiers in Pharmacology, 2020; 11: p.140

### [Summary of Invention]

### [Technical Problem]

The present invention provides a novel compound having a superior B0AT1 inhibitory action, and a medicament containing same as an active ingredient.

### [Solution to Problem]

The present inventors have conducted intensive studies and found that a compound represented by the following formula (I): 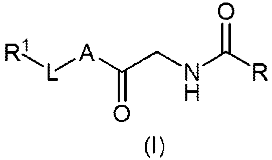 wherein
R is an optionally substituted C₆₋₁₀ aryl-ethenyl group;
R¹ is a carboxy group or a group biologically equivalent to a carboxy group;
L is a divalent organic group comprising a group selected from the group consisting of a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, each of which is optionally further substituted, and
A is a divalent nitrogen-containing heterocyclic group optionally further substituted
(hereinafter sometimes abbreviated as "compound (I)"), and a pharmaceutically acceptable salt thereof have a superior inhibitory action on neutral amino acid transporter B0AT1, and completed the present invention.

That is, the present invention provides the following.
[1] A compound represented by the formula (I): 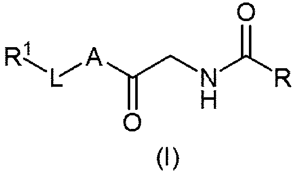 wherein
   R is an optionally substituted C₆₋₁₀ aryl-ethenyl group;
   R¹ is a carboxy group, or a group biologically equivalent to a carboxy group;
   L is a divalent organic group comprising a group selected from the group consisting of a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, each of which is optionally further substituted; and
   A is a divalent nitrogen-containing heterocyclic group optionally further substituted,
   or a pharmaceutically acceptable salt thereof.
[2] The compound described in the above-mentioned [1] or a pharmaceutically acceptable salt thereof, wherein, in the formula (I),
   R is a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group;
   R¹ is a carboxy group, or a group biologically equivalent to a carboxy group;
   L is a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from substituent group a; and
   A is a divalent nitrogen-containing non-aromatic heterocyclic group represented by the following formula: 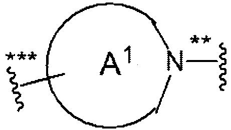
   wherein ** is a bonding site with a carbonyl group, *** is a bonding site with group L, and ring A¹ is a 3- to 10-membered monocycle, a 8- to 14-membered fused cycle, a 6- to 16-membered spiro cycle, or a 6- to 14-membered bridged cycle, and optionally has, as a ring-constituting atom besides a nitrogen atom and a carbon atom, hetero atom(s) selected from the group consisting of a nitrogen atom, an oxygen atom, and a sulfur atom, which is optionally further substituted by 1 to 3 substituents selected from substituent group a
      (Substituent group a):
         a halogen atom;
         a hydroxy group;
         a cyano group;
         an oxo group;
         a C₁₋₆ alkyl group optionally substituted by substituent(s) selected from the group consisting of a halogen atom, a hydroxy group, a cyano group, a C₁₋₆ alkoxy group, a C₆₋₁₄ aryl group, a carboxy group, a C₁₋₆ alkoxy-carbonyl group, a carbamoyl group optionally substituted by 1 or 2 C₁₋₆ alkyl groups, and a C₆₋₁₄ aryloxy group;
         a C₁₋₆ alkoxy group optionally substituted by halogen atom(s);
         a C₁₋₆ alkylsulfonyl group optionally substituted by halogen atom(s);
         a C₁₋₆ alkyl-carbonyl group;
         a C₁₋₆ alkoxy-carbonyl group;
         a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
         a di-C₁₋₆ alkylamino group;
         a tri-substituted silyl group;
         a tri-substituted silyl oxy group;
         a C₆₋₁₄ hydrocarbocyclic group optionally substituted by 1 to 3 substituents selected from substituent group b or substituent group b';
         a 5- to 14-membered aromatic heterocyclic group optionally substituted by 1 to 3 substituents selected from substituent group b or substituent group b'; and
         a 3- to 14-membered non-aromatic heterocyclic group optionally substituted by 1 to 3 substituents selected from substituent group b or substituent group b'
      (Substituent group b):
         a halogen atom;
         a hydroxy group;
         a cyano group;
         an oxo group;
         a carboxy group;
         a C₁₋₆ alkyl group optionally substituted by substituent(s) selected from the group consisting of a halogen atom, a hydroxy group, a cyano group, and a C₁₋₆ alkoxy group;
         a C₃₋₈ cycloalkyl group optionally substituted by substituent(s) selected from the group consisting of a halogen atom, a hydroxy group, a cyano group, and a C₁₋₆ alkoxy group;
         a C₆₋₁₀ aryl group optionally substituted by substituent(s) selected from the group consisting of a halogen atom, a hydroxy group, a cyano group, a C₁₋₆ alkyl group, and a C₁₋₆ alkoxy group;
         a 5- to 14-membered aromatic heterocyclic group optionally substituted by substituent(s) selected from the group consisting of a halogen atom, a hydroxy group, a cyano group, a C₁₋₆ alkyl group, and a C₁₋₆ alkoxy group;
         a 3- to 14-membered non-aromatic heterocyclic group optionally substituted by substituent(s) selected from the group consisting of a halogen atom, a hydroxy group, a cyano group, a C₁₋₆ alkyl group, and a C₁₋₆ alkoxy group;
         a C₁₋₆ alkoxy group optionally substituted by substituent(s) selected from the group consisting of a halogen atom, a hydroxy group, a cyano group, and a C₁₋₆ alkoxy group;
         a C₁₋₆ alkylsulfanyl group optionally substituted by substituent(s) selected from the group consisting of a halogen atom, a hydroxy group, a cyano group, and a C₁₋₆ alkoxy group;
         a C₃₋₈ cycloalkyloxy group optionally substituted by substituent(s) selected from the group consisting of a halogen atom, a hydroxy group, a cyano group, and a C₁₋₆ alkoxy group;
         a C₁₋₆ alkylsulfonyl group optionally substituted by halogen atom(s);
         a C₁₋₆ alkyl-carbonyl group optionally substituted by halogen atom(s);
         a C₁₋₆ alkoxy-carbonyl group;
         a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
         a sulfamoyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from the group consisting of a halogen atom, a hydroxy group, a cyano group, and a C₁₋₆ alkoxy group; and
         a pentafluorosulfanyl group
      (Substituent group b'):
         a halogen atom;
         a hydroxy group;
         a cyano group;
         an oxo group;
         a carboxy group;
         a C₁₋₆ alkyl group optionally substituted by substituent(s) selected from the group consisting of a halogen atom, a hydroxy group, a cyano group, and a C₁₋₆ alkoxy group;
         a C₃₋₈ cycloalkyl group optionally substituted by substituent(s) selected from the group consisting of a halogen atom, a hydroxy group, a cyano group, and a C₁₋₆ alkoxy group;
         a C₁₋₆ alkoxy group optionally substituted by substituent(s) selected from the group consisting of a halogen atom, a hydroxy group, a cyano group, and a C₁₋₆ alkoxy group;
         a C₁₋₆ alkylsulfanyl group optionally substituted by substituent(s) selected from the group consisting of a halogen atom, a hydroxy group, a cyano group, and a C₁₋₆ alkoxy group;
         a C₃₋₈ cycloalkyloxy group optionally substituted by substituent(s) selected from the group consisting of a halogen atom, a hydroxy group, a cyano group, and a C₁₋₆ alkoxy group;
         a C₁₋₆ alkylsulfonyl group optionally substituted by halogen atom(s);
         a C₁₋₆ alkyl-carbonyl group optionally substituted by halogen atom(s);
         a C₁₋₆ alkoxy-carbonyl group;
         a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
         a sulfamoyl group substituted by one substituent selected from the group consisting of a C₁₋₆ alkyl group, a C₃₋₈ cycloalkyl group and a non-aromatic heterocyclic group, each of which is optionally substituted by 1 to 3 substituents selected from the group consisting of a halogen atom, a hydroxy group, a cyano group, and a C₁₋₆ alkoxy group; and
         a pentafluorosulfanyl group.
[3] The compound described in the above-mentioned [1] or a pharmaceutically acceptable salt thereof, wherein, in the formula (I),
   R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group;
   R¹ is a carboxy group or a group biologically equivalent to a carboxy group;
   L is a C₁₋₆ alkylene group optionally having, in the inside or at the end, one or more divalent groups selected from the group consisting of
      -NR²- (wherein R² is a hydrogen atom or a C₁₋₆ alkyl group), -O-, -S-, -C(=O)-, a C₆₋₁₀ arylene group, a C₃₋₁₀ cycloalkylene group, a C₂₋₆ alkynylene group, and a 3- to 8-membered divalent non-aromatic heterocyclic group, and optionally further substituted by 1 to 3 substituents selected from the aforementioned substituent group a,
   a C₆₋₁₀ arylene group optionally further substituted by 1 to 3 substituents selected from the aforementioned substituent group a,
   a C₃₋₁₀ cycloalkylene group optionally further substituted by 1 to 3 substituents selected from the aforementioned substituent group a,
   a C₂₋₄ alkynylene group optionally further substituted by 1 to 3 substituents selected from the aforementioned substituent group a, or
   a 3- to 8-membered divalent non-aromatic heterocyclic group optionally further substituted by 1 to 3 substituents selected from the aforementioned substituent group a; and
   A is a divalent nitrogen-containing non-aromatic heterocyclic group represented by the following formula: 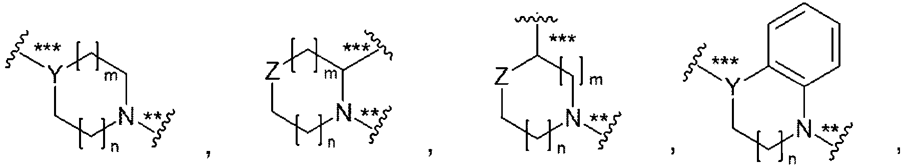 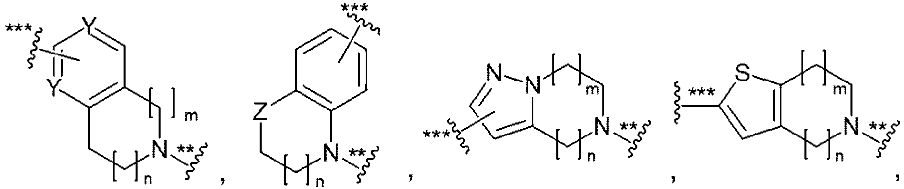 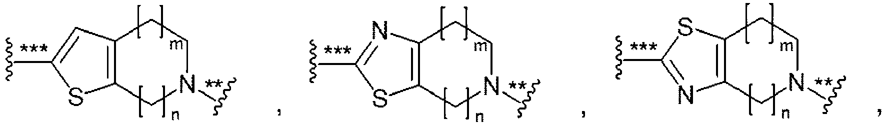 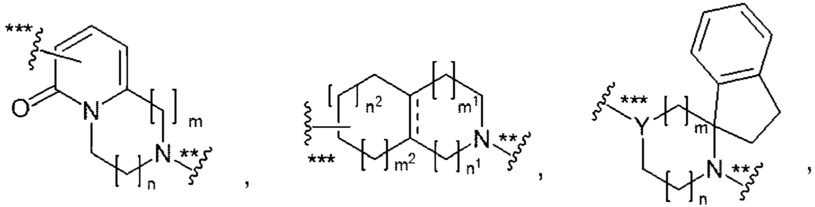 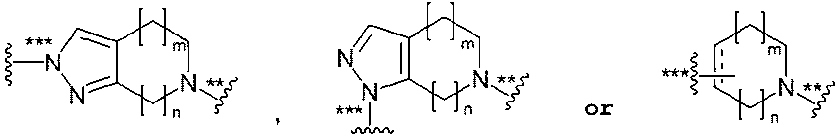
   wherein a wavy line is a bonding site of the group represented by the above-mentioned chemical formula,
   a bond represented by -̅ -̅ -̅ -̅ -̅ -̅
   is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L,
   which non-aromatic heterocyclic group is optionally further substituted by 1 to 3 substituents selected from the aforementioned substituent group a.
[3'] The compound described in the above-mentioned [1] or a pharmaceutically acceptable salt thereof, wherein, in the formula (I),
   R is a 2-(E)-phenyl-ethenyl group optionally substituted by 1 to 3 substituents selected from the group consisting of a halogen atom, a C₁₋₆ alkyl group optionally substituted by halogen atom(s), and a pentafluorosulfanyl group;
   R¹ is a carboxy group or a group biologically equivalent to a carboxy group;
   L is a C₁₋₆ alkylene group optionally having, in the inside or at the end, one or more divalent groups selected from the group consisting of
      -NR²- (wherein R² is a hydrogen atom or a C₁₋₆ alkyl group), -O-, -S-, -C(=O)-, a C₆₋₁₀ arylene group, a C₃₋₁₀ cycloalkylene group, a C₂₋₆ alkynylene group, and a 3- to 8-membered divalent non-aromatic heterocyclic group, and optionally further substituted by 1 to 3 substituents selected from the aforementioned substituent group a,
   a C₆₋₁₀ arylene group optionally further substituted by 1 to 3 substituents selected from the aforementioned substituent group a,
   a C₃₋₁₀ cycloalkylene group optionally further substituted by 1 to 3 substituents selected from the aforementioned substituent group a,
   a C₂₋₄ alkynylene group optionally further substituted by 1 to 3 substituents selected from the aforementioned substituent group a, or
   a 3- to 8-membered divalent non-aromatic heterocyclic group optionally further substituted by 1 to 3 substituents selected from the aforementioned substituent group a; and
   A is a divalent nitrogen-containing non-aromatic heterocyclic group represented by the following formula: 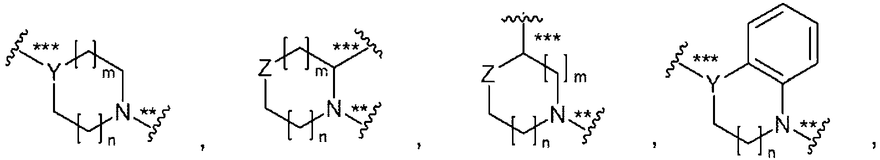 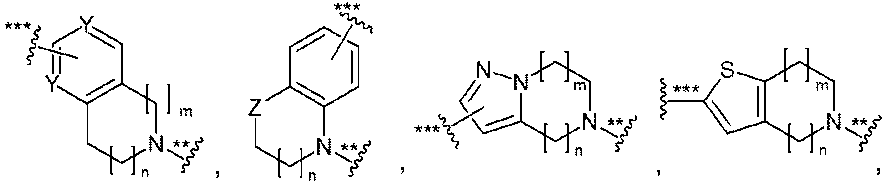 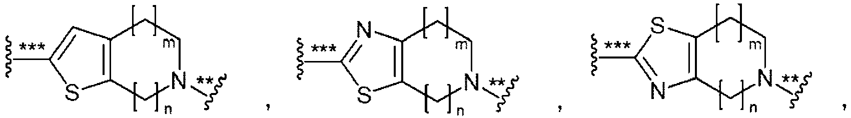 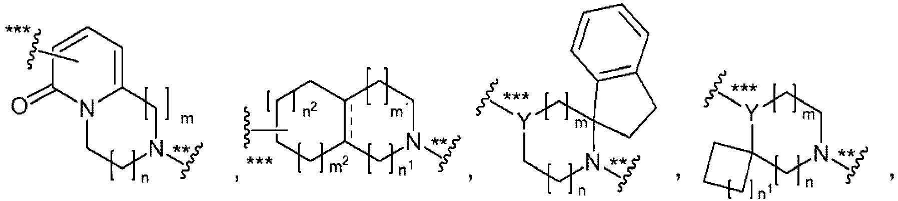 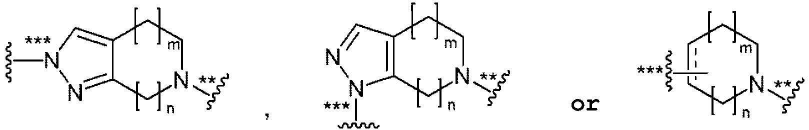
   wherein a wavy line is a bonding site of the group represented by the above-mentioned chemical formula,
   a bond represented by
      -̅ -̅ -̅ -̅ -̅ -̅
   is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L,
   which non-aromatic heterocyclic group is optionally further substituted by 1 to 3 substituents selected from the aforementioned substituent group a.
[4] The compound described in the above-mentioned [3] or [3'] or a pharmaceutically acceptable salt thereof, wherein, in the formula (I),
   L is a C₁₋₆ alkylene group optionally having, in the inside or at the end, one or more divalent groups selected from the group consisting of
      -NR²- (wherein R² is a hydrogen atom or a C₁₋₆ alkyl group), -O-, -S-, a C₃₋₁₀ cycloalkylene group, and a 3- to 8-membered divalent non-aromatic heterocyclic group, and optionally further substituted by 1 to 3 substituents selected from the aforementioned substituent group a,
   a C₃₋₁₀ cycloalkylene group optionally further substituted by 1 to 3 substituents selected from the aforementioned substituent group a, or
   a 3- to 8-membered divalent non-aromatic heterocyclic group optionally further substituted by 1 to 3 substituents selected from the aforementioned substituent group a.
[5] The compound described in the above-mentioned [3] or [3'] or a pharmaceutically acceptable salt thereof, wherein, in the formula (I),
   L is a C₁₋₆ alkylene group optionally further substituted by 1 to 3 substituents selected from the aforementioned substituent group a.
[6] The compound described in the above-mentioned [5] or a pharmaceutically acceptable salt thereof, wherein, in the formula (I),
   A is a divalent nitrogen-containing non-aromatic heterocyclic group represented by the following formula: 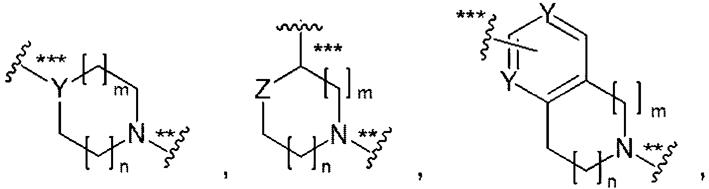 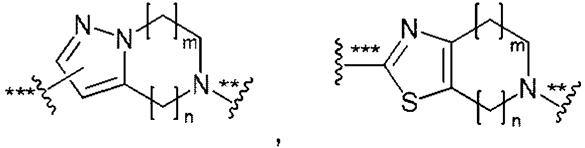 or 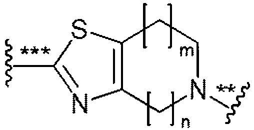
   (wherein each symbol is as defined above), and optionally further substituted by 1 to 3 substituents selected from the aforementioned substituent group a.
[7] The compound described in the above-mentioned [5] or a pharmaceutically acceptable salt thereof, wherein, in the formula (I),
   A is a divalent nitrogen-containing non-aromatic heterocyclic group represented by the following formula: 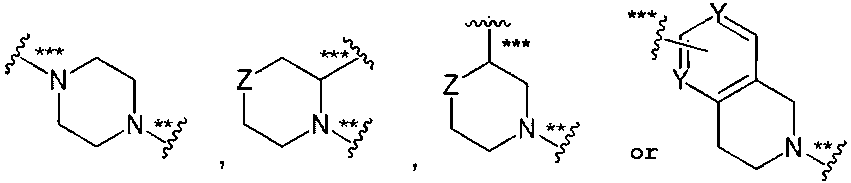
   (wherein each symbol is as defined above, provided that when Z is CR⁵R⁶, the optionally further substituted substituent is selected from the group consisting of, among the substituents in the aforementioned substituent group a, a C₆₋₁₄ hydrocarbocyclic group optionally substituted by 1 to 3 substituents selected from substituent group b or substituent group b'; a 5- to 14-membered aromatic heterocyclic group optionally substituted by 1 to 3 substituents selected from substituent group b or substituent group b'; and a 3- to 14-membered non-aromatic heterocyclic group optionally substituted by 1 to 3 substituents selected from substituent group b or substituent group b'), and optionally further substituted by 1 to 3 substituents selected from the aforementioned substituent group a.
[8] The compound described in the above-mentioned [5] or a pharmaceutically acceptable salt thereof, wherein, in the formula (I),
   A is a divalent nitrogen-containing non-aromatic heterocyclic group represented by the following formula: 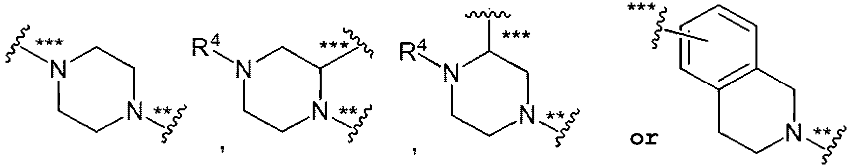
   (wherein each symbol is as defined above), and optionally further substituted by 1 to 3 substituents selected from the aforementioned substituent group a.
[9] The compound described in the above-mentioned [5] or a pharmaceutically acceptable salt thereof, wherein, in the formula (I),
   A is a divalent nitrogen-containing non-aromatic heterocyclic group represented by the following formula: 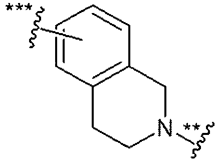
   (wherein each symbol is as defined above), and optionally further substituted by 1 to 3 substituents selected from the aforementioned substituent group a.
[9'] The compound described in the above-mentioned [5] or a pharmaceutically acceptable salt thereof, wherein, in the formula (I),
   A is a divalent nitrogen-containing non-aromatic heterocyclic group represented by the following formula: 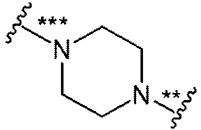
   (wherein each symbol is as defined above), and optionally further substituted by 1 to 3 substituents selected from the aforementioned substituent group a.
[10] The compound described in any of the above-mentioned [1] to [9], [3'], and [9'], or a pharmaceutically acceptable salt thereof, wherein, in the formula (I),
   R¹ is a carboxy group, an optionally substituted C₁₋₆ alkoxy-carbonyl group, or an optionally substituted carbamoyl group.
[11] A compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
   3-[2-[2-[[(E)-3-[4-(trifluoromethyl)phenyl]prop-2-enoyl]amino]acetyl]-3,4-dihydro-1H-isoquinolin-6-yl]propanoic acid;
   N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
   1-[7-[2-[[(E)-3-[4-(trifluoromethyl)phenyl]prop-2-enoyl]amino]acetyl]-6,8-dihydro-5H-2,7-naphthyridin-3-yl]piperidine-4-carboxylic acid;
   3-[3-bromo-5-[2-[[(E)-3-[4-(trifluoromethyl)phenyl]prop-2-enoyl]amino]acetyl]-6,7-dihydro-4H-pyrazolo[1,5-a]pyrazin-2-yl]propanoic acid;
   2-[[2-[2-[[(E)-3-[4-(trifluoromethyl)phenyl]prop-2-enoyl]amino]acetyl]-3,4-dihydro-lH-isoquinolin-6-yl]oxy]acetic acid;
   2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
   (1R*,2R*)-2-[2-[2-[[(E)-3-[4-(trifluoromethyl)phenyl]prop-2-enoyl]amino]acetyl]-3,4-dihydro-1H-isoquinolin-6-yl]cyclopropane-1-carboxylic acid and both enantiomers thereof;
   2-[2-[2-[[(E)-3-[4-(trifluoromethyl)phenyl]prop-2-enoyl]amino]acetyl]-3,4-dihydro-1H-isoquinolin-6-yl]propanoic acid;
   2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[7-[2-[[(E)-3-[4-(trifluoromethyl)phenyl]prop-2-enoyl]amino]acetyl]-6,8-dihydro-5H-2,7-naphthyridin-3-yl]acetic acid;
   3-[3-bromo-5-[2-[[(E)-3-[4-(pentafluoro-λ6-sulfanyl)phenyl]prop-2-enoyl]amino]acetyl]-6,7-dihydro-4H-pyrazolo[1,5-a]pyrazin-2-yl]propanoic acid;
   2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[[2-[2-[[(E)-3-[4-(trifluoromethyl)phenyl]prop-2-enoyl]amino]acetyl]-3,4-dihydro-1H-isoquinolin-6-yl]amino]acetic acid;
   2-[2-[2-[[(E)-3-(4-iodophenyl)prop-2-enoyl]amino]acetyl]-3,4-dihydro-1H-isoquinolin-6-yl]acetic acid;
   2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[8-cyano-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-(2-fluoro-4-iodophenyl)prop-2-enoyl]amino]acetyl]-3,4-dihydro-1H-isoquinolin-6-yl]acetic acid;
   2-[8-fluoro-2-[2-[[(E)-3-(4-iodophenyl)prop-2-enoyl]amino]acetyl]-3,4-dihydro-1H-isoquinolin-6-yl]acetic acid;
   2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[[5-[2-[[(E)-3-[4-(trifluoromethyl)phenyl]prop-2-enoyl]amino]acetyl]-6,7-dihydro-4H-[1,3]thiazolo[5,4-c]pyridin-2-yl]sulfanyl]acetic acid;
   2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
   2-[8-cyano-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   (E)-N-[2-[6-(2-amino-2-oxoethyl)-3,4-dihydro-1H-isoquinolin-2-yl]-2-oxoethyl]-3-[4-(trifluoromethyl)phenyl]prop-2-enamide;
   2-[5-[2-[[(E)-3-[4-(trifluoromethyl)phenyl]prop-2-enoyl]amino]acetyl]-6,7-dihydro-4H-[1,3]thiazolo[4,5-c]pyridin-2-yl]acetic acid;
   (E)-N-[2-[6-[2-(methylamino)-2-oxoethyl]-3,4-dihydro-1H-isoquinolin-2-yl]-2-oxoethyl]-3-[4-(trifluoromethyl)phenyl]prop-2-enamide;
   4-[4-[2-[[(E)-3-[2-fluoro-4-(trifluoromethyl)phenyl]prop-2-enoyl]amino]acetyl]-3-phenylpiperazin-1-yl]butanoic acid and both enantiomers thereof;
   2-[[1-[2-[[(E)-3-[4-(trifluoromethyl)phenyl]prop-2-enoyl]amino]acetyl]-2,3-dihydroindol-4-yl]oxy]acetic acid;
   1-[1-[2-[[(E)-3-[4-(trifluoromethyl)phenyl]prop-2-enoyl]amino]acetyl]piperidin-3-yl]bicyclo[2.1.1]hexane-5-carboxylic acid;
   2-[[8-fluoro-2-[2-[[(E)-3-[4-(trifluoromethyl)phenyl]prop-2-enoyl]amino]acetyl]-3,4-dihydro-1H-isoquinolin-6-yl]oxy]-2-methylpropanoic acid;
   3-[[8-fluoro-2-[2-[[(E)-3-[4-(trifluoromethyl)phenyl]prop-2-enoyl]amino]acetyl]-3,4-dihydro-1H-isoquinolin-6-yl]oxy]-2,2-dimethylpropanoic acid;
   4-[4-[2-[[(E)-3-[2-fluoro-4-(trifluoromethyl)phenyl]prop-2-enoyl]amino]acetyl]-2-methylpiperazin-1-yl]butanoic acid;
   4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
   4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
   4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
   3-[[3-(4-chlorophenyl)-4-[2-[[(E)-3-[2-fluoro-4-(trifluoromethyl)phenyl]prop-2-enoyl]amino]acetyl]piperazin-1-yl]methyl]cyclobutane-1-carboxylic acid and both enantiomers thereof;
   4-[3-(4-chlorophenyl)-4-[2-[[(E)-3-[2-fluoro-4-(trifluoromethyl)phenyl]prop-2-enoyl]amino]acetyl]piperazin-1-yl]-2,2-dimethylbutanoic acid;
   4-[3-(3-cyclopropylphenyl)-4-[2-[[(E)-3-[2-fluoro-4-(trifluoromethyl)phenyl]prop-2-enoyl]amino]acetyl]piperazin-1-yl]butanoic acid;
   4-[3-(4-chlorophenyl)-4-[2-[[(E)-3-[2-fluoro-4-(trifluoromethyl)phenyl]prop-2-enoyl]amino]acetyl]piperazin-1-yl]butanoic acid and both enantiomers thereof;
   4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
   4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
   4-[3-(4-chloro-3-fluorophenyl)-4-[2-[[(E)-3-[2-fluoro-4-(trifluoromethyl)phenyl]prop-2-enoyl]amino]acetyl]piperazin-1-yl]butanoic acid;
   4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
   4-[(2R,5S)-5-(4-chlorophenyl)-4-[2-[[(E)-3-[4-(trifluoromethyl)phenyl]prop-2-enoyl]amino]acetyl]-2-methylpiperazin-1-yl]butanoic acid;
   3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
   4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
   3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
   4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
   4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid or a pharmaceutically acceptable salt thereof.
[12] A compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
   3-[2-[2-[[(E)-3-[4-(trifluoromethyl)phenyl]prop-2-enoyl]amino]acetyl]-3,4-dihydro-1H-isoquinolin-6-yl]propanoic acid;
   N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
   2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
   (1R*,2R*)-2-[2-[2-[[(E)-3-[4-(trifluoromethyl)phenyl]prop-2-enoyl]amino]acetyl]-3,4-dihydro-1H-isoquinolin-6-yl]cyclopropane-1-carboxylic acid and both enantiomers thereof;
   2-[2-[2-[[(E)-3-[4-(trifluoromethyl)phenyl]prop-2-enoyl]amino]acetyl]-3,4-dihydro-1H-isoquinolin-6-yl]propanoic acid;
   2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[2-[2-[[(E)-3-(4-iodophenyl)prop-2-enoyl]amino]acetyl]-3,4-dihydro-1H-isoquinolin-6-yl]acetic acid;
   2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[8-cyano-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-(2-fluoro-4-iodophenyl)prop-2-enoyl]amino]acetyl]-3,4-dihydro-1H-isoquinolin-6-yl]acetic acid;
   2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
   2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[5-[2-[[(E)-3-[4-(trifluoromethyl)phenyl]prop-2-enoyl]amino]acetyl]-6,7-dihydro-4H-[1,3]thiazolo[4,5-c]pyridin-2-yl]acetic acid;
   4-[(3S)-4-[2-[[(E)-3-[2-fluoro-4-(trifluoromethyl)phenyl]prop-2-enoyl]amino]acetyl]-3-phenylpiperazin-1-yl]butanoic acid; 1-[1-[2-[[(E)-3-[4-(trifluoromethyl)phenyl]prop-2-enoyl]amino]acetyl]piperidin-3-yl]bicyclo[2.1.1]hexane-5-carboxylic acid;
   4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
   4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
   4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
   3-[[3-(4-chlorophenyl)-4-[2-[[(E)-3-[2-fluoro-4-(trifluoromethyl)phenyl]prop-2-enoyl]amino]acetyl]piperazin-1-yl]methyl]cyclobutane-1-carboxylic acid and both enantiomers thereof;
   4-[3-(4-chlorophenyl)-4-[2-[[(E)-3-[2-fluoro-4-(trifluoromethyl)phenyl]prop-2-enoyl]amino]acetyl]piperazin-1-yl]butanoic acid and both enantiomers thereof;
   4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
   4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
   4-[3-(4-chloro-3-fluorophenyl)-4-[2-[[(E)-3-[2-fluoro-4-(trifluoromethyl)phenyl]prop-2-enoyl]amino]acetyl]piperazin-1-yl]butanoic acid;
   4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
   4-[(2R,5S)-5-(4-chlorophenyl)-4-[2-[[(E)-3-[4-(trifluoromethyl)phenyl]prop-2-enoyl]amino]acetyl]-2-methylpiperazin-1-yl]butanoic acid;
   3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
   4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
   3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
   4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
   4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid,
   or a pharmaceutically acceptable salt thereof.
[13] A compound selected from the group consisting of 2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[2-[2-[[(E)-3-[4-(trifluoromethyl)phenyl]prop-2-enoyl]amino]acetyl]-3,4-dihydro-1H-isoquinolin-6-yl]propanoic acid;
   2-[2-[2-[[(E)-3-(4-iodophenyl)prop-2-enoyl]amino]acetyl]-3,4-dihydro-1H-isoquinolin-6-yl]acetic acid;
   2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
   2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
   2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   4-[(3S)-4-[2-[[(E)-3-[2-fluoro-4-(trifluoromethyl)phenyl]prop-2-enoyl]amino]acetyl]-3-phenylpiperazin-1-yl]butanoic acid;
   1-[1-[2-[[(E)-3-[4-(trifluoromethyl)phenyl]prop-2-enoyl]amino]acetyl]piperidin-3-yl]bicyclo[2.1.1]hexane-5-carboxylic acid;
   4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
   4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
   4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
   4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
   4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
   4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
   4-[(2R,5S)-5-(4-chlorophenyl)-4-[2-[[(E)-3-[4-(trifluoromethyl)phenyl]prop-2-enoyl]amino]acetyl]-2-methylpiperazin-1-yl]butanoic acid;
   3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
   4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
   3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
   4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
   4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid,
   or a pharmaceutically acceptable salt thereof.
[14] A compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
   3-[2-[2-[[(E)-3-[4-(trifluoromethyl)phenyl]prop-2-enoyl]amino]acetyl]-3,4-dihydro-1H-isoquinolin-6-yl]propanoic acid;
   N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
   2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
   2-[2-[2-[[(E)-3-[4-(trifluoromethyl)phenyl]prop-2-enoyl]amino]acetyl]-3,4-dihydro-1H-isoquinolin-6-yl]propanoic acid;
   2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
   2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
   2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
   2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
   4-[(3R)-3-(4-chlorophenyl)-4-[2-[[(E)-3-[2-fluoro-4-(trifluoromethyl)phenyl]prop-2-enoyl]amino]acetyl]piperazin-1-yl]butanoic acid;
   4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
   3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
   3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
   4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
   4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
   4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid,
   or a pharmaceutically acceptable salt thereof.
[15] A B0AT1 inhibitor comprising a compound described in any of the above-mentioned [1] to [14], [3'], and [9'] or a pharmaceutically acceptable salt thereof.
[15'] The B0AT1 inhibitor described in the above-mentioned [15], which is an agent for the prophylaxis and/or treatment of an amino acid metabolism disorder.
[15''] The B0AT1 inhibitor described in the above-mentioned [15'], wherein the amino acid metabolism disorder is phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, or isovaleric acidemia.
[16] A pharmaceutical composition comprising a compound described in any of the above-mentioned [1] to [14], [3'], and [9'] or a pharmaceutically acceptable salt thereof, and a pharmaceutically acceptable carrier.
[17] The pharmaceutical composition described in the above-mentioned [16], for the prophylaxis and/or treatment of a disease whose symptoms can be alleviated by the B0AT1 inhibitory action.
[18] The pharmaceutical composition described in the above-mentioned [17], wherein the disease whose symptoms can be alleviated by the B0AT1 inhibitory action is an amino acid metabolism disorder.
[19] The pharmaceutical composition described in the above-mentioned [18], wherein the amino acid metabolism disorder is phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, or isovaleric acidemia.
[20] The pharmaceutical composition described in the above-mentioned [18], wherein the amino acid metabolism disorder is phenylketonuria.
[21] The pharmaceutical composition described in any of the above-mentioned [16] to [20], which is administered in combination with other medicament.
[22] The pharmaceutical composition described in the above-mentioned [21], wherein the other medicament is a B0AT1 inhibitor.
[23] The pharmaceutical composition described in the above-mentioned [21], wherein the other medicament is a vitamin, a drug for alleviating various symptoms of amino acid metabolism disorders, an antidepressant, an antianxiety drug, or a therapeutic drug for ADHD.
[24] The pharmaceutical composition of any of the above-mentioned [21] to [23], wherein a pharmaceutical composition of any of the above-mentioned [16] to [20] and the other medicament are separately administered.
[25] The pharmaceutical composition of any of the above-mentioned [21] to [23], wherein
   the pharmaceutical composition of any of the above-mentioned [16] to [20] and the other medicament are administered simultaneously or sequentially.
[26] A method for the prophylaxis or treatment of an amino acid metabolism disorder in a mammal, comprising administering an effective amount of a compound described in any of the above-mentioned [1] to [14], [3'], and [9'] or a pharmaceutically acceptable salt thereof to the mammal.
[27] A method for the prophylaxis or treatment of a disease selected from the group consisting of phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, and isovaleric acidemia in a mammal, comprising administering an effective amount of a compound described in any of the above-mentioned [1] to [14], [3'], and [9'] or a pharmaceutically acceptable salt thereof to the mammal.
[28] A method for the prophylaxis or treatment of an amino acid metabolism disorder in a mammal, comprising administering an effective amount of a pharmaceutical composition described in any of the above-mentioned [16] to [20] and other medicament simultaneously or sequentially to the mammal.
[29] A method for the prophylaxis or treatment of a disease selected from the group consisting of phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, and isovaleric acidemia in a mammal, comprising administering an effective amount of a pharmaceutical composition described in any of the above-mentioned [16] to [20] and other medicament simultaneously or sequentially to the mammal.
[30] Use of a compound described in any of the above-mentioned [1] to [14], [3'], and [9'] or a pharmaceutically acceptable salt thereof in the production of a therapeutic agent or a prophylactic agent for an amino acid metabolism disorder.
[31] Use of a compound described in any of the above-mentioned [1] to [14], [3'], and [9'] or a pharmaceutically acceptable salt thereof in the production of a therapeutic agent or a prophylactic agent for a disease selected from the group consisting of phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, and isovaleric acidemia.
[32] A compound described in any of the above-mentioned [1] to [14], [3'], and [9'] or a pharmaceutically acceptable salt thereof for use in the prophylaxis or treatment of an amino acid metabolism disorder.
[33] A compound described in any of the above-mentioned [1] to [14], [3'], and [9'] or a pharmaceutically acceptable salt thereof for use in the prophylaxis or treatment of a disease selected from the group consisting of phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, and isovaleric acidemia.
[34] A method for producing a compound described in any of the above-mentioned [1] to [14], [3'], and [9'] or a pharmaceutically acceptable salt thereof.
[35] A prodrug of a compound described in any of the above-mentioned [1] to [14], [3'], and [9'] or a pharmaceutically acceptable salt thereof.

### [Advantageous Effects of Invention]

Compound (I) of the present invention or a pharmaceutically acceptable salt thereof has a superior inhibitory activity against B0AT1. Therefore, a pharmaceutical composition containing said compound is useful for the treatment and/or prophylaxis of diseases whose symptoms can be alleviated by a B0AT1 inhibitory action.

### [Description of Embodiments]

The definitions of the terms and symbols used in the present specification are explained below.

In the present specification, the "halogen atom" means a fluorine atom, a chlorine atom, a bromine atom, or an iodine atom.

In the present specification, the "alkyl (group)" means a linear or branched chain monovalent group with one or more carbon atoms, excluding one hydrogen atom from any carbon atom of an alkane. While the number of carbon atoms is not particularly limited, it is a C₁₋₂₀ alkyl group, preferably a C₁₋₆ alkyl group.

In the present specification, the "C₁₋₂₀ alkyl (group)" means an alkyl group having 1 to 20 carbon atoms and, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, dodecyl, tridecyl, eicosyl, and the like can be mentioned.

In the present specification, the "C₁₋₆ alkyl (group)" means an alkyl group having 1 to 6 carbon atoms and, for example, methyl, ethyl, propyl, isopropyl, butyl, isobutyl, sec-butyl, tert-butyl, pentyl, isopentyl, neopentyl, sec-pentyl (pentan-2-yl), 3-pentyl (pentan-3-yl), tert-pentyl (1,1-dimethylpropyl), hexyl, isohexyl, 1,1-dimethylbutyl, 2,2-dimethylbutyl, 3,3-dimethylbutyl, 2-ethylbutyl, and the like can be mentioned.

In the present specification, the "C₁₋₄ alkyl (group)" means an alkyl group having 1 to 4 carbon atoms and, for example, methyl, ethyl, propyl, isopropyl, butyl, isobutyl, sec-butyl, tert-butyl, and the like can be mentioned.

In the present specification, the "C₁₋₆ alkyl (group) optionally substituted by halogen atom(s)" means the aforementioned unsubstituted C₁₋₆ alkyl group, or a C₁₋₆ alkyl group in which one or more (preferably, 1 to 6, more preferably 1 to 3) hydrogen atoms in the aforementioned C₁₋₆ alkyl group are substituted by halogen. Specifically, for example, methyl, ethyl, propyl, isopropyl, butyl, isobutyl, pentyl, neopentyl, hexyl, fluoromethyl, difluoromethyl, trifluoromethyl, 2-chloroethyl, 2-bromoethyl, 2-iodoethyl, 2-fluoroethyl, 2,2-difluoroethyl, 2,2,2-trifluoroethyl, pentafluoroethyl, 2,2,3,3-tetrafluoropropyl, 3,3,3-trifluoropropyl, 4,4,4-trifluorobutyl, 5,5,5-trifluoropentyl, 6,6,6-trifluorohexyl and the like can be mentioned. Among these, a "C₁₋₄ alkyl (group) optionally substituted by halogen atom(s)" is more preferred.

In the present specification, the "C₁₋₆ alkyl (group) optionally substituted by substituent(s) selected from the group" means the aforementioned unsubstituted C₁₋₆ alkyl group, or a C₁₋₆ alkyl group in which one or more (preferably, 1 to 6, more preferably 1 to 3) hydrogen atoms in the aforementioned C₁₋₆ alkyl group are substituted by the same or different substituents selected from the substituent group. Among those, a "C₁₋₄ alkyl (group) optionally substituted by the same or different substituents selected from the substituent group" is more preferred.

In the present specification, the "cycloalkyl (group)" means a cyclic alkyl group. When the number of carbon atoms is not particularly limited, it is preferably a C₃₋₁₄ cycloalkyl group, more preferably a C₃₋₈ cycloalkyl group.

In the present specification, the "C₃₋₁₄ cycloalkyl (group)" means a cyclic alkyl group having 3 to 14 carbon atoms, and may be any of monocyclic, fused cyclic, spirocyclic, and bridged cyclic. Specific examples of the "C₃₋₁₄ cycloalkyl (group)" include monocyclic C₃₋₁₄ cycloalkyl such as cyclopropyl, cyclobutyl, cyclopentyl, cyclohexyl, cycloheptyl, cyclooctyl and the like; fused cyclic C₅₋₁₄ cycloalkyl such as 1-decalinyl, 2-decalinyl and the like; spirocyclic C₆₋₁₄ cycloalkyl such as spiro[3.4]octyl, spiro[4.5]decyl, spiro[3.6]decyl and the like; bridged cyclic C₅₋₁₄ cycloalkyl such as bicyclo[1.1.1]pentyl, bicyclo[2.2.1]heptyl, bicyclo[2.2.2]octyl and the like, and the like. Among these, a C₃₋₈ cycloalkyl group is preferred.

In the present specification, the "C₃₋₈ cycloalkyl (group)" means a cyclic alkyl group having 3 to 8 carbon atoms and, for example, cyclopropyl, cyclobutyl, cyclopentyl, cyclohexyl, cycloheptyl, cyclooctyl, and the like can be mentioned. Among these, a C₃₋₆ cycloalkyl group is preferred.

In the present specification, the "C₃₋₈ cycloalkyl (group) optionally substituted by substituent(s) selected from the group" means the aforementioned unsubstituted C₃₋₈ cycloalkyl group or the aforementioned C₃₋₈ cycloalkyl group in which one or more (preferably 1 to 6, more preferably 1 to 3) hydrogen atoms are substituted by the same or different substituents selected from the substituent group.

In the present specification, the "C₃₋₈ cycloalkyloxy (group)" means a group resulting from the binding of the aforementioned C₃₋₈ cycloalkyl group to an oxygen atom and specifically, for example, cyclopropyloxy, cyclobutyloxy, cyclopentyloxy, cyclohexyloxy, cycloheptyloxy, cyclooctyloxy, and the like can be mentioned.

In the present specification, the "C₃₋₈ cycloalkyloxy (group) optionally substituted by substituent(s) selected from the group" means the aforementioned unsubstituted C₃₋₈ cycloalkyloxy group, or the aforementioned C₃₋₈ cycloalkyloxy group in which one or more (preferably 1 to 6, more preferably 1 to 3) hydrogen atoms are substituted by the same or different substituents selected from the substituent group.

In the present specification, the "C₃₋₈ cycloalkylsulfanyl (group)" means a group resulting from the binding of the aforementioned C₃₋₈ cycloalkyl group to a sulfur atom and specifically, for example, cyclopropylsulfanyl, cyclobutylsulfanyl, cyclopentylsulfanyl, cyclohexylsulfanyl, cycloheptylsulfanyl, cyclooctylsulfanyl, and the like can be mentioned.

In the present specification, the "alkoxy (group)" means a group resulting from the binding of a linear or branched chain alkyl group to an oxygen atom. While the number of carbon atoms is not particularly limited, it is a C₁₋₂₀ alkoxy group, preferably a C₁₋₆ alkoxy group.

In the present specification, the "C₁₋₆ alkoxy (group)" means an alkoxy group having 1 to 6 carbon atoms and, for example, methoxy, ethoxy, propoxy, isopropoxy, butoxy, isobutoxy, sec-butoxy, tert-butoxy, pentyloxy, isopentyloxy, neopentyloxy, hexyloxy, and the like can be mentioned. Among these, a C₁₋₄ alkoxy group is preferred.

In the present specification, the "C₁₋₆ alkoxy (group) optionally substituted by halogen atom(s)" is the aforementioned unsubstituted C₁₋₆ alkoxy group, or the aforementioned C₁₋₆ alkoxy group in which one or more hydrogen atoms are substituted by halogen. Specifically, for example, methoxy, ethoxy, propoxy, isopropoxy, butoxy, pentyloxy, hexyloxy, fluoromethoxy, difluoromethoxy, trifluoromethoxy, 2-chloroethoxy, 2-bromoethoxy, 2-iodoethoxy, 2-fluoroethoxy, 2,2-difluoroethoxy, 2,2,2-trifluoroethoxy, pentafluoroethoxy, 2,2,3,3-tetrafluoropropoxy, 3,3,3-trifluoropropoxy, 4,4,4-trifluorobutoxy, 5,5,5-trifluoropentyloxy, 6,6,6-trifluorohexyloxy and the like can be mentioned. Among these, a "C₁₋₄ alkoxy (group) optionally substituted by halogen atom(s))" is more preferred.

In the present specification, the "C₁₋₆ alkoxy (group) optionally substituted by substituent(s) selected from the group" means the aforementioned unsubstituted C₁₋₆ alkoxy group, or the aforementioned C₁₋₆ alkoxy group in which one or more (preferably 1 to 6, more preferably 1 to 3) hydrogen atoms are substituted by the same or different substituents selected from the substituent group. Among these, a "C₁₋₄ alkoxy (group) optionally substituted by the same or different substituents selected from the group" is more preferred.

In the present specification, the "alkylsulfanyl (group)" means a group resulting from the binding of a linear or branched chain alkyl group to a sulfur atom, and a C₁₋₆ alkylsulfanyl group is preferred.

In the present specification, the "C₁₋₆ alkylsulfanyl (group)" means an alkylsulfanyl group having 1 to 6 carbon atoms and, for example, methylsulfanyl, ethylsulfanyl, propylsulfanyl, isopropylsulfanyl, butylsulfanyl, isobutylsulfanyl, sec-butylsulfanyl, tert-butylsulfanyl, pentylsulfanyl, isopentylsulfanyl, neopentylsulfanyl, hexylsulfanyl, and the like can be mentioned.

In the present specification, the "C₁₋₆ alkylsulfanyl (group) optionally substituted by substituent(s) selected from the group" means the aforementioned unsubstituted C₁₋₆ alkylsulfanyl group, or the aforementioned C₁₋₆ alkylsulfanyl group in which one or more (preferably 1 to 6, more preferably 1 to 3) hydrogen atoms are substituted by the same or different substituents selected from the substituent group.

In the present specification, the "C₁₋₆ alkylsulfonyl (group)" means a group resulting from the binding of the aforementioned "C₁₋₆ alkyl" group to the sulfur atom of a sulfonyl group (-S(=O)₂-), that is, a linear or branched chain alkylsulfonyl group having 1 to 6 carbon atoms. As the "C₁₋₆ alkylsulfonyl (group)", methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, 1-ethylpropylsulfonyl, hexylsulfonyl, and the like can be mentioned.

In the present specification, the "C₁₋₆ alkylsulfonyl (group) optionally substituted by halogen atom(s)" is the aforementioned unsubstituted C₁₋₆ alkylsulfonyl group, or the aforementioned C₁₋₆ alkylsulfonyl group in which one or more (preferably 1 to 6, more preferably 1 to 3) hydrogen atoms are substituted by halogen. Specifically, for example, methylsulfonyl, ethylsulfonyl, propylsulfonyl, butylsulfonyl, pentylsulfonyl, hexylsulfonyl, fluoromethylsulfonyl, difluoromethylsulfonyl, trifluoromethylsulfonyl, 2-chloroethylsulfonyl, 2-bromoethylsulfonyl, 2-iodoethylsulfonyl, 2-fluoroethylsulfonyl, 2,2-difluoroethylsulfonyl, 2,2,2-trifluoroethylsulfonyl, pentafluoroethylsulfonyl, 2,2,3,3-tetrafluoropropylsulfonyl, 3,3,3-trifluoropropylsulfonyl, 4,4,4-trifluorobutylsulfonyl, 5,5,5-trifluoropentylsulfonyl, 6,6,6-trifluorohexylsulfonyl and the like can be mentioned.

In the present specification, the "C₁₋₆ alkyl-carbonyl (group)" means a group in which the aforementioned C₁₋₆ alkyl group is bonded to a carbonyl group. Specifically, for example, acetyl, ethylcarbonyl, propylcarbonyl, butylcarbonyl, pentylcarbonyl, hexylcarbonyl and the like can be mentioned.

In the present specification, the "C₁₋₆ alkyl-carbonyl (group) optionally substituted by halogen atom(s)" is the aforementioned unsubstituted C₁₋₆ alkyl-carbonyl group, or the aforementioned C₁₋₆ alkyl-carbonyl group in which one or more (preferably 1 to 6, more preferably 1 to 3) hydrogen atoms are substituted by halogen. Specifically, for example, acetyl, ethylcarbonyl, propylcarbonyl, butylcarbonyl, pentylcarbonyl, hexylcarbonyl, fluoromethylcarbonyl, difluoromethylcarbonyl, trifluoromethylcarbonyl, 2-chloroethylcarbonyl, 2-bromoethylcarbonyl, 2-iodoethylcarbonyl, 2-fluoroethylcarbonyl, 2,2-difluoroethylcarbonyl, 2,2,2-trifluoroethylcarbonyl, pentafluoroethylcarbonyl, 2,2,3,3-tetrafluoropropylcarbonyl, 3,3,3-trifluoropropylcarbonyl, 4,4,4-trifluorobutylcarbonyl, 5,5,5-trifluoropentylcarbonyl, 6,6,6-trifluorohexylcarbonyl and the like can be mentioned.

In the present specification, the "C₁₋₆ alkoxy-carbonyl (group)" means a group in which the aforementioned C₁₋₆ alkoxy group is bonded to a carbonyl group. Specifically, for example, methoxycarbonyl, ethoxycarbonyl, propoxycarbonyl, butoxycarbonyl, pentyloxycarbonyl, hexyloxycarbonyl and the like can be mentioned.

In the present specification, the "optionally substituted carbamoyl (group)" means a group (mono- or di-substituted carbamoyl group) in which 1 or 2 hydrogen atoms of carbamoyl group (-CONH₂) are each independently optionally substituted by other substituents. The di-substituted carbamoyl group includes 1-pyrrolidinylcarbonyl group and 1-piperidylcarbonyl group.

In the present specification, the "carbamoyl (group) optionally substituted by 1 or 2 C₁₋₆ alkyl groups optionally substituted by substituent(s) selected from the group consisting of a halogen atom, a hydroxy group, a di-C₁₋₆ alkylamino group and a C₁₋₆ alkoxy group " means a group in which 1 or 2 hydrogen atoms of carbamoyl group (-CONH₂) are each independently optionally substituted by 1 or 2 C₁₋₆ alkyl groups optionally substituted by substituent(s) selected from the group consisting of a halogen atom, a hydroxy group, a di-C₁₋₆ alkylamino group and a C₁₋₆ alkoxy group, and a mono- or di-C₁₋₆ alkyl-carbamoyl group is preferred.

In the present specification, the "C₂₋₆ alkenyl group" means a linear or branched monovalent group having 2 to 6 carbon atoms in which one hydrogen atom is removed from any carbon atom of C₂₋₆ alkene. Specifically, for example, vinyl, 1-propenyl, allyl, isopropenyl, butenyl, isobutenyl and the like can be mentioned.

In the present specification, the "C₂₋₆ alkynyl group" means a linear or branched monovalent group having 2 to 6 carbon atoms in which one hydrogen atom is removed from any carbon atom of C₂₋₆ alkyne. Specifically, for example, ethynyl, propargyl, 3-butynyl, 2-butynyl, 2-pentynyl, 3-pentynyl, 4-pentynyl and the like can be mentioned.

In the present specification, the "aryl (group)" means an aromatic monocyclic or polycyclic (fused) hydrocarbon group. Among them, C₆₋₁₄ aryl group is preferred, and C₆₋₁₀ aryl group is more preferred.

In the present specification, as the "C₆₋₁₄ aryl (group)", phenyl, 1-naphthyl, 2-naphthyl, biphenylyl, phenanthryl, 2-anthryl, fluorenyl and the like can be mentioned.

In the present specification, as the "C₆₋₁₀ aryl (group)", phenyl, 1-naphthyl, and 2-naphthyl can be mentioned. Among these, phenyl is preferred.

In the present specification, the "aryl-carbonyl (group)" means a group resulting from the binding of the aforementioned aryl group to a carbonyl group. While the number of carbon atoms is not particularly limited, it is preferably a C₆₋₁₄ aryl-carbonyl group.

In the present specification, the "C₆₋₁₄ aryloxy (group)" means a group resulting from the binding of the aforementioned C₆₋₁₄ aryl group to an oxygen atom, and specifically, for example, phenyloxy, 1-naphthyloxy, 2-naphthyloxy, biphenylyloxy, phenanthryloxy, 2-anthryloxy, and the like can be mentioned. Among these, a C₆₋₁₀ aryloxy group is preferred and a phenyloxy group is particularly preferred.

In the present specification, the "C₆₋₁₄ hydrocarbocycle (group)" means a monovalent, saturated or partially unsaturated aliphatic hydrocarbocyclic (group) having 6 to 14 carbon atoms, or a monovalent aromatic hydrocarbocyclic (group) having 6 to 14 carbon atoms.

In the present specification, the "C₆₋₁₄ arylsulfonyl (group)" means a group resulting from the binding of the aforementioned C₆₋₁₄ aryl group to a sulfur atom of a sulfonyl group (-S(=O)₂-) and, for example, phenylsulfonyl, 1-naphthylsulfonyl, 2-naphthylsulfonyl, and the like can be mentioned.

In the present specification, the "C₆₋₁₄ arylsulfonyloxy (group)" means a group resulting from the binding of the aforementioned C₆₋₁₄ aryl group to a sulfur atom of a sulfonyloxy group (-S(=O)₂-O-) and, for example, phenylsulfonyloxy, 1-naphthylsulfonyloxy, 2-naphthylsulfonyloxy, and the like can be mentioned.

In the present specification, the "C₆₋₁₀ aryl-ethenyl group" means a group in which the terminal hydrogen atom of an ethenyl group (-CH=CH₂) is substituted by the aforementioned C₆₋₁₀ aryl group. Among them, 2-phenylethenyl group is preferred, and 2-(E)-phenylethenyl group is particularly preferred.

In the present specification, "(E)" in the chemical formula of each example compound or "(E)-" in the compound name shows that the double bond is in the E configuration.

In the present specification, the "C₇₋₁₈ aralkyl (group)" means a group resulting from the binding of the aforementioned C₆₋₁₄ aryl group to the aforementioned C₁₋₄ alkyl group and specifically, for example, benzyl, phenethyl, naphthylmethyl, biphenylylmethyl, and the like can be mentioned. Among these, a C₇₋₁₀ aralkyl group is preferred, and a benzyl group is particularly preferred.

In the present specification, the "C₇₋₁₈ aralkyloxy (group)" means a group resulting from the binding of the aforementioned C₇₋₁₈ aralkyl group to an oxygen atom, and specifically, for example, benzyloxy, phenethyloxy, naphthylmethyloxy, biphenylylmethyloxy, and the like can be mentioned. Among these, a benzyloxy group is particularly preferred.

In the present specification, the "acyl (group)" means a formyl group, a linear or branched chain alkyl-carbonyl group, or an aryl-carbonyl group. While the number of carbon atoms is not particularly limited, it is preferably a formyl group, a C₁₋₆ alkyl-carbonyl group, or a C₆₋₁₄ aryl-carbonyl group. Specific preferable examples of the acyl (group) include formyl, acetyl, propionyl, butyryl, isobutyryl, pentanoyl, tert-butylcarbonyl (pivaloyl), hexanoyl, heptanoyl, benzoyl, 1-naphthoyl, 2-naphthoyl, and the like.

In the present specification, the "acyloxy (group)" means a group resulting from the binding of the aforementioned acyl group to an oxygen atom, and it is preferably a C₁₋₆ alkyl-carbonyloxy group or a C₆₋₁₄ aryl-carbonyloxy group.

In the present specification, the "di-C₁₋₆ alkylamino (group)" means a group in which two hydrogen atoms of the amino group are substituted by the same or different aforementioned C₁₋₆ alkyl groups.

In the present specification, the "C₁₋₆ alkylene (group)" means a divalent group in which one hydrogen atom is removed from the aforementioned C₁₋₆ alkyl group. As the C₁₋₆ alkylene (group), specifically, for example, methylene, dimethylene, methylmethylene, dimethylmethylene, trimethylene, propane-1,2-diyl, tetramethylene, pentamethylene, isopentane-2,4-diyl, hexamethylene and the like can be mentioned.

In the present specification, the "C₆₋₁₀ arylene (group)" means a divalent group in which one hydrogen atom is removed from the aforementioned "C₆₋₁₀ aryl group". As the C₆₋₁₀ arylene(group), specifically, for example, 1,2-phenylene, 1,3-phenylene, 1,4-phenylene, 1,2-naphthylene, 1,3-naphthylene, 1,4-naphthylene group, 2,6-naphthylene and the like can be mentioned.

In the present specification, the "C₂₋₆ alkenylene (group)" means a divalent group in which one hydrogen atom is removed from the aforementioned "C₂₋₆ alkenyl group". Among them, C₂₋₃ alkenylene is preferred. As the C₂₋₃ alkenylene (group), specifically, for example, ethenylene (-CH=CH-), propenylene (-CH=CHCH₂-) and the like can be mentioned.

In the present specification, the "C₂₋₆ alkynylene (group)" means a divalent group in which one hydrogen atom is removed from the aforementioned "C₂₋₆ alkynyl group". Among them, C₂₋₃ alkynylene is preferred. As the C₂₋₃ alkynylene (group), specifically, for example, ethynylene (-C≡C-), propynylene (-C≡C-CH₂-) and the like can be mentioned.

In the present specification, the "C₃₋₁₄ cycloalkylene (group)" means a divalent group in which one hydrogen atom is removed from the aforementioned "C₃₋₁₄ cycloalkyl group". Among them, C₃₋₈ cycloalkylene is preferred. As the C₃₋₈ cycloalkylene(group), specifically, for example, cyclopropane-1,1-diyl, cyclopropane-1,2-diyl, cyclobutane-1,3-diyl, bicyclo[2.1.1]hexane-1,2-diyl, bicyclo[1.1.1]pentane-1,3-diyl and the like can be mentioned.

In the present specification, the "3- to 14-membered divalent non-aromatic heterocycle (group)" means a divalent group in which one hydrogen atom is removed from the below-mentioned "3- to 14-membered non-aromatic heterocycle (group)". Among them, 3- to 8-membered divalent non-aromatic heterocycle is preferred. As the 3- to 8-membered divalent non-aromatic heterocycle (group), specifically, for example, aziridine-1,2-diyl, oxetane-2,4-diyl, azetidine-1,3-diyl, pyrrolidine-1,3-diyl, piperidine-1,2-diyl, piperidine-1,3-diyl, piperidine-1,4-diyl and the like can be mentioned.

In the present specification, the "divalent organic group including a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group " means a divalent group having the aforementioned "C₁₋₆ alkylene group, C₆₋₁₀ arylene group, C₃₋₁₄ cycloalkylene group, C₂₋₆ alkenylene group, C₂₋₆ alkynylene group, or 3- to 14-membered divalent non-aromatic heterocyclic group", and the moiety other than the "C₁₋₆ alkylene group, C₆₋₁₀ arylene group, C₃₋₁₄ cycloalkylene group, C₂₋₆ alkenylene group, C₂₋₆ alkynylene group, or 3- to 14-membered divalent non-aromatic heterocyclic group" in the "organic group having a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group " can be freely determined. Preferable examples of the "divalent organic group including a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group" include a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²-(wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from the aforementioned substituent group a. Here, the two bonds of the divalent (organic) group may originate from the same carbon atom or different carbon atoms in the (organic) group, or from the hetero atom constituting the (organic) group. In the present specification, the "C₃₋₁₀ cycloalkylene group", "C₂₋₄ alkenylene group" and "C₂₋₄ alkynylene group" are respectively the same as the aforementioned "C₃₋₁₄ cycloalkylene group", "C₂₋₆ alkenylene group" and "C₂₋₆ alkynylene group" except that the carbon number range is different.

In the present specification, the "heterocycle (group)" is (i) an aromatic heterocyclic group or (ii) a non-aromatic heterocyclic group, each of which contains 1 to 4 hetero atoms selected from a nitrogen atom, a sulfur atom, and an oxygen atom as ring-constituting atom besides carbon atom, and (i) aromatic heterocyclic group includes 5- or 6-membered monocyclic aromatic heterocyclic group and 8- to 14-membered fused aromatic heterocyclic group, and (ii) non-aromatic heterocyclic group includes 3- to 10-membered monocyclic non-aromatic heterocyclic group, 9- to 14-membered fused non-aromatic heterocyclic group, 6- to 14-membered bridged non-aromatic heterocyclic group, and 6- to 16-membered spirocyclic non-aromatic heterocyclic group.

In the present specification, as the "aromatic heterocyclic group", a 5- to 14-membered aromatic heterocyclic group containing 1 to 4 hetero atoms selected from a nitrogen atom, a sulfur atom, and an oxygen atom as ring-constituting atoms besides carbon atom can be mentioned.

Preferred examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and
8- to 14-membered fused aromatic heterocyclic groups (fused polycyclic (preferably bi- or tri-cyclic) aromatic heterocyclic groups) such as benzothiophenyl, benzofuranyl, benzoimidazolyl, benzoxazolyl, benzisoxazolyl, benzothiazolyl, benzisothiazolyl, benzotriazolyl, imidazopyridyl, thienopyridyl, furopyridyl, pyrrolopyridyl, pyrazolopyridyl, oxazolopyridyl, thiazolopyridyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acrydinyl, phenazinyl, phenothiazinyl, phenoxazinyl and the like.

In the present specification, as the "non-aromatic heterocyclic group", a 3- to 14-membered non-aromatic heterocyclic group containing 1 to 4 hetero atoms selected from a nitrogen atom, a sulfur atom, and an oxygen atom as ring-constituting atoms besides carbon atom can be mentioned.

Preferred examples of the "non-aromatic heterocyclic group" include 3- to 10-membered monocyclic non-aromatic heterocyclic groups (preferably 5- to 8-membered monocyclic non-aromatic heterocyclic groups) such as aziridinyl, oxiranyl, thiiranyl, azetidinyl, oxetanyl, thietanyl, tetrahydrothienyl, tetrahydrofuryl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidyl, piperazinyl, tetrahydropyridyl, dihydropyridyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl, and the like; and
8- to 14-membered fused non-aromatic heterocyclic groups (fused polycyclic (preferably bicyclic) non-aromatic heterocyclic groups) such as tetrahydrotriazolopyridyl, tetrahydrotriazolopyrazinyl, tetrahydropyrazolopyrazinyl, dihydropyrazolopyrrolyl, dihydropyrazolopyrazinyl, tetrahydropyrazolopyridyl, tetrahydroimidazolopyridyl, dihydroimidazolopyridyl, dihydroimidazolopyrrolyl tetrahydroimidazolopyrazinyl, dihydropyrimidopyrrolyl, dihydropyridopyrrolyl, tetrahydropyridopyridyl, tetrahydrooxazolopyrazinyl, tetrahydropyridopyridyl, tetrahydrotriazolodiazepinyl, octahydro-1,4-oxazinopyrazinyl, dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, dihydroisoquinolyl, tetrahydroquinolyl, 4H-quinolizinyl, indolinyl, isoindolinyl, tetrahydrothieno[2,3-c]pyridyl, dihydrothiazolo[4,5-c]pyridyl, dihydrothiazolo[5,4-c]pyridyl, tetrahydrobenzoazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, dihydronaphthyridinyl, tetrahydroquinazolinyl, tetrahydrocinnolinyl, tetrahydrocarbazolyl, tetrahydro-β-carbolynyl, tetrahydroacridinyl, tetrahydrophenazinyl, tetrahydrothioxanthenyl, octahydroisoquinolyl, and the like.

More preferable examples of the "non-aromatic heterocyclic group" include pyrrolidinyl, piperidyl, tetrahydrotriazolopyrazinyl (e.g., 5,6,7,8-tetrahydro-[1,2,4]triazolo[4,3-a]pyrazinyl, 5,6,7,8-tetrahydro-[1,2,4]triazolo[1,5-a]pyrazinyl), tetrahydropyrazolopyrazinyl (e.g., 4,5,6,7-tetrahydropyrazolo[1,5-a]pyrazinyl), tetrahydroisoquinolyl (e.g., 1,2,3,4-tetrahydroisoquinolyl), tetrahydronaphthyridinyl (e.g., 5,6,7,8-tetrahydro-1,6-naphthyridinyl, 1,2,3,4-tetrahydro-2,6-naphthyridinyl, 1,2,3,4-tetrahydro-2,7-naphthyridinyl), dihydropyrazolopyrazinyl (e.g., dihydro-4H-pyrazolo[1,5-a]pyrazinyl), dihydronaphthyridinyl (e.g., 6,8-dihydro-5H-2,7-naphthyridinyl), dihydroisoquinolyl (e.g., 3,4-dihydro-1H-isoquinolyl), dihydrothiazolo[5,4-c]pyridyl (e.g., 6,7-dihydro-4H-[1,3]thiazolo[5,4-c]pyridyl), dihydrothiazolo[4,5-c]pyridyl (e.g., 6,7-dihydro-4H-[1,3]thiazolo[4,5-c]pyridyl), indolinyl (e.g., 2,3-dihydroindolyl) and the like.

Other preferable examples of the "non-aromatic heterocyclic group" include 6- to 14-membered bridged cyclic non-aromatic heterocyclic groups such as 3,8-diazabicyclo[3.2.1]octyl, 2,5-diazabicyclo[2.2.2]octyl, 7-azabicyclo[2.2.1]heptanyl, quinuclidinyl and the like; and 6-to 16-membered spirocyclic non-aromatic heterocyclic groups such as 2,8-diazaspiro[4.5]decyl, 2,7-diazaspiro[3.5]nonyl, 2,6-diazaspiro[3.3]heptyl and the like.

In the present specification, as the "nitrogen-containing heterocyclic group", nitrogen-containing aromatic heterocyclic group and nitrogen-containing non-aromatic heterocyclic group can be mentioned.

In the present specification, the "nitrogen-containing aromatic heterocyclic group" or "nitrogen-containing non-aromatic heterocyclic group" means the aforementioned aromatic heterocyclic group or non-aromatic heterocyclic group that has at least one nitrogen atom as a ring-constituting atom.

In the present specification, the "divalent nitrogen-containing aromatic heterocyclic group" means a divalent group in which one hydrogen atom is removed from the aforementioned "nitrogen-containing aromatic heterocyclic group".

In the present specification, the "divalent nitrogen-containing non-aromatic heterocyclic group" means a divalent group in which one hydrogen atom is removed from the aforementioned "nitrogen-containing non-aromatic heterocyclic group".

In the present specification, being "optionally further substituted by 1 to 3 substituents selected from substituent group a (or substituent group b)" means that, besides the two bonds in the divalent groups, 1 to 3 hydrogen atoms are each independently optionally further substituted by 1 to 3 substituents selected from substituent group a (or substituent group b). When substituent group a' (or substituent group b') is adopted instead of substituent group a, this definition similarly applies by replacing substituent group a (or substituent group b) with substituent group a' (or substituent group b').

In the present specification, the "tri-substituted silyl (group)" means a silyl group substituted by the same or different 3 substituents (e.g., C₁₋₆ alkyl group, C₆₋₁₀ aryl group, etc.). Examples thereof include trialkylsilyl groups such as trimethylsilyl group, triethylsilyl group, triisopropylsilyl group, tert-butyldimethylsilyl group, and the like (preferably, tri-C₁₋₆ alkylsilyl group), tert-butyldiphenylsilyl group, triphenylsilyl group, and the like.

In the present specification, the "tri-substituted silyloxy (group)" means a group in which a tri-substituted silyl group is bonded to an oxygen atom. Examples thereof include trialkylsilyloxy groups such as trimethylsilyloxy group, triethylsilyloxy group, triisopropylsilyloxy group, tertbutyldimethylsilyloxy group and the like (preferably, tri C₁₋₆ alkylsilyloxy group), tert-butyldiphenylsilyloxy group, triphenylsilyloxy group, and the like.

In the present specification, the "biologically equivalent group" means "a functional group that exhibits broadly similar biological effects and has chemical and physical similarities" as proposed by Thornber in 1979 in the field of drug discovery chemistry. Examples of the "group biologically equivalent to a carboxy group" include optionally substituted C₁₋₆ alkoxy-carbonyl group, optionally substituted carbamoyl group, cyano group, group selected from the group consisting of the following formulas: 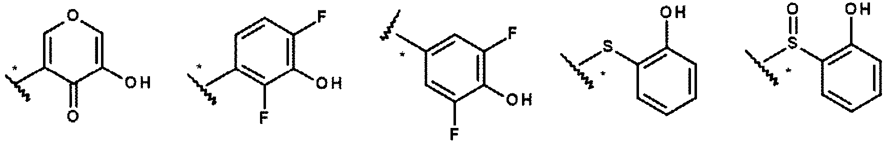 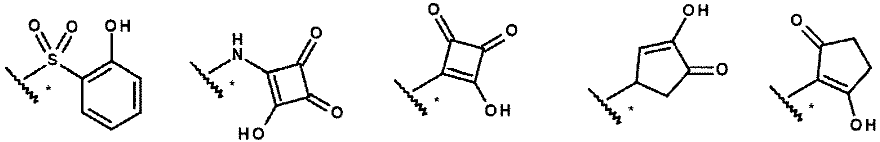 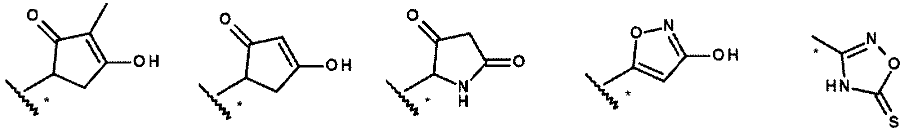 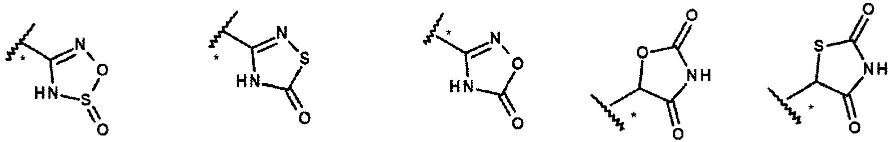 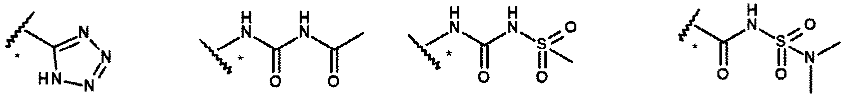 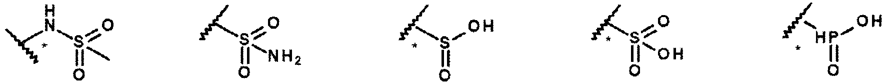 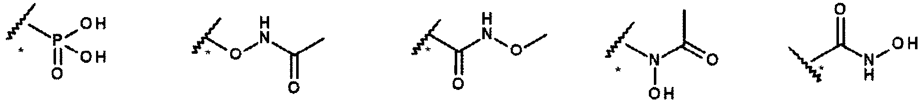
wherein a wavy line is a bonding site of the group represented by the above-mentioned chemical formula, and * shows a bonding position with L,
tautomeric groups thereof, or the like. Among these, optionally substituted C₁₋₆ alkoxy-carbonyl group or optionally substituted carbamoyl group is preferred.

In the present specification, being "optionally substituted" means unsubstituted or substituted by a specific substituent at any substitutable position (any hydrogen atom is replaced with a substituent). The "substituent" is not particularly limited and may be a substituent selected from the following (Substituent group a), (Substituent group a'), (Substituent group b), and (Substituent group b'). When the substituent group is not particularly specified, it means being optionally substituted by one or more substituents selected from the following (Substituent group c) or (Substituent group c'). While the number of the substituents is not particularly limited as long as it is a substitutable number, it is generally 1 to 5, preferably 1 to 3. When plural substituents are present, the respective substituents may be the same or different.

(Substituent group a):
a halogen atom;
a hydroxy group;
a cyano group;
an oxo group;
a C₁₋₆ alkyl group optionally substituted by substituent(s) selected from the group consisting of a halogen atom, a hydroxy group, a cyano group, a C₁₋₆ alkoxy group, a C₆₋₁₄ aryl group, a carboxy group, a C₁₋₆ alkoxy-carbonyl group, a carbamoyl group optionally substituted by 1 or 2 C₁₋₆ alkyl groups, and a C₆₋₁₄ aryloxy group;
a C₁₋₆ alkoxy group optionally substituted by halogen atom(s);
a C₁₋₆ alkylsulfonyl group optionally substituted by halogen atom(s);
a C₁₋₆ alkyl-carbonyl group;
a C₁₋₆ alkoxy-carbonyl group;
a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
a di-C₁₋₆ alkylamino group;
a tri-substituted silyl group;
a tri-substituted silyl oxy group;
a C₆₋₁₄ hydrocarbocyclic group optionally substituted by 1 to 3 substituents selected from substituent group b or substituent group b';
a 5- to 14-membered aromatic heterocyclic group optionally substituted by 1 to 3 substituents selected from substituent group b or substituent group b'; and
a 3- to 14-membered non-aromatic heterocyclic group optionally substituted by 1 to 3 substituents selected from substituent group b or substituent group b'

(Substituent group a'):
a halogen atom;
a hydroxy group;
a cyano group;
an oxo group;
a C₁₋₆ alkyl group optionally substituted by substituent(s) selected from the group consisting of a halogen atom, a hydroxy group, a cyano group, a C₁₋₆ alkoxy group, a C₆₋₁₄ aryl group, and a C₆₋₁₄ aryloxy group;
a C₁₋₆ alkoxy group optionally substituted by halogen atom(s);
a C₁₋₆ alkylsulfonyl group optionally substituted by halogen atom(s);
a C₁₋₆ alkyl-carbonyl group;
a C₁₋₆ alkoxy-carbonyl group;
carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
a di-C₁₋₆ alkylamino group;
a tri-substituted silyl group;
a tri-substituted silyl oxy group;
a C₆₋₁₄ hydrocarbocyclic group optionally substituted by 1 to 3 substituents selected from substituent group b or substituent group b';
a 5- to 14-membered aromatic heterocyclic group optionally substituted by 1 to 3 substituents selected from substituent group b or substituent group b'; and
a 3- to 14-membered non-aromatic heterocyclic group optionally substituted by 1 to 3 substituents selected from substituent group b or substituent group b'.

(Substituent group b):
a halogen atom;
a hydroxy group;
a cyano group;
an oxo group;
a carboxy group;
a C₁₋₆ alkyl group optionally substituted by substituent(s) selected from the group consisting of a halogen atom, a hydroxy group, a cyano group, and a C₁₋₆ alkoxy group;
a C₃₋₈ cycloalkyl group optionally substituted by substituent(s) selected from the group consisting of a halogen atom, a hydroxy group, a cyano group, and a C₁₋₆ alkoxy group;
a C₆₋₁₀ aryl group optionally substituted by substituent(s) selected from the group consisting of a halogen atom, a hydroxy group, a cyano group, a C₁₋₆ alkyl group, and a C₁₋₆ alkoxy group;
a 5- to 14-membered aromatic heterocyclic group optionally substituted by substituent(s) selected from the group consisting of a halogen atom, a hydroxy group, a cyano group, a C₁₋₆ alkyl group, and a C₁₋₆ alkoxy group;
a 3- to 14-membered non-aromatic heterocyclic group optionally substituted by substituent(s) selected from the group consisting of a halogen atom, a hydroxy group, a cyano group, a C₁₋₆ alkyl group, and a C₁₋₆ alkoxy group;
a C₁₋₆ alkoxy group optionally substituted by substituent(s) selected from the group consisting of a halogen atom, a hydroxy group, a cyano group, and a C₁₋₆ alkoxy group;
a C₁₋₆ alkylsulfanyl group optionally substituted by substituent(s) selected from the group consisting of a halogen atom, a hydroxy group, a cyano group, and a C₁₋₆ alkoxy group;
a C₃₋₈ cycloalkyloxy group optionally substituted by substituent(s) selected from the group consisting of a halogen atom, a hydroxy group, a cyano group, and a C₁₋₆ alkoxy group;
a C₁₋₆ alkylsulfonyl group optionally substituted by halogen atom(s);
a C₁₋₆ alkyl-carbonyl group optionally substituted by halogen atom(s);
a C₁₋₆ alkoxy-carbonyl group;
a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
a sulfamoyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from the group consisting of a halogen atom, a hydroxy group, a cyano group, and a C₁₋₆ alkoxy group; and
a pentafluorosulfanyl group.

(Substituent group b'):
a halogen atom;
a hydroxy group;
a cyano group;
an oxo group;
a carboxy group;
a C₁₋₆ alkyl group optionally substituted by substituent(s) selected from the group consisting of a halogen atom, a hydroxy group, a cyano group, and a C₁₋₆ alkoxy group;
a C₃₋₈ cycloalkyl group optionally substituted by substituent(s) selected from the group consisting of a halogen atom, a hydroxy group, a cyano group, and a C₁₋₆ alkoxy group;
a C₁₋₆ alkoxy group optionally substituted by substituent(s) selected from the group consisting of a halogen atom, a hydroxy group, a cyano group, and a C₁₋₆ alkoxy group;
a C₁₋₆ alkylsulfanyl group optionally substituted by substituent(s) selected from the group consisting of a halogen atom, a hydroxy group, a cyano group, and a C₁₋₆ alkoxy group;
a C₃₋₈ cycloalkyloxy group optionally substituted by substituent(s) selected from the group consisting of a halogen atom, a hydroxy group, a cyano group, and a C₁₋₆ alkoxy group;
a C₁₋₆ alkylsulfonyl group optionally substituted by halogen atom(s);
a C₁₋₆ alkyl-carbonyl group optionally substituted by halogen atom(s);
a C₁₋₆ alkoxy-carbonyl group;
a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
a sulfamoyl group substituted by one substituent selected from the group consisting of a C₁₋₆ alkyl group, a C₃₋₈ cycloalkyl group and a non-aromatic heterocyclic group, each of which is optionally substituted by 1 to 3 substituents selected from the group consisting of a halogen atom, a hydroxy group, a cyano group, and a C₁₋₆ alkoxy group; and
a pentafluorosulfanyl group.

(Substituent group c):
a halogen atom;
a hydroxy group;
a carboxy group;
a nitro group;
a cyano group;
an amino group optionally substituted by 1 or 2 substituents selected from the group consisting of a C₁₋₆ alkyl group and a C₁₋₆ alkoxy-carbonyl group;
a C₁₋₆ alkyl group optionally substituted by 1 to 3 substituents selected from substituent group a or substituent group a';
a C₁₋₆ alkoxy group optionally substituted by 1 to 3 substituents selected from substituent group a or substituent group a';
a C₁₋₆ alkoxy-carbonyl group;
a C₁₋₆ alkylsulfonyl group optionally substituted by 1 to 3 substituents selected from substituent group a or substituent group a';
a C₃₋₈ cycloalkyl group optionally substituted by 1 to 3 substituents selected from substituent group a or substituent group a';
an acyl group optionally substituted by 1 to 3 substituents selected from substituent group a or substituent group a';
an acyloxy group optionally substituted by 1 to 3 substituents selected from substituent group a or substituent group a';
a C₆₋₁₄ aryl group optionally substituted by 1 to 3 substituents selected from substituent group b;
a C₆₋₁₄ aryloxy group optionally substituted by 1 to 3 substituents selected from substituent group b;
a C₆₋₁₄ arylsulfonyl group optionally substituted by 1 to 3 substituents selected from substituent group b;
a C₆₋₁₄ arylsulfonyloxy group optionally substituted by 1 to 3 substituents selected from substituent group b;
a C₇₋₁₈ aralkyl group optionally substituted by 1 to 3 substituents selected from substituent group b;
a C₇₋₁₈ aralkyloxy group optionally substituted by 1 to 3 substituents selected from substituent group b;
a carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
a carbamoyloxy group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
an oxo group;
a thioxo group;
a mono- or di-C₁₋₆ alkylamino group;
an azido group;
a tri-substituted silyl group;
a tri-substituted silyloxy group;
an aromatic heterocyclic group optionally substituted by 1 to 3 substituents selected from substituent group b; and
a non-aromatic heterocyclic group optionally substituted by 1 to 3 substituents selected from substituent group b.

(Substituent group c'):
a halogen atom;
a hydroxy group;
a carboxy group;
a nitro group;
a cyano group;
an amino group optionally substituted by 1 or 2 substituents selected from the group consisting of a C₁₋₆ alkyl group and a C₁₋₆ alkoxy-carbonyl group;
a C₁₋₆ alkyl group optionally substituted by 1 to 3 substituents selected from substituent group a or substituent group a';
a C₁₋₆ alkoxy group optionally substituted by 1 to 3 substituents selected from substituent group a or substituent group a';
a C₁₋₆ alkoxy-carbonyl group;
a C₁₋₆ alkylsulfonyl group optionally substituted by 1 to 3 substituents selected from substituent group a or substituent group a';
a C₃₋₈ cycloalkyl group optionally substituted by 1 to 3 substituents selected from substituent group a or substituent group a';
an acyl group optionally substituted by 1 to 3 substituents selected from substituent group a or substituent group a';
an acyloxy group optionally substituted by 1 to 3 substituents selected from substituent group a or substituent group a';
C₆₋₁₄ aryl group optionally substituted by 1 to 3 substituents selected from substituent group b;
a C₆₋₁₄ aryloxy group optionally substituted by 1 to 3 substituents selected from substituent group b';
a C₆₋₁₄ arylsulfonyl group optionally substituted by 1 to 3 substituents selected from substituent group b';
a C₆₋₁₄ arylsulfonyloxy group optionally substituted by 1 to 3 substituents selected from substituent group b';
a C₇₋₁₈ aralkyl group optionally substituted by 1 to 3 substituents selected from substituent group b';
a C₇₋₁₈ aralkyloxy group optionally substituted by 1 to 3 substituents selected from substituent group b';
a carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
a carbamoyloxy group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
an oxo group;
a thioxo group;
a mono- or di-C₁₋₆ alkylamino group;
an azido group;
a tri-substituted silyl group;
a tri-substituted silyloxy group;
an aromatic heterocyclic group optionally substituted by 1 to 3 substituents selected from substituent group b'; and
a non-aromatic heterocyclic group optionally substituted by 1 to 3 substituents selected from substituent group b'.

In the present specification, the "pharmaceutically acceptable salt thereof" means a salt that can be used as a medicament. When the compound (I) of the present invention has an acidic group or a basic group, it shows a basic salt or an acidic salt that can be obtained by reacting with a base or an acid. A water-soluble salt is preferred.

Examples of the pharmaceutically acceptable "basic salt" of the compound (I) of the present invention include alkali metal salts such as sodium salt, potassium salt, lithium salt, and the like; alkaline earth metal salts such as magnesium salt, calcium salt, and the like; ammonium salts such as ammonium salt, tetramethylammonium salt, and the like; salts with organic base such as N-methylmorpholinium salt, triethylamine salt, tributylamine salt, diisopropylethylamine salt, dicyclohexylamine salt, N-methylpiperidinium salt, pyridinium salt, 4-pyrrolidinopyridinium salt, picolinium salt, and the like, basic amino acid salts such as lysine salt, arginine salt and the like and the like. It is preferably an alkali metal salt (particularly, sodium salt or potassium salt) or a basic amino acid salt.

Examples of the pharmaceutically acceptable "acidic salt" of the compound (I) of the present invention include inorganic acid salts such as hydrohalides (e.g., hydrofluoride, hydrochloride, hydrobromide, hydroiodide, and the like), nitride, perchloride, sulfate, phosphate and the like; organic acid salts such as lower alkanesulfonates (e.g., methanesulfonate, trifluoromethanesulfonate, ethanesulfonate, and the like), arylsulfonates (e.g., benzenesulfonate, p-toluenesulfonate, and the like), acetate, malate, fumarate, succinate, citrate, ascorbate, tartrate, oxalate, maleate and the like, and the like. A hydrohalide (particularly, hydrochloride) is preferred.

In the present specification, the "salt thereof" means all salts including the aforementioned "pharmaceutically acceptable salt thereof".

In the present specification, the "prophylaxis" includes prevention of disease onset, delay of disease onset, and prevention of pathogenesis. The "prophylactically effective amount" refers to a dose of the active ingredient sufficient to achieve the prophylactic purposes.

In the present specification, the "treatment" includes curing a disease, improving the pathology of a disease (e.g., one or more symptoms), and inhibiting the progression of the disease (severity of the disease). The "therapeutically effective amount" is a dose of the active ingredient sufficient to achieve the therapeutic purpose. Thus, the "improvement" is a concept encompassed by the "treatment".

In the present specification, the "subject" means an object to whom a medicament (pharmaceutical composition) containing an effective amount of an active ingredient necessary to prevent and/or treat (or ameliorate) a disease or a disease condition is administered. The "subject" may be a human or non-human animal, particularly mammal (e.g., human, mouse, rat, guinea pig, hamster, rabbit, cat, dog, bovine, sheep, monkey etc.).

In the present specification, the "B0AT1 inhibitor" means an agent containing a compound that has the ability to inhibit B0AT1, a transporter responsible for the reabsorption of neutral amino acids such as phenylalanine in the kidney. In particular, the compound (I) of the present invention, or a pharmaceutically acceptable salt thereof, exhibits excellent inhibitory activity against B0AT1. The B0AT1 inhibitory activity can be measured, for example, by the method described in Non-Patent Document 11 (SLAS Discovery, 2019; 24(2): p.111-120) and the method described in the Experimental Examples described below.

In the present specification, the "diseases for which symptoms can be alleviated by B0AT1 inhibition" means diseases caused by an increase in the level of neutral amino acids in the blood due to a mutation in a gene related to an amino acid metabolic pathway or the like. Specific examples of the disease include amino acid metabolism disorders such as phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, isovaleric acidemia, and the like.

### (The compound of the present invention (compound (I)))

Each group in the aforementioned formula (I) of compound (I) is explained below.

R¹ is a carboxy group or a group biologically equivalent to a carboxy group.

R¹ is preferably a carboxy group, an optionally substituted C₁₋₆ alkoxy-carbonyl group, an optionally substituted carbamoyl group, a cyano group, or a group selected from the group consisting of the following formulas: 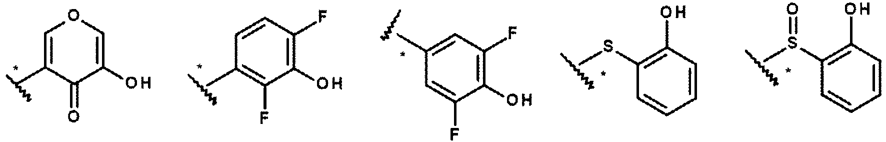 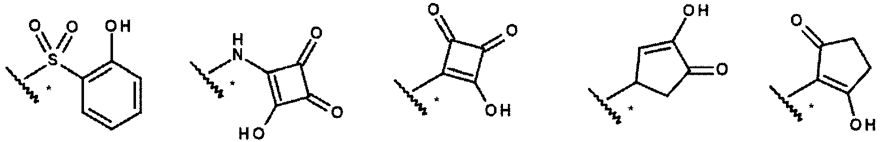 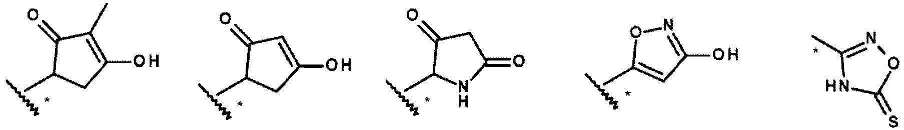 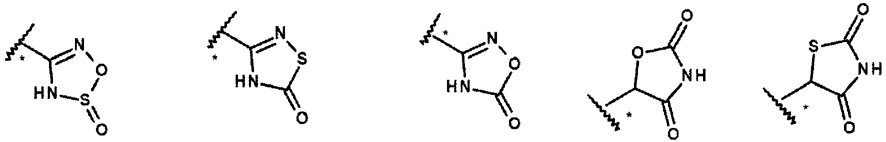 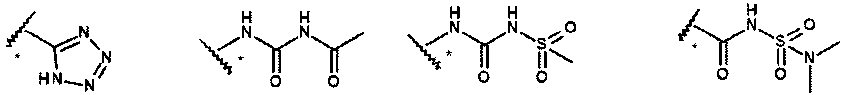 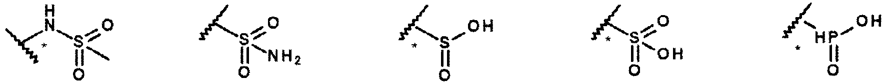 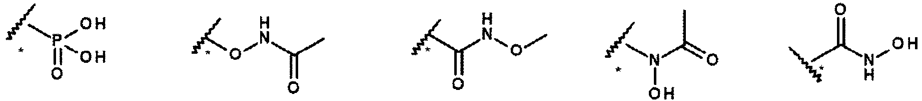
wherein a wavy line is a bonding site of the group represented by the above-mentioned chemical formula, and * is a bonding position with L, or a tautomeric group thereof,
more preferably, a carboxy group, an optionally substituted C₁₋₆ alkoxy-carbonyl group, or an optionally substituted carbamoyl group.

L is a divalent organic group comprising a group selected from the group consisting of a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, each of which is optionally further substituted.

L is preferably a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from the aforementioned substituent group a or substituent group a',
more preferably, a C₁₋₆ alkylene group optionally having, in the inside or at the end, one or more divalent groups selected from the group consisting of -NR²- (wherein R² is a hydrogen atom or a C₁₋₆ alkyl group), -O-, -S-, -C(=O)-, a C₆₋₁₀ arylene group (e.g., 1,2-phenylene, 1,3-phenylene, 1,4-phenylene), a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), C₂₋₆ alkynylene group, and 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
a C₆₋₁₀ arylene group (e.g., 1,2-phenylene, 1,3-phenylene, 1,4-phenylene) optionally further substituted by 1 to 3 substituents selected from the aforementioned substituent group a or substituent group a',
a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a',
a C₂₋₄ alkynylene group optionally further substituted by 1 to 3 substituents selected from the aforementioned substituent group a or substituent group a', or
a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a',
further preferably, a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a',
particularly preferably, a C₁₋₆ alkylene group optionally further substituted by 1 to 3 substituents selected from the aforementioned substituent group a or substituent group a'.

A is a divalent nitrogen-containing heterocyclic group that may be further substituted.

A is preferably a divalent nitrogen-containing non-aromatic heterocyclic group represented by the following formula: 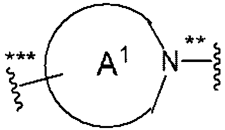
wherein ** is a bonding site with a carbonyl group, *** is a bonding site with group L, and ring A¹ is a 3- to 10-membered monocycle, a 8- to 14-membered fused cycle, a 6- to 16-membered spiro cycle, or a 6- to 14-membered bridged cycle, and optionally has, as a ring-constituting atom besides a nitrogen atom and a carbon atom, hetero atom(s) selected from the group consisting of a nitrogen atom, an oxygen atom, and a sulfur atom, which is optionally further substituted by 1 to 3 substituents selected from the aforementioned substituent group a or substituent group a',
one more preferred embodiment is a divalent nitrogen-containing non-aromatic heterocyclic group represented by the following formula: 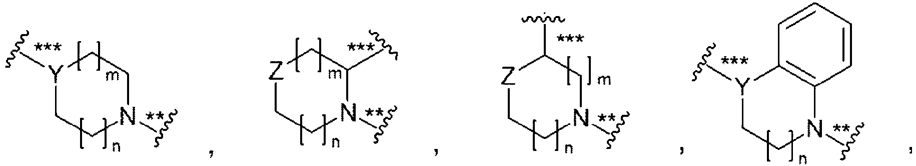 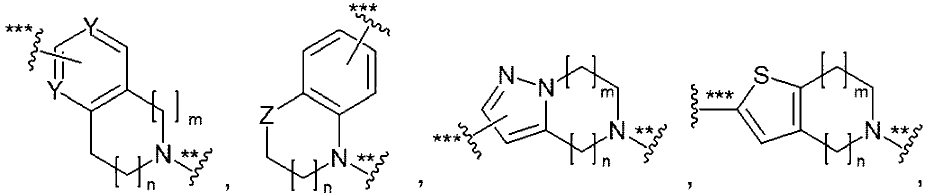 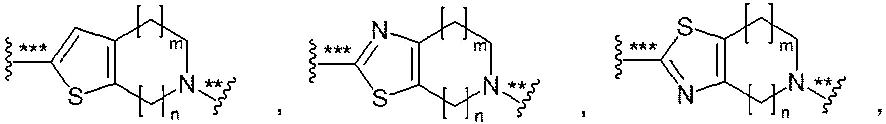 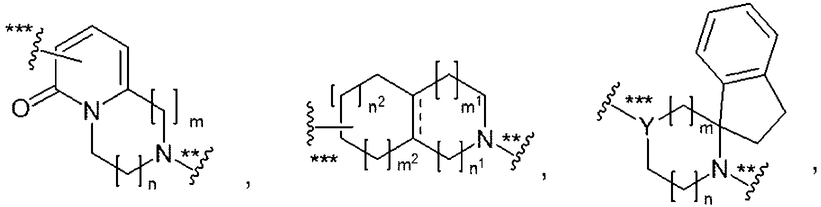 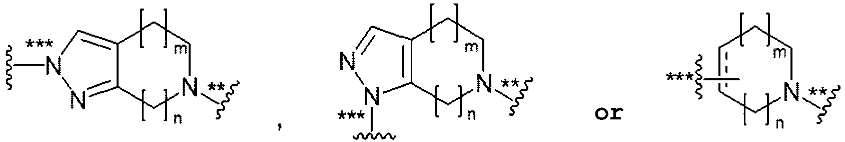
wherein a wavy line is a bonding site of the group represented by the above-mentioned chemical formula,
a bond represented by
   -̅ -̅ -̅ -̅ -̅ -̅
is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 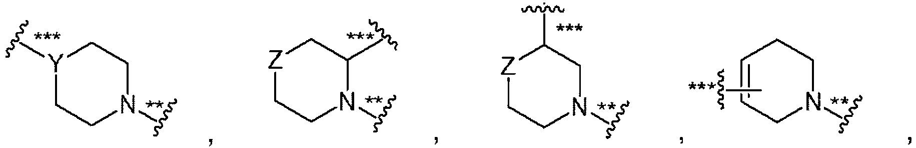 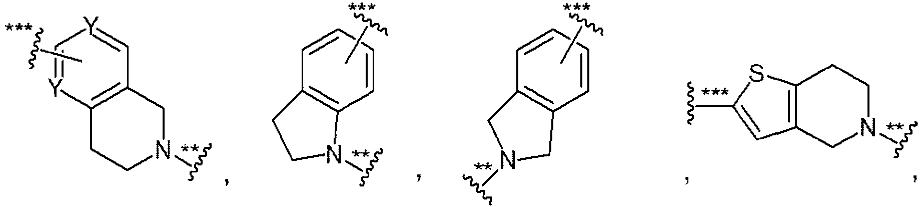 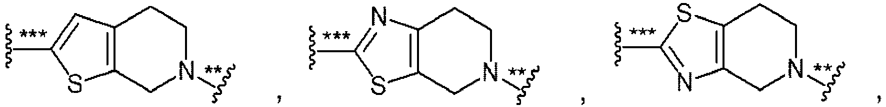 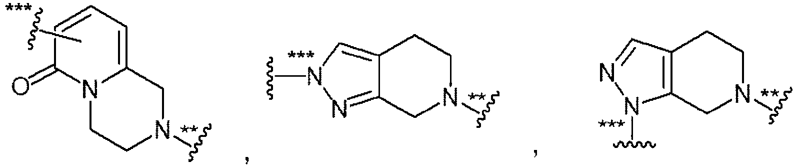 or 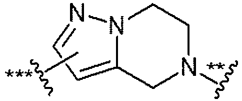
wherein each symbol is as defined above, and optionally further substituted by 1 to 3 substituents selected from the aforementioned substituent group a or substituent group a'), another more preferred embodiment is a divalent nitrogen-containing non-aromatic heterocyclic group represented by the following formula: 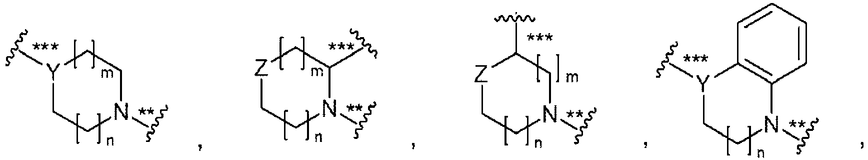 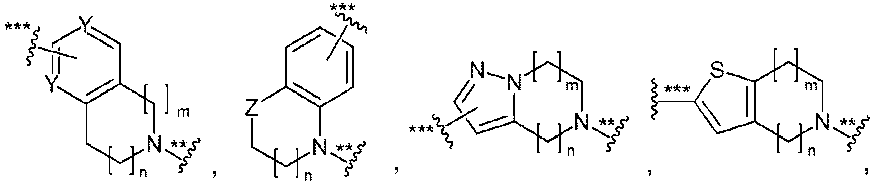 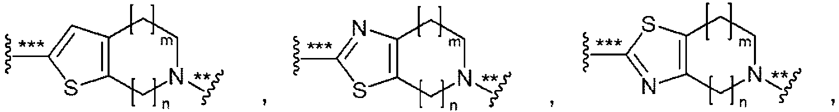 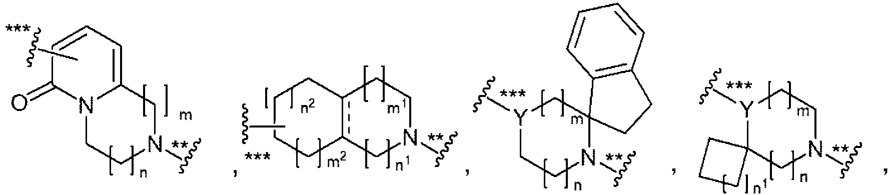 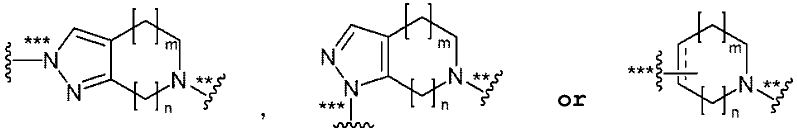
wherein a wavy line is a bonding site of the group represented by the above-mentioned chemical formula,
a bond represented by
   -̅ -̅ -̅ -̅ -̅ -̅
is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to
2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 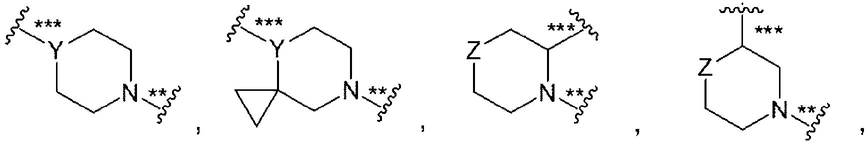 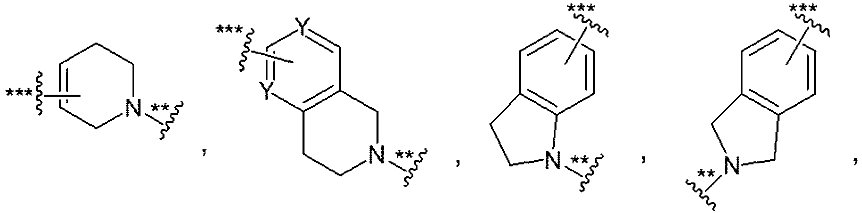 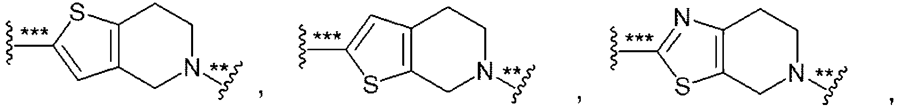 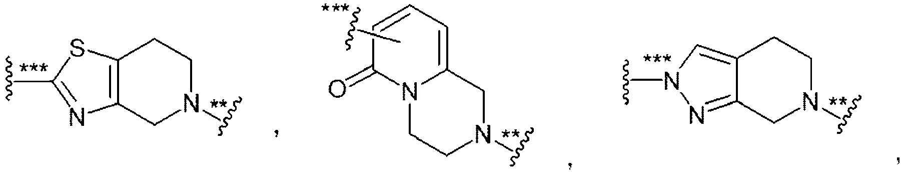 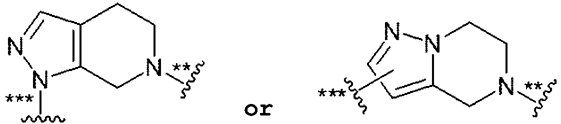
wherein each symbol is as defined above, and optionally further substituted by 1 to 3 substituents selected from the aforementioned substituent group a or substituent group a'), further preferably, a divalent nitrogen-containing non-aromatic heterocyclic group represented by the following formula: 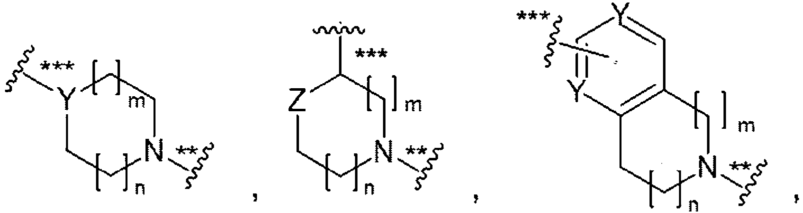 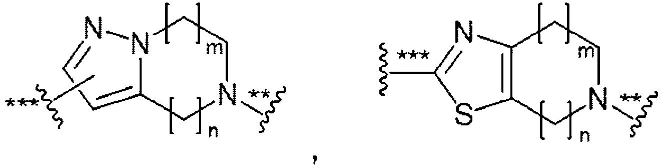 or 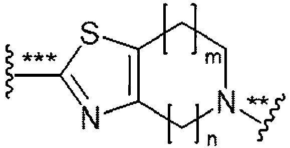
wherein each symbol is as defined above, and optionally further substituted by 1 to 3 substituents selected from the aforementioned substituent group a or substituent group a' (e.g., a divalent nitrogen-containing non-aromatic heterocyclic group represented by the following formula: 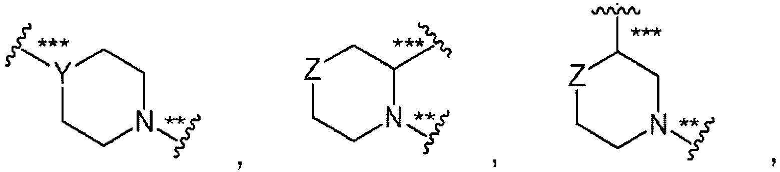 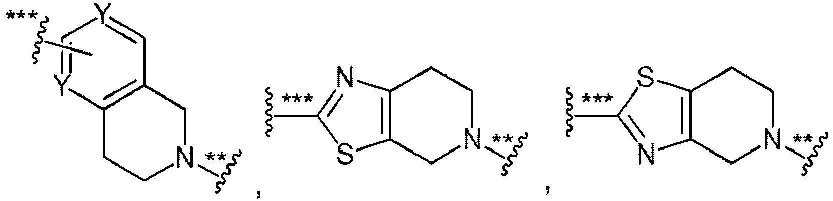 or 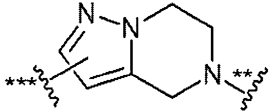
wherein each symbol is as defined above, and optionally further substituted by 1 to 3 substituents selected from the aforementioned substituent group a or substituent group a'), still more preferably, a divalent nitrogen-containing non-aromatic heterocyclic group represented by the following formula: 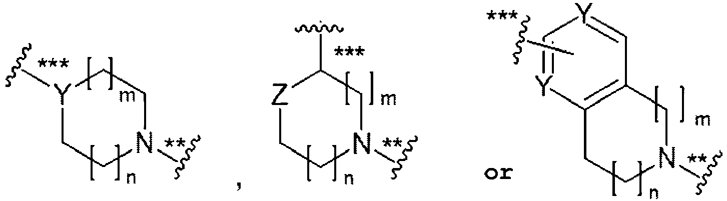
wherein each symbol is as defined above, and optionally further substituted by 1 to 3 substituents selected from the aforementioned substituent group a or substituent group a' (e.g., a divalent nitrogen-containing non-aromatic heterocyclic group represented by the following formula: 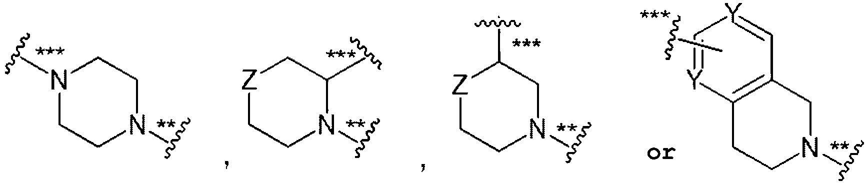
wherein each symbol is as defined above, and optionally further substituted by 1 to 3 substituents selected from the aforementioned substituent group a or substituent group a', provided that when Z is CR⁵R⁶, the optionally further substituted substituent is selected from the group consisting of, among the substituents in the aforementioned substituent group a or substituent group a', a C₆₋₁₄ hydrocarbocyclic group optionally substituted by 1 to 3 substituents selected from substituent group b or substituent group b'; a 5- to 14-membered aromatic heterocyclic group optionally substituted by 1 to 3 substituents selected from substituent group b or substituent group b'; and a 3- to 14-membered non-aromatic heterocyclic group optionally substituted by 1 to 3 substituents selected from substituent group b or substituent group b'),
particularly preferably, a divalent nitrogen-containing non-aromatic heterocyclic group represented by the following formula: 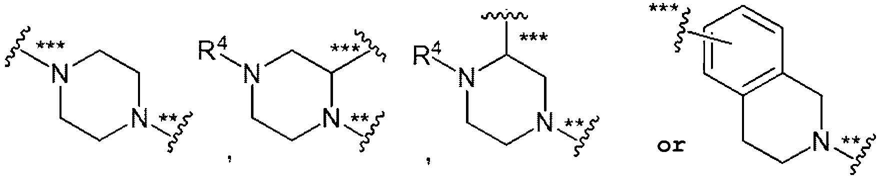
wherein each symbol is as defined above, and optionally further substituted by 1 to 3 substituents selected from the aforementioned substituent group a or substituent group a' (particularly preferably, a divalent nitrogen-containing non-aromatic heterocyclic group represented by the following formula: 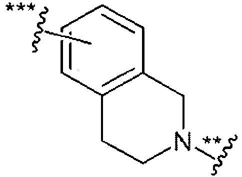
wherein each symbol is as defined above, and optionally further substituted by 1 to 3 substituents selected from the aforementioned substituent group a or substituent group a').

R is an optionally substituted C₆₋₁₀ aryl-ethenyl group.

R is preferably a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group, more preferably, a 2-(E)-phenylethenyl group optionally substituted by 1 to 3 substituents selected from the group consisting of aa halogen atom, a C₁₋₆ alkyl group optionally substituted by halogen atom(s), and a pentafluorosulfanyl group.

As compound (I), the following compounds are preferred.

### [Compound (IA)]

Compound (I), wherein R¹ is a carboxy group, an optionally substituted C₁₋₆ alkoxy-carbonyl group, an optionally substituted carbamoyl group, a cyano group, or a group selected from the group consisting of the following formulas: 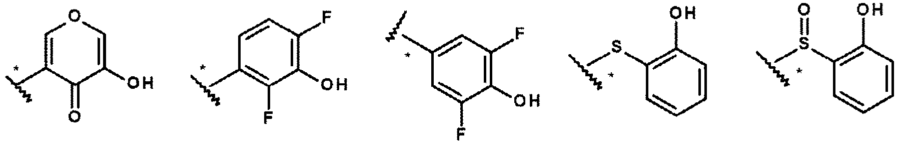 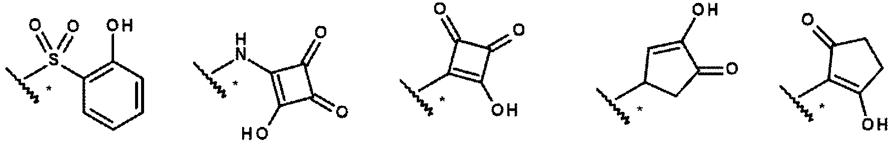 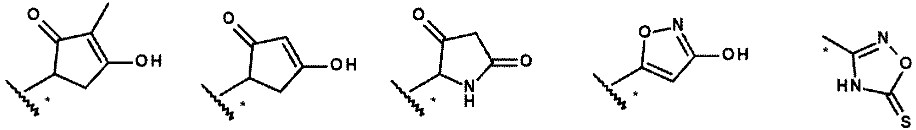 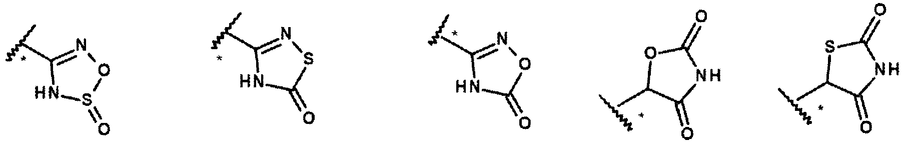 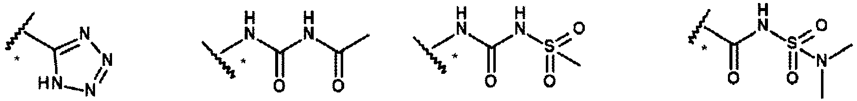 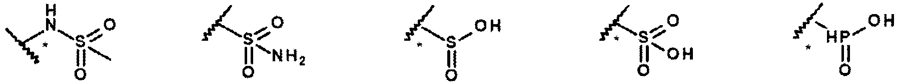 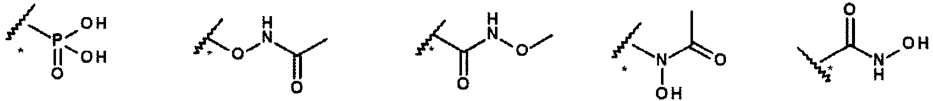
wherein a wavy line is a bonding site of the group represented by the above-mentioned chemical formula, and * is a bonding position with L,
or a tautomeric group thereof;
   L is a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from the aforementioned substituent group a or substituent group a';
   A is a divalent nitrogen-containing non-aromatic heterocyclic group represented by the following formula: 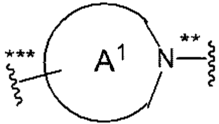
wherein ** is a bonding site with a carbonyl group, *** is a bonding site with group L, and ring A¹ is a 3- to 10-membered monocycle, a 8- to 14-membered fused cycle, a 6- to 16-membered spiro cycle or a 6- to 14-membered bridged cycle, and optionally contains, as a ring-constituting atom besides a nitrogen atom and a carbon atom, hetero atom(s) selected from the group consisting of a nitrogen atom, an oxygen atom, and a sulfur atom, and optionally further substituted by 1 to 3 substituents selected from the aforementioned substituent group a or substituent group a'; and
   R is a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group,
or a pharmaceutically acceptable salt thereof.

### [Compound (IB)]

Compound (I), wherein
R¹ is a carboxy group, an optionally substituted C₁₋₆ alkoxy-carbonyl group, or an optionally substituted carbamoyl group;
L is a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from the aforementioned substituent group a or substituent group a';
A is a divalent nitrogen-containing non-aromatic heterocyclic group represented by the following formula: 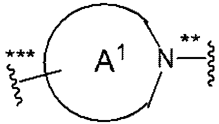 wherein ** is a bonding site with a carbonyl group, *** is a bonding site with group L, and ring A¹ is a 3- to 10-membered monocycle, a 8- to 14-membered fused cycle, a 6- to 16-membered spiro cycle or a 6- to 14-membered bridged cycle, and optionally contains, as a ring-constituting atom besides a nitrogen atom and a carbon atom, hetero atom(s) selected from the group consisting of a nitrogen atom, an oxygen atom, and a sulfur atom, and optionally further substituted by 1 to 3 substituents selected from the aforementioned substituent group a or substituent group a'; and
R is a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group,
or a pharmaceutically acceptable salt thereof.

### [Compound (IC)]

Compound (I), wherein
R¹ is a carboxy group, an optionally substituted C₁₋₆ alkoxy-carbonyl group, an optionally substituted carbamoyl group, a cyano group, or a group selected from the group consisting of the following formulas: 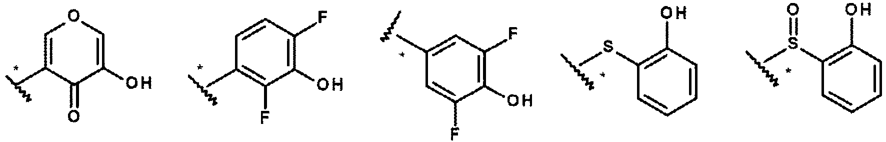 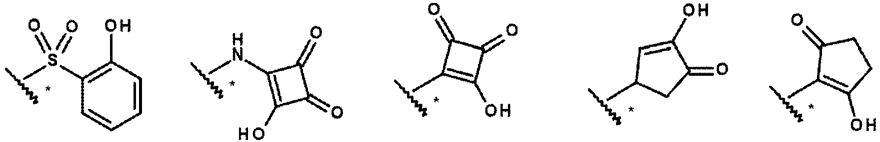 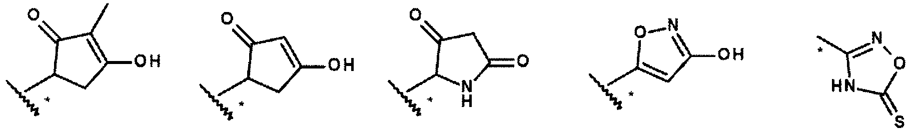 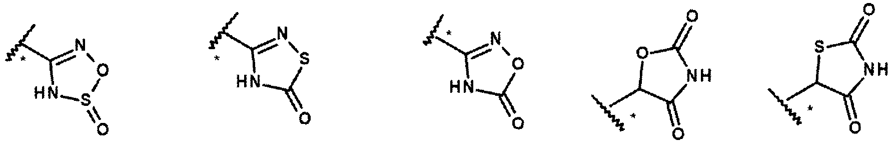 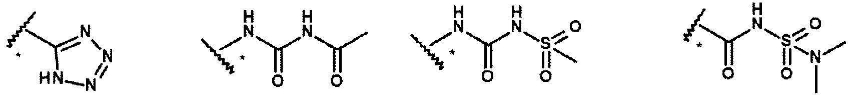 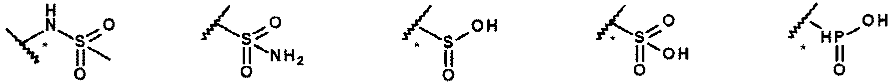 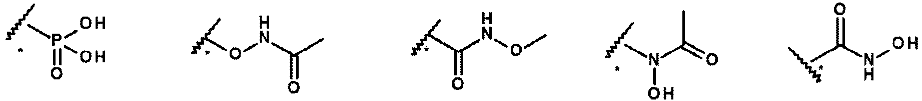 wherein a wavy line is a bonding site of the group represented by the above-mentioned chemical formula, and * is a bonding position with L, or a tautomeric group thereof;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, -C(=O)-, a C₆₋₁₀ arylene group (e.g., 1,2-phenylene, 1,3-phenylene, 1,4-phenylene), a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), C₂₋₆ alkynylene group, and 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₆₋₁₀ arylene group (e.g., 1,2-phenylene, 1,3-phenylene, 1,4-phenylene) optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a',
   a C₂₋₄ alkynylene group optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 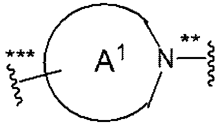 wherein ** is a bonding site with a carbonyl group, *** is a bonding site with group L, and ring A¹ is a 3- to 10-membered monocycle, a 8- to 14-membered fused cycle, a 6- to 16-membered spiro cycle, or a 6- to 14-membered bridged cycle, and optionally has, as a ring-constituting atom besides a nitrogen atom and a carbon atom, hetero atom(s) selected from the group consisting of a nitrogen atom, an oxygen atom, and a sulfur atom, which is optionally further substituted by 1 to 3 substituents selected from the aforementioned substituent group a or substituent group a'; and
R is a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group,
or a pharmaceutically acceptable salt thereof.

### [Compound (ID)]

Compound (I), wherein
R¹ is a carboxy group, an optionally substituted C₁₋₆ alkoxy-carbonyl group, an optionally substituted carbamoyl group, a cyano group, or a group selected from the group consisting of the following formulas: 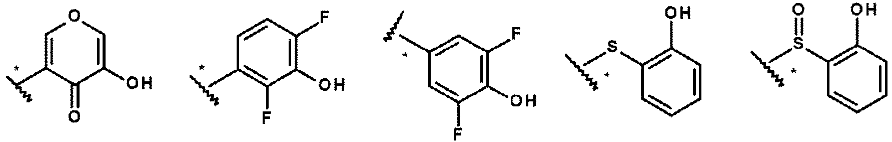 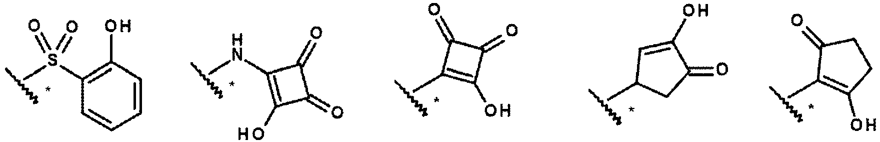 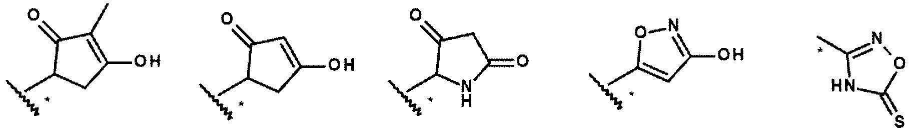 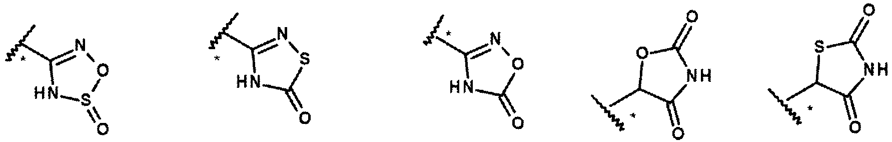 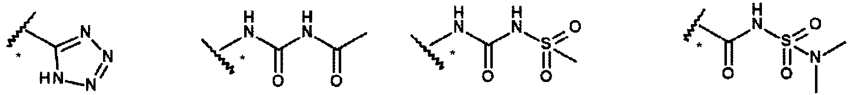 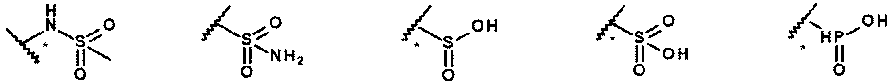 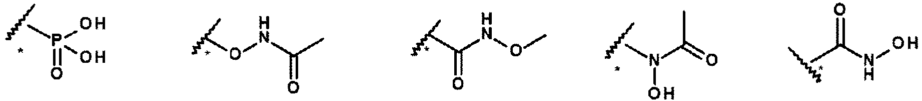 wherein a wavy line is a bonding site of the group represented by the above-mentioned chemical formula, and * is a bonding position with L, or a tautomeric group thereof;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 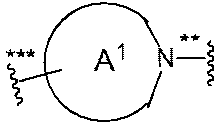 wherein ** is a bonding site with a carbonyl group, *** is a bonding site with group L, and ring A¹ is a 3- to 10-membered monocycle, a 8- to 14-membered fused cycle, a 6- to 16-membered spiro cycle, or a 6- to 14-membered bridged cycle, and optionally has, as a ring-constituting atom besides a nitrogen atom and a carbon atom, hetero atom(s) selected from the group consisting of a nitrogen atom, an oxygen atom, and a sulfur atom, which is optionally further substituted by 1 to 3 substituents selected from the aforementioned substituent group a or substituent group a'; and
R is a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group,
or a pharmaceutically acceptable salt thereof.

### [Compound (IE)]

Compound (I), wherein
R¹ is a carboxy group, an optionally substituted C₁₋₆ alkoxy-carbonyl group, an optionally substituted carbamoyl group, a cyano group, or a group selected from the group consisting of the following formulas: 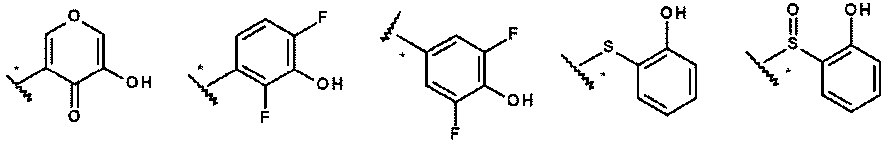 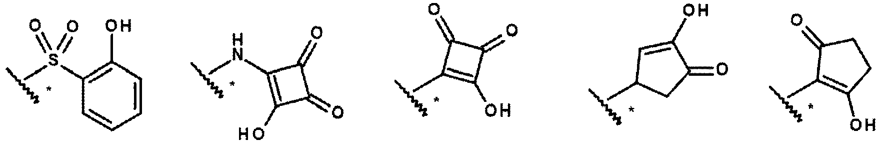 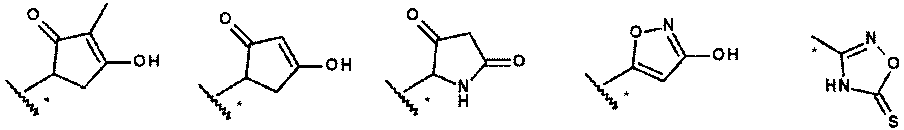 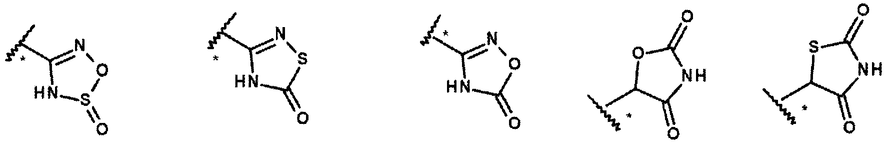 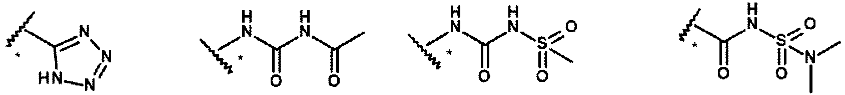 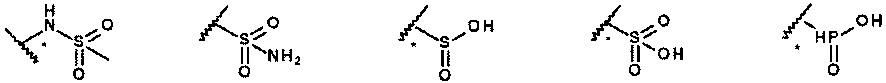 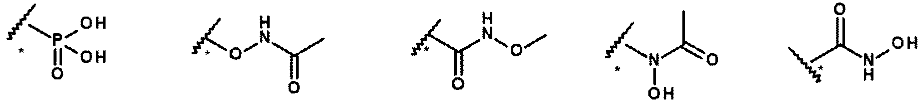 wherein a wavy line is a bonding site of the group represented by the above-mentioned chemical formula, and * is a bonding position with L, or a tautomeric group thereof;
L is a C₁₋₆ alkylene group optionally further substituted by 1 to 3 substituents selected from the aforementioned substituent group a or substituent group a';
A is a divalent nitrogen-containing non-aromatic heterocyclic group represented by the following formula: 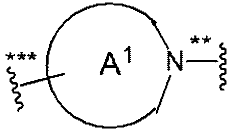 wherein ** is a bonding site with a carbonyl group, *** is a bonding site with group L, and ring A¹ is a 3- to 10-membered monocycle, a 8- to 14-membered fused cycle, a 6- to 16-membered spiro cycle, or a 6- to 14-membered bridged cycle, and optionally has, as a ring-constituting atom besides a nitrogen atom and a carbon atom, hetero atom(s) selected from the group consisting of a nitrogen atom, an oxygen atom, and a sulfur atom, which is optionally further substituted by 1 to 3 substituents selected from the aforementioned substituent group a or substituent group a'; and
R is a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group,
or a pharmaceutically acceptable salt thereof.

### [Compound (IF)]

Compound (I), wherein
R¹ is a carboxy group, an optionally substituted C₁₋₆ alkoxy-carbonyl group, an optionally substituted carbamoyl group, a cyano group, or a group selected from the group consisting of the following formulas: 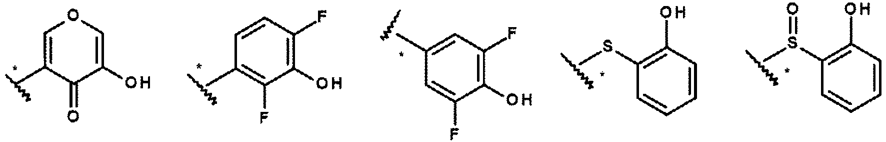 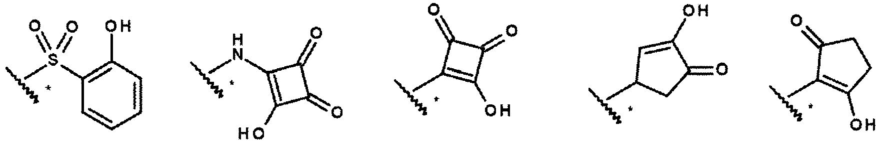 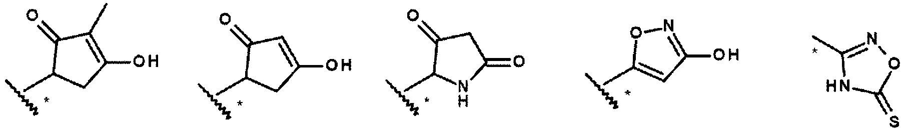 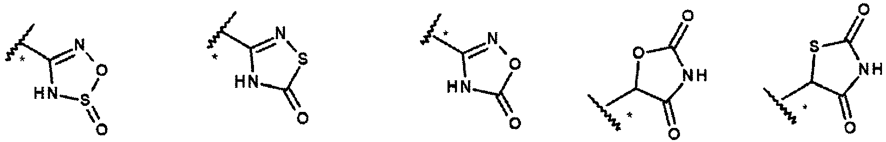 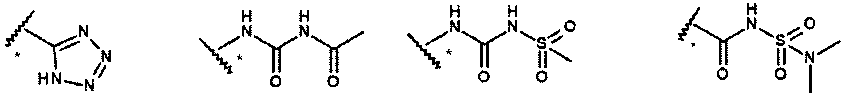 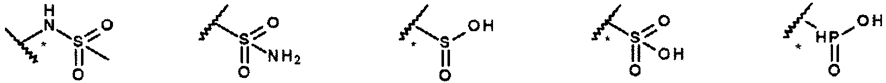 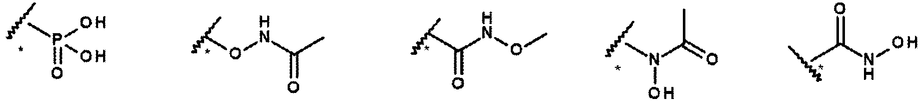 wherein a wavy line is a bonding site of the group represented by the above-mentioned chemical formula, and * is a bonding position with L, or a tautomeric group thereof;
L is a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from the aforementioned substituent group a or substituent group a';
A is a divalent nitrogen-containing non-aromatic heterocyclic group represented by the following formula: 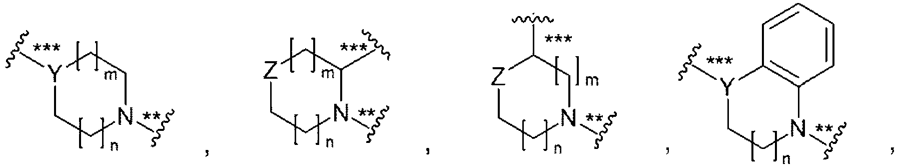 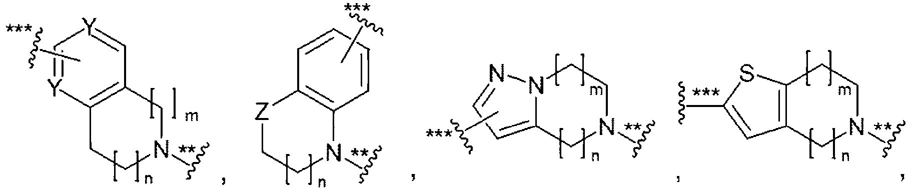 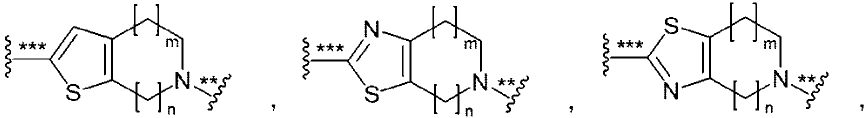 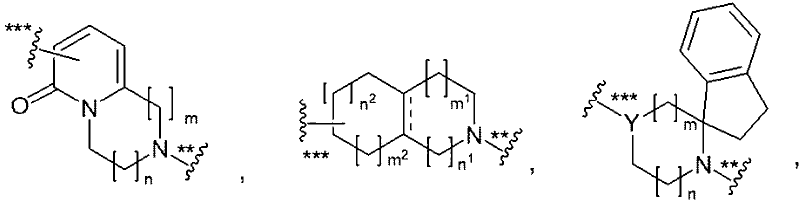 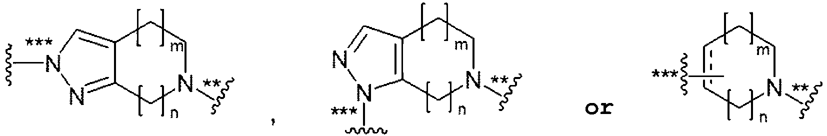
   wherein a wavy line is a bonding site of the group represented by the above-mentioned chemical formula,
   a bond represented by
      -̅ -̅ -̅ -̅ -̅ -̅
   is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 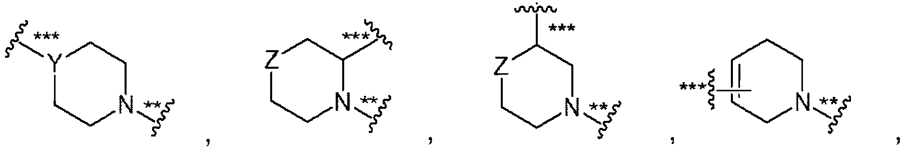 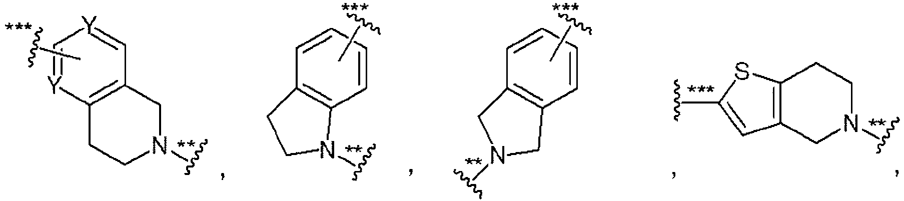 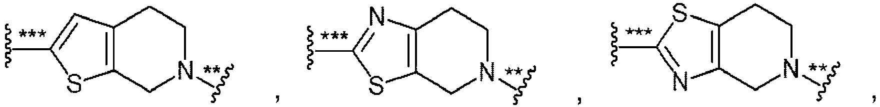 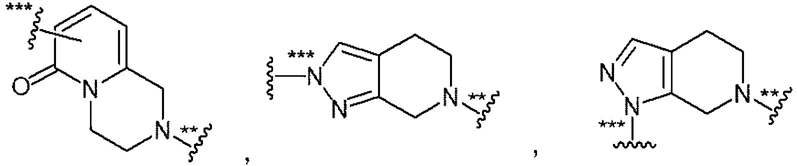 or 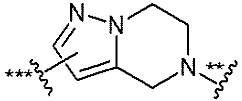
   wherein each symbol is as defined above, and optionally further substituted by 1 to 3 substituents selected from the aforementioned substituent group a or substituent group a'); and
R is a 2-phenylethenyl group optionally substituted by 1 to 3 substituents selected from the group consisting of a halogen atom, an optionally substituted C₁₋₆ alkyl group, an optionally substituted C₃-₈ cycloalkyl group, an optionally substituted C₁₋₆ alkoxy group, an optionally substituted C₃-₈ cycloalkyloxy group, an optionally substituted C₁₋₆ alkylsulfanyl group, an optionally substituted C₃₋₈ cycloalkylsulfanyl group, and a pentafluorosulfanyl group,
or a pharmaceutically acceptable salt thereof.

### [Compound (IF')]

Compound (I), wherein
A is a divalent nitrogen-containing non-aromatic heterocyclic group represented by the following formula: 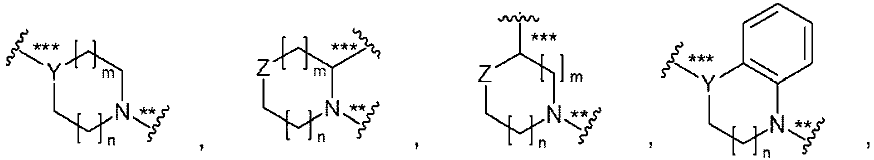 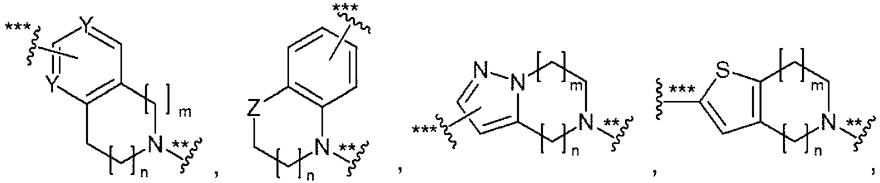 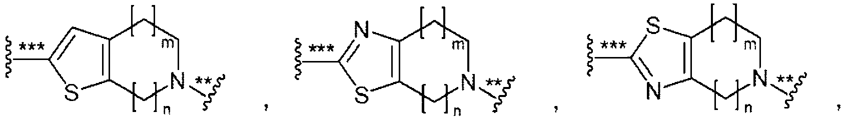 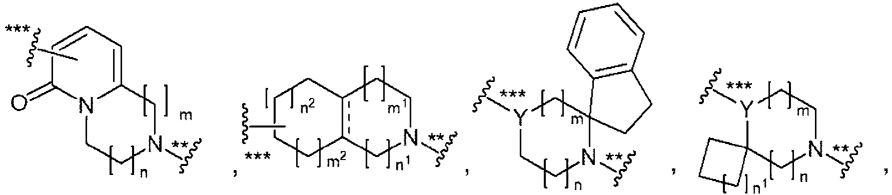 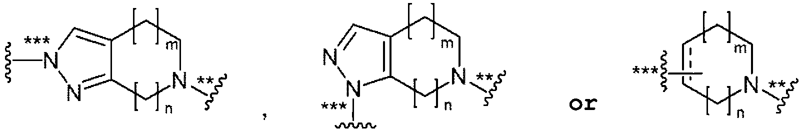
wherein a wavy line is a bonding site of the group represented by the above-mentioned chemical formula,
a bond represented by
   -̅ -̅ -̅ -̅ -̅ -̅
is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 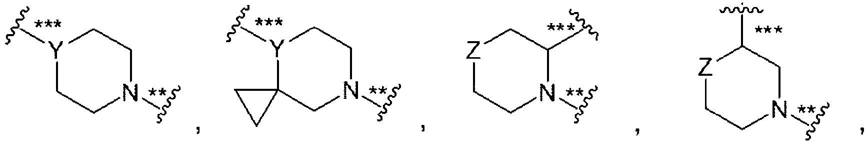 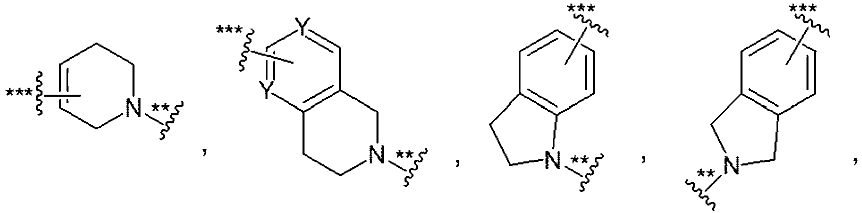 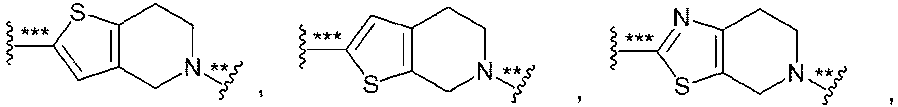 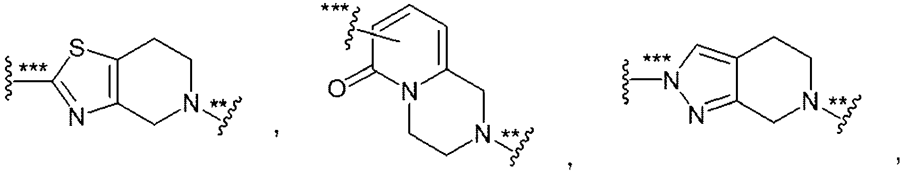 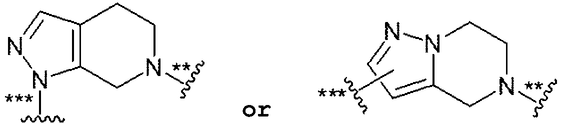
wherein each symbol is as defined above, and optionally further substituted by 1 to 3 substituents selected from the aforementioned substituent group a or substituent group a'); and
other symbols are as defined for the above-mentioned [compound (IF)],
or a pharmaceutically acceptable salt thereof.

### [Compound (IG)]

Compound (I), wherein
R¹ is a carboxy group, an optionally substituted C₁₋₆ alkoxy-carbonyl group, an optionally substituted carbamoyl group, a cyano group, or a group selected from the group consisting of the following formulas: 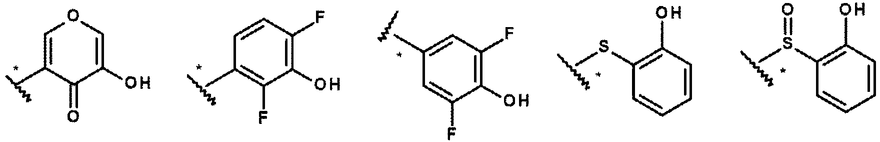 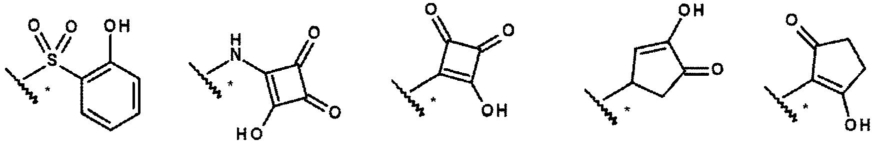 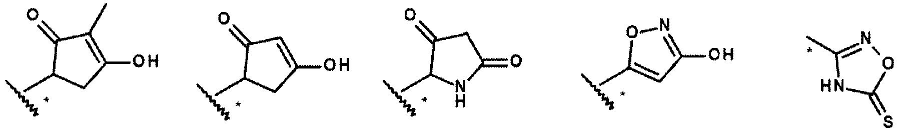 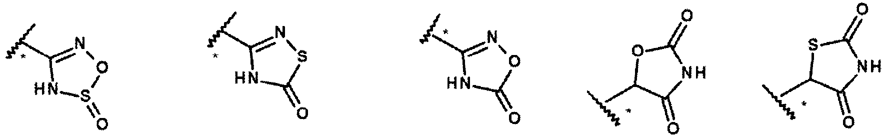 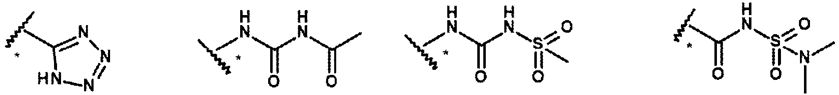 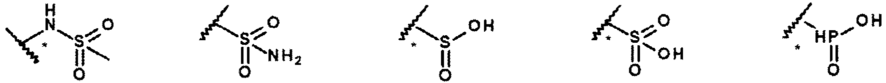 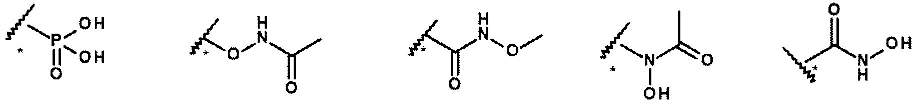 wherein a wavy line is a bonding site of the group represented by the above-mentioned chemical formula, and * is a bonding position with L, or a tautomeric group thereof;
L is a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from the aforementioned substituent group a or substituent group a';
A is a divalent nitrogen-containing non-aromatic heterocyclic group represented by the following formula: 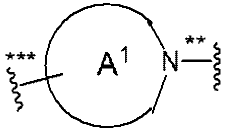 wherein ** is a bonding site with a carbonyl group, *** is a bonding site with group L, and ring A¹ is a 3- to 10-membered monocycle, a 8- to 14-membered fused cycle, a 6- to 16-membered spiro cycle, or a 6- to 14-membered bridged cycle, and optionally has, as a ring-constituting atom besides a nitrogen atom and a carbon atom, hetero atom(s) selected from the group consisting of a nitrogen atom, an oxygen atom, and a sulfur atom, which is optionally further substituted by 1 to 3 substituents selected from the aforementioned substituent group a or substituent group a'; and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group, or a pharmaceutically acceptable salt thereof.

### [Compound (IH)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, -C(=O)-, a C₆₋₁₀ arylene group (e.g., 1,2-phenylene, 1,3-phenylene, 1,4-phenylene), a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), C₂₋₆ alkynylene group, and 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₆₋₁₀ arylene group (e.g., 1,2-phenylene, 1,3-phenylene, 1,4-phenylene) optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a',
   a C₂₋₄ alkynylene group optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 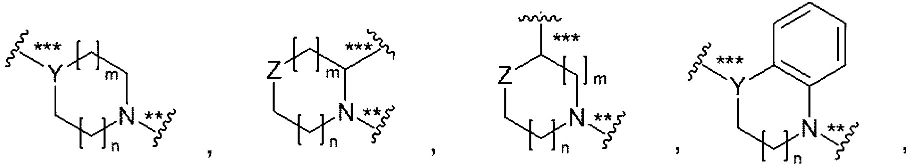 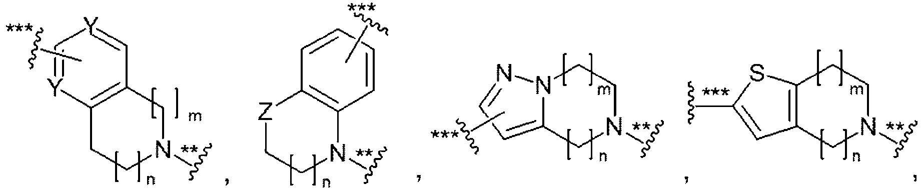 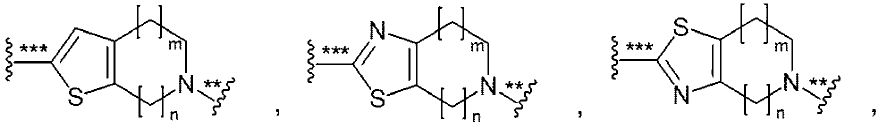 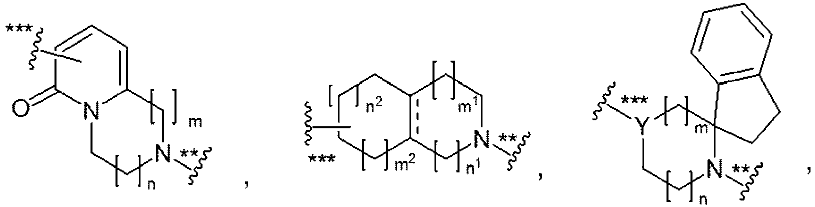 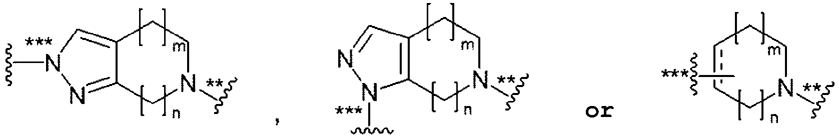
   wherein a wavy line is a bonding site of the group represented by the above-mentioned chemical formula,
   a bond represented by
      -̅ -̅ -̅ -̅ -̅ -̅
   is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 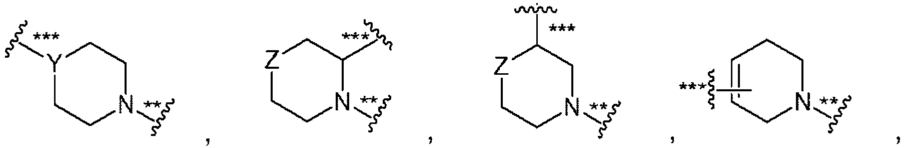 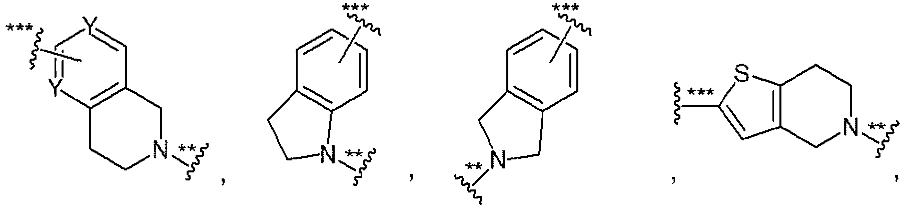 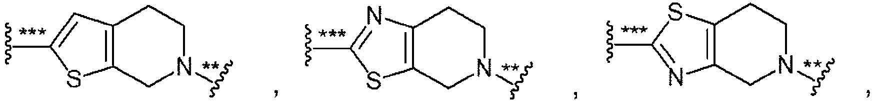 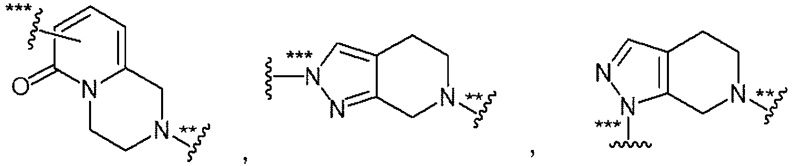 or 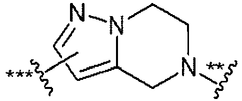
   wherein each symbol is as defined above, and optionally further substituted by 1 to 3 substituents selected from the aforementioned substituent group a or substituent group a'); and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IH')]

Compound (I), wherein
A is a divalent nitrogen-containing non-aromatic heterocyclic group represented by the following formula: 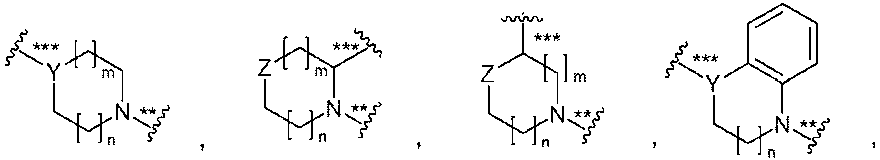 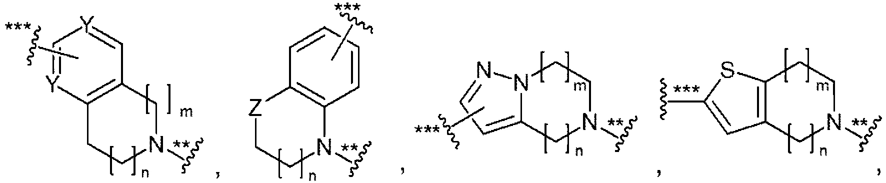 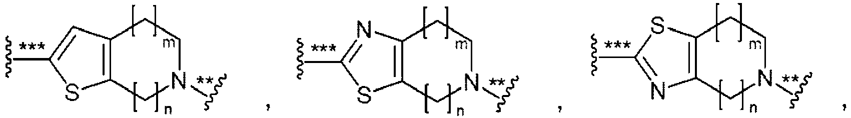 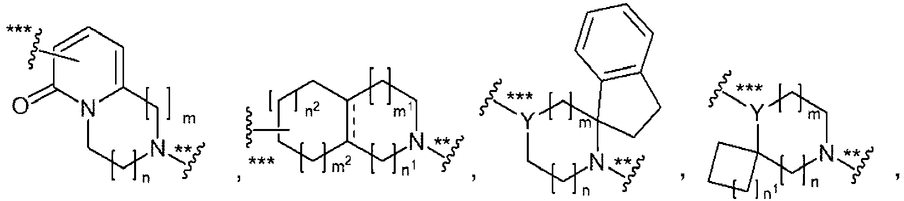 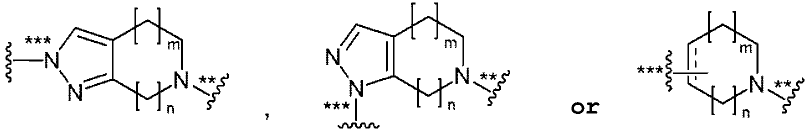
wherein a wavy line is a bonding site of the group represented by the above-mentioned chemical formula,
a bond represented by
   -̅ -̅ -̅ -̅ -̅ -̅
is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 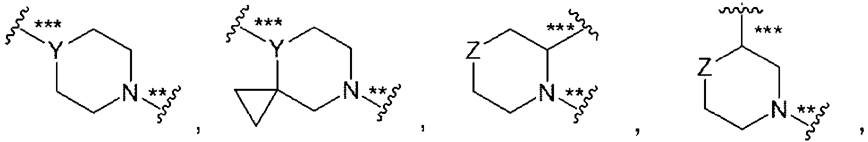 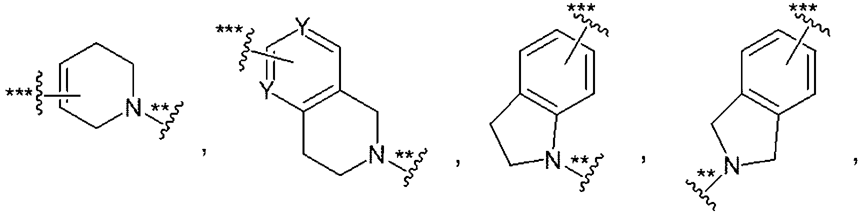 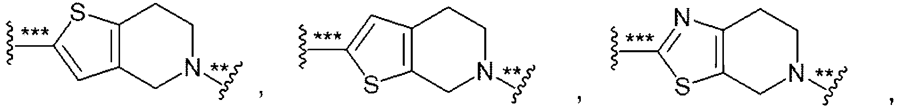 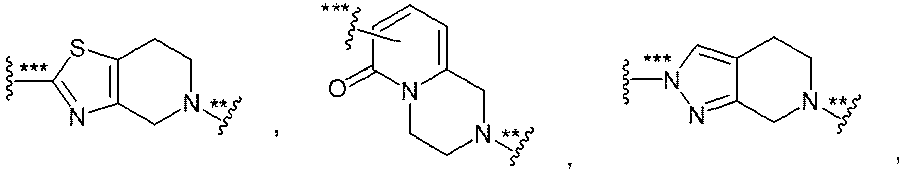 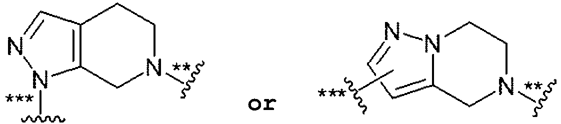
wherein each symbol is as defined above, and optionally further substituted by 1 to 3 substituents selected from the aforementioned substituent group a or substituent group a'); and
other symbols are as defined for the above-mentioned [compound (IH)],
or a pharmaceutically acceptable salt thereof.

### [Compound (IJ)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 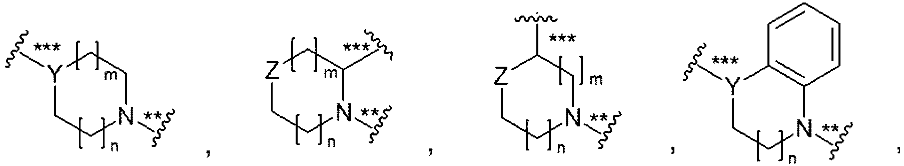 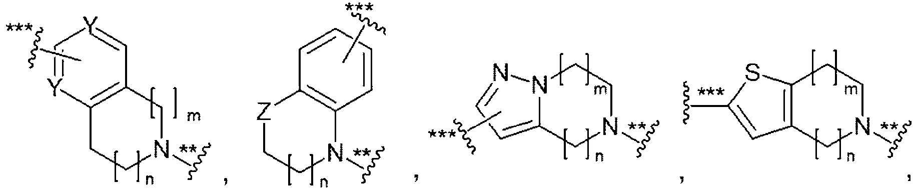 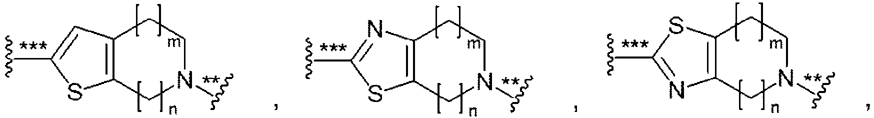 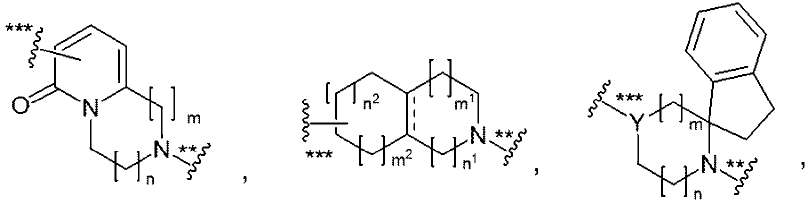 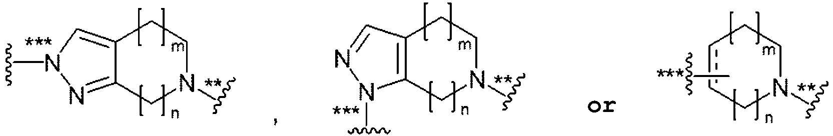
   wherein a wavy line is a bonding site of the group represented by the above-mentioned chemical formula,
   a bond represented by
      -̅ -̅ -̅ -̅ -̅ -̅
   is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 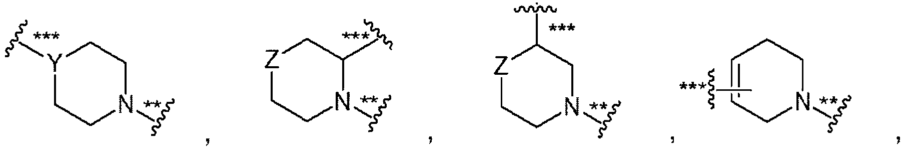 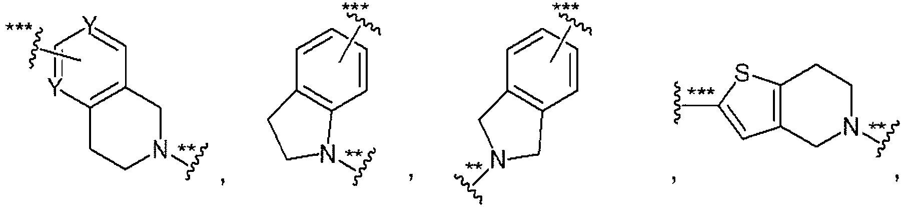 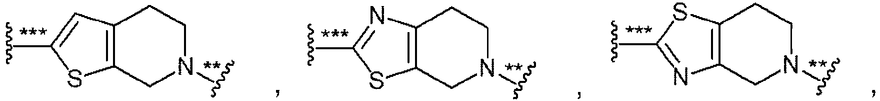 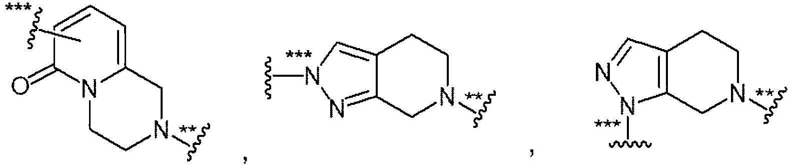 or 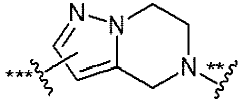
   wherein each symbol is as defined above, and optionally further substituted by 1 to 3 substituents selected from the aforementioned substituent group a or substituent group a'); and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IJ')]

Compound (I), wherein
A is a divalent nitrogen-containing non-aromatic heterocyclic group represented by the following formula: 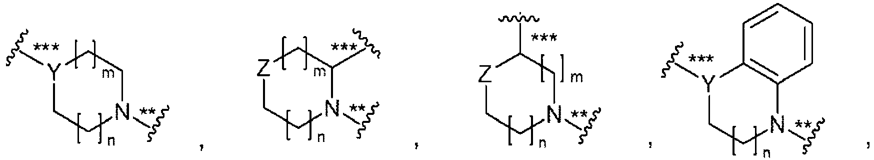 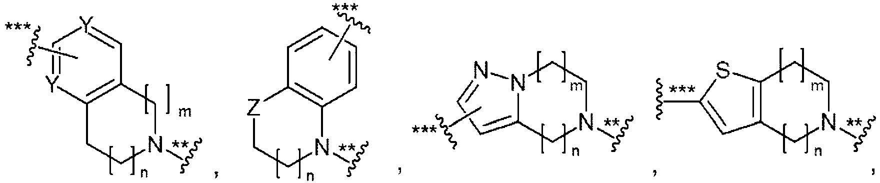 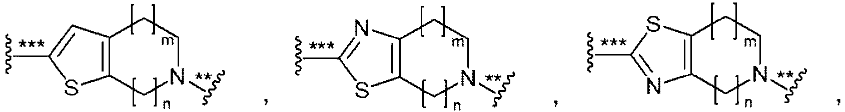 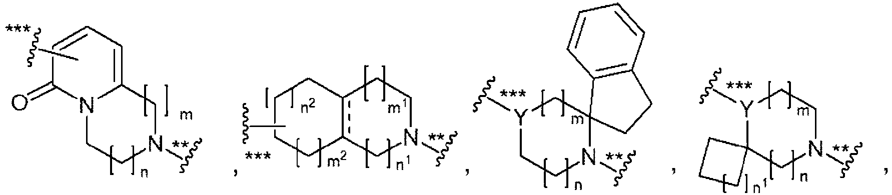 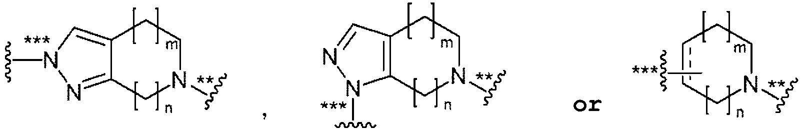
wherein a wavy line is a bonding site of the group represented by the above-mentioned chemical formula,
a bond represented by
   -̅ -̅ -̅ -̅ -̅ -̅
is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 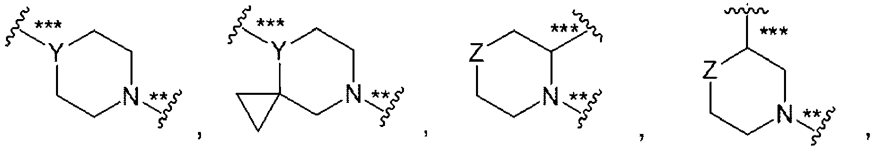 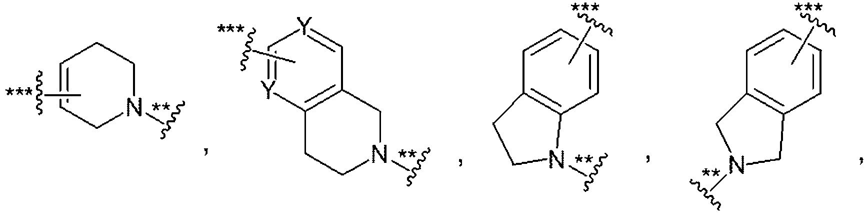 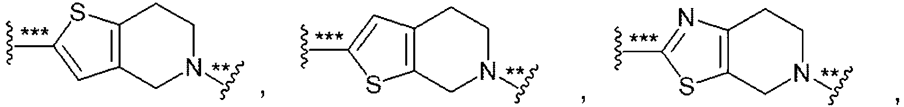 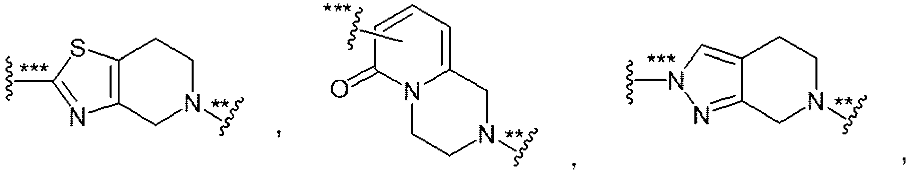 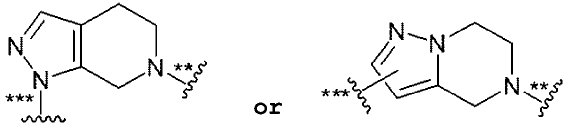
wherein each symbol is as defined above, and optionally further substituted by 1 to 3 substituents selected from the aforementioned substituent group a or substituent group a'); and
other symbols are as defined for the above-mentioned [compound (IJ)],
or a pharmaceutically acceptable salt thereof.

### [Compound (IK)]

Compound (I), wherein
R¹ is a carboxy group, an optionally substituted C₁₋₆ alkoxy-carbonyl group, or an optionally substituted carbamoyl group;
L is a C₁₋₆ alkylene group optionally further substituted by 1 to 3 substituents selected from the aforementioned substituent group a or substituent group a';
A is a divalent nitrogen-containing non-aromatic heterocyclic group represented by the following formula: 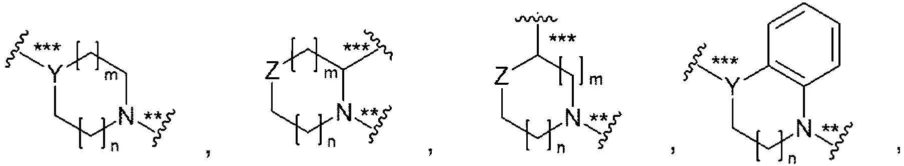 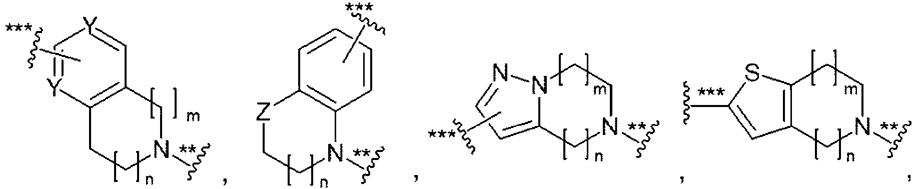 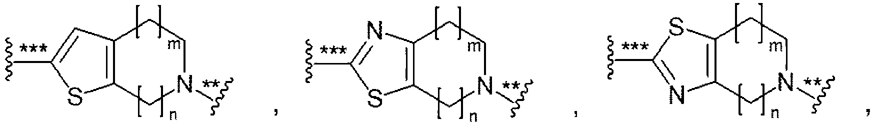 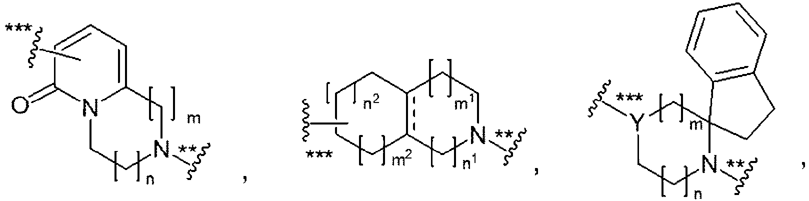 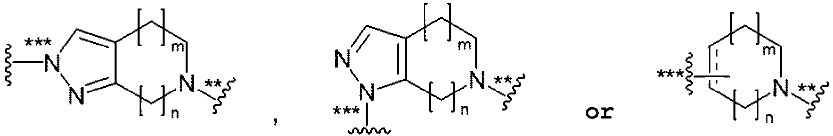
   wherein a wavy line is a bonding site of the group represented by the above-mentioned chemical formula,
   a bond represented by
      -̅ -̅ -̅ -̅ -̅ -̅
   is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 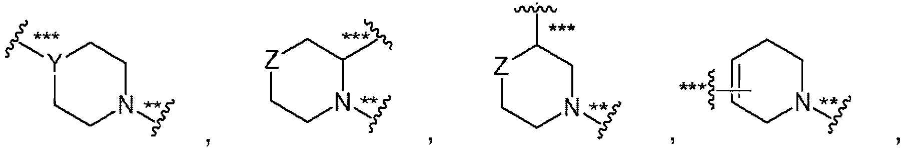 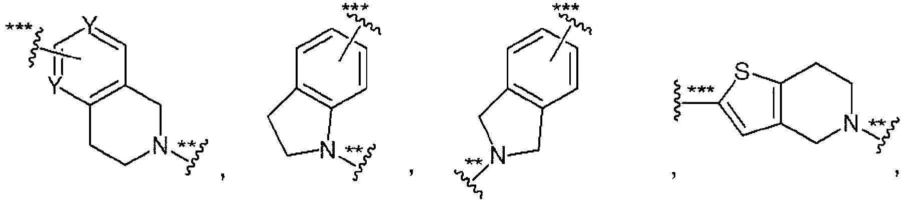 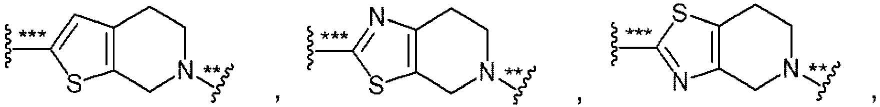 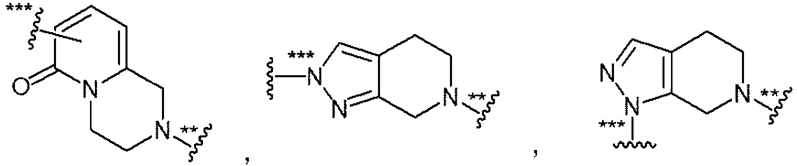 or 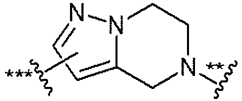
   wherein each symbol is as defined above, and optionally further substituted by 1 to 3 substituents selected from the aforementioned substituent group a or substituent group a');
      and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IK')]

Compound (I), wherein
A is a divalent nitrogen-containing non-aromatic heterocyclic group represented by the following formula: 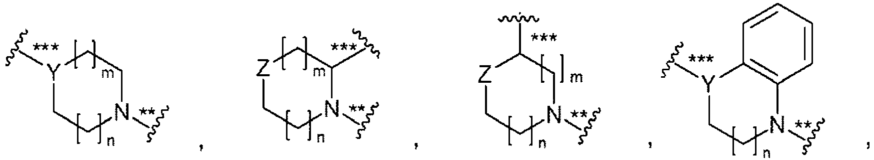 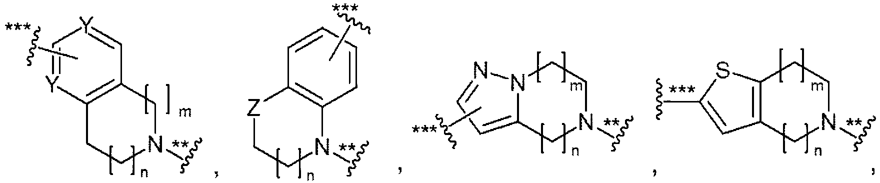 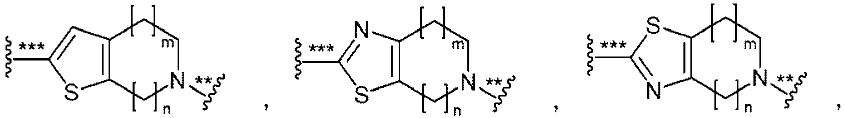 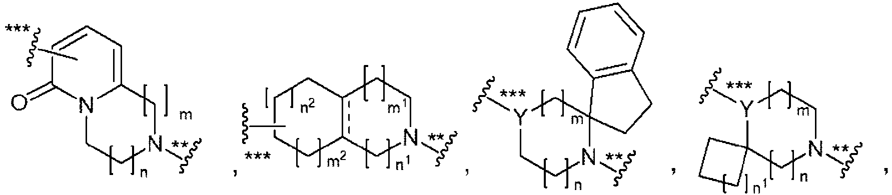 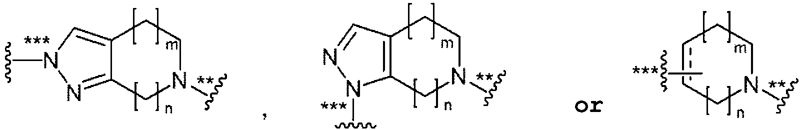
wherein a wavy line is a bonding site of the group represented by the above-mentioned chemical formula,
a bond represented by
   -̅ -̅ -̅ -̅ -̅ -̅
is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L, and optionally further substituted by 1 to 3 substituents selected from the aforementioned substituent group a or substituent group a' (e.g., a divalent nitrogen-containing non-aromatic heterocyclic group represented by the following formula: 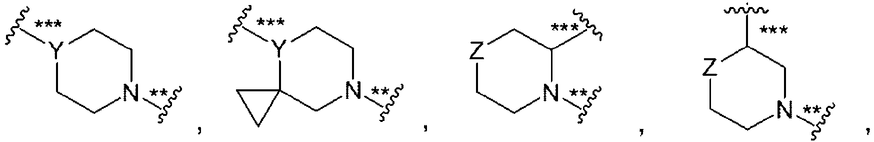 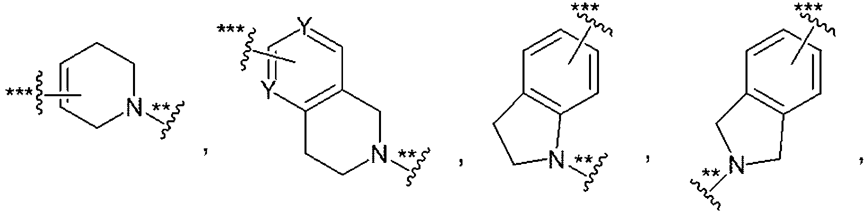 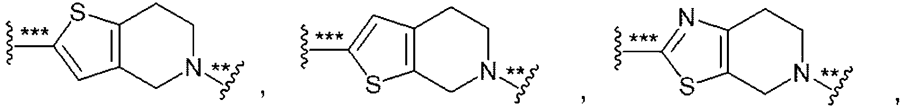 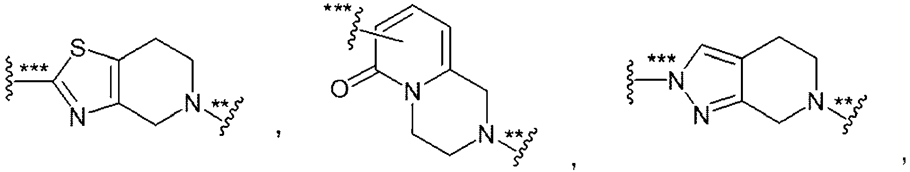 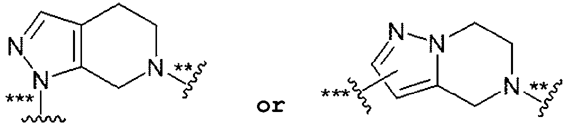
wherein each symbol is as defined above, and optionally further substituted by 1 to 3 substituents selected from the aforementioned substituent group a or substituent group a'); and
other symbols are as defined for the above-mentioned [compound (IK)],
or a pharmaceutically acceptable salt thereof.

### [Compound (IL)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, -C(=O)-, a C₆₋₁₀ arylene group (e.g., 1,2-phenylene, 1,3-phenylene, 1,4-phenylene), a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), C₂₋₆ alkynylene group, and 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₆₋₁₀ arylene group (e.g., 1,2-phenylene, 1,3-phenylene, 1,4-phenylene) optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a',
   a C₂₋₄ alkynylene group optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 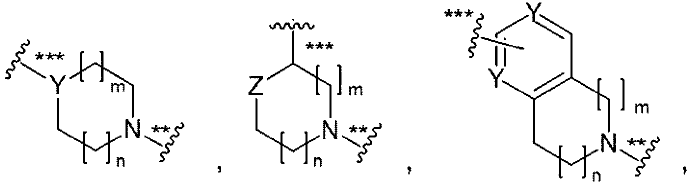 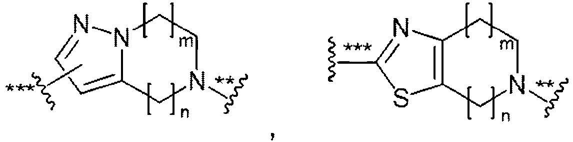 or 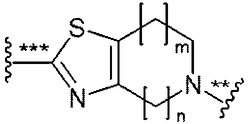 wherein each symbol is as defined above, and optionally further substituted by 1 to 3 substituents selected from the aforementioned substituent group a or substituent group a' (e.g., a divalent nitrogen-containing non-aromatic heterocyclic group represented by the following formula: 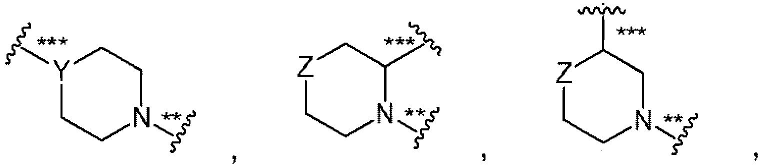 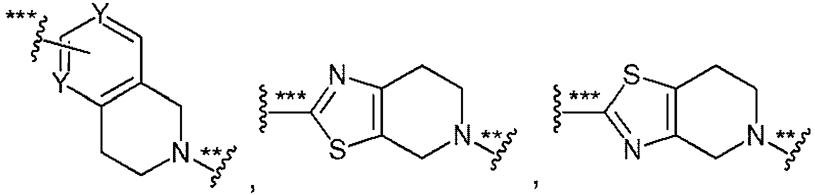 or 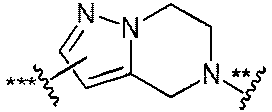 wherein each symbol is as defined above, and optionally further substituted by 1 to 3 substituents selected from the aforementioned substituent group a or substituent group a'); and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IM)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 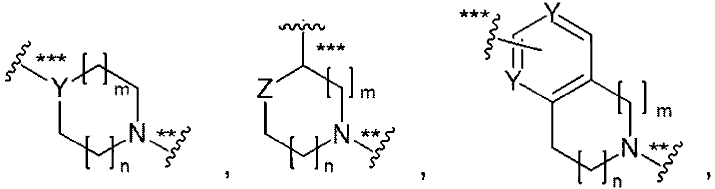 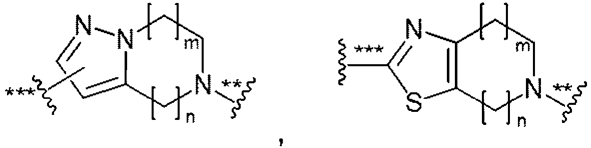 or 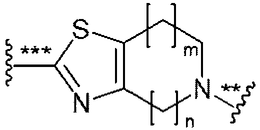 wherein each symbol is as defined above, and optionally further substituted by 1 to 3 substituents selected from the aforementioned substituent group a or substituent group a' (e.g., a divalent nitrogen-containing non-aromatic heterocyclic group represented by the following formula: 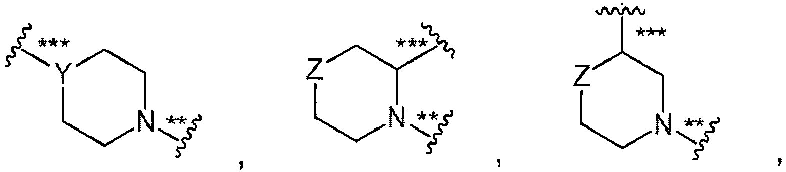 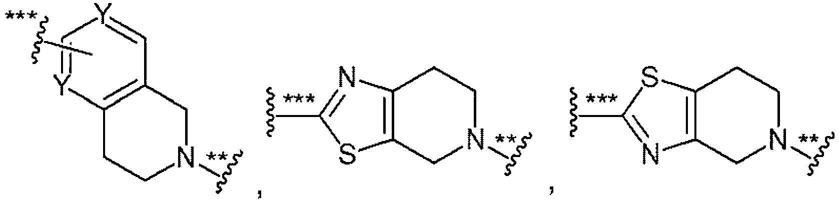 or 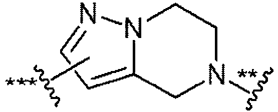 wherein each symbol is as defined above, and optionally further substituted by 1 to 3 substituents selected from the aforementioned substituent group a or substituent group a'); and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IN)]

Compound (I), wherein
R¹ is a carboxy group, an optionally substituted C₁₋₆ alkoxy-carbonyl group, or an optionally substituted carbamoyl group;
L is a C₁₋₆ alkylene group optionally further substituted by 1 to 3 substituents selected from the aforementioned substituent group a or substituent group a';
A is a divalent nitrogen-containing non-aromatic heterocyclic group represented by the following formula: 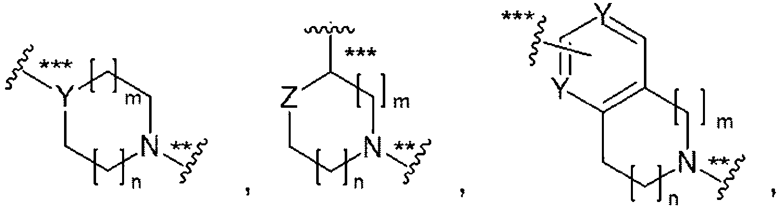 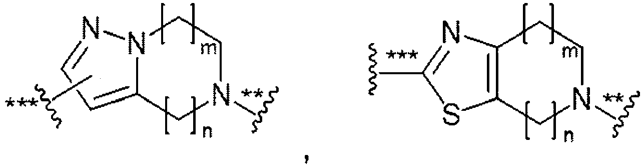 or 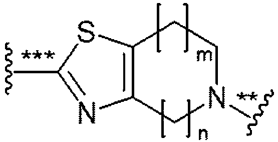 wherein each symbol is as defined above, and optionally further substituted by 1 to 3 substituents selected from the aforementioned substituent group a or substituent group a' (e.g., a divalent nitrogen-containing non-aromatic heterocyclic group represented by the following formula: 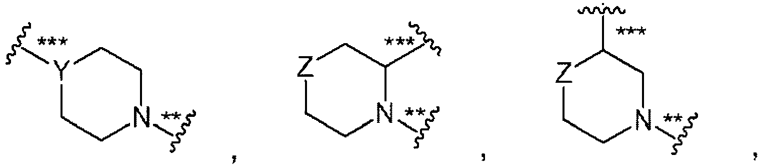 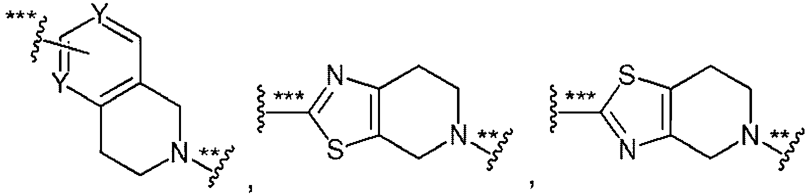 or 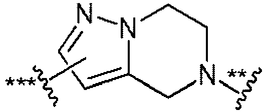 wherein each symbol is as defined above, and optionally further substituted by 1 to 3 substituents selected from the aforementioned substituent group a or substituent group a'); and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IP)]

Compound (I), wherein
R¹ is a carboxy group, an optionally substituted C₁₋₆ alkoxy-carbonyl group, or an optionally substituted carbamoyl group;
L is a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from the aforementioned substituent group a or substituent group a';
A is a divalent nitrogen-containing non-aromatic heterocyclic group represented by the following formula: 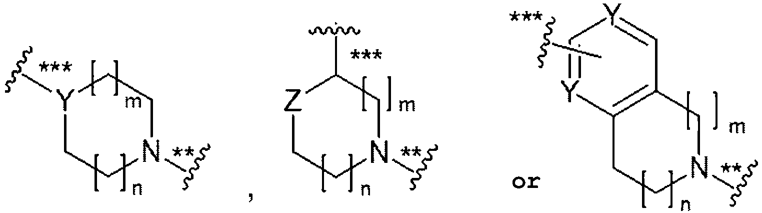 wherein each symbol is as defined above, and optionally further substituted by 1 to 3 substituents selected from the aforementioned substituent group a or substituent group a' (e.g., a divalent nitrogen-containing non-aromatic heterocyclic group represented by the following formula: 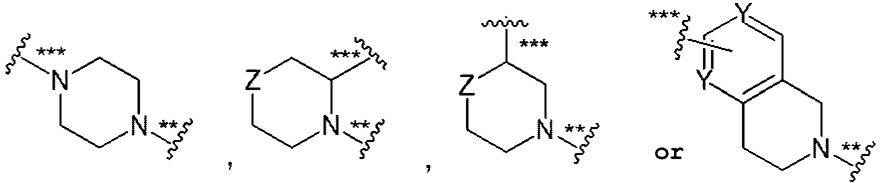 wherein each symbol is as defined above, and optionally further substituted by 1 to 3 substituents selected from the aforementioned substituent group a or substituent group a', provided that when Z is CR⁵R⁶, the optionally further substituted substituent is selected from the group consisting of, among the substituents in the aforementioned substituent group a or substituent group a', a C₆₋₁₄ hydrocarbocyclic group optionally substituted by 1 to 3 substituents selected from substituent group b or substituent group b'; a 5- to 14-membered aromatic heterocyclic group optionally substituted by 1 to 3 substituents selected from substituent group b or substituent group b'; and a 3- to 14-membered non-aromatic heterocyclic group optionally substituted by 1 to 3 substituents selected from substituent group b or substituent group b'); and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IQ)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 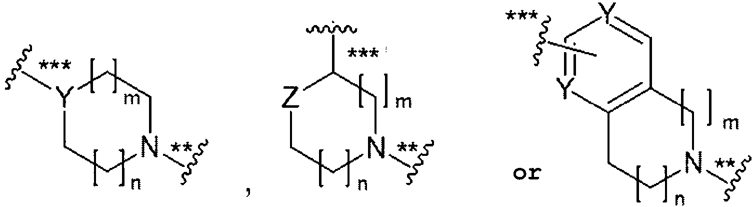 wherein each symbol is as defined above, and optionally further substituted by 1 to 3 substituents selected from the aforementioned substituent group a or substituent group a' (e.g., a divalent nitrogen-containing non-aromatic heterocyclic group represented by the following formula: 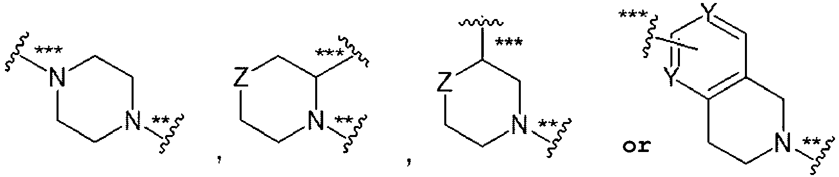 wherein each symbol is as defined above, and optionally further substituted by 1 to 3 substituents selected from the aforementioned substituent group a or substituent group a', provided that when Z is CR⁵R⁶, the optionally further substituted substituent is selected from the group consisting of, among the substituents in the aforementioned substituent group a or substituent group a', a C₆₋₁₄ hydrocarbocyclic group optionally substituted by 1 to 3 substituents selected from substituent group b or substituent group b'; a 5- to 14-membered aromatic heterocyclic group optionally substituted by 1 to 3 substituents selected from substituent group b or substituent group b'; and a 3- to 14-membered non-aromatic heterocyclic group optionally substituted by 1 to 3 substituents selected from substituent group b or substituent group b'); and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IR)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 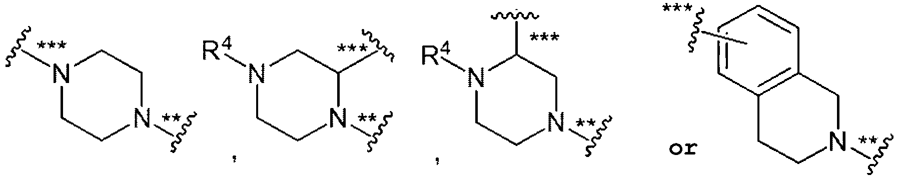 wherein each symbol is as defined above, and optionally further substituted by 1 to 3 substituents selected from the aforementioned substituent group a or substituent group a'; and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IS)]

Compound (I), wherein
R¹ is a carboxy group, an optionally substituted C₁₋₆ alkoxy-carbonyl group, or an optionally substituted carbamoyl group;
L is a C₁₋₆ alkylene group optionally further substituted by 1 to 3 substituents selected from the aforementioned substituent group a or substituent group a';
A is a divalent nitrogen-containing non-aromatic heterocyclic group represented by the following formula: 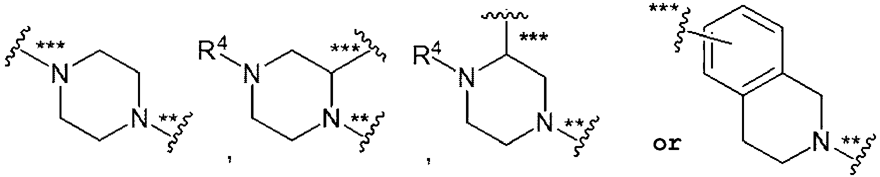 wherein each symbol is as defined above, and optionally further substituted by 1 to 3 substituents selected from the aforementioned substituent group a or substituent group a'; and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IT)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 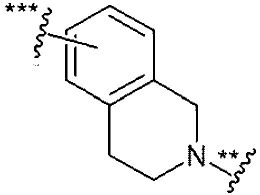 wherein each symbol is as defined above, and optionally further substituted by 1 to 3 substituents selected from the aforementioned substituent group a or substituent group a'; and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IU)]

Compound (I), wherein
R¹ is a carboxy group, an optionally substituted C₁₋₆ alkoxy-carbonyl group, or an optionally substituted carbamoyl group;
L is a C₁₋₆ alkylene group optionally further substituted by 1 to 3 substituents selected from the aforementioned substituent group a or substituent group a';
A is a divalent nitrogen-containing non-aromatic heterocyclic group represented by the following formula: 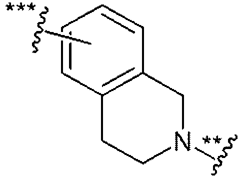 wherein each symbol is as defined above, and optionally further substituted by 1 to 3 substituents selected from the aforementioned substituent group a or substituent group a'; and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IV)]

Compound (I), wherein
R¹ is a carboxy group, an optionally substituted C₁₋₆ alkoxy-carbonyl group, or an optionally substituted carbamoyl group;
L is a C₁₋₆ alkylene group optionally having, in the inside or at the end, one or more divalent groups selected from the group consisting of -NR²- (wherein R² is a hydrogen atom or a C₁₋₆ alkyl group), -O-, -S-, a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl), and a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl), and optionally further substituted by 1 to 3 substituents selected from the aforementioned substituent group a or substituent group a',
   a C₃₋₁₀ cycloalkylene group (e.g., cyclobutane-1,3-diyl, cyclopropane-1,2-diyl, cyclopropane-1,1-diyl, cyclopentane-1,2-diyl, cyclohexane-1,2-diyl, bicyclo[2.1.1]hexane-1,2-diyl) optionally further substituted by 1 to 3 substituents selected from the aforementioned substituent group a or substituent group a', or
   a 3- to 8-membered divalent non-aromatic heterocyclic group (e.g., azetidine-1,3-diyl, pyrrolidine-1,3-diyl, pyrrolidine-1,2-diyl, piperidine-1,2-diyl, piperidine-1,4-diyl, azepane-1,2-diyl) optionally further substituted by 1 to 3 substituents selected from the aforementioned substituent group a or substituent group a';
A is a divalent nitrogen-containing non-aromatic heterocyclic group represented by the following formula: 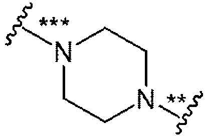 wherein each symbol is as defined above, and optionally further substituted by 1 to 3 substituents selected from the aforementioned substituent group a or substituent group a'; and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

### [Compound (IW)]

Compound (I), wherein
R¹ is a carboxy group, an optionally substituted C₁₋₆ alkoxy-carbonyl group, or an optionally substituted carbamoyl group;
L is a C₁₋₆ alkylene group optionally further substituted by 1 to 3 substituents selected from the aforementioned substituent group a or substituent group a';
A is a divalent nitrogen-containing non-aromatic heterocyclic group represented by the following formula: 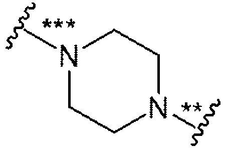 wherein each symbol is as defined above, and optionally further substituted by 1 to 3 substituents selected from the aforementioned substituent group a or substituent group a'; and
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group,
or a pharmaceutically acceptable salt thereof.

Specific preferred examples of compound (I) are the compounds of the below-mentioned Examples 1 to 403 (compound (I-1) to compound (I-403)), or pharmaceutically acceptable salts thereof.

Among them, a compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
3-[2-[2-[[(E)-3-[4-(trifluoromethyl)phenyl]prop-2-enoyl]amino]acetyl]-3,4-dihydro-1H-isoquinolin-6-yl]propanoic acid;
N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
1-[7-[2-[[(E)-3-[4-(trifluoromethyl)phenyl]prop-2-enoyl]amino]acetyl]-6,8-dihydro-5H-2,7-naphthyridin-3-yl]piperidine-4-carboxylic acid;
3-[3-bromo-5-[2-[[(E)-3-[4-(trifluoromethyl)phenyl]prop-2-enoyl]amino]acetyl]-6,7-dihydro-4H-pyrazolo[1,5-a]pyrazin-2-yl]propanoic acid;
2-[[2-[2-[[(E)-3-[4-(trifluoromethyl)phenyl]prop-2-enoyl]amino]acetyl]-3,4-dihydro-1H-isoquinolin-6-yl]oxy]acetic acid;
2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
(1R*,2R*)-2-[2-[2-[[(E)-3-[4-(trifluoromethyl)phenyl]prop-2-enoyl]amino]acetyl]-3,4-dihydro-1H-isoquinolin-6-yl]cyclopropane-1-carboxylic acid and both enantiomers thereof;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[7-[2-[[(E)-3-[4-(trifluoromethyl)phenyl]prop-2-enoyl]amino]acetyl]-6,8-dihydro-5H-2,7-naphthyridin-3-yl]acetic acid;
3-[3-bromo-5-[2-[[(E)-3-[4-(pentafluoro-λ6-sulfanyl)phenyl]prop-2-enoyl]amino]acetyl]-6,7-dihydro-4H-pyrazolo[1,5-a]pyrazin-2-yl]propanoic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[[2-[2-[[(E)-3-[4-(trifluoromethyl)phenyl]prop-2-enoyl]amino]acetyl]-3,4-dihydro-1H-isoquinolin-6-yl]amino]acetic acid;
2-[2-[2-[[(E)-3-(4-iodophenyl)prop-2-enoyl]amino]acetyl]-3,4-dihydro-1H-isoquinolin-6-yl]acetic acid;
2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-cyano-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-(2-fluoro-4-iodophenyl)prop-2-enoyl]amino]acetyl]-3,4-dihydro-1H-isoquinolin-6-yl]acetic acid;
2-[8-fluoro-2-[2-[[(E)-3-(4-iodophenyl)prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[[5-[2-[[(E)-3-[4-(trifluoromethyl)phenyl]prop-2-enoyl]amino]acetyl]-6,7-dihydro-4H-[1,3]thiazolo[5,4-c]pyridin-2-yl]sulfanyl]acetic acid;
2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-cyano-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
(E)-N-[2-[6-(2-amino-2-oxoethyl)-3,4-dihydro-1H-isoquinolin-2-yl]-2-oxoethyl]-3-[4-(trifluoromethyl)phenyl]prop-2-enamide;
2-[5-[2-[[(E)-3-[4-(trifluoromethyl)phenyl]prop-2-enoyl]amino]acetyl]-6,7-dihydro-4H-[1,3]thiazolo[4,5-c]pyridin-2-yl]acetic acid;
(E)-N-[2-[6-[2-(methylamino)-2-oxoethyl]-3,4-dihydro-1H-isoquinolin-2-yl]-2-oxoethyl]-3-[4-(trifluoromethyl)phenyl]prop-2-enamide;
4-[4-[2-[[(E)-3-[2-fluoro-4-(trifluoromethyl)phenyl]prop-2-enoyl]amino]acetyl]-3-phenylpiperazin-1-yl]butanoic acid and both enantiomers thereof;
2-[[1-[2-[[(E)-3-[4-(trifluoromethyl)phenyl]prop-2-enoyl]amino]acetyl]-2,3-dihydroindol-4-yl]oxy]acetic acid;
1-[1-[2-[[(E)-3-[4-(trifluoromethyl)phenyl]prop-2-enoyl]amino]acetyl]piperidin-3-yl]bicyclo[2.1.1]hexane-5-carboxylic acid;
2-[[8-fluoro-2-[2-[[(E)-3-[4-(trifluoromethyl)phenyl]prop-2-enoyl]amino]acetyl]-3,4-dihydro-1H-isoquinolin-6-yl]oxy]-2-methylpropanoic acid;
3-[[8-fluoro-2-[2-[[(E)-3-[4-(trifluoromethyl)phenyl]prop-2-enoyl]amino]acetyl]-3,4-dihydro-1H-isoquinolin-6-yl]oxy]-2,2-dimethylpropanoic acid;
4-[4-[2-[[(E)-3-[2-fluoro-4-(trifluoromethyl)phenyl]prop-2-enoyl]amino]acetyl]-2-methylpiperazin-1-yl]butanoic acid;
4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
3-[[3-(4-chlorophenyl)-4-[2-[[(E)-3-[2-fluoro-4-(trifluoromethyl)phenyl]prop-2-enoyl]amino]acetyl]piperazin-1-yl]methyl]cyclobutane-1-carboxylic acid and both enantiomers thereof;
4-[3-(4-chlorophenyl)-4-[2-[[(E)-3-[2-fluoro-4-(trifluoromethyl)phenyl]prop-2-enoyl]amino]acetyl]piperazin-1-yl]-2,2-dimethylbutanoic acid;
4-[3-(3-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid and both enantiomers thereof;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[3-(4-chloro-3-fluorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
4-[(2R,5S)-5-(4-chlorophenyl)-4-[2-[[(E)-3-[4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid,
or a pharmaceutically acceptable salt thereof is preferred, a compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
3-[2-[2-[[(E)-3-[4-(trifluoromethyl)phenyl]prop-2-enoyl]amino]acetyl]-3,4-dihydro-1H-isoquinolin-6-yl]propanoic acid;
N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
(1R*,2R*)-2-[2-[2-[[(E)-3-[4-(trifluoromethyl)phenyl]prop-2-enoyl]amino]acetyl]-3,4-dihydro-1H-isoquinolin-6-yl]cyclopropane-1-carboxylic acid and both enantiomers thereof;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[2-[2-[[(E)-3-(4-iodophenyl)prop-2-enoyl]amino]acetyl]-3,4-dihydro-1H-isoquinolin-6-yl]acetic acid;
2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-cyano-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-(2-fluoro-4-iodophenyl)prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[5-[2-[[(E)-3-[4-(trifluoromethyl)phenyl]prop-2-enoyl]amino]acetyl]-6,7-dihydro-4H-[1,3]thiazolo[4,5-c]pyridin-2-yl]acetic acid;
4-[(3S)-4-[2-[[(E)-3-[2-fluoro-4-(trifluoromethyl)phenyl]prop-2-enoyl]amino]acetyl]-3-phenylpiperazin-1-yl]butanoic acid;
1-[1-[2-[[(E)-3-[4-(trifluoromethyl)phenyl]prop-2-enoyl]amino]acetyl]piperidin-3-yl]bicyclo[2.1.1]hexane-5-carboxylic acid;
4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
3-[[3-(4-chlorophenyl)-4-[2-[[rac-(E)-3-[2-fluoro-4-(trifluoromethyl)phenyl]prop-2-enoyl]amino]acetyl]piperazin-1-yl]methyl]cyclobutane-1-carboxylic acid and both enantiomers thereof;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid and both enantiomers thereof;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[3-(4-chloro-3-fluorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
4-[(2R,5S)-5-(4-chlorophenyl)-4-[2-[[(E)-3-[4-(trifluoromethyl)phenyl]prop-2-enoyl]amino]acetyl]-2-methylpiperazine-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid,
or a pharmaceutically acceptable salt thereof is more preferred, a compound selected from the group consisting of 2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[2-[2-[[(E)-3-(4-iodophenyl)prop-2-enoyl]amino]acetyl]-3,4-dihydro-1H-isoquinolin-6-yl]acetic acid;
2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
4-[(3S)-4-[2-[[(E)-3-[2-fluoro-4-(trifluoromethyl)phenyl]prop-2-enoyl]amino]acetyl]-3-phenylpiperazin-1-yl]butanoic acid;
1-[1-[2-[[(E)-3-[4-(trifluoromethyl)phenyl]prop-2-enoyl]amino]acetyl]piperidin-3-yl]bicyclo[2.1.1]hexane-5-carboxylic acid;
4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
4-[(2R,5S)-5-(4-chlorophenyl)-4-[2-[[(E)-3-[4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid,
or a pharmaceutically acceptable salt thereof is further preferred, and
a compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
3-[2-[2-[[(E)-3-[4-(trifluoromethyl)phenyl]prop-2-enoyl]amino]acetyl]-3,4-dihydro-1H-isoquinolin-6-yl]propanoic acid;
N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
4-[(3R)-3-(4-chlorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid,
or a pharmaceutically acceptable salt thereof is particularly preferred.

When compound (I) of the present invention has asymmetric carbon atom(s) in the molecule, it can exist as multiple stereoisomers (i.e., diastereomeric isomers, optical isomers) based on said asymmetric carbon atom(s), and the present invention includes both any one of these stereoisomers and mixtures containing those multiple stereoisomers in any ratio. Isomers due to conformation or tautomerism may also be formed, and such isomers or mixtures thereof are also included in compound (I) of the present invention.

Compound (I) of the present invention may be labeled or substituted with isotopes (e.g., ²H, ³H, ¹³C, ¹⁴C, ¹⁵N, ¹⁸F, ³²P, ³⁵S, ¹²³I, ¹²⁵I, ¹³¹I, etc.). The isotope-labeled or isotopically-substituted compounds can be used as tracers (PET tracers) for, for example, Single Photon Emission Computed Tomography (SPECT) and positron emission tomography (PET) and are useful in the field of medical diagnosis and the like.

The compound (I) of the present invention or a pharmaceutically acceptable salt thereof may be crystal, and may have a single crystal form or a mixture of several crystal forms.

The compound (I) of the present invention or a pharmaceutically acceptable salt thereof may also include intramolecular salts or adducts thereof and their solvates. Their solvates are those in which a solvent molecule is coordinated to compound (I) or a salt thereof, and hydrates are also included. For example, hydrates, ethanol solvates or dimethyl sulfoxide solvates of compound (I) or a salt thereof are included.

The compound (I) of the present invention may be a prodrug.

Prodrugs of compound (I) of the present invention are compounds that are converted to compound (I) in vivo by reactions with enzymes or stomach acid. As a prodrug of compound (I), monoesters or diesters of the phosphate group can be considered, preferably whose ester functional groups have structures that are easily hydrolyzed or metabolized after administration to a patient.
As a prodrug of compound (I) may be a monoester or diester of a phosphate group, the ester functional group of which preferably has a structure that is easily hydrolyzed or metabolized after administration to the patient. Examples of ester functional groups of such prodrugs include C₁₋₆ alkyl esters optionally substituted with acyloxy groups, phenyl esters, benzyl esters, and the like (see Bioorganic Chemistry, 1984; 12: p. 118-129). In addition, as prodrugs other than the above monoesters or diesters of phosphate groups, see, for example, compounds having groups derived from phosphate groups, etc. as described in Current opinion in investigational drugs, 2006; 7: p. 109-117, J. Med. Chem. 1994; 37: p. 1857-1864, and J. Med. Chem. 2000; 43: p.4570-4574.

As another form of a prodrug of compound (I), for example, when compound (I) has an amino group, a compound in which the amino group is acylated, alkylated, or phosphorylated (e.g., the amino group of compound (I) is eicosanoylated, alanylated, pentylaminocarbonylated, (5-methyl-2-oxo-1,3-dioxolen-4-yl)methoxycarbonylated, tetrahydrofurylated, pyrrolidylmethylated, pivaloyloxymethylated, acetoxymethylated, tert-butylated, etc.); when compound (I) has a hydroxy group, a compound in which the hydroxy group is acylated, alkylated, phosphorylated, borylated (e.g. the hydroxy group of compound (I) is acetylated, palmitoylated, propanoylated, pivaloylated, succinylated, fumarylated, alanylated, dimethylaminomethylcarbonylated, etc.); when compound (I) has a carboxy group, a compound in which the carboxy group is esterified or amidated (e.g., the carboxy group of compound (I) is ethyl esterified, phenyl esterified, carboxymethyl esterified, dimethylaminomethyl esterified, pivaloyloxymethyl esterified, 1-{(ethoxycarbonyl)oxy}ethyl esterified, phthalidyl esterified, (5-methyl-2-oxo-1,3-dioxolen-4-yl)methyl esterified, 1-{[(cyclohexyloxy)carbonyl]oxy}ethyl esterified, methylamidated, etc.). These compounds can be produced by methods known per se. Furthermore, prodrugs of compound (I) may be hydrated or unhydrated. Prodrugs of compound (I) may also be those that transform into compound (I) under physiological conditions, as described in "Molecular Design", pp. 163-198 in "Development of Pharmaceuticals", Vol. 7, published by Hirokawa Shoten 1990.

(Production methods of the compound (I) of the present invention)
present invention or a pharmaceutically acceptable salt thereof are explained below.

As an example of the production method of compound (I), representative production methods (A) to (F) are described below. However, the production method is not limited thereto. In addition, the following production methods and steps may be combined with each other.

Compound (I) (e.g., compound (I-1) to compound (I-403) described in the below-mentioned Examples) can be produced by the following production methods (A) to (F), the below-mentioned Reference Examples, Examples, or a method analogous thereto, and the like.

Each starting material compound may form a salt if the reaction is not inhibited, and such salts are the same as those of compound (I). If a specific manufacturing method is not described, the starting material compounds can be easily obtained and used commercially, or can be manufactured according to methods known per se or methods analogous thereto. The intermediates produced in the following manufacturing process may be isolated and purified by column chromatography, recrystallization, distillation, or the like, or may be used in the following process without isolation.

The contents of all patent documents, non-patent documents, or reference documents that are expressly cited herein may all be incorporated herein by reference.

### [Production method (A)]

In this production method, compound (1-1) and compound (2-1) are condensed to give compound (3-1), and the protecting group (P¹) is removed to give compound (Ia).

Compound (1-1) synthesized by a method known per se (e.g., US Patent No. 10,836,736, etc.) can be preferably used.

### Production method (A)

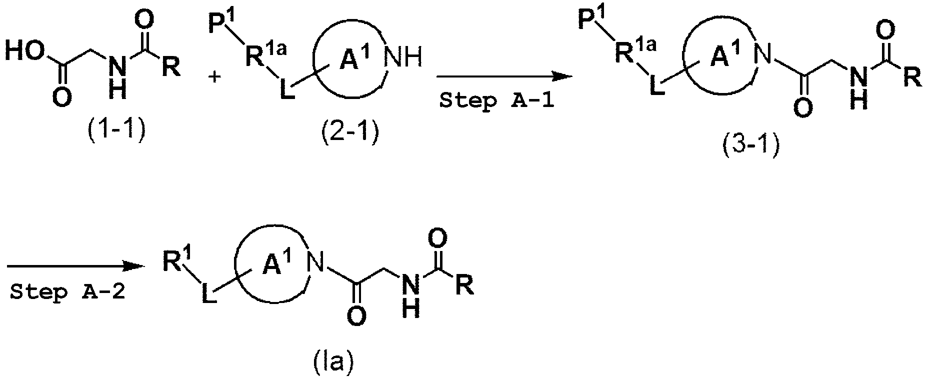

wherein R^{1a}-P¹ is protected R¹, and other symbols are as defined above.

### (Step A-1)

In this step, compound (1-1) and compound (2-1) are condensed in the presence of a condensing agent to produce compound (3-1).

The amount of compound (2-1) to be used is 0.8 mol to 5 mol, preferably 0.8 mol to 3 mol, per 1 mol of compound (1-1).

The condensing agent includes o-(7-azabenzotriazol-1-yl)-N,N,N',N'-tetramethyluronium hexafluorophosphate (HATU), 1-ethyl-3-(3'-dimethylaminopropyl)carbodiimide hydrochloride (EDC-HCl) (WSC hydrochloride), dicyclohexylcarbodiimide (DCC), diisopropylcarbodiimide (DIC), benzotriazol-1-yl-oxy-tris-pyrrolidino-phosphonium hexafluorophosphate (PyBop), O-(benzotriazol-1-yl)-N,N,N',N'-tetramethyluronium tetrafluoroborate (TBTU), 1-[bis(dimethylamino)methylene]-5-chloro-1H-benzotriazolium 3-oxide hexafluorophosphate (HCTU), O-benzotriazole-N,N,N',N'-tetramethyluronium hexafluoroborate (HBTU), propylphosphonic acid anhydride (T3P) solution, and the like, preferably HATU or WSC hydrochloride.

The amount of the condensing agent to be used is generally 1 mol to 10 mol, preferably 1 mol to 5 mol, per 1 mol of compound (1-1).

The reaction may be performed in the copresence of 1-hydroxybenzotriazole (HOBt), ethyl 1-hydroxy-1H-1,2,3-triazole-5-carboxylate (HOCt), 1-hydroxy-7-azabenzotriazole (HOAt), or the like as an additive.

The amount of the additive to be used is generally 0 to 1.5 mol per 1 mol of compound (1-1).

Examples of the base include organic bases such as triethylamine, pyridine, N,N-diisopropylethylamine, and the like. Among these, triethylamine or N,N-diisopropylethylamine is preferred.

The amount of the base to be used is generally 1 mol to 5 mol, preferably 1.5 mol, per 1 mol of compound (1-1).

This reaction can be performed in a solvent that does not affect the reaction. Examples of the reaction solvent include, but are not particularly limited to, aromatic hydrocarbons such as toluene, xylene, and the like; amide solvents such as N,N-dimethylformamide, N,N-dimethylacetamide, and the like; ethers such as diethyl ether, tetrahydrofuran, dioxane, and the like; halogenated hydrocarbons such as chloroform, dichloromethane, and the like; nitriles such as acetonitrile and the like, or a mixture thereof can be mentioned.

The reaction temperature is generally -78°C to room temperature, preferably 0°C to room temperature, and the reaction time is generally 1 hr to 48 hr.

### (Step A-2)

In this step, the amino-protecting group (P¹) is eliminated to obtain compound (Ia).

For the deprotection step of step A-2, the reaction conditions can be selected according to the kind of protecting group (P1). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protective Groups in Organic Synthesis, 3rd Ed. 2004, "Protecting Groups 3rd Ed." by P. J. Kocienski, etc., or as described in the examples below.

### [Production method (B)]

In this production method, compound (1-1) and compound (4-1) are condensed to give compound (5-1), the compound is converted to compound (7-1) by a coupling reaction with compound (6-1), and the protecting group (P²) is removed to give compound (Ib).

Compound (1-1) synthesized by a method known per se (e.g., US Patent No. 10,836,736, etc.) can be preferably used.

### Production method (B)

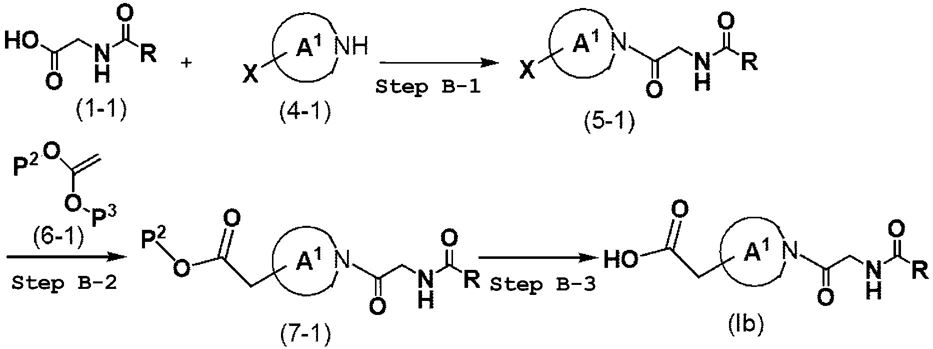

wherein X is a leaving group, P² and P³ are each independently protecting groups, and other symbols are as defined above.

As the protecting group P³ in compound (6-1), a trisubstituted silyl group such as a trimethylsilyl group, a triethylsilyl group, a tert-butyldimethylsilyl group, a tert-butyldiphenylsilyl group, a triisopropylsilyl group or the like is used. The protecting group P³ is removed by a reaction process in step B-2.

### (Step B-1)

In this step, compound (1-1) and compound (4-1) are condensed in the presence of a condensing agent to produce compound (5-1).

The amount of compound (4-1) to be used is 0.8 mol to 5 mol, preferably 0.8 mol to 3 mol, per 1 mol of compound (1-1).

In step B-1, the reaction can be performed under the same conditions as in the aforementioned step A-1 except that compound (4-1) is used instead of compound (2-1).

### (Step B-2)

This step can be performed by a cross coupling reaction (Heck reaction) of compound (5-1) and compound (6-1) in a solvent that does not affect the reaction, in the presence of a metal catalyst (e.g., palladium catalyst) and a base as necessary.

The amount of compound (6-1) to be used is 0.8 mol to 5 mol, preferably 0.8 mol to 3 mol, per 1 mol of compound (5-1).

Metal catalysts include palladium catalysts such as palladium(II) acetate, palladium(II) chloride, dichlorobis(tricyclohexylphosphine)palladium(II), tris(dibenzylideneacetone)dipalladium(O), bis(dibenzylideneacetone)palladium(0) (Pd₂(dba)₃), tetrakis(triphenylphosphine)palladium(O), bis(tri-tert-butylphosphine)palladium(0), palladium(II) chloride and diphenylphosphinoferrocene (PdCl₂(dppf)), PdCl₂(dppf) dichloromethane complex, palladium carbon(0), bis(triphenylphosphine)palladium dichloride, dichloro bis(trio-tolylphosphine)palladium(II), bis(tris(2-tolyl)phosphine)palladium(0), and the like; copper catalysts such as copper(I) iodide, copper(I) bromide, copper (I) chloride, copper(II) chloride, and the like; iron catalysts such as iron(III) acetylacetonate, and the like; nickel catalysts such as bis(1,5-cyclooctadiene)nickel(0), bis(acetylacetonate)nickel(II), and the like; ruthenium catalysts such as dichloro(p-cymene)ruthenium(II) dimer, ruthenium alumina, and the like. A palladium catalyst is preferred, and among which dichlorobis(tricyclohexylphosphine)palladium(II), dichlorobis(tri-o-tolylphosphine)palladium(II) or bis(tri-tert-butylphosphine)palladium(0) is more preferred.

Where necessary, phosphine ligands such as 1,1'-bis(diphenylphosphino)ferrocene (dppf), 2-dicyclohexylphosphino-2',6'-dimethoxybiphenyl (SPhos), 2-dicyclohexylphosphino-2',4',6'-triisopropylbiphenyl (XPhos), 2-dicyclohexylphosphino-2',6'-diisopropoxybiphenyl (RuPhos), 3,6-dimethoxy-2-dicyclohexylphosphino-2',4',6'-triisopropylbiphenyl (BrettPhos), tri-t-butylphosphine, tricyclohexylphosphine, [4-(N,N-dimethylamino)phenyl]di-tert-butylphosphine (AmPhos), (S)-1-[(RP)-2-(dicyclohexylphosphino)ferrocenyl]ethyl di-tert-butylphosphine (JosiPhos), and the like; phenanthrolin and the like may also be added.

The amount of the metal catalyst to be used is generally 0.01 mol to 1 mol, preferably, 0.05 mol to 0.3 mol, per 1 mol of compound (5-1).

The amount of the ligand to be used is generally 0.05 mol to 1 mol, preferably 0.1 mol to 0.4 mol, per 1 mol of compound (5-1).

The reaction may be performed in the presence of zinc fluoride (ZnF₂), copper fluoride (CuF₂), lithium fluoride (LiF), or the like as additive.

The amount of additive to be used is generally 0 to 1.5 mol per 1 mol of compound (5-1).

Examples of the base include alkali metal amides such as lithium diisopropylamide, sodium amide, lithium bis trimethylsilylamide, and the like; alkali metal carbonate salts such as sodium carbonate, potassium carbonate, sodium hydrogen carbonate, potassium hydrogen carbonate, and the like; alkali metal phosphate salts such as sodium phosphate, potassium phosphate, and the like; and amines such as triethylamine, N,N-diisopropylethylamine, pyridine, N-methylmorpholine, and the like.

The amount of the base to be used is generally 1 mol to 5 mol, preferably 1 mol to 2.5 mol, per 1 mol of compound (5-1).

The reaction solvent is not particularly limited and includes, for example, amides such as N,N-dimethylformamide, N-methylpyrrolidone, and the like; ethers such as tetrahydrofuran, 1,4-dioxane, and the like; halogenated hydrocarbons such as chloroform, dichloromethane, and the like; aromatic hydrocarbons such as toluene, xylene, and the like; nitriles such as acetonitrile and the like; water; a mixture of these, and the like.

The reaction temperature is generally -78°C to 200°C, preferably -78°C to 120°C.

The reaction time is generally 0.5 hr to 12 hr.

### (Step B-3)

In this step, compound (Ib) is obtained by removing the protecting group (P²).

For the deprotection process in step B-3, the reaction conditions can be selected according to the kind of protecting group (P2). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### [Production method (C)]

In this production method, compound (1-1) and compound (8-1) are condensed to give compound (9-1), the protecting group (P⁴) is removed to give compound (10-1), compound (10-1) is converted to compound (12-1) by reacting with compound (11-1), and protecting group (P⁵) is removed to obtain compound (Ic).

### Production method (C)

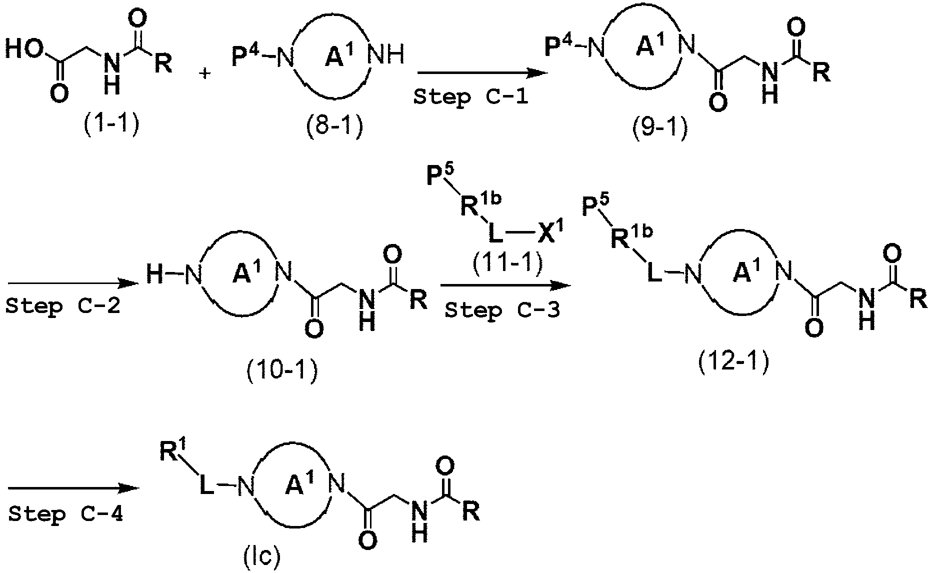

wherein R^{1b}-P⁵ is a protected R¹, X¹ is a leaving group, P⁴ and P⁵ are each independently protecting groups, and other symbols are as defined above.

### (Step C-1)

In this step, compound (1-1) and compound (8-1) are condensed in the presence of a condensing agent to produce compound (9-1).

The amount of compound (8-1) to be used is 0.8 mol to 5 mol, preferably 0.8 mol to 3 mol, per 1 mol of compound (1-1).

In step C-1, the reaction can be performed under conditions similar to those in the aforementioned step A-1 except that compound (8-1) is used instead of compound (2-1).

### (Step C-2)

In this step, compound (10-1) is obtained by removing the amino-protecting group (P⁴).

For the deprotection process in step C-2, the reaction conditions can be selected according to the kind of protecting group (P⁴). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### (Step C-3)

In this step, compound (10-1) and compound (11-1) are reacted in the presence of a base to produce compound (12-1).

The amount of compound (11-1) to be used is 1 mol to 5 mol, preferably 1 mol to 3 mol, per 1 mol of compound (10-1).

As the base, organic bases such as triethylamine, pyridine, N,N-diisopropylethylamine and the like; and inorganic bases such as sodium carbonate, potassium carbonate, cesium carbonate and the like can be mentioned. Among these, triethylamine or N,N-diisopropylethylamine is preferred.

The amount of the base to be used is generally 1 mol to 5 mol, preferably 1.5 mol, per 1 mol of compound (10-1).

The reaction may be performed in the presence of tetrabutylammonium iodide (TBAI), sodium iodide, silver trifluoromethanesulfonate, or the like as an additive.

The amount of the additive to be used is generally 0 to 1.5 mol per 1 mol of compound (10-1).

This reaction can be performed in a solvent that does not affect the reaction. The reaction solvent is not particularly limited and, for example, aromatic hydrocarbons such as toluene, xylene and the like; amide solvents such as N,N-dimethylformamide, N,N-dimethylacetamide and the like; ethers such as diethyl ether, tetrahydrofuran, dioxane and the like; halogenated hydrocarbons such as chloroform, dichloromethane and the like; nitriles such as acetonitrile and the like, and a mixture thereof can be mentioned.

The reaction temperature is generally 0°C to 100°C, preferably 0°C to 50°C, and the reaction time is generally 1 hr to 48 hr.

When the resultant product obtained by this reaction contains a halogen, the halo group can be converted to an alkyl group or a cycloalkyl group by the subsequent coupling reaction.

### (Step C-4)

In this step, compound (Ic) is obtained by removing the amino-protecting group (P⁵).

For the deprotection process in step C-4, the reaction conditions can be selected according to the kind of protecting group (P⁵). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

When the resultant product obtained by this reaction contains a halogen, the halo group can be converted to an alkyl group or a cycloalkyl group by the subsequent coupling reaction.

### [Production method (D)]

In this production method, compound (14-1) is obtained by a reductive alkylation reaction of compound (10-1) and compound (13-1), and protecting group (P⁶) is removed to obtain compound (Id).

### Production method (D)

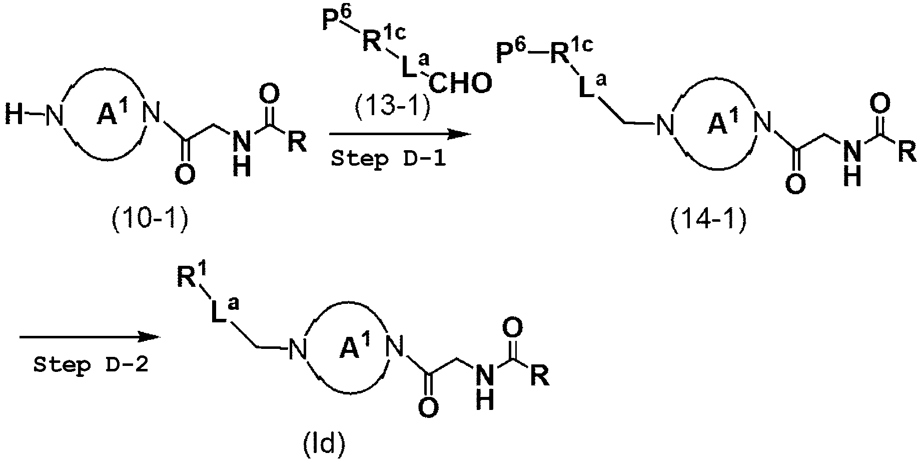

wherein R^{1c}-P⁶ is a protected R¹, P⁶ is a protecting group, L^{a} is a divalent organic group, and other symbols are as defined above.

### (Step D-1)

In this step, compound (10-1) is reacted with a reducing agent and compound (13-1) (reductive alkylation reaction) in a solvent that does not affect the reaction to obtain compound (14-1).

The amount of compound (13-1) to be used is 1 mol to 3 mol, preferably 1 mol to 2 mol, per 1 mol of compound (10-1).

The reducing agent is not particularly limited and, for example, sodium triacetoxyborohydride, sodium borohydride and the like can be mentioned.

The amount of reducing agent to be used is 1 mol to 10 mol, preferably 1 mol to 3 mol, per 1 mol of compound (10-1).

As the additive, the reaction may be performed in the presence of, for example, organic acids such as acetic acid and the like; Lewis acids such as titanium(IV) tetrachloride, titanium tetraisopropoxide and the like, and the like.

The amount of additive to be used is 1 mol to 10 mol, preferably 1 mol to 3 mol, per 1 mol of compound (10-1).

The reaction solvent is not particularly limited and, for example, aromatic hydrocarbons such as benzene, toluene, xylene and the like; alcohols such as methanol, ethanol and the like; ethers such as tetrahydrofuran and the like; halogenated hydrocarbons such as chloroform, dichloromethane and the like, and a mixture thereof can be mentioned.

The reaction temperature is generally -10°C to 100°C, preferably 10°C to 50°C, and the reaction time is generally 1 hr to 48 hr.

### (Step D-2)

In this step, compound (Id) is obtained by removing the amino-protecting group (P⁶).

For the deprotection process in step D-2, the reaction conditions can be selected according to the kind of protecting group (P⁶). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### [Production method (E)]

In this production method, compound (8-1) and compound (11-1) are reacted to give compound (15-1), the protecting group (P⁴) is removed to give compound (16-1), compound (16-1) is converted to compound (12-1) by condensing with compound (1-1), and protecting group (P⁵) is removed to obtain compound (Ic).

Compound (1-1) synthesized by a method known per se (e.g., US Patent No. 10,836,736, etc.) can be preferably used.

### Production method (E)

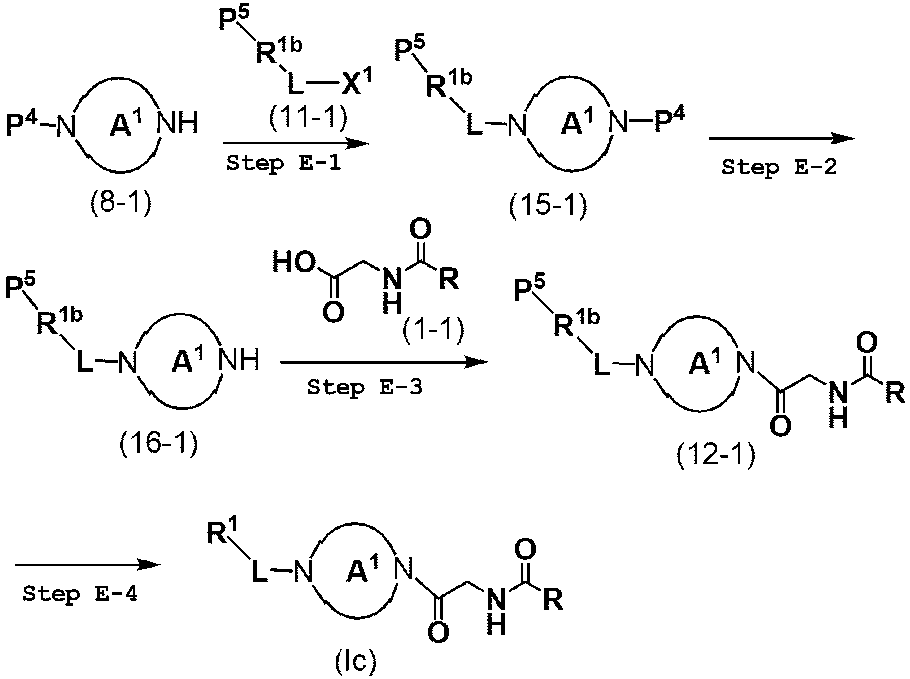

(wherein each symbol is as defined above.)

### (Step E-1)

In this step, compound (8-1) and compound (11-1) are reacted in the presence of a base to produce compound (15-1).

The amount of compound (11-1) to be used is 1 mol to 5 mol, preferably 1 mol to 3 mol, per 1 mol of compound (8-1).

In step E-1, the reaction can be performed under conditions similar to those in the aforementioned step C-3 except that compound (8-1) is used instead of compound (10-1).

### (Step E-2)

In this step, compound (16-1) is obtained by removing the amino-protecting group (P⁴).

For the deprotection process in step E-2, the reaction conditions can be selected according to the kind of protecting group (P⁴). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### (Step E-3)

In this step, compound (16-1) and compound (1-1) are condensed in the presence of a condensing agent to produce compound (12-1).

The amount of compound (16-1) to be used is 0.8 mol to 5 mol, preferably 0.8 mol to 3 mol, per 1 mol of compound (1-1).

In step E-3, the reaction can be performed under conditions similar to those in the aforementioned step A-1 except that compound (16-1) is used instead of compound (2-1).

When the resultant product obtained by this reaction contains a halogen, the halo group can be converted to an alkyl group or a cycloalkyl group by the subsequent coupling reaction.

### (Step E-4)

In this step, compound (Ic) is obtained by removing the amino-protecting group (P⁵).

For the deprotection process in step E-4, the reaction conditions can be selected according to the kind of protecting group (P⁵). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

When the resultant product obtained by this reaction contains a halogen, the halo group can be converted to an alkyl group or a cycloalkyl group by the subsequent coupling reaction.

### [Production method (F)]

In this production method, compound (8-1) and compound (13-1) are reacted to give compound (17-1), the protecting group (P⁴) is removed to give compound (18-1), compound (18-1) is converted to compound (14-1) by reacting with compound (1-1), and the protecting group (P⁶) is removed to obtain compound (Id).

### Production method (F)

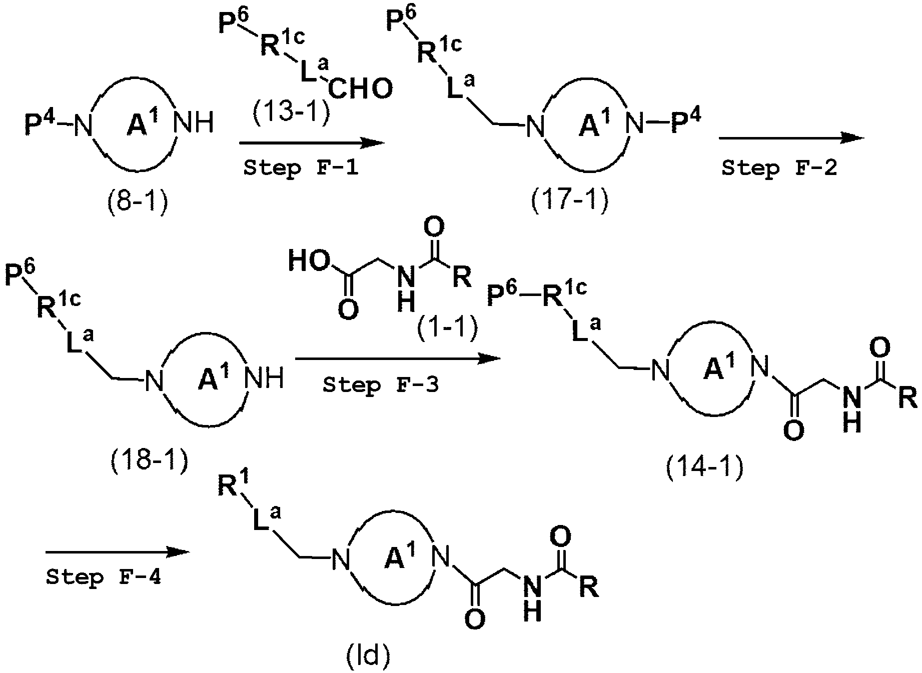

(wherein each symbol is as defined above.)

### (Step F-1)

In this step, compound (8-1) is reacted with a reducing agent and compound (13-1) (reductive alkylation reaction) in a solvent that does not affect the reaction to obtain compound (17-1).

The amount of compound (13-1) to be used is 1 mol to 3 mol, preferably 1 mol to 2 mol, per 1 mol of compound (8-1).

In step F-1, the reaction can be performed under conditions similar to those in the aforementioned step D-1 except that compound (8-1) is used instead of compound (10-1).

### (Step F-2)

In this step, compound (18-1) is obtained by removing the amino-protecting group (P⁴).

For the deprotection process in step F-2, the reaction conditions can be selected according to the kind of protecting group (P⁴). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### (Step F-3)

In this step, compound (18-1) and compound (1-1) are condensed in the presence of a condensing agent to produce compound (14-1).

The amount of compound (18-1) to be used is 0.8 mol to 5 mol, preferably 0.8 mol to 3 mol, per 1 mol of compound (1-1).

In step F-3, the reaction can be performed under conditions similar to those in the aforementioned step A-1 except that compound (18-1) is used instead of compound (2-1).

When the resultant product obtained by this reaction contains a halogen, the halo group can be converted to an alkyl group or a cycloalkyl group by the subsequent coupling reaction.

### (Step F-4)

In this step, compound (Id) is obtained by removing the amino-protecting group (P⁶).

For the deprotection process in step F-4, the reaction conditions can be selected according to the kind of protecting group (P⁶). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

When the resultant product obtained by this reaction contains a halogen, the halo group can be converted to an alkyl group or a cycloalkyl group by the subsequent coupling reaction.

### [Production method (G)]

In this production method, compound (8-1) and compound (19-1) are reacted to give compound (20-1), the protecting group (P⁴) is removed to give compound (21-1), compound (21-1) is converted to compound (22-1) by reacting with compound (11-1), the protecting group (P⁷) is removed to obtain compound (23-1), compound (23-1) is converted to compound (12-1) by reacting with compound (24-1), and the protecting group (P⁵) is removed to obtain compound (Ic).

### Production method (G)

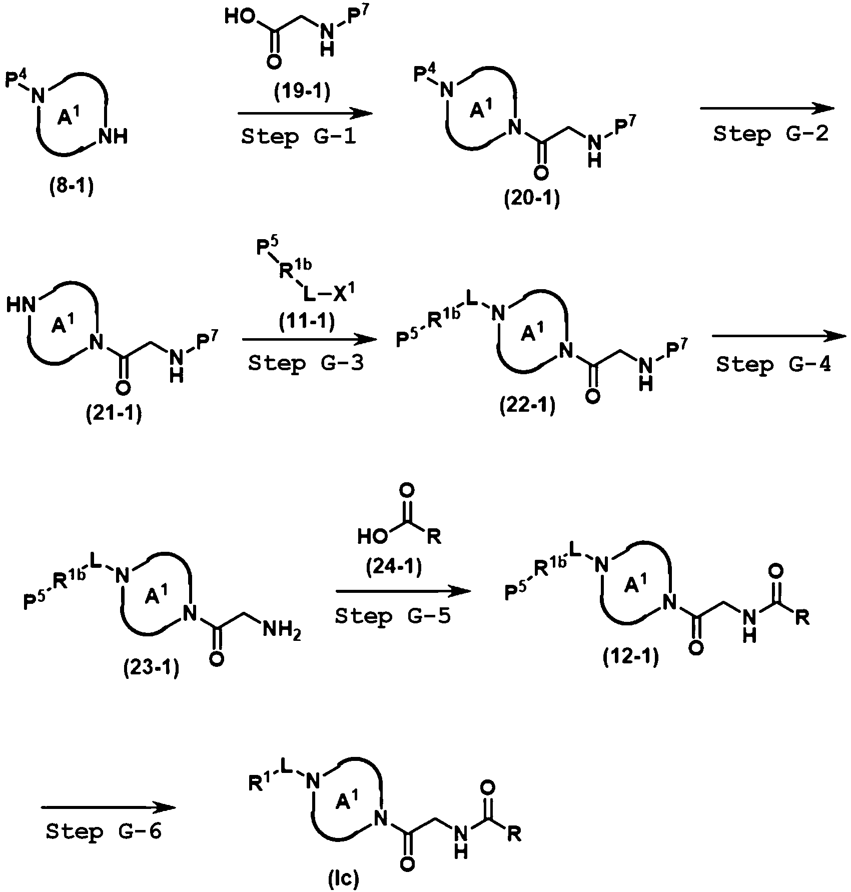

(wherein each symbol is as defined above.)

### (Step G-1)

In this step, compound (19-1) is reacted with a halogenating agent in a solvent that does not affect the reaction to form an acid halide, and the acid halide is reacted with compound (8-1) to obtain compound (20-1).

The amount of compound (19-1) to be used is 1 mol to 3 mol, preferably 1 mol to 2 mol, per 1 mol of compound (8-1).

The halogenating agent is not particularly limited and, for example, thionyl chloride, sulfuryl chloride, phosphorus trichloride, phosphorus pentachloride, phosphorus oxychloride, oxalyl chloride and the like can be mentioned.

The amount of halogenating agent to be used is 1 mol to 10mol, preferably 1 mol to 3 mol, per 1 mol of compound (19-1).

As the additive, the reaction may be performed in the presence of, for example, DMF and the like.

The amount of additive to be used is 0.05 mol to 1 mol, preferably 10.05 mol to 0.1 mol, per 1 mol of compound (19-1).

The reaction solvent is not particularly limited and, for example, aromatic hydrocarbons such as benzene, toluene, xylene and the like; ethers such as tetrahydrofuran and the like; halogenated hydrocarbons such as chloroform, dichloromethane and the like, and a mixture thereof can be mentioned.

The reaction temperature is generally -10°C to 100°C, preferably 10°C to 50°C, and the reaction time is generally 1 hr to 48 hr.

### (Step G-2)

In this step, compound (21-1) is obtained by removing the amino-protecting group (P⁴).

For the deprotection process in step G-2, the reaction conditions can be selected according to the kind of protecting group (P⁴). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### (Step G-3)

In this step, compound (21-1) and compound (11-1) are reacted in the presence of a base to produce compound (22-1).

The amount of compound (11-1) to be used is 1 mol to 5 mol, preferably 1 mol to 3 mol, per 1 mol of compound (21-1).

In step G-3, the reaction can be performed under conditions similar to those in the aforementioned step C-3 except that compound (21-1) is used instead of compound (10-1).

### (Step G-4)

In this step, compound (23-1) is obtained by removing the amino-protecting group (P⁷).

For the deprotection process in step G-4, the reaction conditions can be selected according to the kind of protecting group (P⁷). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### (Step G-5)

In this step, compound (23-1) and compound (24-1) are condensed in the presence of a condensing agent to produce compound (12-1).

The amount of compound (23-1) to be used is 0.8 mol to 5 mol, preferably 0.8 mol to 3 mol, per 1 mol of compound (24-1).

In step G-5, the reaction can be performed under conditions similar to those in the aforementioned step A-1 except that compound (23-1) is used instead of compound (2-1).

### (Step G-6)

In this step, compound (Ic) is obtained by removing the amino-protecting group (P⁵).

For the deprotection process in step G-6, the reaction conditions can be selected according to the kind of protecting group (P⁵). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

Compound (I) or a pharmaceutically acceptable salt thereof obtained by the above-mentioned production methods can be isolated and purified by a general separation means, for example, recrystallization, distillation, chromatography, and the like.

When the compound (I) of the present invention or a pharmaceutically acceptable salt thereof exists as an optical isomer based on an asymmetric carbon, it can be separated into individual optical isomers by conventional optical resolution means (e.g., fractional crystallization method, resolution using a chiral column). Alternatively, the optical isomers can be synthesized using optically pure starting materials. Furthermore, optical isomers can also be synthesized by stereoselectively carrying out each reaction using chiral auxiliary groups or asymmetric catalysts.

### (Medicament (pharmaceutical composition) of the present invention)

Medicament of the present invention is a drug for preventing and/or treating diseases whose symptoms can be alleviated by B0AT1 inhibitory action, containing compound (I), or a pharmaceutically acceptable salt thereof as the active ingredient. Specifically, the medicament of the present invention is at least one of a medicament containing the aforementioned active ingredient for preventing the onset of a disease whose symptoms can be alleviated by the B0AT1 inhibitory action, and a medicament for improving the symptoms of the disease.

The medicament of the present invention may be a medicament comprising only compound (I) or a pharmaceutically acceptable salt thereof, or a pharmaceutical composition comprising said compound (I) or a pharmaceutically acceptable salt thereof and a pharmaceutically acceptable carrier, etc. The medicament of the present invention can be administered to a subject (e.g., mouse, rat, hamster, rabbit, cat, dog, bovine, sheep, monkey, human, etc.) in a prophylactically effective amount or a therapeutically effective amount.

Examples of the pharmaceutically acceptable carrier include excipient (e.g., starch, lactose, sugar, calcium carbonate, calcium phosphate, etc.), binder (e.g., starch, gum arabic, carboxymethylcellulose, hydroxypropylcellulose, crystalline cellulose, etc.), lubricant (e.g., magnesium stearate, talc, etc.), disintegrant (e.g., carboxymethylcellulose, talc, etc.), solvent (e.g., water for injection, physiological brine, Ringer's solution, alcohol, propylene glycol, polyethylene glycol, sesame oil, corn oil, olive oil, cottonseed oil, etc.), solubilizing agent (e.g., polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, etc.), suspending agent (e.g., surfactants such as stearyltriethanolamine, sodium lauryl sulfate, laurylaminopropionate, lecithin, benzalkonium chloride, benzethonium chloride, glyceryl monostearate, and the like; hydrophilic polymers such as poly(vinyl alcohol), polyvinylpyrrolidone, carboxymethylcellulose sodium, methylcellulose, hydroxymethylcellulose, hydroxyethylcellulose, hydroxypropylcellulose and the like; polysorbates, polyoxyethylene hydrogenated castor oil, etc.), isotonic agent (e.g., sodium chloride, glycerol, D-mannitol, D-sorbitol, glucose, etc.), buffering agent (e.g., buffers such as phosphate, acetate, carbonate, citrate, and the like, etc.), soothing agent (e.g., benzyl alcohol, etc.), antiseptic (e.g., p-hydroxybenzoic acid esters, chlorobutanol, benzyl alcohol, phenethyl alcohol, dehydroacetic acid, sorbic acid, etc.), antioxidant (e.g., sulfite, ascorbate, etc.), colorant (e.g., water-soluble food tar color (e.g., food colors such as Food Color Red Nos. 2 and 3, Food Color yellow No.4 and No.5, Food Color Blue Nos. 1 and 2, and the like), water insoluble lake pigment (e.g., the aforementioned aluminum salts of the above water-soluble food tar color), natural dye (e.g., β-carotene, chlorophyll, red iron oxide), etc.), sweetening agent (e.g., saccharin sodium, dipotassium glycyrrhizinate, stevia, etc.), and the like.

The medicament (pharmaceutical composition) of the present invention is prepared by mixing the above-mentioned ingredients, and then preparing the mixture according to known means, for example, oral administration dosage forms such as tablets, fine granules, granules, capsules, dry syrups, and the like, or parenteral dosage forms such as injections (e.g., subcutaneous injection, intravenous injection, intramuscular injection, intraperitoneal injection, intravenous infusion, etc.), topical formulations (e.g., transdermal, ointment, lotion, patch), suppository (e.g., rectal suppository, vaginal suppository), pellet, nasal agent, transpulmonary agent (inhalation agent), ophthalmic agent, implantable agent, microcapsule, liposome, and the like.

Among them, the medicament of the present invention is preferably a preparation for oral administration, such as tablets and the like.

The content of the compound (I) of the present invention, or a pharmaceutically acceptable salt thereof, in the medicament (pharmaceutical composition) of the present invention varies depending on the preparation form. It is generally within the range of about 0.01 to 100 wt%, preferably about 0.1 to 50 wt%, further preferably about 0.5 to 20 wt%, based on the whole preparation.

The dose of the compound (I) of the present invention, or a pharmaceutically acceptable salt thereof, can be selected according to the subject of administration (age, weight, general health condition, sex, degree of disease, etc. of the subject), route of administration, type of disease, kind of concomitant drug, and the like. For example, in the case of a human, when administered orally to an adult patient (weighing approximately 60 kg), the dose of compound (I), or a pharmaceutically acceptable salt thereof, is usually 0.001 mg to 1000 mg, preferably 0.01 mg to 100 mg in terms of the active ingredient, once or several times per day. It can be administered before, after, or between meals. The duration of administration is not limited.

The compound (I) of the present invention, or a pharmaceutically acceptable salt thereof is useful for the prevention of the onset of diseases whose symptoms can be alleviated by a B0AT1 inhibitory action or therapeutic use for the diseases. Particularly, it is effective for the prophylactic or therapeutic use for the aforementioned disease including amino acid metabolism disorders such as phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, isovaleric acidemia, and the like.

The compound (I) of the present invention, or a pharmaceutically acceptable salt thereof can be administered in combination with other drug (concomitant drug) (combined use), as long as the efficacy thereof is not impaired. The concomitant drug is not particularly limited and, for example, one or more known drugs conventionally used for the treatment of "diseases whose symptoms can be alleviated by B0AT1 inhibitory action" exemplified above can be preferably used.

Specifically, when the compound (I) of the present invention or a pharmaceutically acceptable salt thereof is used for the treatment and/or prevention of amino acid metabolism disorders such as phenylketonuria, urea cycle disorder, hypertyrosinemia (type 1-3), hypermethioninemia, maple syrup urine disease, homocystinuria, nonketotic hyperglycinemia, propionic acidemia, methylmalonic acidemia, isovaleric acidemia, and the like, concomitant drugs to be used in combination include, for example, vitamins (e.g., folic acid (vitamin B9), nicotinamide (vitamin B3), thiamine (vitamin B1), pyridoxine (vitamin B6), etc.) and drugs to alleviate various symptoms of amino acid metabolism disorders (e.g., L-dopa, LNAA such as 5-hydroxytryptophan, sapropterin hydrochloride, pegvaliase, nicotinic acid, nitisinone, S-adenosylmethionine, betaine, aspirin, dipyridamole, sodium benzoate, sodium phenylbutyrate, dextromethorphan, ketamine, antidepressants, anxiolytics, ADHD medications, etc.).

When a concomitant drug is used, the administration period is not limited and can be administered simultaneously or with a time interval to the subject of administration. In the administration with a time interval, the medicament of the present invention may be administered first and the concomitant drug may be administered later, or the concomitant drug may be administered first and the medicament of the present invention may be administered later. Each administration method may be the same or different. In addition, a single preparation containing the compound (I) of the present invention or a pharmaceutically acceptable salt thereof, and a concomitant drug in combination can also be administered.

The dose of the concomitant drug can be appropriately selected with the dose used clinically as the standard. The mixing ratio of the compound of the present invention or a pharmaceutically acceptable salt thereof, and a concomitant drug can be appropriately determined according to the subject of administration (age, body weight, general health condition, gender, severity of the disease, etc.), administration route, type of disease, type of concomitant drug, and the like.

The mass ratio of the compound (I) or a pharmaceutically acceptable salt thereof and the concomitant drug is not particularly limited.

The concomitant drug that complements and/or enhances the therapeutic effect of the compound (I) or a pharmaceutically acceptable salt thereof includes not only those that have been found to date but also those that will be found in the future based on the mechanism described above.

In addition, to complement and/or enhance the therapeutic effect of compound (I) or its pharmaceutically acceptable salt, it may also be useful in combination with dietary therapy or enzyme replacement therapy to avoid the intake of certain amino acids.

The medicament or pharmaceutical composition of the present invention may be provided in the form of a kit together with instructions on how to administer the medicament or pharmaceutical composition. The drug in the kit is supplied by a container manufactured from a material that will effectively sustain the activity of the components of the medicament or pharmaceutical composition for a long period of time, will not adsorb on the inside of the container, and will not alter the components. For example, a sealed glass ampoule may contain buffer and the like sealed in the presence of a neutral, inert gas such as nitrogen gas.

The kit may also be accompanied by instructions for use. The instructions for use of the kit may be printed on paper or other media, or may be stored on an electromagnetically readable medium such as a CD-ROM or DVD-ROM and supplied to the user.

### [Example]

The present invention is explained in more detail in the following by referring to Reference Examples, Examples, and Experimental Examples, which do not limit the present invention. The present invention may be modified without departing from the scope of the invention. The reagents, apparatuses, materials and the like used in the present invention are commercially available unless particularly indicated.

% indicates mol/mol % for yields, and weight % for others unless otherwise specified. Room temperature indicates a temperature of 15°C to 30°C unless otherwise specified. * in the following structural formulas indicates racemic carbon. Other abbreviations used in the text show the following meanings.
THF: tetrahydrofuran
DMSO: dimethyl sulfoxide
DMF: N,N-dimethylformamide
HATU: O-(7-aza benzotriazol-1-yl)-N,N,N',N'-tetramethyluronium hexafluorophosphate
WSC: 1-ethyl-3-(3'-dimethylaminopropyl)carbodiimide
HOBt: 1-hydroxybenzotriazole

The starting compounds used in the following Reference Examples and Examples are known compounds unless particularly indicated, and those synthesized and identified according to methods known per se (e.g., US Patent No. 10,836,736; Bioorganic & Medicinal Chemistry Letters, 2017, 27(21), 4805-4811; J. Med. Chem., 2014, 57, 3687-3706; WO 2013/067274), or methods analogous thereto were used.

### Reference Example 1:

### Production of 6-bromo-4-methoxy-1,2,3,4-tetrahydroisoquinoline

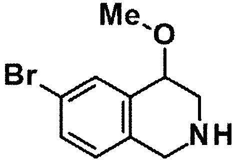

(1) To a solution of 6-bromo-2-(2-nitrophenyl)sulfonyl-3,4-dihydro-1H-isoquinolin-4-ol (300 mg) in toluene (2 ml) were added tetrabutylammonium hydrogensulfate (25 mg), iodomethane (206 mg), and potassium hydroxide (50%w/v, 2 ml), and the reaction mixture was stirred at 50°C for 1 hr. Water was added to the reaction mixture, and the aqueous layer was extracted with ethyl acetate. The organic layer was washed with saturated brine, dried over anhydrous sodium sulfate, and concentrated under reduced pressure. The residue was purified by silica gel column chromatography (solvent: n-hexane/ethyl acetate =100/0 - 50/50) to give 6-bromo-4-methoxy-2-(2-nitrophenyl)sulfonyl-3,4-dihydro-1H-isoquinoline (295 mg; yield 95%) as a colorless viscous oil.
   MS(ESI) m/z: 397.1/399.1[M-OMe+H]⁺
(2) To a solution of 6-bromo-4-methoxy-2-(2-nitrophenyl)sulfonyl-3,4-dihydro-1H-isoquinoline (295 mg) obtained in the aforementioned (1) in DMF (2 ml) were added potassium carbonate (191 mg) and phenylmethanethiol (172 mg), and the reaction mixture was stirred for 3 hr at room temperature. Water was added to the reaction mixture, and the aqueous layer was extracted with ethyl acetate. The organic layer was washed with saturated brine, dried over anhydrous sodium sulfate, concentrated under reduced pressure, and dried under reduced pressure to give a crude product (167 mg; yield 99%) of the title compound as a brown viscous oil.

### Reference Example 2 :

### Production of methyl 3-(1-methylpiperazin-2-yl)benzoate

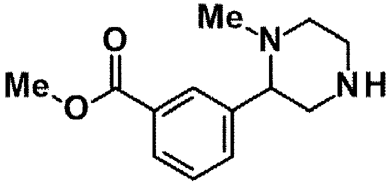

(1) Benzyl 3-(3-bromophenyl)piperazine-1-carboxylate (1.4 g) was dissolved in THF (12 ml), acetic acid (2 ml), aqueous formalin solution (37%, 1.4 ml) and sodium triacetoxyborohydride (1.58 g) were added, and the reaction mixture was stirred at room temperature for 30 min. To the reaction mixture were added aqueous sodium hydroxide solution (1 mol/l) and chloroform, the organic layer was separated, and the aqueous layer was extracted with chloroform. The organic layers were combined, dried over anhydrous magnesium sulfate, and filtered. The filtrate was evaporated under reduced pressure to give a crude product (1.47 g) of benzyl 3-(3-bromophenyl)-4-methylpiperazine-1-carboxylate as a yellow oil.
(2) The crude product (350 mg) of benzyl 3-(3-bromophenyl)-4-methylpiperazine-1-carboxylate obtained in the aforementioned (1) was dissolved in acetonitrile (6 ml), methanol (3 ml), molybdenum hexacarbonyl (239 mg) and DBU (0.28 ml) were added thereto, and the mixture was subjected to nitrogen replacement. To the reaction mixture was added bis(tri-tert-butylphosphine)palladium(0) (46 mg), and the mixture was stirred under microwave irradiation at 140°C for 30 min. The reaction mixture was allowed to cool to room temperature, diluted with ethyl acetate and filtered. The filtrate was concentrated under reduced pressure, and the obtained residue was purified by silica gel column chromatography (solvent: n-hexane/ethyl acetate =50/50 - 25/75) to give benzyl 3-(3-methoxycarbonylphenyl)-4-methylpiperazine-1-carboxylate (306 mg; yield 92.4%) as a yellow oil.
   MS(ESI) m/z: 369.2[M+H]⁺
(3) Benzyl 3-(3-methoxycarbonylphenyl)-4-methylpiperazine-1-carboxylate (306 mg) obtained in the aforementioned (2) was dissolved in methanol (2.8 ml), palladium hydroxide (30 mg) was added, and the mixture was subjected to hydrogen replacement. The reaction mixture was stirred at 60°C for 4 hr 30 min, allowed to cool to room temperature, filtered through celite, and washed with methanol. The filtrate was concentrated under reduced pressure to give the title compound (265 mg; yield 136.2%) as a yellow amorphous.
   MS(ESI) m/z: 235.2[M+H]⁺

### Reference Example 3:

### Production of methyl 2-[2-(1,2,3,6-tetrahydropyridin-5-yl)phenyl]acetate hydrochloride

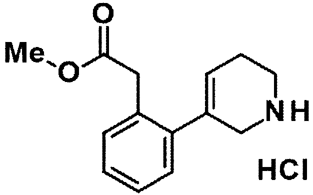

(1) tert-Butyl 5-(4,4,5,5-tetramethyl-1,3,2-dioxa borolan-2-yl)-3,6-dihydro-2H-pyridine-1-carboxylate (1.0 g) was dissolved in 1,4-dioxane (40 ml), methyl 2-(2-bromophenyl)acetate (890 mg), tetrakis(triphenylphosphine)palladium (380 mg), cesium carbonate (2.1 g) and water (10 ml) were added, and the reaction mixture was stirred at 90°C for 1 hr. The reaction mixture was allowed to cool to room temperature, water was added thereto, and the mixture was extracted with ethyl acetate. The organic layer was washed with saturated brine, dried over anhydrous magnesium sulfate, and filtered. The filtrate was concentrated under reduced pressure, and the obtained residue was purified by silica gel column chromatography (solvent: n-hexane/ethyl acetate =80/20 - 50/50) to give tert-butyl 5-[2-(2-methoxy-2-oxoethyl)phenyl]-3,6-dihydro-2H-pyridine-1-carboxylate (1.0 g; yield 93.3%) as a colorless oil.
(2) tert-Butyl 5-[2-(2-methoxy-2-oxoethyl)phenyl]-3,6-dihydro-2H-pyridine-1-carboxylate (300 mg) obtained in the aforementioned (1) was dissolved in methanol (3 ml), and hydrogen chloride -1,4-dioxane solution (4 mol/l.3 ml) was added. The reaction mixture was stirred at room temperature for 1 hr, and concentrated under reduced pressure at 40°C to give a crude product (242 mg) of the title compound as a colorless amorphous.

### Reference Example 4:

### Production of methyl 2-(3-piperidin-3-ylphenyl)acetate hydrochloride

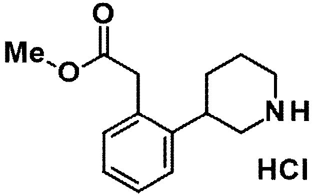

(1) tert-Butyl 5-[2-(2-methoxy-2-oxoethyl)phenyl]-3,6-dihydro-2H-pyridine-1-carboxylate (700 mg) obtained in Reference Example 3, step (1), was dissolved in methanol (10 ml), and palladium-carbon (10%, 200 mg) was added. The reaction mixture was subjected to hydrogen replacement, and stirred at room temperature for 2 hr. The reaction mixture was filtered through celite, and the filtrate was concentrated under reduced pressure. The obtained residue was purified by silica gel column chromatography (solvent: n-hexane/ethyl acetate =95/5 - 80/20) to give tert-butyl 3-[3-(2-methoxy-2-oxoethyl)phenyl]piperidine-1-carboxylate (580 mg; yield 82.3%) as a colorless oil.
   MS(ESI) m/z: 334.1[M+H]⁺
(2) tert-Butyl 3-[3-(2-methoxy-2-oxoethyl)phenyl]piperidine-1-carboxylate (580 mg) obtained in the aforementioned (1) was dissolved in methanol (6 ml), and hydrogen chloride -1,4-dioxane solution (4 mol/l, 6 ml) was added thereto. The reaction mixture was stirred at room temperature for 1 hr, and concentrated under reduced pressure at 40°C to give a crude product (469 mg) of the title compound as a colorless amorphous.

### Reference Example 5:

### Production of tert-butyl 3-(5,6,7,8-tetrahydropyrido[4,3-d]pyrimidin-2-yl)propanoate

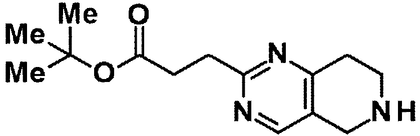

(1) To a mixture of 2-chloro-5,6,7,8-tetrahydropyrido[4,3-d]pyrimidine hydrochloride (400 mg), benzyloxycarbonylchloride (0.48 ml), sodium hydrogen carbonate (326 mg) and 1,4-dioxane (6.4 ml) was added water (1.8 ml), and the mixture was stirred at room temperature for 18 hr. Water was added to the reaction mixture. The organic layer was extracted with ethyl acetate, and the combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 30/70) to give benzyl 2-chloro-7,8-dihydro-5H-pyrido[4,3-d]pyrimidine-6-carboxylate (535 mg; yield 80%) as a colorless viscous oil.
   MS(ESI) m/z: 304.1/306.1[M+H]⁺
(2) To a mixture of benzyl 2-chloro-7,8-dihydro-5H-pyrido[4,3-d]pyrimidine-6-carboxylate (535 mg) obtained in the aforementioned (1), palladium(II) chloride-bis[4-(N,N-dimethylamino)phenyl]-di-tert-butylphosphine (124 mg), tert-butyl (E)-3-(4,4,5,5-tetramethyl-1,3,2-dioxaborolan-2-yl)prop-2-enoate (538 mg), cesium carbonate (1.15 g) and 1,4-dioxane (3 ml) was added water (0.8 ml), and the mixture was stirred under microwave irradiation at 140°C for 1 hr. Water was added to the reaction mixture. The organic layer was extracted with ethyl acetate, and the combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 30/70) to give benzyl 2-[(E)-3-[(2-methylpropan-2-yl)oxy]-3-oxoprop-1-enyl]-7,8-dihydro-5H-pyrido[4,3-d]pyrimidine-6-carboxylate (318 mg; yield 46%) as a yellow viscous oil.
   MS(ESI) m/z: 396.2[M+H]⁺
(3) To a solution of benzyl 2-[(E)-3-[(2-methylpropan-2-yl)oxy]-3-oxoprop-1-enyl]-7,8-dihydro-5H-pyrido[4,3-d]pyrimidine-6-carboxylate (52 mg) obtained in the aforementioned (2) in ethanol (1.4 ml) was added palladium(II) hydroxide (20 mg), and the mixture was stirred under hydrogen atmosphere at room temperature for 3 hr. The reaction mixture was filtered through celite, and the filtrate was concentrated under reduced pressure to give a crude product (35 mg) of the title compound as a colorless viscous oil.

### Reference Example 6:

### Production of methyl 3-(indolin-4-yl)propanoate trifluoroacetate

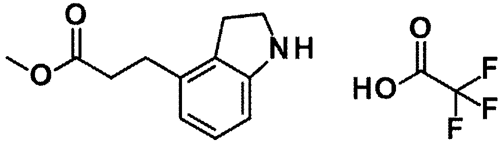

(1) tert-Butyl 4-bromoindoline-1-carboxylate (500 mg) was dissolved in 1,4-dioxane (4.5 ml), to the reaction mixture were added water (1 ml), cesium carbonate (1.1 g), ethyl (E)-3-(4,4,5,5-tetramethyl-1,3,2-dioxa borolan-2-yl)prop-2-enoate (570 mg) and bis(4-di-tert-butylphosphanyl-N,N-dimethylaniline)dichloro palladium(II) (100 mg). The reaction mixture was stirred under microwave irradiation at 110°C for 2 hr 30 min. The reaction mixture was allowed to cool to room temperature and extracted with ethyl acetate. The organic layer was dried over sodium sulfate, filtered, and the filtrate was concentrated under reduced pressure to give a crude product (560 mg) of tert-butyl 4-[(E)-3-ethoxy-3-oxoprop-1-enyl]indoline-1-carboxylate as a brown solid.
(2) The crude product (560 mg) of tert-butyl 4-[(E)-3-ethoxy-3-oxoprop-1-enyl]indoline-1-carboxylate obtained in the aforementioned (1) was dissolved in methanol (25 ml). Magnesium (500 mg) was added thereto, and the mixture was stirred at room temperature for 14 hr. The reaction mixture was washed with hydrochloric acid (1 mol/l), and the organic layer was dried over sodium sulfate, NH₂ silica gel was added and the mixture was filtered. The filtrate was concentrated under reduced pressure to give a crude product (550 mg) of tert-butyl 4-(3-methoxy-3-oxopropyl)indoline-1-carboxylate as a yellow oil.
(3) The crude product (247 mg) of tert-butyl 4-(3-methoxy-3-oxopropyl)indoline-1-carboxylate obtained in the aforementioned (2) was dissolved in dichloromethane (1 ml), trifluoroacetic acid (1.3 ml) was added thereto, and the mixture was stirred at room temperature for 1 hr. The solvent was evaporated under reduced pressure, toluene was added to the residue and the mixture was concentrated under reduced pressure again to give a crude product (258.2 mg) of the title compound.

### Reference Example 7:

### Production of tert-butyl 2-(5-cyano-1,2,3,4-tetrahydroisoquinolin-6-yl)acetate

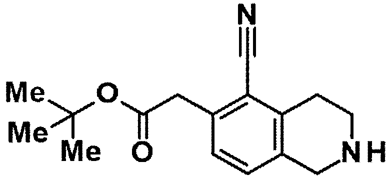

(1) To a solution of 6-bromoisoquinoline (1 g) in sulfuric acid (10 ml) was added 1-chloropyrrolidine-2,5-dione (1.3 g), and the mixture was stirred at 50°C for 3 hr. 1-Chloropyrrolidine-2,5-dione (1.3 g) was added thereto, and the mixture was further stirred at 50°C for 15 hr. To the reaction mixture were added water and sodium hydroxide, and the organic layer was extracted with ethyl acetate. The combined organic layer was washed with water and saturated brine, dried over anhydrous magnesium sulfate, and concentrated under reduced pressure. The obtained residue was purified by silica gel column chromatography (solvent: n-hexane/ethyl acetate =100/0 - 30/70) to give 6-bromo-5-chloroisoquinoline (824 mg; yield 71%) as a white solid. MS(ESI) m/z: 242.0/244.0[M+H]⁺
(2) To a solution of 6-bromo-5-chloroisoquinoline (824 mg) obtained in the aforementioned (1) in acetic acid (10 ml) was added sodium borohydride (141 mg) under ice-cooling, and the mixture was stirred at room temperature for 1 hr. Under ice-cooling, sodium borohydride (71 mg) was added, and the mixture was stirred at room temperature for 2 hr more. The reaction mixture was neutralized with aqueous sodium hydroxide solution, and the organic layer was extracted with ethyl acetate. The combined organic layer was washed with saturated brine, dried over anhydrous magnesium sulfate, and concentrated under reduced pressure to give a crude product (837 mg) of 6-bromo-5-chloro-1,2,3,4-tetrahydroisoquinoline as a white solid.
(3) To the crude product (837 mg) of 6-bromo-5-chloro-1,2,3,4-tetrahydroisoquinoline obtained in the aforementioned (2) were added ethyl acetate (4 ml) and hydrogen chloride (4 mol/l 1,4-dioxane solution) (0.93 ml), and the precipitated solid was collected by filtration to give 6-bromo-5-chloro-1,2,3,4-tetrahydroisoquinoline hydrochloride (766 mg; yield 80%) as a white solid.
   MS(ESI) m/z: 246.0/248.0[M+H]⁺
(4) To a mixture of 6-bromo-5-chloro-1,2,3,4-tetrahydroisoquinoline hydrochloride (1 g) obtained in the aforementioned (3), pyridine (7 ml) and N,N-dimethylpyridine-4-amine (43 mg), was added trifluoroacetic anhydride (1.5 g) under ice-cooling, and the mixture was stirred at room temperature for 2 hr. To the reaction mixture were added water and 1 mol/l hydrochloric acid, and the organic layer was extracted with ethyl acetate. The combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 80/20) to give 1-(6-bromo-5-chloro-3,4-dihydro-1H-isoquinolin-2-yl)-2,2,2-trifluoroethanone (1.05 g; yield 87%) as a white solid.
   ¹H-NMR (400 MHz, CDCl₃) δ ppm: 3.05(2H, t), 3.87(2H, t), 4.76(2H, s), 6.96(1H, d), 7.53(1H, d) (*rotamers were observed at NMR integration ratio of about 2:1.)
(5) 1-(6-Bromo-5-chloro-3,4-dihydro-1H-isoquinolin-2-yl)-2,2,2-trifluoroethanone (1.05 g) obtained in the aforementioned (4), 1-tert-butoxyvinyloxy-tert-butyl-dimethylsilane (285 mg), potassium carbonate (114 mg) and bis(tri-tert-butylphosphine) palladium(0) (63 mg) were dissolved in DMF (7 ml), and the mixture was stirred under microwave irradiation at 120°C for 1 hr. Water was added to the reaction mixture, and the organic layer was extracted with ethyl acetate. The combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 30/70) to give tert-butyl 2-[5-chloro-2-(2,2,2-trifluoroacetyl)-3,4-dihydro-1H-isoquinolin-6-yl]acetate (1.02 g; yield 76%) as a colorless viscous oil.
   MS(ESI) m/z: 322.1[M-C₄H₈+H]⁺
(6) tert-Butyl 2-[5-chloro-2-(2,2,2-trifluoroacetyl)-3,4-dihydro-1H-isoquinolin-6-yl]acetate (1.02 g) obtained in the aforementioned (5), potassium hexacyanoferrate(II) trihydrate (1.65 g), [2-(2-aminophenyl)phenyl]-chloropalladium; 2-dicyclohexylphosphino-2',4',6'-triisopropylbiphenyl (212 mg) and potassium acetate (530 mg) were dissolved in 1,4-dioxane (50 ml), water (15 ml) was added and the mixture was stirred at 100°C for 2 hr. The reaction mixture was filtered, water was added to the filtrate, and the organic layer was extracted with ethyl acetate. The combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 80/20) to give tert-butyl 2-[5-cyano-2-(2,2,2-trifluoroacetyl)-3,4-dihydro-1H-isoquinolin-6-yl]acetate (551 mg; yield 55%) as a brown viscous oil.
   ¹H-NMR (400 MHz, CDCl₃) δ ppm: 1.47(9H, s),3.18(2H, t), 3.79(2H, s), 3.91(2H, t),4.81(2H, s), 7.29(1H, d), 7.34(1H, d). (*rotamers were observed at NMR integration ratio of about 2:1.)
(7) tert-Butyl 2-[5-cyano-2-(2,2,2-trifluoroacetyl)-3,4-dihydro-1H-isoquinolin-6-yl]acetate (551 mg) obtained in the aforementioned (6) was dissolved in ethanol (9 ml), 1 mol/l aqueous sodium hydroxide solution (5.4 ml) was added and the mixture was stirred at room temperature for 1 hr. Water was added to the reaction mixture and the organic layer was extracted with ethyl acetate. The combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 0/100) to give the title compound (172 mg; yield 42%) as a white solid. MS(ESI) m/z: 273.3[M+H]⁺

### Reference Example 8:

### Production of tert-butyl 2-(1,2,3,4-tetrahydroisoquinolin-6-yl)acetate

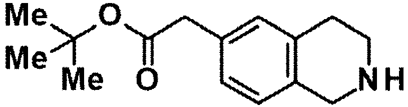

(1) Benzyl 6-bromo-3,4-dihydro-1H-isoquinoline-2-carboxylate (2 g), tert-butyl-(1-methoxyvinyloxy)-dimethylsilane (2.5 ml), potassium carbonate (1.6 g) and bis(tri-tert-butylphosphine)palladium(0) (886 mg) were dissolved in DMF (19 ml), and the mixture was stirred under microwave irradiation at 120°C for 2 hr. Water was added to the reaction mixture, and the organic layer was extracted with ethyl acetate. The combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 70/30) to give benzyl 6-(2-methoxy-2-oxoethyl)-3,4-dihydro-1H-isoquinoline-2-carboxylate (978 mg; yield 48%) as a yellow viscous oil.
   ¹H-NMR (400 MHz, CDCl₃) δ ppm: 2.84(2H,br-s), 3.58(2H, s), 3.69(3H, s), 3.72(2H, s), 4.63(2H, s), 5.18(2H, s), 7.01-7.12(3H, m), 7.29-7.42(5H, m).
(2) Benzyl 6-(2-methoxy-2-oxoethyl)-3,4-dihydro-1H-isoquinoline-2-carboxylate (1.41 g) obtained in the aforementioned (1) was dissolved in methanol (31 ml), 4 mol/l aqueous lithium hydroxide solution (9.2 ml) was added and the mixture was stirred at room temperature for 3 hr. The reaction mixture was neutralized, and the organic layer was extracted with ethyl acetate. The combined organic layer was washed with saturated brine, dried over anhydrous magnesium sulfate and concentrated under reduced pressure to give a crude product (1.33 g) of 2-(2-phenylmethoxycarbonyl-3,4-dihydro-1H-isoquinolin-6-yl)acetic acid as a yellow viscous oil.
(3) The crude product (1.33 g) of 2-(2-phenylmethoxycarbonyl-3,4-dihydro-1H-isoquinolin-6-yl)acetic acid obtained in the aforementioned (2) was dissolved in toluene (13.6 ml), 1,1-di-tert-butoxy-N,N-dimethylmethanamine (4 ml) was added and the mixture was stirred at 120°C for 3 hr. The reaction mixture was allowed to cool, concentrated under reduced pressure, and the obtained residue was purified by silica gel column chromatography (solvent: n-hexane/ethyl acetate =100/0 - 70/30) to give benzyl 6-[2-[(2-methylpropan-2-yl)oxy]-2-oxoethyl]-3,4-dihydro-1H-isoquinoline-2-carboxylate (428 mg; yield 26%) as a colorless viscous oil.
   MS(ESI) m/z: 380.4[M-H]⁻
(4) To a solution of benzyl 6-[2-[(2-methylpropan-2-yl)oxy]-2-oxoethyl]-3,4-dihydro-1H-isoquinoline-2-carboxylate (400 mg) obtained in the aforementioned (3) in ethanol (3.3 ml) was added palladium(II) hydroxide (40 mg), and the mixture was stirred under hydrogen atmosphere at room temperature for 3 hr. The reaction mixture was filtered through a membrane filter, and the filtrate was concentrated under reduced pressure to give a crude product (244 mg) of the title compound as a white solid.

### Reference Examples 9 to 21:

The corresponding starting compounds were treated in the same manner as in Reference Example 8 to obtain crude products of the compounds described in the following Table 1-1 and Table 1-2.

**[Table 1-1]**

| **Ref. Ex.No.** | **structural formula** | **Ref. Ex.No.** | **structural formula** |
|---|---|---|---|
| 9 | 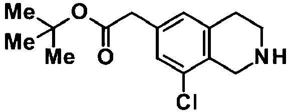 | 14 | 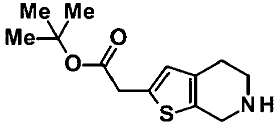 |
| 10 | 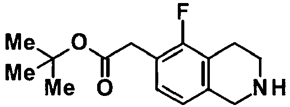 | 15 | 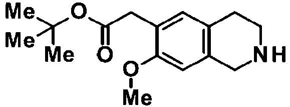 |
| 11 | 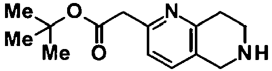 | 16 | 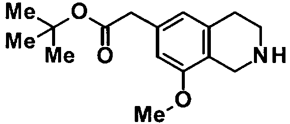 |
| 12 | 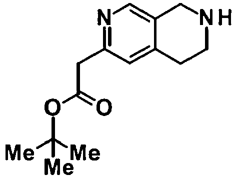 | 17 | 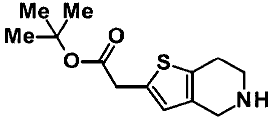 |
| 13 | 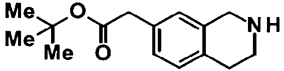 | 18 | 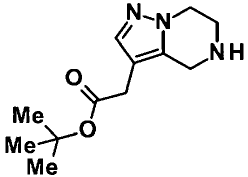 |

**[Table 1-2]**

| **Ref. Ex.No.** | **structural formula** |
|---|---|
| 19 | 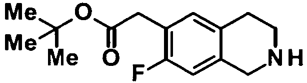 |
| 20 | 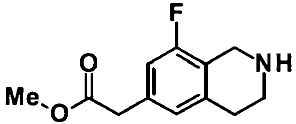 |
| 21 | 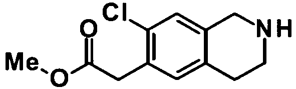 |

### Reference Example 22:

### Production of tert-butyl (2S)-1-(1,2,3,4-tetrahydroisoquinolin-6-yl)piperidine-2-carboxylate

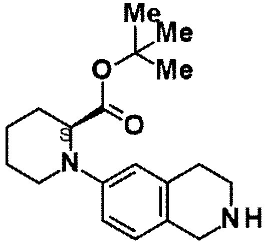

(1) To a mixture of benzyl 6-bromo-3,4-dihydro-1H-isoquinoline-2-carboxylate (1.00 g), tert-butyl (2S)-piperidine-2-carboxylate (800 mg), bis(dibenzylideneacetone)palladium(0) (270 mg), 2-dicyclohexylphosphino-2',6'-diisopropoxybiphenyl (135 mg) and toluene (30 ml) was added sodium tert-butoxide (1.10 g) and the mixture was stirred at 120°C for 1 hr. The reaction mixture was allowed to cool, water was added thereto, and the organic layer was extracted with ethyl acetate. The combined organic layer was dried over anhydrous magnesium sulfate and concentrated under reduced pressure. The obtained residue was purified by silica gel column chromatography (solvent: n-hexane/ethyl acetate =90/10 - 60/40) to give benzyl 6-[(2S)-2-[(2-methylpropan-2-yl)oxycarbonyl]piperidin-1-yl]-3,4-dihydro-1H-isoquinoline-2-carboxylate (113 mg; yield 9%) as a brown viscous oil.
   MS(ESI) m/z: 451.4[M+H]⁺
(2) Benzyl 6-[(2S)-2-[(2-methylpropan-2-yl)oxycarbonyl]piperidin-1-yl]-3,4-dihydro-1H-isoquinoline-2-carboxylate (113 mg) obtained in the aforementioned (1) was dissolved in methanol (5 ml), 10% palladium-carbon (100 mg) was added, and the mixture was stirred under hydrogen atmosphere at room temperature for 3 hr. The reaction mixture was filtered through celite, and the filtrate was concentrated under reduced pressure to give a crude product of the title compound as a brown solid.

### Reference Example 23:

### Production of tert-butyl 1-(1,2,3,4-tetrahydroisoquinolin-6-yl)pyrrolidine-3-carboxylate

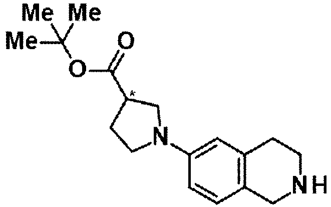

(1) To a mixture of benzyl 6-bromo-3,4-dihydro-1H-isoquinoline-2-carboxylate (1.00 g), tert-butyl pyrrolidine-3-carboxylate (600 mg), bis(dibenzylideneacetone)palladium(0) (270 mg), 2-dicyclohexylphosphino-2',6'-diisopropoxybiphenyl (135 mg) and toluene (30 ml) was added sodium tert-butoxide (1.10 g), and the mixture was stirred at 120°C for 1 hr. The reaction mixture was allowed to cool, water was added thereto, and the organic layer was extracted with ethyl acetate. The combined organic layer was dried over anhydrous magnesium sulfate and concentrated under reduced pressure. The obtained residue was purified by silica gel column chromatography (solvent: n-hexane/ethyl acetate =90/10 - 60/40) to give benzyl 6-[3-[(2-methylpropan-2-yl)oxycarbonyl]pyrrolidin-1-yl]-3,4-dihydro-1H-isoquinoline-2-carboxylate (517 mg; yield 41%) as a brown viscous oil. MS(ESI) m/z: 436.9[M+H]⁺
(2) Benzyl 6-[3-[(2-methylpropan-2-yl)oxycarbonyl]pyrrolidin-1-yl]-3,4-dihydro-1H-isoquinoline-2-carboxylate (517 mg) obtained in the aforementioned (1) was dissolved in methanol (30 ml), 10% palladium-carbon (200 mg) was added, and the mixture was stirred under hydrogen atmosphere at room temperature for 3 hr. The reaction mixture was filtered through celite, and the filtrate was concentrated under reduced pressure to give the title compound (311 mg; yield 87%) as a yellow solid.
   MS(ESI) m/z: 303.3[M+H]⁺

### Reference Example 24:

### Production of tert-butyl 1-(1,2,3,4-tetrahydroisoquinolin-6-yl)piperidine-4-carboxylate

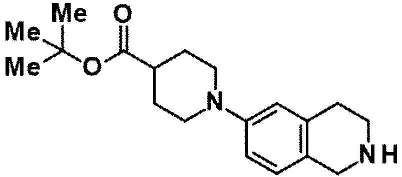

(1) To a mixture of benzyl 6-bromo-3,4-dihydro-1H-isoquinoline-2-carboxylate (1.00 g), tert-butyl piperidine-4-carboxylate hydrochloride (770 mg), bis(dibenzylideneacetone)palladium(0) (270 mg), 2-dicyclohexylphosphino-2',6'-diisopropoxy biphenyl (135 mg) and toluene (30 ml) were added N,N-diisopropylethylamine (0.6 ml) and sodium tert-butoxide (840 mg), and the mixture was stirred at 120°C for 1 hr. The reaction mixture was allowed to cool, water was added thereto, and the organic layer was extracted with ethyl acetate. The combined organic layer was dried over anhydrous magnesium sulfate and concentrated under reduced pressure. The obtained residue was purified by silica gel column chromatography (solvent: n-hexane/ethyl acetate =90/10 - 60/40) to give benzyl 6-[4-[(2-methylpropan-2-yl)oxycarbonyl]piperidin-1-yl]-3,4-dihydro-1H-isoquinoline-2-carboxylate (1.11 g; yield 85%) as a brown viscous oil.
   MS(ESI) m/z: 450.9[M+H]⁺
(2) Benzyl 6-[4-[(2-methylpropan-2-yl)oxycarbonyl]piperidin-1-yl]-3,4-dihydro-1H-isoquinoline-2-carboxylate (1.11 g) obtained in the aforementioned (1) was dissolved in methanol (10 ml), 10% palladium-carbon (200 mg) was added, and the mixture was stirred under hydrogen atmosphere at room temperature for 3 hr. The reaction mixture was filtered through celite, and the filtrate was concentrated under reduced pressure to give the title compound (707 mg; yield 91%)as a brown solid.
   MS(ESI) m/z: 317.3[M+H]⁺

### Reference Example 25:

### Production of tert-butyl 2-methyl-3-(1,2,3,4-tetrahydroisoquinolin-6-yl)propanoate

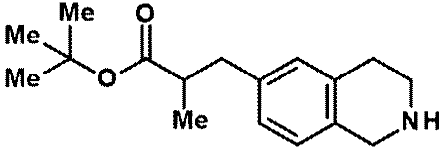

(1) Benzyl 6-bromo-3,4-dihydro-1H-isoquinoline-2-carboxylate (1 g), tert-butyl 2-methylprop-2-enoate (821 mg), potassium carbonate (567 mg) and [1,3-bis(2,6-diisopropyl phenyl)imidazol-2-ylidene] (3-chloropyridyl)palladium(II) dichloride (394 mg) were dissolved in DMF (6 ml), and the mixture was stirred under microwave irradiation at 120°C for 2 hr. Water was added to the reaction mixture, and the organic layer was extracted with ethyl acetate. The combined organic layer was washed successively with water and saturated brine, dried over anhydrous magnesium sulfate, and concentrated under reduced pressure. The obtained residue was purified by silica gel column chromatography (solvent: n-hexane/ethyl acetate =100/0 - 30/70) to give benzyl 6-[2-methyl-3-[(2-methylpropan-2-yl)oxy]-3-oxoprop-1-enyl]-3,4-dihydro-1H-isoquinoline-2-carboxylate (1.06 g; yield 32%) as a colorless viscous oil.
   MS(ESI) m/z: 352.2[M-C₄H₈+H]⁺
(2) To a solution of benzyl 6-[2-methyl-3-[(2-methylpropan-2-yl)oxy]-3-oxoprop-1-enyl]-3,4-dihydro-1H-isoquinoline-2-carboxylate (1.06 g) obtained in the aforementioned (1) in ethanol (4 ml) was added palladium(II) hydroxide (48 mg), and the mixture was stirred under hydrogen atmosphere at room temperature for 3 hr. The reaction mixture was filtered through celite, and the filtrate was concentrated under reduced pressure to give a crude product (1.04 g) of the title compound as a colorless viscous oil.

### Reference Example 26:

### Production of tert-butyl 3-(1,2,3,4-tetrahydroisoquinolin-6-yl)butanoate

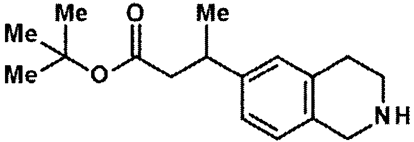

(1) Benzyl 6-bromo-3,4-dihydro-1H-isoquinoline-2-carboxylate (1 g), tributyl(1-ethoxyvinyl)tin (1.27 ml) and bis(tri-tert-butylphosphine)palladium(0) (74 mg) were dissolved in DMF (9 ml), and the mixture was stirred under microwave irradiation at 100°C for 1 hr. To the reaction mixture was added 1 mol/l hydrochloric acid, and the mixture was stirred at room temperature for 1 hr. Water was added to the reaction mixture, and the organic layer was extracted with ethyl acetate. The combined organic layer was washed with saturated brine, dried over anhydrous magnesium sulfate, and concentrated under reduced pressure. The obtained residue was purified by silica gel column chromatography (solvent: n-hexane/ethyl acetate =100/0 - 60/40) to give benzyl 6-acetyl-3,4-dihydro-1H-isoquinoline-2-carboxylate (67.1 mg; yield 8%) as a colorless viscous oil.
   ¹H-NMR (400 MHz, CDCl₃) δ ppm: 2.58(3H, s),2.91(2H, br-s), 3.75(2H, t), 4.70(2H, s),5.19(2H, s), 7.20(1H, br-s), 7.29-7.45(5H, m), 7.74(1H, s), 7.77(1H, d).
(2) To a solution of tert-butyl 2-diethoxyphosphoryl acetate (109 mg) in THF (1.4 ml) was added sodium hydride (60%, 17 mg). To the reaction mixture was added a solution of benzyl 6-acetyl-3,4-dihydro-1H-isoquinoline-2-carboxylate (67.1 mg) obtained in the aforementioned (1) in THF, and the mixture was stirred at room temperature for 18 hr. Water was added to the reaction mixture, and the organic layer was extracted with ethyl acetate. The combined organic layer was washed with saturated brine, dried over anhydrous magnesium sulfate, and concentrated under reduced pressure. The obtained residue was purified by silica gel column chromatography (solvent: n-hexane/ethyl acetate =100/0 - 70/30) to give benzyl 6-[4-[(2-methylpropan-2-yl)oxy]-4-oxobut-2-en-2-yl]-3,4-dihydro-1H-isoquinoline-2-carboxylate (68.5 mg; yield 78%) as a colorless viscous oil.
   ¹H-NMR (400 MHz, CDCl₃) δ ppm: 1.52(9H, s),2.51(3H, d), 2.86(2H, br-s), 3.73(2H, br-s), 4.66(2H, s), 5.18(2H, s), 6.03(1H, d), 6.94-7.16(2H, m), 7.20-7.43(6H, m). (*regio isomeric mixture of about 3:1 of NMR integration ratio)
(3) To a solution of benzyl 6-[4-[(2-methylpropan-2-yl)oxy]-4-oxobut-2-en-2-yl]-3,4-dihydro-1H-isoquinoline-2-carboxylate (68.5 mg) obtained in the aforementioned (2) in ethanol (1 ml) was added palladium(II) hydroxide (8 mg), and the mixture was stirred under hydrogen atmosphere at room temperature for 3 hr. The reaction mixture was filtered through celite, and the filtrate was concentrated under reduced pressure to give a crude product (46.3 mg) of the title compound as a colorless viscous oil.

### Reference Example 27:

### Production of tert-butyl 1-(1,2,3,4-tetrahydroisoquinolin-6-yl)cyclopropane-1-carboxylate

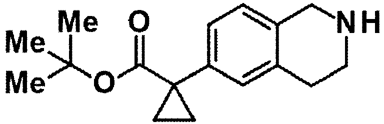

(1) A mixture of benzyl 6-(4,4,5,5-tetramethyl-1,3,2-dioxaborolan-2-yl)-3,4-dihydro-1H-isoquinoline-2-carboxylate (2.2 g), tert-butyl 2-bromoacrylate (1.1 g), bis(di-tert-butyl(4-dimethylaminophenyl)phosphine)dichloropalladium(II) (190 mg), cesium carbonate (3.5 g), 1,4-dioxane (20 ml) and water (4 ml) was stirred in a microwave reactor at 140°C for 1 hr. After cooling to room temperature, the reaction mixture was filtered and concentrated under reduced pressure. The residue was purified by silica gel column chromatography (solvent: n-hexane/ethyl acetate =100/0 - 70/30) to give benzyl 6-[3-[(2-methylpropan-2-yl)oxy]-3-oxoprop-1-en-2-yl]-3,4-dihydro-1H-isoquinoline-2-carboxylate (725 mg; yield 31%) as a brown oil. MS(ESI) m/z: 338.1[M-C₄H₈+H]⁺
(2) To a DMSO solution (6 ml) of trimethylsulfoxonium iodide (810 mg) was added 60% sodium hydride (148 mg) at 0°C, and the mixture was stirred for 10 min. A DMSO solution of benzyl 6-[3-[(2-methylpropan-2-yl)oxy]-3-oxoprop-1-en-2-yl]-3,4-dihydro-1H-isoquinoline-2-carboxylate (725 mg; 89 mass%) obtained in the aforementioned (1) was added thereto, and the mixture was stirred at room temperature for 18 hr. Water was added to the reaction mixture, and the mixture was extracted with ethyl acetate. The organic layer was washed with saturated brine, dried over anhydrous magnesium sulfate, and concentrated under reduced pressure. The obtained residue was purified by silica gel column chromatography (solvent: n-hexane/ethyl acetate =100/0 - 70/30) to give benzyl 6-[1-[(2-methylpropan-2-yl)oxycarbonyl]cyclopropyl]-3,4-dihydro-1H-isoquinoline-2-carboxylate (454 mg; yield 55%) as a yellow oil.
   ¹H-NMR (400 MHz, CDCl₃) δ ppm: 1.08-1.10(2H,m),1.38(9H,s),1.47-1.51(2H,m),3.71(2H,br),2.83(2H,br),4.62(2H,s),5.17(2H,s),7.00-7.15(3H,m),7.30-7.39(5H,m).
(3) To benzyl 6-[1-[(2-methylpropan-2-yl)oxycarbonyl]cyclopropyl]-3,4-dihydro-1H-isoquinoline-2-carboxylate (454 mg) obtained in the aforementioned (2) were added ethanol (4 ml) and 20% palladium hydroxide (45 mg), and the mixture was stirred under hydrogen atmosphere at room temperature for 3 hr. The reaction mixture was filtered through celite, and the filtrate was concentrated under reduced pressure to give a crude product (277 mg) of the title compound as a colorless oil.

### Reference Example 28:

### Production of tert-butyl 2-methyl-2-(1,2,3,4-tetrahydroisoquinolin-6-yl)propanoate

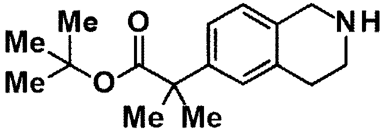

(1) 60% Sodium hydride (25.6 mg) was added to DMSO (1.6 ml). To the reaction mixture were added methyl iodide (90 mg) and a solution of benzyl 6-[2-[(2-methylpropan-2-yl)oxy]-2-oxoethyl]-3,4-dihydro-1H-isoquinoline-2-carboxylate (130 mg) in THF (1.6 ml) at 0°C, and the reaction mixture was stirred at room temperature for 4 hr. Water was added to the reaction mixture, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =100/0 - 50/50) to give benzyl 6-[1-[(2-methylpropan-2-yl)oxy]-1-oxopropan-2-yl]-3,4-dihydro-1H-isoquinoline-2-carboxylate (79.7 mg; yield 52%) as a colorless oil.
   MS(ESI) m/z: 340.1[M-C₄H₈+H]⁺
(2) 60% Sodium hydride (26 mg) was added to DMSO (1.2 ml). To the reaction mixture were added methyl iodide (91 mg) and a solution of benzyl 6-[1-[(2-methylpropan-2-yl)oxy]-1-oxopropan-2-yl]-3,4-dihydro-1H-isoquinoline-2-carboxylate (79.7 mg) obtained in the aforementioned (1) in THF (1.2 ml) at 0°C, and the reaction mixture was stirred at room temperature for 18 hr. Water was added to the reaction mixture, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =100/0 - 85/15) to give benzyl 6-[2-methyl-1-[(2-methylpropan-2-yl)oxy]-1-oxopropan-2-yl]-3,4-dihydro-1H-isoquinoline-2-carboxylate (22.4 mg; yield 33%) as a colorless oil.
   MS(ESI) m/z: 354.2[M-C₄H₈+H]⁺
(3) To benzyl 6-[2-methyl-1-[(2-methylpropan-2-yl)oxy]-1-oxopropan-2-yl]-3,4-dihydro-1H-isoquinoline-2-carboxylate (22.4 mg) obtained in the aforementioned (2) were added ethanol (0.6 ml) and 20% palladium hydroxide (4 mg), and the mixture was stirred under hydrogen atmosphere at room temperature for 3 hr. The reaction mixture was filtered through celite and concentrated under reduced pressure to give a crude product (15.1 mg) of the title compound as a colorless oil.

### Reference Example 29:

### Production of tert-butyl 4-(1,2,3,4-tetrahydroisoquinolin-6-yl)butanoate

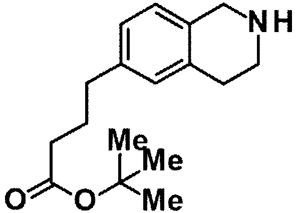

(1) To a solution of benzyl 6-bromo-3,4-dihydro-1H-isoquinoline-2-carboxylate (1 g) in 1,4-dioxane (10 ml) were added [1,3-bis(2,6-diisopropylphenyl)imidazol-2-ylidene] (3-chloropyridyl)palladium(II) dichloride (197 mg) and 4-ethoxy-4-oxobutylzinc bromide (1 mol/l, 3.5 ml), and the mixture was stirred nitrogen atmosphere at room temperature for 3 hr. Water was added to the reaction mixture, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =100/0 - 0/100) to give benzyl 6-(4-ethoxy-4-oxobutyl)-3,4-dihydro-1H-isoquinoline-2-carboxylate (183 mg; yield 17%) as a yellow oil. ¹H-NMR (400 MHz, CDCl₃) δ ppm: 1.25(3H,t),1.89-1.94(2H,m),2.29-2.33(2H,m),2.60-2.61(2H,m), 2.82(2H,br), 3.72(2H,br), 4.13(2H,q), 4.62(2H,s), 5.18(2H,s), 6.95-7.00(3H,m), 7.30-7.40(5H,m).
(2) To a reaction mixture of benzyl 6-(4-ethoxy-4-oxobutyl)-3,4-dihydro-1H-isoquinoline-2-carboxylate (183 mg) obtained in the aforementioned (1) and methanol (1.6 ml) was added 4N aqueous lithium hydroxide solution (0.48 ml), and the mixture was stirred at room temperature for 12 hr. The reaction mixture was neutralized with 1N hydrochloric acid and extracted with ethyl acetate. The organic layer was washed with saturated brine, dried over anhydrous magnesium sulfate, and concentrated under reduced pressure to give a crude product (159 mg) of 4-(2-phenylmethoxycarbonyl-3,4-dihydro-1H-isoquinolin-6-yl)butanoic acid as a yellow oil.
(3) To a mixed solution of the crude product (159 mg) of 4-(2-phenylmethoxycarbonyl-3,4-dihydro-1H-isoquinolin-6-yl)butanoic acid obtained in the aforementioned (2) and toluene (1.5 ml) was added N,N-dimethylformamide di-tert-butylacetal (0.3 ml), and the mixture was stirred at 120°C for 1 hr. The reaction mixture was cooled to room temperature, and concentrated under reduced pressure. The residue was purified by silica gel column chromatography (solvent: n-hexane/ethyl acetate =100/0 - 70/30) to give benzyl 6-[4-[(2-methylpropan-2-yl)oxy]-4-oxobutyl]-3,4-dihydro-1H-isoquinoline-2-carboxylate (57 mg; yield 31%) as a colorless oil.
   ¹H-NMR (400 MHz, CDCl₃) δ ppm: 1.44(9H,s), 1.84-1.92(2H,m), 2.21-2.24(2H,m),2.56-1.61(2H,m), 2.82(2H,br), 3.71(2H,br), 4.62(2H,s), 5.18(2H,s), 6.95-7.03(3H,m), 7.36-7.40(5H,m).
(4) To benzyl 6-[4-[(2-methylpropan-2-yl)oxy]-4-oxobutyl]-3,4-dihydro-1H-isoquinoline-2-carboxylate (57 mg) obtained in the aforementioned (3) were added ethanol (0.9 ml) and 20% palladium hydroxide (4 mg), and the mixture was stirred under hydrogen atmosphere at room temperature for 3 hr. The reaction mixture was filtered through celite and concentrated under reduced pressure to give a crude product (39 mg) of the title compound as a colorless oil.

### Reference Example 30:

### Production of tert-butyl 2-(1,2,3,4-tetrahydroisoquinolin-6-yl)propanoate

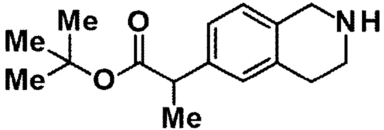

To benzyl 6-[1-[(2-methylpropan-2-yl)oxy]-1-oxopropan-2-yl]-3,4-dihydro-1H-isoquinoline-2-carboxylate (85.4 mg) obtained in Reference Example 28, (1), were added ethanol (1.4 ml) and 20% palladium hydroxide (10 mg), and the mixture was stirred under hydrogen atmosphere at room temperature for 3 hr. The reaction mixture was filtered through celite, and the filtrate was concentrated under reduced pressure to give a crude product (56.4 mg) of the title compound as a yellow oil.

### Reference Example 31:

### Production of tert-butyl rac-(1R,2R)-2-(1,2,3,4-tetrahydroisoquinolin-6-yl)cyclopropane-1-carboxylate

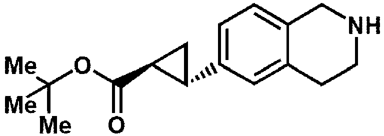

(1) A mixture of benzyl 6-bromo-3,4-dihydro-1H-isoquinoline-2-carboxylate (1.5 g), 4,4,5,5-tetramethyl-2-vinyl-1,3,2-dioxaborolane (800 mg), bis(di-tert-butyl(4-dimethylaminophenyl)phosphine)dichloropalladium(II) (155 mg), cesium carbonate (2.8 g), 1,4-dioxane (14 ml) and water (2 ml) was stirred in a microwave reactor at 140°C for 1 hr. After cooling to room temperature, the reaction solution was concentrated under reduced pressure. The residue was purified by silica gel column chromatography (solvent: n-hexane/ethyl acetate =100/0 - 80/20) to give benzyl 6-ethenyl-3,4-dihydro-1H-isoquinoline-2-carboxylate (734 mg; yield 55%) as a colorless oil.
   MS(ESI) m/z: 294.1[M+H]⁺
(2) Benzyl 6-ethenyl-3,4-dihydro-1H-isoquinoline-2-carboxylate (734 mg) obtained in the aforementioned (1) was dissolved in 1,4-dioxane (8 ml), sodium periodate (1 g), 2,6-lutidine (0.56 ml) and 2.5 wt% osmium tetraoxide-tert-butanol solution (2.4 ml) were added and the mixture was stirred at room temperature for 18 hr. The reaction solution was filtered and concentrated under reduced pressure. The residue was purified by silica gel column chromatography (solvent: n-hexane/ethyl acetate =100/0 - 70/30) to give benzyl 6-formyl-3,4-dihydro-1H-isoquinoline-2-carboxylate (565 mg; yield 74%) as a colorless oil.
   MS(ESI) m/z: 296.2[M+H]⁺
(3) A reaction mixture of benzyl 6-formyl-3,4-dihydro-1H-isoquinoline-2-carboxylate (1.06 g) obtained in the aforementioned (2), tert-butyl diethoxyphosphoryl acetate (1.8 g), 1,1,3,3-tetramethylguanidine (830 mg) and THF (11 ml) was stirred at room temperature for 12 hr. The reaction solution was concentrated under reduced pressure. The residue was purified by silica gel column chromatography (solvent: n-hexane/ethyl acetate =100/0 - 70/30) to give benzyl 6-[(E)-3-[(2-methylpropan-2-yl)oxy]-3-oxoprop-1-enyl]-3,4-dihydro-1H-isoquinoline-2-carboxylate (698 mg; yield 46%) as a white solid. MS(ESI) m/z: 392.4[M-H]⁻
(4) To a DMSO solution (12 ml) of trimethylsulfoxonium iodide (1.6 g) was added 60% sodium hydride (288 mg) at 0°C, and the mixture was stirred for 10 min. To the reaction mixture was added a DMSO solution of benzyl 6-[(E)-3-[(2-methylpropan-2-yl)oxy]-3-oxoprop-1-enyl]-3,4-dihydro-1H-isoquinoline-2-carboxylate (698 mg) obtained in the aforementioned (3), and the mixture was stirred at room temperature for 18 hr. Water was added to the reaction mixture, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =100/0 - 70/30) to give benzyl 6-[rac-(1R,2R)-2-[(2-methylpropan-2-yl)oxycarbonyl]cyclopropyl]-3,4-dihydro-1H-isoquinoline-2-carboxylate (129 mg; yield 19%) as a colorless oil.
   ¹H-NMR (400 MHz, CDCl₃) δ ppm: 1.18-1.21(1H,m),1.46(9H.s),1.47-1.50(1H,m),1.80-1.82(1H,m), 2.38-2.41(1H,m), 2.81(br, 1H), 3.70(1H,br), 4.61(2H,s), 5.17(2H,s), 6.87-7.02(3H,m), 7.33-7.39(5H,m).
(5) To benzyl 6-[rac-(1R,2R)-2-[(2-methylpropan-2-yl)oxycarbonyl]cyclopropyl]-3,4-dihydro-1H-isoquinoline-2-carboxylate (129 mg) obtained in the aforementioned (4) were added ethanol (1 ml) and 20% palladium hydroxide (12 mg), and the mixture was stirred under hydrogen atmosphere at room temperature for 3 hr. The reaction mixture was filtered through celite, and the filtrate was concentrated under reduced pressure to give the title compound (107 mg; yield 97%) as a gray oil. MS(ESI) m/z: 274.3[M+H]⁺

### Reference Example 32:

### Production of tert-butyl 1-(5,6,7,8-tetrahydro-2,7-naphthyridin-3-yl)piperidine-4-carboxylate

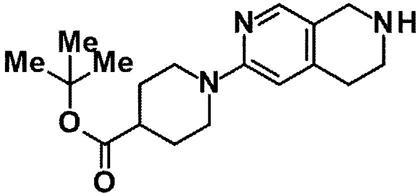

(1) A mixture of benzyl 6-chloro-3,4-dihydro-1H-2,7-naphthyridine-2-carboxylate (300 mg), DMSO (3 ml), diisopropylethylamine (0.33 ml) and tert-butyl piperidine-4-carboxylate hydrochloride (250 mg) was stirred in a microwave reactor at 180°C for 1 hr. After cooling to room temperature, water was added to the reaction mixture, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =100/0 - 40/60) to give benzyl 6-[4-[(2-methylpropan-2-yl)oxycarbonyl]piperidin-1-yl]-3,4-dihydro-1H-2,7-naphthyridine-2-carboxylate (117 mg; yield 22%) as a yellow oil.
   MS(ESI) m/z: 452.3[M+H]⁺
(2) To benzyl 6-[4-[(2-methylpropan-2-yl)oxycarbonyl]piperidin-1-yl]-3,4-dihydro-1H-2,7-naphthyridine-2-carboxylate (117 mg) obtained in the aforementioned (1) were added ethanol (1 ml) and 20% palladium hydroxide (12 mg), and the mixture was stirred under hydrogen atmosphere at room temperature for 3 hr. The reaction mixture was filtered through celite, and the filtrate was concentrated under reduced pressure to give a crude product (65 mg) of the title compound as a yellow solid.

### Reference Example 33:

### Production of tert-butyl 3-(1,2,3,4-tetrahydroisoquinolin-6-yl)propanoate

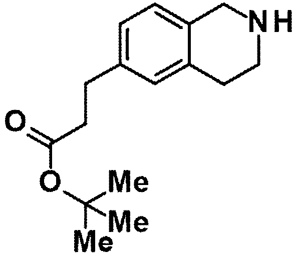

(1) 6-Bromo-1,2,3,4-tetrahydroisoquinoline (12 g) was dissolved in pyridine (47 ml), N,N-dimethylpyridine-4-amine (172 mg), trifluoroacetic anhydride (6 g) were added, and the mixture was stirred at room temperature for 18 hr. The reaction mixture was concentrated, ethyl acetate was added to the residue and the mixture was washed with 1N hydrochloric acid. The organic layer was dried and concentrated, and the residue was purified by silica gel column chromatography (solvent: n-hexane/ethyl acetate =100/0 - 70/30) to give 1-(6-bromo-3,4-dihydro-1H-isoquinolin-2-yl)-2,2,2-trifluoroethanone (11.8 g; yield 68%) as a white solid.
   MS(ESI) m/z: 308.0/310.0[M+H]⁺
(2) 1-(6-Bromo-3,4-dihydro-1H-isoquinolin-2-yl)-2,2,2-trifluoroethanone (2.4 g) obtained in the aforementioned (1) was dissolved in 1,4-dioxane (16 ml), water (4 ml), 4,4,5,5-tetramethyl-2-vinyl-1,3,2-dioxaborolane (1.16 g), bis[di-tert-butyl (4-dimethylaminophenyl)phosphine]dichloropalladium (II) (178 mg) and cesium carbonate (3.3 g) were mixed therewith, and stirred using a microwave reactor at 140°C for 1 hr. The reaction solution was filtered, and the filtrate was concentrated. The residue was purified by silica gel column chromatography (solvent: n-hexane/ethyl acetate =100/0 - 80/20) to give 1-(6-ethenyl-3,4-dihydro-1H-isoquinolin-2-yl)-2,2,2-trifluoroethanone (903 mg; 44%) as a white solid.
   MS(ESI) m/z: 256.2[M+H]⁺
(3) 1-(6-Ethenyl-3,4-dihydro-1H-isoquinolin-2-yl)-2,2,2-trifluoroethanone (903 mg) obtained in the aforementioned (2) was dissolved in 1,4-dioxane (12 ml), water (3 ml), 2,6-lutidine (0.78 ml), 2.5 wt% osmium tetraoxide-tert-butanol solution (3.5 ml) and sodium periodate (1.4 g) were added, and the mixture was stirred at room temperature for 18 hr. The reaction solution was filtered, and the filtrate was concentrated. The residue was purified by silica gel column chromatography (solvent: n-hexane/ethyl acetate =80/20 - 50/50) to give 2-(2,2,2-trifluoroacetyl)-3,4-dihydro-1H-isoquinoline -6-carbaldehyde (573 mg; 66%) as a white solid.
   MS(ESI) m/z: 258.0[M+H]⁺
(4) 2-(2,2,2-Trifluoroacetyl)-3,4-dihydro-1H-isoquinoline-6-carbaldehyde (573 mg) obtained in the aforementioned (3) was dissolved in THF (7 ml), 1,1,3,3-tetramethylguanidine (513 mg) and tert-butyl 2-diethoxyphosphoryl acetate (843 mg) were added thereto, and the mixture was stirred at room temperature for 3 hr. The reaction solution was concentrated, and the residue was purified by silica gel column chromatography (solvent: n-hexane/ethyl acetate =100/0 - 70/30) to give tert-butyl (E)-3-[2-(2,2,2-trifluoroacetyl)-3,4-dihydro-1H-isoquinolin-6-yl]prop-2-enoate (544 mg; 63%) as a colorless oil.
   ¹H-NMR (400 MHz, CDCl₃) δ ppm: 1.56(9H,s), 2.99(2H,t), 3.88(2H,t), 4.82(2H,s),6.38(1H,d), 7.19(1H,d), 7.32(1H,s), 7.41(1H,d), 7.56(1H,d). (*rotameric mixture of about 2:1 of NMR integration ratio)
(5) tert-Butyl (E)-3-[2-(2,2,2-trifluoroacetyl)-3,4-dihydro-1H-isoquinolin-6-yl]prop-2-enoate (544 mg) obtained in the aforementioned (4) was dissolved in ethanol (6 ml), palladium hydroxide-activated carbon (55 mg) was added, and the mixture was stirred under hydrogen atmosphere at room temperature for 3 hr. The reaction solution was filtered through membrane filter, and the filtrate was concentrated under reduced pressure, a crude product (527 mg) of tert-butyl 3-[2-(2,2,2-trifluoroacetyl)-3,4-dihydro-1H-isoquinolin-6-yl]propanoate as a colorless oil.
   ¹H-NMR (400 MHz, CDCl₃) δ ppm: 1.35(9H,s), 2.45(2H,t), 2.80(2H,t), 2.85(2H,t), 3.76(2H,t), 4.69(2H,s), 7.01-6.92(3H,m). (*rotameric mixture of about 2:1 of NMR integration ratio)
(6) The crude product (527 mg) of tert-Butyl 3-[2-(2,2,2-trifluoroacetyl)-3,4-dihydro-1H-isoquinolin-6-yl]propanoate obtained in the aforementioned (5) was dissolved in ethanol (5 ml), aqueous sodium hydroxide solution (4 mol/l, 1.5 ml) was added, and the mixture was stirred at room temperature for 3 hr. Water was added to the reaction mixture, and the mixture was extracted with ethyl acetate. The organic layer was washed with saturated brine, dried, and concentrated to give a crude product (377 mg) of the title compound as a colorless oil.

### Reference Example 34:

### Production of tert-butyl 2-[(8-bromo-1,2,3,4-tetrahydroisoquinolin-6-yl)oxy]acetate

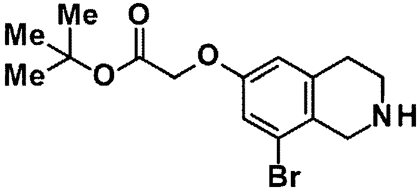

(1) A mixture of 8-bromo-6-methoxy-1,2,3,4-tetrahydroisoquinoline (180 mg) and 50% hydrobromic acid (4 ml) was stirred at 130°C for 8 hr. After cooling to room temperature, the reaction mixture was concentrated under reduced pressure. To a mixture of the obtained residue, 1,4-dioxane (1 ml) and water (1 ml) were added 1N aqueous sodium hydroxide solution (2 ml) and benzyl chloroformate (0.15 ml) under ice bath cooling, and the mixture was stirred for 1 hr. To the reaction mixture was added saturated aqueous ammonium chloride solution, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =90/10 - 50/50) to give benzyl 8-bromo-6-hydroxy-3,4-dihydro-1H-isoquinoline-2-carboxylate (53 mg; yield 23%) as a colorless oil.
   MS(ESI) m/z: 360.1/362.1[M-H]⁻
(2) To a mixture of benzyl 8-bromo-6-hydroxy-3,4-dihydro-1H-isoquinoline-2-carboxylate (53 mg) obtained in the aforementioned (1), DMF (0.5 ml) and THF (0.1 ml) was added 60% sodium hydride (9 mg) under ice bath cooling, and the mixture was stirred for 5 min. The mixture was warmed to room temperature, tert-butyl bromoacetate (47 mg) was added, and the mixture was stirred for 1 hr. To the reaction mixture was added saturated aqueous ammonium chloride solution, and the mixture was extracted with chloroform. The organic layer was washed with saturated brine, dried over anhydrous magnesium sulfate, and concentrated under reduced pressure. The residue was purified by silica gel column chromatography (solvent: n-hexane/ethyl acetate =90/10 - 70/30) to give benzyl 8-bromo-6-[2-[(2-methylpropan-2-yl)oxy]-2-oxoethoxy]-3,4-dihydro-1H-isoquinoline-2-carboxylate (64 mg; yield 92%) as a colorless oil. MS(ESI) m/z: 493.0/495.0 [M+NH₄]⁺
(3) To a mixture of palladium acetate (7 mg) and dichloromethane (1.0 ml) were added triethylamine (0.02 ml), triethylsilane (0.032 ml) and benzyl 8-bromo-6-[2-[(2-methylpropan-2-yl)oxy]-2-oxoethoxy]-3,4-dihydro-1H-isoquinoline-2-carboxylate (64 mg) obtained in the aforementioned (2) under nitrogen atmosphere, and the mixture was stirred at room temperature for 30 min. The reaction mixture was filtered through celite, and the filtrate was concentrated under reduced pressure to give a crude product of tert-butyl 2-[(8-bromo-1,2,3,4-tetrahydroisoquinolin-6-yl)oxy]acetate as a brown oil.
   MS(ESI) m/z: 342.1/344.1 [M+H]⁺

### Reference Example 35:

### Production of tert-butyl 2-[(8-fluoro-1,2,3,4-tetrahydroisoquinolin-6-yl)oxy]-2-methylpropanoate

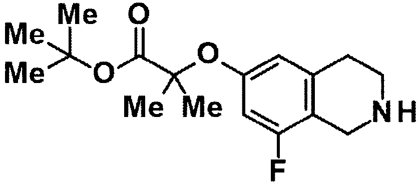

(1) Benzyl 8-fluoro-6-hydroxy-3,4-dihydro-1H-isoquinoline-2-carboxylate (101 mg) was dissolved in DMF (1 ml), potassium carbonate (52 mg) and tert-butyl 2-bromo-2-methylpropanoate (0.064 ml) were added, and the reaction mixture was stirred at room temperature for 5 hr. To the reaction mixture were added potassium carbonate (49 mg) and tert-butyl 2-bromo-2-methylpropanoate (0.064 ml), and the mixture was stirred at room temperature for 15 hr 30 min, at 50°C for 7 hr, and further at 80°C for 1 hr. To the reaction mixture were added potassium carbonate (102 mg) and tert-butyl 2-bromo-2-methylpropanoate (0.128 ml), and the mixture was stirred at 80°C for 15 hr 30 min. The reaction mixture was cooled to room temperature, water was added and the mixture was extracted with ethyl acetate. The organic layer was washed with 2-fold diluted saturated brine, filtered by Phase-separator (manufactured by Biotage AB), and the filtrate was concentrated under reduced pressure. The obtained residue was purified by silica gel column chromatography (solvent: n-hexane/ethyl acetate =91/9 - 70/30) to give benzyl 8-fluoro-6-[2-methyl-1-[(2-methylpropan-2-yl)oxy]-1-oxopropan-2-yl]oxy-3,4-dihydro-1H-isoquinoline-2-carboxylate (97.2 mg; yield 69.8%) as a colorless oil.
   MS(ESI) m/z: 444.4[M+H]⁺
(2) Benzyl 8-fluoro-6-[2-methyl-1-[(2-methylpropan-2-yl)oxy]-1-oxopropan-2-yl]oxy-3,4-dihydro-1H-isoquinoline-2-carboxylate (97 mg) obtained in the aforementioned (1) was dissolved in ethanol (1 ml), palladium-carbon (10%, 50 mg) was added, and the mixture was stirred under hydrogen atmosphere at room temperature for 2 hr 30 min. After nitrogen replacement, the reaction mixture was filtered through a membrane filter, and the filtrate was concentrated under reduced pressure and dried under vacuum to give the title compound (65.2 mg; yield 96.2%) as a colorless oil.
   MS(ESI) m/z: 310.3[M+H]⁺

### Reference Example 36:

The corresponding starting compounds were treated in the same manner as in Reference Example 35 to obtain a crude product of the compound described in the following Table 2.

**[Table 2]**

| **Ref. Ex.No.** | **structural formula** |
|---|---|
| 36 | 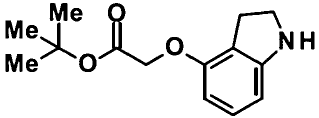 |

### Reference Example 37:

### Production of tert-butyl 3-[(8-fluoro-1,2,3,4-tetrahydroisoquinolin-6-yl)oxy]-2,2-dimethylpropanoate

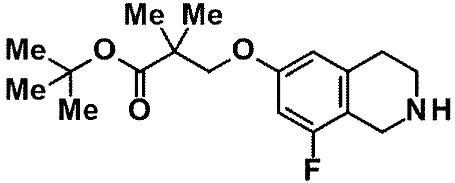

(1) Benzyl 8-fluoro-6-hydroxy-3,4-dihydro-1H-isoquinoline-2-carboxylate (108 mg) was dissolved in THF (1 ml), triphenyl phosphine (117 mg), tert-butyl 3-hydroxy-2,2-dimethyl-propanoate (115 mg) and diethyl azodicarboxylate (2.2 mol/l toluene solution, 0.31 ml) were added thereto, and the reaction mixture was stirred at 70°C for 21 hr. Thereafter, and the mixture was stirred for 4 days while allowing to cool to room temperature. The reaction mixture was concentrated under reduced pressure, and the residue was diluted with diisopropyl ether. The precipitate was filtered, and the filtrate was concentrated under reduced pressure. The obtained residue was purified by silica gel column chromatography (solvent: n-hexane/ethyl acetate =95/5 - 75/25) to give benzyl 6-[2,2-dimethyl-3-[(2-methylpropan-2-yl)oxy]-3-oxopropoxy]-8-fluoro-3,4-dihydro-1H-isoquinoline-2-carboxylate (85.8 mg; yield 44.4%) as a colorless oil.
   MS(ESI) m/z: 402.3[M-C₄H₈+H]⁺
(2) Benzyl 6-[2,2-dimethyl-3-[(2-methylpropan-2-yl)oxy]-3-oxopropoxy]-8-fluoro-3,4-dihydro-1H-isoquinoline-2-carboxylate (85.8 mg) obtained in the aforementioned (1) was dissolved in ethanol (2 ml), palladium-carbon (10%, 23 mg) was added, and the mixture was stirred under hydrogen atmosphere at room temperature for 30 min. After nitrogen replacement, the reaction mixture was filtered through a membrane filter, and the filtrate was concentrated under reduced pressure and dried under vacuum to give the title compound (57.1 mg; yield 104%) as a colorless oil.
   MS(ESI) m/z: 324.3[M+H]⁺

### Reference Example 38:

### Production of tert-butyl 3-(4,5,6,7-tetrahydropyrazolo[1,5-a]pyrazin-2-yl)propanoate

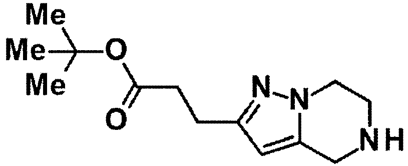

(1) To a mixture of ethyl 3-(4,5,6,7-tetrahydropyrazolo[1,5-a]pyrazin-2-yl)propanoate (1.0 g) and dichloromethane (13 ml) were added triethylamine (1.8 ml) and benzyl chloroformate (0.6 ml), and the mixture was stirred at room temperature for 3 hr. To the reaction mixture were added saturated aqueous ammonium chloride solution, and the mixture was extracted with chloroform. The organic layer was dried over anhydrous magnesium sulfate, and concentrated under reduced pressure to give benzyl 2-(3-ethoxy-3-oxopropyl)-6,7-dihydro-4H-pyrazolo[1,5-a]pyrazine-5-carboxylate (1.4 g; yield 102%) as a white solid.
   MS(ESI) m/z: 358.2[M+H]⁺
(2) To a mixture of benzyl 2-(3-ethoxy-3-oxopropyl)-6,7-dihydro-4H-pyrazolo[1,5-a]pyrazine-5-carboxylate (1.4 g) obtained in the aforementioned (1) and ethanol (13 ml) was added 4N aqueous sodium hydroxide solution (1.6 ml), and the mixture was stirred at 50°C for 1 hr. The reaction mixture was neutralized with 1N hydrochloric acid and extracted with ethyl acetate. The organic layer was washed with saturated brine, dried over anhydrous magnesium sulfate, and concentrated under reduced pressure to give 3-(5-phenylmethoxycarbonyl-6,7-dihydro-4H-pyrazolo[1,5-a]pyrazin-2-yl)propanoic acid (940 mg; yield 73%) as a colorless oil.
   MS(ESI) m/z: 328.1[M-H]⁻
(3) To a mixed solution of 3-(5-phenylmethoxycarbonyl-6,7-dihydro-4H-pyrazolo[1,5-a]pyrazin-2-yl)propanoic acid (940 mg) obtained in the aforementioned (2) and toluene (10 ml) was added N,N-dimethylformamide di-tert-butylacetal (2.2 ml), and the mixture was stirred under nitrogen atmosphere at 120°C for 30 min. The reaction mixture was cooled to room temperature and concentrated under reduced pressure. The residue was purified by silica gel column chromatography (solvent: n-hexane/ethyl acetate =60/40 - 40/60) to give benzyl 2-[3-[(2-methylpropan-2-yl)oxy]-3-oxopropyl]-6,7-dihydro-4H-pyrazolo[1,5-a]pyrazine-5-carboxylate (611 mg; yield 56%) as a colorless oil.
   MS(ESI) m/z: 386.1[M+H]⁺
(4) To a mixture of benzyl 2-[3-[(2-methylpropan-2-yl)oxy]-3-oxopropyl]-6,7-dihydro-4H-pyrazolo[1,5-a]pyrazine-5-carboxylate (95 mg) obtained in the aforementioned (3) and methanol (1 ml) was added 20% palladium hydroxide (15 mg), and the mixture was stirred under hydrogen atmosphere at 60°C for 2 hr. The reaction mixture was filtered through celite, and the filtrate was concentrated under reduced pressure to give a crude product (62 mg) of the title compound as a gray oil.

### Reference Example 39:

Production of methyl 2-(4,5,6,7-tetrahydro-[1,3]thiazolo[5,4-c]pyridin-2-yl)acetate trifluoroacetate 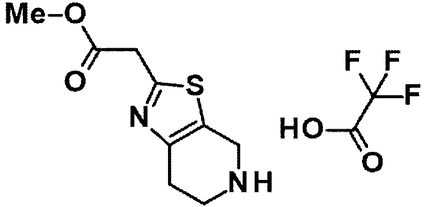
(1) To a mixed solution of tert-butyl 4-oxopiperidine-1-carboxylate (15 g) and toluene (70 ml) were added pyrrolidine (6.6 L) and p-toluenesulfonic acid monohydrate (5 mg), and the mixture was heated under reflux for 8 hr using a Dean-Stark apparatus. The reaction mixture was cooled to room temperature and concentrated under reduced pressure. Methanol (30 ml) and sulfur (2.41 g) were added to the residue, and a solution of cyanamide (3.17 g) in methanol (10 ml) was added dropwise under ice bath cooling. After completion of adding dropwise, and the mixture was stirred under ice bath cooling for 5 hr. The precipitated solid was collected by filtration, washed with ethyl acetate, and dried under reduced pressure to give tert-butyl 2-amino-6,7-dihydro-4H-[1,3]thiazolo[5,4-c]pyridine-5-carboxylate (11.6 g; yield 60%) as a pale yellow solid.
   MS(APCI)m/z:256.1[M+H]⁺
(2) To a mixture of copper(II) bromide (11.9 g) and DMF (40 ml) was added hexyl nitrite (6.83 g), and the mixture was stirred at 50°C for 5 min. To the reaction mixture was added tert-butyl 2-amino-6,7-dihydro-4H-[1,3]thiazolo[5,4-c]pyridine-5-carboxylate (11.3 g) obtained in the aforementioned (1), and the mixture was stirred at 50°C for 2 hr. The reaction mixture was cooled to room temperature and concentrated under reduced pressure. The residue was purified by silica gel column chromatography (solvent: n-hexane/ethyl acetate =83/17) to give tert-butyl 2-bromo-6,7-dihydro-4H-[1,3]thiazolo[5,4-c]pyridine-5-carboxylate (4.1 g; yield 24%) as a pale yellow solid.
   MS(APCI)m/z:319.0/321.0[M+H]⁺
(3) A mixture of tert-butyl 2-bromo-6,7-dihydro-4H-[1,3]thiazolo[5,4-c]pyridine-5-carboxylate (150 mg) obtained in the aforementioned (2), 1-(tert-butyldimethylsilyloxy)-1-methoxyethene (266 mg), potassium carbonate (130 mg), bis(tri-tert-butylphosphine)palladium(0) (72 mg) and DMF (1 ml) was stirred in a microwave reactor at 120°C for 1 hr. The reaction mixture was cooled to room temperature, saturated aqueous ammonium chloride solution was added, and the mixture was extracted with ethyl acetate. The organic layer was washed with saturated brine, dried over anhydrous magnesium sulfate, and concentrated under reduced pressure. The residue was purified by reversed-phase HPLC (10 mmol/l aqueous ammonium carbonate solution/acetonitrile =60/40 - 30/70) to give tert-butyl 2-(2-methoxy-2-oxoethyl)-6,7-dihydro-4H-[1,3]thiazolo[5,4-c]pyridine-5-carboxylate (36 mg; yield 25%) as a yellow amorphous.
   MS(ESI) m/z: 313.2[M+H]⁺
(4) To a mixture of tert-butyl 2-(2-methoxy-2-oxoethyl)-6,7-dihydro-4H-[1,3]thiazolo[5,4-c]pyridine-5-carboxylate (34 mg) obtained in the aforementioned (3) and dichloromethane (0.5 ml) was added trifluoroacetic acid (0.1 ml), and the mixture was stirred at room temperature for 1 hr. The reaction mixture was concentrated under reduced pressure to give a crude product (36 mg) of the title compound as a colorless amorphous.

### Reference Example 40:

### Production of methyl 2-(8-cyano-1,2,3,4-tetrahydroisoquinolin-6-yl)acetate trifluoroacetate

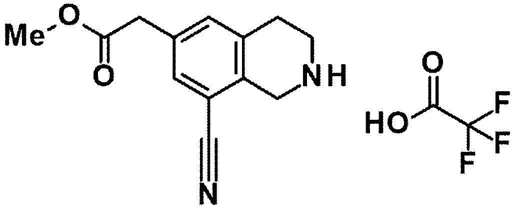

(1) To a mixture of 6-bromo-8-chloro-1,2,3,4-tetrahydroisoquinoline (58 mg) and dichloromethane (1.5 ml) were added di-tert-butyl dicarbonate (77 mg) and triethylamine (0.05 ml), and the mixture was stirred at room temperature for 3 hr. Water was added to the reaction mixture, and the mixture was extracted with chloroform. The organic layer was dried over anhydrous magnesium sulfate, and concentrated under reduced pressure. The residue was purified by reversed-phase HPLC (10 mmol/l aqueous ammonium carbonate solution/acetonitrile =30/70 - 0/100) to give tert-butyl 6-bromo-8-chloro-3,4-dihydro-1H-isoquinoline-2-carboxylate (63 mg; yield 77%) as a colorless amorphous.
   MS(ESI) m/z: 346.0/348.0[M+H]⁺
(2) A mixture of tert-butyl 6-bromo-8-chloro-3,4-dihydro-1H-isoquinoline-2-carboxylate (60 mg) obtained in the aforementioned (1), 1-(tert-butyldimethylsilyloxy)-1-methoxyethene (98 mg), potassium carbonate (48 mg), bis(tri-tert-butylphosphine)palladium(0) (27 mg) and DMF (0.35 ml) was stirred in a microwave reactor at 120°C for 1 hr. The reaction mixture was cooled to room temperature, water was added, and the mixture was extracted with ethyl acetate. The organic layer was washed with saturated brine, dried over anhydrous magnesium sulfate, and concentrated under reduced pressure. The residue was purified by reversed-phase HPLC (10 mmol/l aqueous ammonium carbonate solution/acetonitrile =50/50 - 20/80) to give tert-butyl 8-chloro-6-(2-methoxy-2-oxoethyl)-3,4-dihydro-1H-isoquinoline-2-carboxylate (31 mg; yield 53%) as a pale yellow amorphous.
   MS(ESI) m/z: 240.1/242.1[M-Boc+H]⁺
(3) To a solution of tert-butyl 8-chloro-6-(2-methoxy-2-oxoethyl)-3,4-dihydro-1H-isoquinoline-2-carboxylate (30 mg) obtained in the aforementioned (2) in water (1.5 ml) and dioxane (5 ml) were added potassium hexacyanoferrate(II) trihydrate (24 mg), chloro(2-dicyclohexylphosphino-2',4',6'-triisopropyl-1,1'-biphenyl) [2-(2'-amino-1,1'-biphenyl)]palladium(II) (9 mg) and potassium acetate (9 mg), and the mixture was stirred at 100°C for 2 hr. The reaction mixture was allowed to cool, saturated aqueous ammonium chloride solution was added. The mixture was extracted with ethyl acetate, dried over sodium sulfate, and concentrated under reduced pressure. The residue was purified by reversed-phase HPLC (10 mmol/l aqueous ammonium carbonate solution/acetonitrile =80/20 - 50/50) to give 2-[8-cyano-2-[(2-methylpropan-2-yl)oxycarbonyl]-3,4-dihydro-1H-isoquinolin-6-yl]acetic acid (27 mg; yield 97%) as a colorless oil.
   MS(ESI) m/z: 217.2[M-Boc+H]⁺
(4) To a solution of 2-[8-cyano-2-[(2-methylpropan-2-yl)oxycarbonyl]-3,4-dihydro-1H-isoquinolin-6-yl]acetic acid (24 mg) obtained in the aforementioned (3) in methanol (0.3 ml) and toluene (1.2 ml) was added dropwise trimethylsilyldiazomethane (10% hexane solution) (0.3 ml), and the mixture was stirred at room temperature for 2 hr. The reaction mixture was concentrated under reduced pressure to give tert-butyl 8-cyano-6-(2-methoxy-2-oxoethyl)-3,4-dihydro-1H-isoquinoline-2-carboxylate (24 mg; yield 96%) as a colorless amorphous.
   MS(ESI) m/z: 331.3[M+H]⁺
(5) To a mixture of tert-butyl 8-cyano-6-(2-methoxy-2-oxoethyl)-3,4-dihydro-1H-isoquinoline-2-carboxylate (22 mg) obtained in the aforementioned (4) and dichloromethane (1 ml) was added trifluoroacetic acid (0.1 ml), and the mixture was stirred at room temperature overnight. The reaction mixture was concentrated under reduced pressure to give a crude product (23 mg) of the title compound as a colorless amorphous.

### Reference Example 41:

### Production of methyl 4-(3-phenylpiperazin-1-yl)butanoate hydrochloride

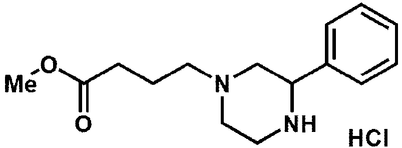

(1) Diethyl oxalate (2.3 ml) was added to a solution of 2-phenylpiperazine (1.84 g) in THF (ml), and the reaction mixture was stirred overnight. To the reaction mixture was added di-tert-butyl dicarbonate (3.25 g), and the mixture was stirred at room temperature overnight. The solvent of the reaction mixture was evaporated, and methanol (23 ml) and water (23 ml) were added to the residue, 10 mol/l aqueous sodium hydroxide solution (11.3 ml) was added, and the mixture was stirred at 80°C for 12 hr. Water was added to the reaction mixture, the mixture was extracted with ethyl acetate, and the organic layer was washed successively with water and saturated brine, dried over sodium sulfate, and concentrated under reduced pressure. The residue was purified by NH silica gel column chromatography (hexane/ethyl acetate =75/25 - 50/50), a fraction containing the desired compound was concentrated under reduced pressure to give tert-butyl 2-phenylpiperazine-1-carboxylate (1.1958 g; yield 40%) as a pale yellow oil.
   MS(ESI) m/z: 263.3[M+H]⁺
(2) tert-Butyl 2-phenylpiperazine-1-carboxylate (200 mg) obtained in the aforementioned (1) was added to DMF (1.9 ml), methyl 4-bromobutyrate (207 mg), potassium carbonate (316 mg) and potassium iodide (127 mg) were successively added, and the mixture was stirred at 80°C for 5 hr 5 min. Water was added to the reaction mixture, the mixture was extracted with ethyl acetate, and the organic layer was washed successively with water and saturated brine, dried over sodium sulfate, and concentrated under reduced pressure to give tert-butyl 4-(4-methoxy-4-oxobutyl)-2-phenylpiperazine-1-carboxylate (270 mg; yield 98%) as a pale yellow oil.
   MS(ESI) m/z: 363.3[M+H]⁺
(3) tert-Butyl 4-(4-methoxy-4-oxobutyl)-2-phenylpiperazine-1-carboxylate (270 mg) obtained in the aforementioned (2) was added to dioxane (1.86 ml), 4 M hydrogen chloride-dioxane solution (1.86 ml) was added, and the mixture was stirred at room temperature for 4 hr 10 min. The reaction mixture was evaporated under reduced pressure, dioxane was added, and the solvent was evaporated under reduced pressure to give the title compound as a crude product.
   MS(ESI) m/z: 263.3[M+H]⁺

### Reference Examples 42 to 46:

The corresponding starting compounds were treated in the same manner as in Reference Example 41 to obtain the compounds described in the following Table 3.

**[Table 3]**

| **Ref. Ex.No.** | **structural formula** | **instrument analysis data** |
|---|---|---|
| 42 | 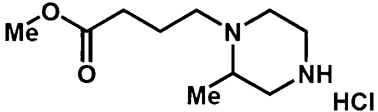 | MS (ESI) m/z:201.1 [M+H]+ |
| 43 | 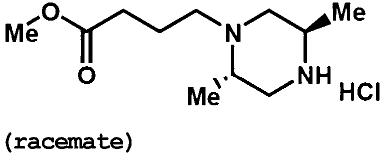 | MS(ESI) m/z:215.2 [M+H]+ |
| 44 | 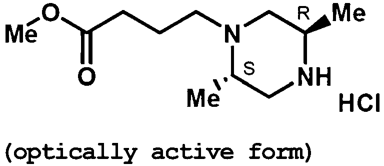 | |
| 45 | 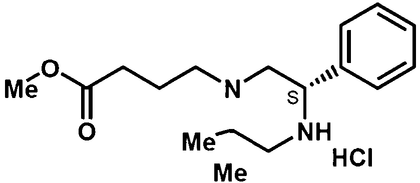 | |
| 46 | 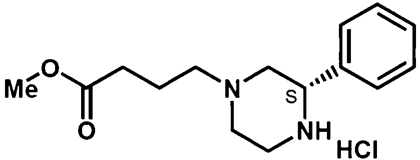 | MS(ESI) m/z:263.2 [M+H]+ |

### Reference Example 47:

### Production of methyl 4-[(2R,5S)-2-methyl-5-phenylpiperazin-1-yl]butanoate

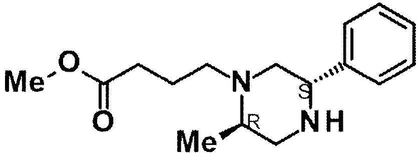

(1) To a solution of methyl (2S)-2-(benzylamino)-2-phenylacetate (2.3 g) in DMF (20 ml) were added (2R)-2-(tert-butoxycarbonylamino)propanoic acid (5.0 g), HATU (12.3 g) and N,N-diisopropylethylamine (5.62 ml), and the reaction mixture was stirred at room temperature for 4 days. Water was added to the reaction mixture, and the mixture was extracted with ethyl acetate. The organic layer was washed with saturated brine, dried over anhydrous sodium sulfate, and concentrated under reduced pressure. The obtained residue was purified by silica gel column chromatography (hexane/ethyl acetate =80/20 - 40/60) to give methyl (2S)-2-[benzyl-[(2R)-2-(tert-butoxycarbonylamino)propanoyl]amino]-2-phenylacetate (1.74 g; yield 45.3%) as a colorless oil.
   MS(ESI) m/z: 425.3[M-H]⁻
(2) Methyl (2S)-2-[benzyl-[(2R)-2-(tert-butoxycarbonylamino)propanoyl]amino]-2-phenylacetate (1.74 g) obtained in the aforementioned (1) was dissolved in dichloromethane (10 ml), trifluoroacetic acid (10 ml) was added, and the reaction mixture was stirred at room temperature for 1 hr. The reaction mixture was concentrated under reduced pressure at 40°C, and the obtained residue was purified by silica gel column chromatography (chloroform/methanol=100/0 - 90/10) to give (3R,6S)-1-benzyl-3-methyl-6-phenylpiperazine-2,5-dione (1.0 g; yield 83.3%) as a colorless amorphous.
   MS(ESI) m/z: 295.1[M+H]⁺
(3) (3R,6S)-1-Benzyl-3-methyl-6-phenylpiperazine-2,5-dione (1.0 g) obtained in the aforementioned (2) was dissolved in THF (7 ml), borane dimethylsulfide (6.59 ml) was added, and the reaction mixture was stirred at 60°C for 15 hr. The reaction mixture was cooled to 0°C, water was slowly added, hydrochloric acid (1 mol/l, 20 ml) was added, and the mixture was stirred at room temperature for 10 min. The reaction mixture was neutralized with aqueous sodium hydroxide solution (1 mol/l) and extracted with chloroform. The organic layer was washed with saturated brine, dried over anhydrous magnesium sulfate, and filtered. The filtrate was concentrated under reduced pressure, methanol (20 ml) and aqueous sodium hydroxide solution (1 mol/l, 20 ml) were added, and the reaction mixture was stirred at 90°C for 15 hr. The reaction mixture was cooled to room temperature and extracted with chloroform. The organic layer was washed with saturated brine, dried over anhydrous magnesium sulfate, and concentrated under reduced pressure. The obtained residue was purified by silica gel column chromatography (hexane/ethyl acetate =80/20 - 50/50) to give (2S,5R)-1-benzyl-5-methyl-2-phenylpiperazine (231 mg; yield 25.5%) as a colorless solid. MS(ESI) m/z: 267.2[M+H]⁺
(4) (2S,5R)-1-Benzyl-5-methyl-2-phenylpiperazine (231 mg) obtained in the aforementioned (3) was dissolved in DMF (3 ml), and methyl 4-bromobutanoate (240 mg), potassium carbonate (160 mg) and potassium iodide (150 mg) were added. The reaction mixture was stirred at 80°C for 2 hr. The reaction mixture was cooled to room temperature, water was added, and the mixture was extracted with ethyl acetate. The organic layer was dried over anhydrous sodium sulfate and concentrated under reduced pressure. The obtained residue was purified by silica gel column chromatography (hexane/ethyl acetate =80/20 - 60/40) to give methyl 4-[(2R,5S)-4-benzyl-2-methyl-5-phenylpiperazin-1-yl]butanoate (272 mg; yield 85.6%) as a colorless oil.
   MS(ESI) m/z: 367.2[M+H]⁺
(5) Methyl 4-[(2R,5S)-4-benzyl-2-methyl-5-phenylpiperazin-1-yl]butanoate (272 mg) obtained in the aforementioned (4) was dissolved in methanol (6 ml), and palladium hydroxide-carbon (20%, 200 mg) was added. The reaction mixture was subjected to hydrogen replacement, and the mixture was stirred at room temperature for 15 hr. The reaction mixture was filtered through celite, and the filtrate was concentrated under reduced pressure at 40°C to give a crude product (205 mg) of the title compound as a colorless oil.

### Reference Example 48:

The corresponding starting compounds were treated in the same manner as in Reference Example 47 to obtain a crude product of the compound described in the following Table 4.

**[Table 4]**

| **Ref. Ex.No.** | **structural formula** |
|---|---|
| 48 | 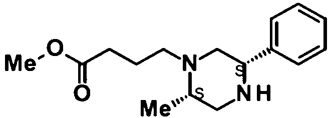 |

### Reference Example 49:

### Production of methyl 2-(1-ethyl-1,2,3,4-tetrahydroisoquinolin-6-yl)acetate

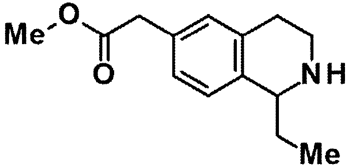

(1) To a solution (24.99 ml) of 2-(3-bromophenyl)ethanamine (1.84 g) in dichloromethane was added triethylamine (2.8 ml) at room temperature, propanoyl chloride (0.48 ml) was added under ice-cooling, and the mixture was stirred for 30 min. To the reaction mixture was added aqueous ammonium chloride solution, and the mixture was extracted with chloroform, and the organic layer was dried over anhydrous magnesium sulfate, and concentrated under reduced pressure to give N-[2-(3-bromophenyl)ethyl]propanamide (1.28 g; yield 100%) as a colorless oil.
   MS(ESI) m/z: 256.1/258.1[M+H]⁺
(2) To N-[2-(3-bromophenyl)ethyl]propanamide (1.2802 g) obtained in the aforementioned (1) was added polyphosphoric acid (5 ml), and the mixture was stirred at an outer temperature of 160°C for 5 hr. The reaction mixture was added to ice water, adjusted to pH 8 with 1 mol/l aqueous sodium hydroxide solution, and the aqueous layer was extracted with chloroform. The organic layer was dried over anhydrous magnesium sulfate, and concentrated under reduced pressure to give 6-bromo-1-ethyl-3,4-dihydroisoquinoline (1.1901 g; yield 100%) as a yellow oil.
   MS(ESI) m/z: 238.0/240.0[M+H]⁺
(3) A solution of 6-bromo-1-ethyl-3, 4-dihydroisoquinoline (1.19 g) obtained in the aforementioned (2) in methanol (16.66 ml) was ice-cooled, sodium borohydride (208 mg) was added, and the mixture was stirred for 30 min. To the reaction mixture was added aqueous ammonium chloride solution, and the aqueous layer was extracted with ethyl acetate. The organic layer was washed with saturated brine, dried over anhydrous magnesium sulfate, and concentrated under reduced pressure. The obtained residue was purified by NH silica gel column chromatography (chloroform/methanol=100/0 - 85/15) to give 6-bromo-1-ethyl-1,2,3,4-tetrahydroisoquinoline (594 mg; yield 49%) as a colorless oil.
   MS(ESI) m/z: 240.1/242.1[M+H]⁺
(4) To a solution of 6-bromo-1-ethyl-1,2,3,4-tetrahydroisoquinoline (325 mg) obtained in the aforementioned (3) in dichloromethane (4 ml) were added triethylamine (0.3 ml) and benzyl chloroformate (0.23 ml) at room temperature, and the mixture was stirred for 30 min. To the reaction mixture was added aqueous ammonium chloride solution, and the aqueous layer was extracted with chloroform. The organic layer was dried over anhydrous magnesium sulfate and concentrated under reduced pressure. The obtained residue was purified by silica gel column chromatography (hexane/ethyl acetate =100/0 - 80/20) to give benzyl 6-bromo-1-ethyl-3,4-dihydro-1H-isoquinoline-2-carboxylate (360 mg; yield 71%) as a colorless oil.
   MS(ESI) m/z: 374.1/376.1[M+H]⁺
(5) To a solution of benzyl 6-bromo-1-ethyl-3,4-dihydro-1H-isoquinoline-2-carboxylate (730 mg) obtained in the aforementioned (4) in DMF (2 ml) were added 1-(tert-butyldimethylsilyloxy)-1-methoxyethene (1.26 ml), potassium carbonate (530 mg) and bis(tri-tert-butylphosphine)palladium (245 mg), and the mixture was stirred under microwave irradiation at 110°C for 1 hr. To the reaction mixture was added aqueous ammonium chloride solution, and the aqueous layer was extracted with ethyl acetate. The organic layer was washed with saturated brine, dried over anhydrous magnesium sulfate, and concentrated under reduced pressure. The obtained residue was purified by silica gel column chromatography (hexane/ethyl acetate =80/20 - 65/35) to give benzyl 1-ethyl-6-(2-methoxy-2-oxoethyl)-3,4-dihydro-1H-isoquinoline-2-carboxylate (360 mg; yield 71%) as a yellow oil.
   MS(ESI) m/z: 366.1[M-H]⁻
(6) To a solution of benzyl 1-ethyl-6-(2-methoxy-2-oxoethyl)-3,4-dihydro-1H-isoquinoline-2-carboxylate (520 mg) obtained in the aforementioned (5) in methanol (4 ml) was added palladium hydroxide (65 mg) at room temperature, and the mixture was stirred under hydrogen atmosphere and at an outer temperature of 60°C for 5 hr. The reaction mixture was allowed to cool, filtered through celite, washed with methanol, and the filtrate was concentrated under reduced pressure to give a crude product (330.1 mg) of the title compound as a yellow solid.

### Reference Example 50:

### Production of methyl 2-(1,2,3,4-tetrahydroisoquinolin-7-ylamino)acetate hydrochloride

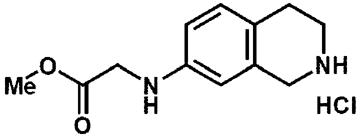

(1) To an acetonitrile solution (2.6 ml) of tert-butyl 7-amino-3,4-dihydro-1H-isoquinoline-2-carboxylate (200 mg) were added diisopropylethylamine (0.2 ml) and bromomethyl acetate (0.09 ml) at room temperature, and the mixture was stirred overnight. To the reaction mixture was added 10% aqueous potassium carbonate solution, and the mixture was extracted with ethyl acetate, and the organic layer was washed with saturated brine, dried over anhydrous magnesium sulfate, and concentrated under reduced pressure. The obtained residue was purified by silica gel column chromatography (chloroform/methanol=100/0 - 93/7), and a fraction containing the desired compound was concentrated under reduced pressure to give tert-butyl 7-[(2-methoxy-2-oxoethyl)amino]-3,4-dihydro-1H-isoquinoline-2-carboxylate (200 mg; yield 78%) as a yellow oil. MS(ESI) m/z: 321.2[M+H]⁺
(2) To a solution of tert-butyl 7-[(2-methoxy-2-oxoethyl)amino]-3,4-dihydro-1H-isoquinoline-2-carboxylate (170 mg) obtained in the aforementioned (1) in dichloromethane (0.5 ml) was added trifluoroacetic acid (1 ml) at room temperature, and the mixture was stirred for 1 hr. The reaction mixture was concentrated under reduced pressure, 4N hydrogen chloride-ethyl acetate solution (2 ml) was added, and the resulting solid was collected by filtration, washed with diisopropylether, and dried under reduced pressure at 60°C to give a crude product (125 mg) of the title compound as a beige solid.

### Reference Example 51:

### Production of methyl 2-(1,2,3,4-tetrahydroisoquinolin-6-ylamino)acetate hydrochloride

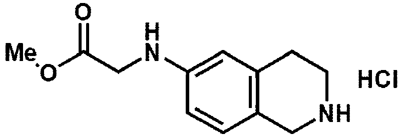

(1) To an acetonitrile solution (2.7 ml) of tert-butyl 6-amino-3,4-dihydro-1H-isoquinoline-2-carboxylate (200 mg) were added N,N-diisopropylethylamine (0.2 ml) and bromomethyl acetate (0.09 ml) at room temperature, and the mixture was stirred overnight. To the reaction mixture was added 10% aqueous potassium carbonate solution, the mixture was extracted with ethyl acetate, and the organic layer was washed with saturated brine, dried over anhydrous magnesium sulfate, and concentrated under reduced pressure. The obtained residue was purified by silica gel column chromatography (chloroform/methanol=100/0 - 93/7), and a fraction containing the desired compound was concentrated under reduced pressure to give tert-butyl 6-[(2-methoxy-2-oxoethyl)amino]-3,4-dihydro-1H-isoquinoline-2-carboxylate (190 mg; yield 74%) as a yellow oil.
   MS(ESI) m/z: 321.2[M+H]⁺
(2) To a solution of tert-butyl 6-[(2-methoxy-2-oxoethyl)amino]-3,4-dihydro-1H-isoquinoline-2-carboxylate (170 mg) obtained in the aforementioned (1) in dichloromethane (0.5 ml) was added trifluoroacetic acid (1 ml) at room temperature, and the mixture was stirred for 1 hr. The reaction mixture was concentrated under reduced pressure, crystallized by adding 4N hydrogen chloride-ethyl acetate solution (2 ml), and the solid was collected by filtration, washed with diisopropylether, and dried under reduced pressure at 60°C to give a crude product (150 mg) of the title compound as a beige solid.

### Reference Example 52:

### Production of methyl 2-(3-methyl-4,5,6,7-tetrahydropyrazolo[4,3-c]pyridin-1-yl)acetate hydrochloride

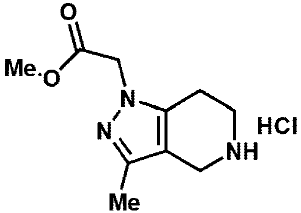

(1) To a DMF solution (20 ml) of tert-butyl 3-methyl-1,4,6,7-tetrahydropyrazolo[4,3-c]pyridine-5-carboxylate (200 mg) were added bromomethyl acetate (3.90 g), potassium carbonate (1.5 g) and potassium iodide (1.4 g) at room temperature, and the mixture was stirred for 30 min. The mixture was further stirred at 80°C for 1 hr, water was added to the reaction mixture, and the mixture was extracted with ethyl acetate. The organic layer was dried over dried over anhydrous magnesium sulfate and concentrated under reduced pressure. The obtained residue was purified by silica gel column chromatography (chloroform/methanol=100/0 - 95/5) and a fraction containing the desired compound was concentrated under reduced pressure to give tert-butyl 1-(2-methoxy-2-oxoethyl)-3-methyl-6,7-dihydro-4H-pyrazolo[4,3-c]pyridine-5-carboxylate (2.6 g; yield 100%) (regio isomeric mixture of 2:1) as a brown oil.
   MS(ESI) m/z: 310.2[M+H]⁺
(2) To a solution of tert-butyl 1-(2-methoxy-2-oxoethyl)-3-methyl-6,7-dihydro-4H-pyrazolo[4,3-c]pyridine-5-carboxylate (2.6 g) obtained in the aforementioned (1) in methanol (10 ml) was added 4N hydrogen chloride-dioxane solution (20 ml) at room temperature, and the mixture was stirred for 1 hr. The reaction mixture was evaporated under reduced pressure, and crystallized by adding acetonitrile. The solvent was evaporated under reduced pressure. Ethyl acetate was added to the residue, and the resulting solid was collected by filtration and dried under reduced pressure to give the title compound (1.56 g; yield 76%) (regio isomer mixture of 2:1) as a white solid.
   MS(ESI) m/z: 210.1[M+H]⁺

### Reference Example 53:

### Production of methyl 3-(4,5,6,7-tetrahydrothieno[2,3-c]pyridin-2-yl)propanoate

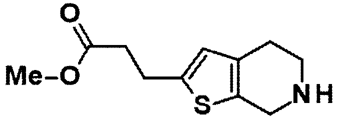

(1) To a solution of tert-butyl 2-formyl-5,7-dihydro-4H-thieno[2,3-c]pyridine-6-carboxylate (1.25 g) in dichloromethane (40 ml) was added methyl (triphenylphosphoranylidene)acetate (1.9 g) at room temperature, and the mixture was stirred for 15 hr. The reaction mixture was concentrated under reduced pressure, the obtained residue was crystallized by adding diisopropyl ether, the solid was filtered off, and the filtrate was concentrated under reduced pressure. The obtained residue was purified by silica gel column chromatography (hexane/ethyl acetate =90/10 - 70/30) and a fraction containing the desired compound was concentrated under reduced pressure to give tert-butyl 2-[(E)-3-methoxy-3-oxoprop-1-enyl]-5,7-dihydro-4H-thieno[2,3-c]pyridine-6-carboxylate (1.25 g; yield 54%) as a pale yellow solid. MS(ESI) m/z: 267.9[M-C₄H₈+H]⁺
(2) To a solution of tert-butyl 2-[(E)-3-methoxy-3-oxoprop-1-enyl]-5,7-dihydro-4H-thieno[2,3-c]pyridine-6-carboxylate (1.2 g) obtained in the aforementioned (1) in methanol (20 ml) was added 10% palladium-carbon (M) (200 mg) at room temperature, and the mixture was stirred under hydrogen atmosphere at room temperature for 3 hr. The reaction mixture was filtered through celite, and the filtrate was concentrated under reduced pressure. The obtained residue was purified by silica gel column chromatography (hexane/ethyl acetate =90/10 - 70/30) and a fraction containing the desired compound was concentrated under reduced pressure to give tert-butyl 2-(3-methoxy-3-oxopropyl)-5,7-dihydro-4H-thieno[2,3-c]pyridine-6-carboxylate (956 mg; yield 79%) as a pale yellow solid.
   MS(ESI) m/z: 226.1[M-Boc+H]⁺
(3) To a solution of tert-butyl 2-(3-methoxy-3-oxopropyl)-5,7-dihydro-4H-thieno[2,3-c]pyridine-6-carboxylate (956 mg) obtained in the aforementioned (2) in dichloromethane (10 ml) was added trifluoroacetic acid (10 ml) at room temperature, and the mixture was stirred for 1 hr. The reaction mixture was concentrated under reduced pressure, dried under reduced pressure, and the residue was purified by silica gel column chromatography (chloroform/methanol=100/0 - 95/5), and a fraction containing the desired compound was concentrated under reduced pressure to give the title compound (647 mg; yield 98%) as a pale yellow oil.
   MS(ESI) m/z: 226.1[M+H]⁺

### Reference Example 54:

### Production of methyl 2-(7-chloro-1,2,3,4-tetrahydroisoquinolin-6-yl)acetate trifluoroacetate

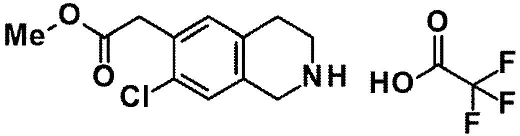

(1) To a solution of tert-butyl 6-bromo-7-chloro-3,4-dihydro-1H-isoquinoline-2-carboxylate (1.3934 g) in DMF (8 ml) were added 1-(tert-butyldimethylsilyloxy)-1-methoxyethene (2.7 ml), potassium carbonate (1111 mg) and bis(tri-tert-butylphosphine)palladium (600 mg), and the mixture was stirred under microwave irradiation at 110°C for 1 hr. The reaction mixture was filtered through celite, water was added to the filtrate, and the aqueous layer was extracted with chloroform. The organic layer was washed with water, dried over sodium sulfate, and concentrated under reduced pressure. The obtained residue was purified by silica gel column chromatography (hexane/ethyl acetate =100/0 - 80/20) and a fraction containing the desired compound was concentrated under reduced pressure to give tert-butyl 7-chloro-6-(2-methoxy-2-oxoethyl)-3,4-dihydro-1H-isoquinoline-2-carboxylate (592.2 mg; yield 43%) as a yellow solid.
   MS(ESI) m/z: 284.2/286.2[M-C₄H₈+H]⁺
(2) To a solution of tert-butyl 7-chloro-6-(2-methoxy-2-oxoethyl)-3,4-dihydro-1H-isoquinoline-2-carboxylate (63.4 mg) obtained in the aforementioned (1) in dichloromethane (2 ml) was added trifluoroacetic acid (0.3 ml) at room temperature, and the mixture was stirred for 30 min. The reaction mixture was concentrated under reduced pressure to give a crude product (66 mg) of the title compound.

### Reference Example 55:

### Production of methyl 2-(7-cyano-1,2,3,4-tetrahydroisoquinolin-6-yl)acetate trifluoroacetate

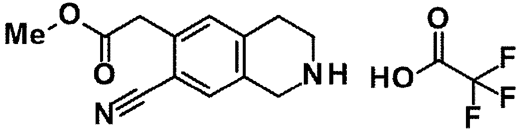

(1) A mixture of tert-butyl 6-bromo-7-chloro-3,4-dihydro-1H-isoquinoline-2-carboxylate (1.30 g), 1-(tert-butyldimethylsilyloxy)-1-methoxyethene (2.12 g), potassium carbonate (1.04 g), bis(tri-tert-butylphosphine)palladium(0) (574 mg) and DMF (12.5 ml) was stirred in a microwave reactor at 110°C for 1 hr. The reaction mixture was cooled to room temperature, water was added, and the mixture was extracted with chloroform. The organic layer was washed with saturated brine, dried over anhydrous magnesium sulfate, and concentrated under reduced pressure. The residue was purified by silica gel column chromatography (solvent: n-hexane/ethyl acetate =100/0 - 80/20) to give tert-butyl 7-chloro-6-(2-methoxy-2-oxoethyl)-3,4-dihydro-1H-isoquinoline-2-carboxylate (488 mg; yield 38%) as a white solid.
   MS(ESI) m/z: 283.9/285.9[M-C₄H₈+H]⁺
(2) A mixture of tert-butyl 7-chloro-6-(2-methoxy-2-oxoethyl)-3,4-dihydro-1H-isoquinoline-2-carboxylate (488 mg) obtained in the aforementioned (1), potassium hexacyanoferrate(II) trihydrate (400 mg), chloro(2-dicyclohexylphosphino-2,4,6-triisopropyl-1,1-biphenyl) (2-amino-1,1-biphenyl-2-yl)palladium(II) (150 mg), potassium acetate (140 mg), 1,4-dioxane (15 ml) and water (4.5 ml) was stirred at 105°C for 4 hr. To the reaction mixture were further added potassium hexacyanoferrate(II) trihydrate (400 mg), chloro(2-dicyclohexylphosphino-2,4,6-triisopropyl-1,1-biphenyl) (2-amino-1,1-biphenyl-2-yl)palladium(II) (300 mg) and potassium acetate (280 mg), and the mixture was stirred for 2 hr. The reaction mixture was cooled to room temperature, water was added, and the mixture was extracted with chloroform. The organic layer was washed with saturated brine, dried over anhydrous magnesium sulfate, and concentrated under reduced pressure. The residue was purified by silica gel column chromatography (solvent: n-hexane/ethyl acetate =100/0 - 70/30) to give tert-butyl 7-cyano-6-(2-methoxy-2-oxoethyl)-3,4-dihydro-1H-isoquinoline-2-carboxylate (370 mg; yield 78%) as a white solid.
   MS(ESI) m/z: 275.2[M-C₄H₈+H] ⁺
(3) To tert-butyl 7-cyano-6-(2-methoxy-2-oxoethyl)-3,4-dihydro-1H-isoquinoline-2-carboxylate (60 mg) obtained in the aforementioned (2) were added dichloromethane (2 ml) and trifluoroacetic acid (0.27 ml), and the mixture was stirred at room temperature for 1 hr. The reaction mixture was concentrated under reduced pressure to give the title compound crude product (62.5 mg) as a yellow solid.

### Reference Example 56:

### Production of 7-bromo-1,2,3,4-tetrahydropyrido[1,2-a]pyrazin-6-one hydrochloride

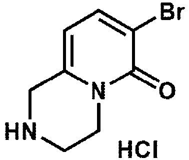

(1) To a solution of tert-butyl 6-oxo-3,4-dihydro-1H-pyrido[1,2-a]pyrazine-2-carboxylate (182 mg) in dichloromethane (1.8 ml) was added N-bromosuccinimide (65 mg), and the mixture was stirred at room temperature for 3 hr. To the reaction mixture was added aqueous sodium thiosulfate solution, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =100/0 - 40/60) to give tert-butyl 7-bromo-6-oxo-3,4-dihydro-1H-pyrido[1,2-a]pyrazine-2-carboxylate (42.2 mg; yield 18%) as a colorless amorphous.
   MS(ESI) m/z: 329.2/331.2[M+H]⁺
(2) To tert-butyl 7-bromo-6-oxo-3,4-dihydro-1H-pyrido[1,2-a]pyrazine-2-carboxylate (42.2 mg) obtained in the aforementioned (1) was added 4N hydrogen chloride-ethyl acetate solution (0.8 ml), and the mixture was stirred at room temperature for 18 hr. The reaction mixture was concentrated under reduced pressure to give the title compound (33.2 mg; yield 98%) as a white solid.
   MS(ESI) m/z: 229.0/231.0[M+H]⁺

### Reference Example 57:

### Production of 6-bromo-1-(methoxymethyl)-1,2,3,4-tetrahydroisoquinoline

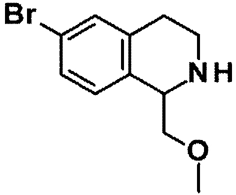

(1) To a solution of 2-(3-bromophenyl)ethanamine (1 g), 2-methoxyacetic acid (540 mg) and HATU (2.4 g) in dichloromethane (17 ml) was added triethylamine (1.4 ml), and the mixture was stirred at room temperature for 3 hr. Water was added to the reaction mixture, and the mixture was extracted with ethyl acetate. The organic layer was washed with 1N hydrochloric acid and 1N aqueous sodium hydroxide solution, dried over anhydrous magnesium sulfate, and concentrated under reduced pressure to give a crude product (1.36 g) of N-[2-(3-bromophenyl)ethyl]-2-methoxyacetamide as a yellow oil.
(2) To a solution of the crude product (1.36 g) of N-[2-(3-bromophenyl)ethyl]-2-methoxyacetamide obtained in the aforementioned (1) in toluene (17 ml) was added phosphorus(V) oxide, and the mixture was stirred at 140°C for 2 hr. The reaction mixture was cooled to 0°C, water was added, and the mixture was extracted with ethyl acetate. The organic layer was washed with saturated brine, dried over anhydrous magnesium sulfate, and concentrated under reduced pressure to give a crude product (1.27 g) of 6-bromo-1-(methoxymethyl)-3,4-dihydroisoquinoline as a brown oil.
(3) To a solution of the crude product (1.27 g) of 6-bromo-1-(methoxymethyl)-3,4-dihydroisoquinoline obtained in the aforementioned (2) in methanol (17 ml) was added sodium borohydride (756 mg), and the mixture was stirred at room temperature for 18 hr. Water was added to the reaction mixture, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =100/0 - 0/100) to give the title compound (64.1 mg; yield 5.0%) as an orange oil.
   MS(ESI) m/z: 256.1/258.1[M+H]⁺

### Reference Example 58:

### Production of ethyl 5-(1,2,3,4-tetrahydroisoquinolin-6-yl)pentanoate

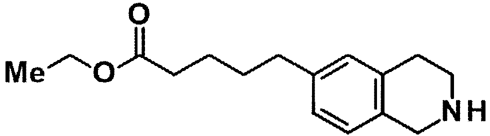

(1) A reaction mixture of benzyl 6-bromo-3,4-dihydro-1H-isoquinoline-2-carboxylate (1 g), ethyl 4-pentenoate (555 mg), potassium carbonate (399 mg), bis(tri-tert-butylphosphine)palladium(0) (221 mg) and DMF (4.8 ml) was stirred in a microwave reactor at 120°C for 2 hr. The reaction mixture was cooled to room temperature, water was added, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =90/10 - 80/20) to give benzyl 6-(5-ethoxy-5-oxopent-1-enyl)-3,4-dihydro-1H-isoquinoline-2-carboxylate (306 mg; yield 54%) as a pale yellow oil.
   MS(ESI) m/z: 394.2[M+H]⁺
(2) To benzyl 6-(5-ethoxy-5-oxopent-1-enyl)-3,4-dihydro-1H-isoquinoline-2-carboxylate (306 mg) obtained in the aforementioned (1) were added ethanol (5.2 ml) and 10% palladium-carbon (83 mg), and the mixture was stirred under hydrogen atmosphere at room temperature for 15 hr. The residue was purified by NH silica gel column chromatography (solvent: n-hexane/ethyl acetate =60/40 - 20/80) to give the title compound (152 mg; yield 75%) as a colorless oil.
   MS(ESI) m/z: 262.3[M+H]⁺

### Reference Example 59:

### Production of tert-butyl 3-(4,5,6,7-tetrahydropyrazolo[1,5-a]pyrazin-3-yl) propanoate

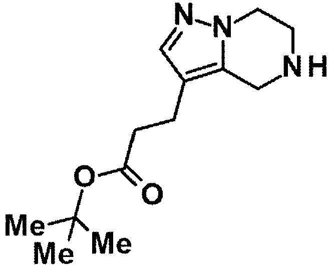

(1) To a solution of benzyl 3-iodo-6,7-dihydro-4H-pyrazolo[1,5-a]pyrazine-5-carboxylate (300 mg) in THF (3 ml) was added 2 mol/l isopropylmagnesium chloride-THF solution (0.5 ml) at - 15°C, and the mixture was stirred for 10 min. To the reaction mixture was added a solution of DMF (0.09 ml) in THF (1 ml), and the mixture was stirred for 30 min. To the reaction mixture was added diluted hydrochloric acid, and the mixture was extracted with ethyl acetate. The organic layer was washed with saturated brine, dried over anhydrous magnesium sulfate, and concentrated under reduced pressure to give benzyl 3-formyl-6,7-dihydro-4H-pyrazolo[1,5-a]pyrazine-5-carboxylate (240 mg; yield 108%) as a pale yellow oil.
   MS(ESI) m/z: 286.1[M+H]⁺
(2) To a solution of tert-butyl diethoxyphosphoryl acetate (257 mg) in THF (3 ml) was added 60% sodium hydride (140 mg) at 0°C, and the mixture was stirred for 5 min. To the reaction mixture was added a solution of benzyl 3-formyl-6,7-dihydro-4H-pyrazolo[1,5-a]pyrazine-5-carboxylate (240 mg) obtained in the aforementioned (1) in THF (2 ml), and the mixture was stirred at room temperature for 30 min. To the reaction mixture was added aqueous ammonium chloride solution, and the mixture was extracted with ethyl acetate. The organic layer was washed with saturated brine, dried over anhydrous magnesium sulfate, and concentrated under reduced pressure. The residue was purified by silica gel column chromatography (solvent: n-hexane/ethyl acetate =67/33 - 45/55) to give benzyl 3-[(E)-3-[(2-methylpropan-2-yl)oxy]-3-oxoprop-1-enyl]-6,7-dihydro-4H-pyrazolo[1,5-a]pyrazine-5-carboxylate (190 mg; yield 59%) as a colorless oil.
   MS(ESI) m/z: 384.1[M+H]⁺
(3) To benzyl 3-[(E)-3-[(2-methylpropan-2-yl)oxy]-3-oxoprop-1-enyl]-6,7-dihydro-4H-pyrazolo[1,5-a]pyrazine-5-carboxylate (117 mg) obtained in the aforementioned (2) were added methanol (2.5 ml) and 20% palladium hydroxide (38 mg), and the mixture was stirred under hydrogen atmosphere at room temperature overnight. The reaction mixture was filtered through celite, and the filtrate was concentrated under reduced pressure to give a crude product (125 mg) of the title compound as a gray solid.

### Reference Example 60:

### Production of (E)-N-[2-(7-bromo-1,2,4,5-tetrahydro-3-benzazepin-3-yl)-2-oxoethyl]-3-[4-(trifluoromethyl)phenyl]prop-2-enamide

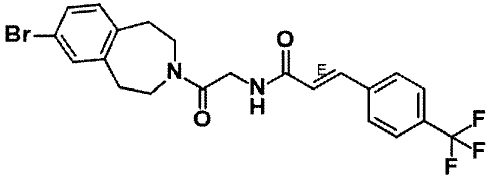

To a solution of 2-[[(E)-3-[4-(trifluoromethyl)phenyl]prop-2-enoyl]amino]acetic acid (130 mg) in DMF (2.4 ml) were added 7-bromo-2,3,4,5-tetrahydro-1H-3-benzazepine hydrochloride (125 mg), N,N-diisopropylethylamine (0.25 ml) and HATU (199 mg), and the reaction mixture was stirred at room temperature for 1 hr. To the reaction mixture was added a saturated aqueous ammonium chloride solution, and the mixture was extracted with chloroform. The organic layer was concentrated under reduced pressure, and the residue was purified by reversed-phase HPLC (10 mmol/l aqueous ammonium carbonate solution/acetonitrile =50/50 - 20/80) to give the title compound (168 mg; yield 73%) as a colorless solid. MS(ESI) m/z: 481.2[M+H]⁺

### Reference Examples 61 to 99:

The corresponding starting compounds were treated in the same manner as in Reference Example 60 to obtain the compounds described in the following Table 5-1 to Table 5-8.

**[Table 5-1]**

| **Ref. Ex.No.** | **structural formula** | **instrument analysis data** |
|---|---|---|
| 61 | 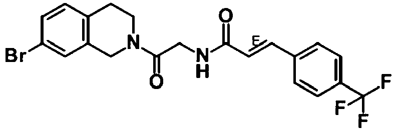 | MS (ESI) m/z:467.2/4 69.2[M+H]+ |
| 62 | 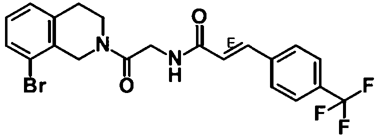 | MS (ESI) m/z:467.2/4 69.2[M+H]+ |
| 63 | 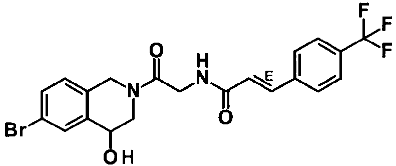 | MS(ESI) m/z: 483.2/ 485.2[M+H]+ |
| 64 | 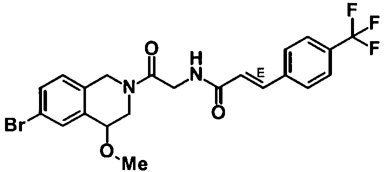 | MS(ESI) m/z:197.2/4 99.2[M+H]+ |
| 65 | 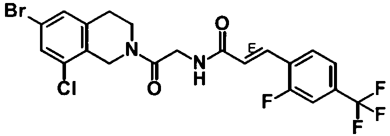 | MS (EST) m/z: 519.0/ 521.0[M+H]+ |

**[Table 5-2]**

| **Ref. Ex.No.** | **structural formula** | **instrument analysis data** |
|---|---|---|
| 66 | 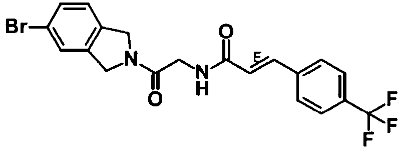 | MS(ESI) m/z:453.0/4 55.0[M+H]+ |
| 67 | 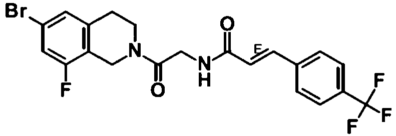 | MS(ESI) m/z:485.2/4 87.2 [M+H]+ |
| 68 | 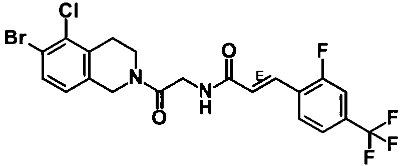 | MS(ESI) m/z:519.2/5 21.2 [M+H]+ |
| 69 | 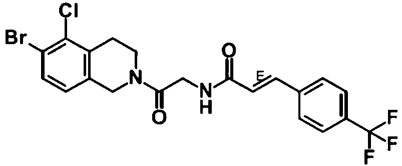 | MS(ESI) m/z: 501.1/5 03.1[M+H]+ |
| 70 | 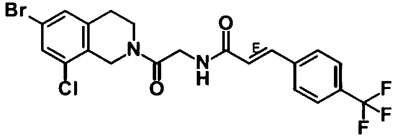 | MS(EST) m/z:501.1/5 03.1[M+H]+ |

**[Table 5-3]**

| **Ref. Ex.No.** | **structural formula** | **instrument analysis data** |
|---|---|---|
| 71 | 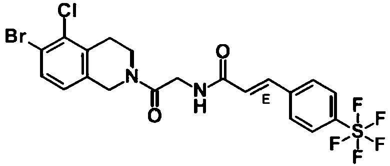 | MS(ESI) m/z:559.1/5 61.1 [M+H]+ |
| 72 | 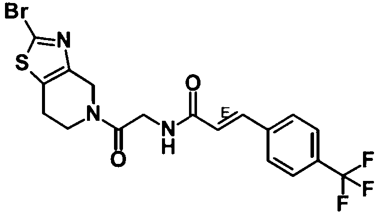 | MS(ESI) m/z:474.2/4 76.2[M+H]+ |
| 73 | 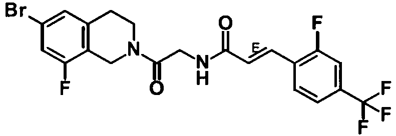 | MS (ESI) m/z:503.2/5 05.2 [M+H]+ |
| 74 | 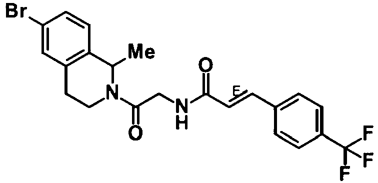 | MS(EST) m/z:481.1/4 83.2 [M+H]+ |
| 75 | 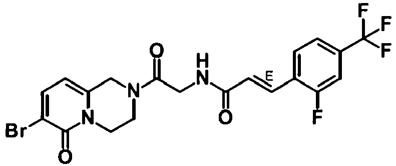 | MS(ESI) m/z:502.2/5 04.2 [M+H]+ |

**[Table 5-4]**

| **Ref. Ex.No.** | **structural formula** | **instrument analysis data** |
|---|---|---|
| 76 | 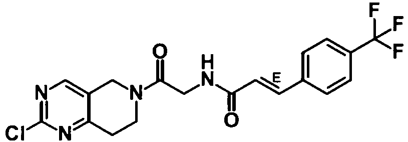 | MS(EST) m/z:425.2/4 27.2[M+H]+ |
| 77 | 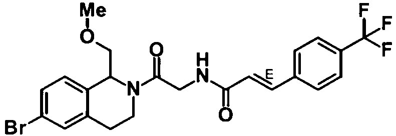 | MS(ESI) m/z:511.2/5 13.2[M+H]+ |
| 78 | 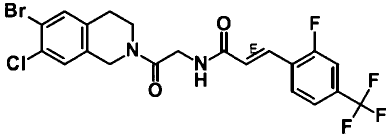 | MS (ESI) m/z:519.1/5 21.1/523.2[M+H]+ |
| 79 | 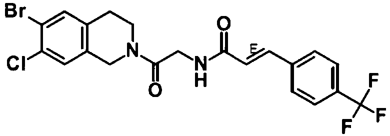 | MS(EST) m/z:501. 1/5 03.2/505.1[M+H]+ |
| 80 | 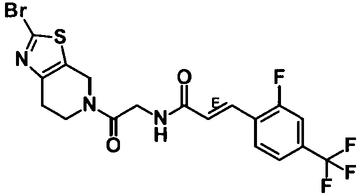 | MS(ESI) m/z:491.0/4 93.0[M+H]+ |

**[Table 5-5]**

| **Ref. Ex.No.** | **structural formula** | **instrument analysis data** |
|---|---|---|
| 81 | 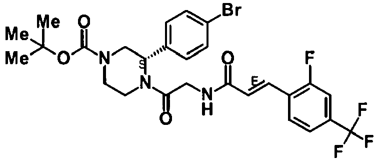 | MS(ESI) m/z:612.3/61 4.3 [M-H]- |
| 82 | 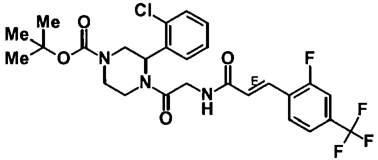 | MS(ESI) m/z:568.3/57 0.2 [M-H]- |
| 83 | 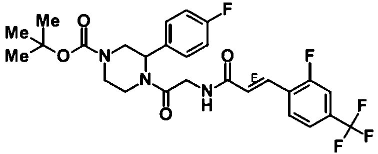 | MS(ESI) m/z:552.3 [M -H]- |
| 84 | 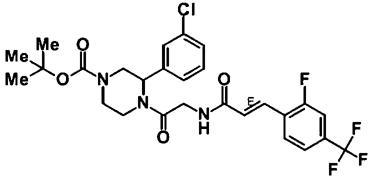 | MS(ESI) m/z:56.2/57 0.2 [M-H]- |
| 85 | 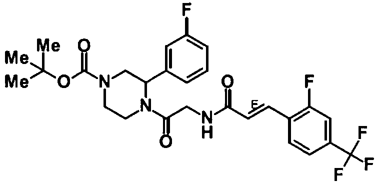 | MS(ESI) m/z:552.3 [M -H]- |

**[Table 5-6]**

| **Ref. Ex.No.** | **structural formula** | **instrument analysis data** |
|---|---|---|
| 86 | 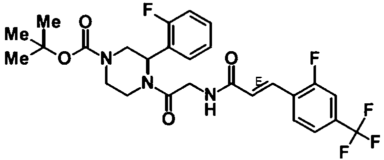 | MS(ESI) m/z:552.3 [M -H]- |
| 87 | 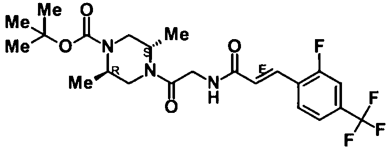 | MS(ESI) m/z:188.3 [M +H]+ |
| 88 | 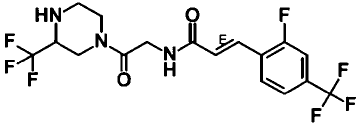 | MS (EST) m/z : 426.2 [M -H]- |
| 89 | 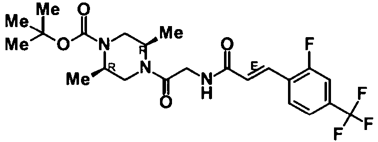 | MS(ESI) m/z:188.2 [M +H]+ |
| 90 | 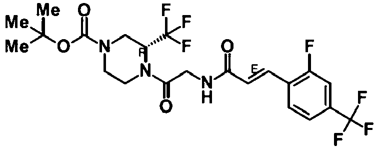 | MS(ESI) m/z:526.2 [M -H]- |

**[Table 5-7]**

| Ref. **Ex.No.** | **structural formula** | **instrument analysis data** |
|---|---|---|
| 91 | 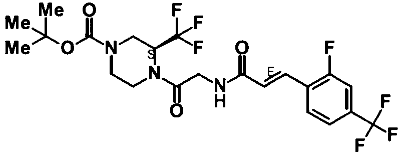 | MS (EST) m/z:526.3 [M-H]- |
| 92 | 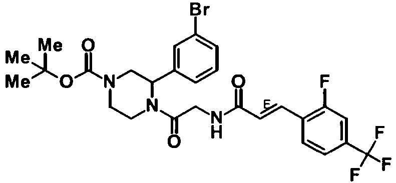 | MS(ESI) m/z:612.3/6 14.2 [M-H]- |
| 93 | 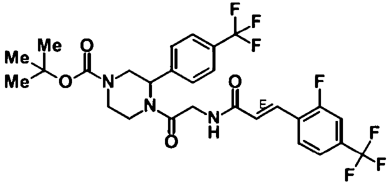 | MS(ESI) m/z:602.3 [M-H]- |
| 94 | 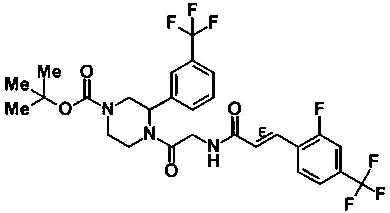 | MS(ESI) m/z:602.3 [M-H]- |
| 95 | 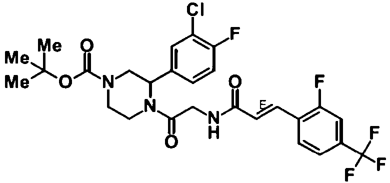 | MS (ESI) m/z:586.2/5 88. 2 [M-H]- |

**[Table 5-8]**

| **Ref. Ex.No.** | **structural formula** | **instrument analysis data** |
|---|---|---|
| 96 | 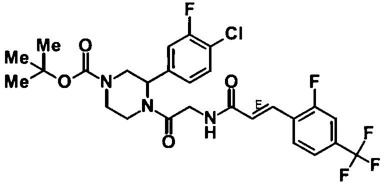 | MS (EST) m/z:586.2/5 88.2 [M-H]- |
| 97 | 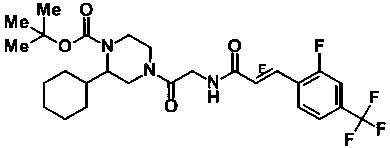 | MS (EST) m/z:540.2 [M-H]- |
| 98 | 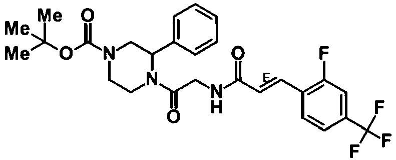 | MS(EST) m/z:534.3 [M-H]- |
| 99 | 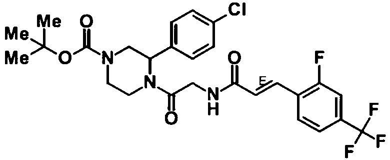 | MS (ESI) m/z:568. 3/5 70.3 [M-H]- |

### Reference Example 100:

### Production of methyl 2-[3-(1-methylpiperazin-2-yl)phenyl]acetate

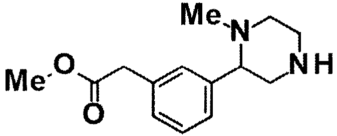

(1) Benzyl 3-(3-bromophenyl)-4-methylpiperazine-1-carboxylate (497 mg) was dissolved in DMF (4.3 ml), 1-(tert-butyldimethylsilyloxy)-1-methoxyethene (0.845 ml) and potassium carbonate (201 mg) were added, and the mixture was subjected to nitrogen replacement. To the reaction mixture was added bis(tri-tert-butylphosphine)palladium(0) (197 mg), and the mixture was stirred under microwave irradiation at 110°C for 1 hr. The reaction mixture was allowed to cool to room temperature, aqueous potassium carbonate solution was added and the mixture was extracted with ethyl acetate. The organic layer was washed with saturated brine, dried over anhydrous magnesium sulfate, and concentrated under reduced pressure. The obtained residue was purified by silica gel column chromatography (solvent: n-hexane/ethyl acetate =45/55 - 25/75) to give benzyl 3-[3-(2-methoxy-2-oxoethyl)phenyl]-4-methylpiperazine-1-carboxylate (377 mg; yield 77%) as a yellow oil.
   MS(ESI) m/z: 383.2[M+H]⁺
(3) Benzyl 3-[3-(2-methoxy-2-oxoethyl)phenyl]-4-methylpiperazine-1-carboxylate (368 mg) obtained in the aforementioned (2) was dissolved in methanol (3.2 ml), palladium hydroxide (37 mg) was added, and the mixture was subjected to hydrogen replacement. The reaction mixture was stirred at 50°C for 2 hr, and allowed to cool to room temperature, filtered through celite, and washed with methanol. The filtrate was concentrated under reduced pressure to give the title compound (239 mg; yield 100%) as a yellow amorphous.

### Reference Example 101:

### Production of methyl 2-[(3S)-1-piperidin-3-yl pyrrolidin-3-yl]acetate trifluoroacetate

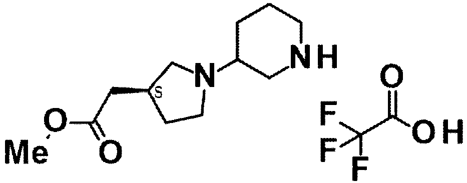

(1) 2-[(3S)-1-[(2-Methylpropan-2-yl)oxycarbonyl]pyrrolidin-3-yl]acetic acid (350 mg) was added to a mixed solution of toluene (16 ml) and methanol (4 ml), then 2 mol/l diazomethyl(trimethyl)silane/toluene solution (3 ml) was slowly added, and the mixture was stirred at room temperature for 2 hr. The solvent of the reaction mixture was evaporated, and the obtained residue was purified by reversed-phase HPLC (10 mmol/l aqueous ammonium carbonate solution/acetonitrile =60/40 - 30/70) to give tert-butyl (3S)-3-(2-methoxy-2-oxoethyl)pyrrolidine-1-carboxylate (330 mg; yield 89%) as a colorless amorphous.
   MS(ESI) m/z: 144.0[M+H-Boc]⁺
(2) To a solution of tert-butyl (3S)-3-(2-methoxy-2-oxoethyl)pyrrolidine-1-carboxylate (327 mg) obtained in the aforementioned (1) in dichloromethane (5 ml) was added trifluoroacetic acid (0.5 ml) at room temperature, and the mixture was stirred for 2 hr. The reaction mixture was concentrated under reduced pressure, toluene was added thereto, and the solvent was evaporated under reduced pressure to give a crude product of methyl 2-[(3S)-pyrrolidin-3-yl]acetate trifluoroacetate. To the obtained crude product was added 1,2-dichloroethane (7 ml), then tert-butyl 3-oxopiperidine-1-carboxylate (268 mg) and N,N'-diisopropylethylamine (0.23 mL) were successively added, and the mixture was stirred at room temperature. After confirming that the reaction mixture became a solution, acetic acid (0.3 ml) and sodium triacetoxyborohydride (569 mg) were added, and the mixture was stirred at room temperature overnight. To the reaction mixture was added saturated sodium hydrogen carbonate solution, chloroform was added thereto for extraction, and the solvent was evaporated. The obtained residue was purified by reversed-phase HPLC (10 mmol/l aqueous ammonium carbonate solution/acetonitrile =60/40 - 30/70) to give tert-butyl 3-[(3S)-3-(2-methoxy-2-oxoethyl)pyrrolidin-1-yl]piperidine-1-carboxylate (217 mg; yield 49%) as a pale-yellow amorphous. MS(ESI) m/z: 327.2[M+H]⁺
(3) To a solution of tert-butyl 3-[(3S)-3-(2-methoxy-2-oxoethyl)pyrrolidin-1-yl]piperidine-1-carboxylate (214 mg) obtained in the aforementioned (2) in dichloromethane (3 ml) was added trifluoroacetic acid (0.5 ml) at room temperature and the mixture was stirred for 2 hr. The reaction mixture was concentrated under reduced pressure, toluene was added thereto, and the solvent was evaporated under reduced pressure to give the title compound (223.1 mg) as a yellow amorphous.

### Reference Examples 102 to 104:

The corresponding starting compounds were treated in the same manner as in Reference Example 101 to obtain crude products of the compounds described in the following Table 6.

**[Table 6]**

| **Ref. Ex.No.** | **structural formula** |
|---|---|
| 102 | 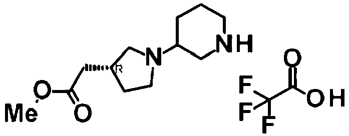 |
| 103 | 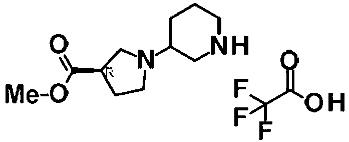 |
| 104 | 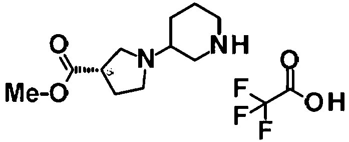 |

### Reference Example 105:

The corresponding starting compounds were treated in the same manner as in Reference Example 47 to obtain a crude product of the compound described in the following Table 7.

**[Table 7]**

| **Ref. Ex.No.** | **structural formula** |
|---|---|
| 105 | 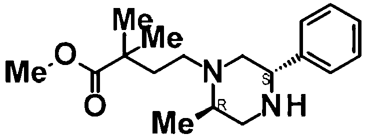 |

### Reference Example 106:

### Production of tert-butyl (2R,5S)-2-methyl-5-phenyl-piperazine-1-carboxylate

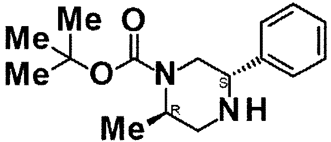

(1) A solution (19 ml) of (2S,5R)-1-benzyl-5-methyl-2-phenylpiperazine (990 mg) in dichloromethane were added triethylamine (0.78 ml) and di-tert-butyl dicarbonate (217 mg) at room temperature, and the mixture was stirred overnight. To the reaction mixture were added saturated aqueous ammonium chloride solution, the mixture was extracted with chloroform, and the organic layer was concentrated under reduced pressure. The obtained residue was purified by silica gel column chromatography (hexane/ethyl acetate =90/10 - 80/20) and a fraction containing the desired compound was concentrated under reduced pressure to give tert-butyl (2R,5S)-4-benzyl-2-methyl-5-phenyl-piperazine-1-carboxylate (1.3 g; yield 95%).
   MS(ESI) m/z: 367.2[M+H-]⁺
(2) To a solution of tert-butyl (2R,5S)-4-benzyl-2-methyl-5-phenyl-piperazine-1-carboxylate (350 mg) obtained in the aforementioned (1) in methanol (10 ml) was added palladium hydroxide (210 mg) at room temperature, and the mixture was stirred under hydrogen atmosphere for 4 hr. The reaction mixture was filtered through celite, and washed with chloroform. The filtrate was concentrated under reduced pressure to give a crude product (264 mg) of the title compound.

### Reference Example 107:

The corresponding starting compounds were treated in the same manner as in Reference Example 41 to obtain the compound described in the following Table 8.

**[Table 8]**

| **Ref. Ex.No.** | **structural formula** |
|---|---|
| 107 | 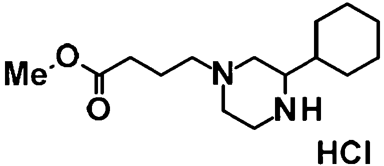 |

### Reference Example 108:

### Production of methyl 4-[(6S)-6-phenyl-4,7-diazaspiro[2.5]octan-4-yl]butanoate hydrochloride

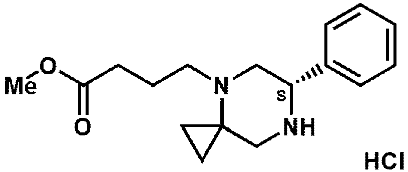

(1) To a solution of methyl (2S)-2-amino-2-phenylacetate hydrochloride (4.2 g) in DMF (60 ml) were added 1-(benzyloxycarbonylamino)cyclopropanecarboxylic acid (5.0 g), HATU (9.5 g) and N,N-diisopropylethylamine (8.03 ml), and the reaction mixture was stirred at room temperature for 1 hr. Water was added to the reaction mixture, and the mixture was extracted with ethyl acetate. The organic layer was washed with saturated brine, dried over anhydrous sodium sulfate, and concentrated under reduced pressure. The obtained residue was purified by silica gel column chromatography (hexane/ethyl acetate =80/20 - 20/80) to give methyl (2S)-2-phenyl-[[1-(benzyloxycarbonylamino)cyclopropanecarbonyl]amino]acetate (7.33 g; yield 92%) as a white solid.
   MS(ESI) m/z: 383.3[M+H]⁺
(2) Methyl (2S)-2-phenyl-[[1-(benzyloxycarbonylamino)cyclopropanecarbonyl]amino]acetate (7.33 g) obtained in the aforementioned (1) was dissolved in THF (80 ml), palladium carbon (1.0 g) was added, and the mixture was stirred under hydrogen atmosphere at room temperature for 1 hr. The reaction mixture was filtered through celite, and the filtrate was concentrated under reduced pressure at 40°C to give methyl (2S)-2-[(1-aminocyclopropanecarbonyl)amino]-2-phenylacetate (4.8 g) as a crude product.
(3) Methyl (2S)-2-[(1-aminocyclopropanecarbonyl)amino]-2-phenylacetate (4.8 g) obtained in the aforementioned (2) was dissolved in toluene (25 ml), 2-butanol (50 ml) and diisopropylethylamine (5.0 g) were added, and the reaction mixture was stirred at 110°C for 15 hr. The reaction mixture was cooled to room temperature, and the solvent was concentrated under reduced pressure to give (6S)-6-phenyl-4,7-diazaspiro[2.5]octane-5,8-dione (2.61 g; yield 63%) as a white solid.
   MS(ESI) m/z: 217.0[M+H]+
(4) (6S)-6-Phenyl-4,7-diazaspiro[2.5]octane-5,8-dione (231 mg) obtained in the aforementioned (3) was dissolved in THF (12 ml), borane dimethylsulfide (12 ml) was added, and the reaction mixture was stirred at 60°C for 15 hr. The reaction mixture was cooled to 0°C, water was slowly added, hydrochloric acid (1 mol/l, 75 ml) was added, and the mixture was stirred at room temperature for 10 min. To the reaction mixture was neutralized with aqueous sodium hydroxide solution (1 mol/l) and extracted with chloroform. The organic layer was washed with saturated brine, dried over anhydrous magnesium sulfate, and filtered. The filtrate was concentrated under reduced pressure, methanol (63 ml) and aqueous sodium hydroxide solution (1 mol/l, 30 ml) were added, and the reaction mixture was stirred at 90°C for 15 hr. The reaction mixture was cooled to room temperature and extracted with chloroform. The organic layer was washed with saturated brine, dried over anhydrous magnesium sulfate, and concentrated under reduced pressure. The obtained residue was purified by silica gel column chromatography (methanol/ethyl acetate =0/100 - 5/95) to give (6S)-6-phenyl-4,7-diazaspiro[2.5]octane (1.25 g; yield 55%) as a colorless solid.
   MS(ESI) m/z: 189.1[M+H]⁺
(5) (6S)-6-Phenyl-4,7-diazaspiro[2.5]octane (1.25 g) obtained in the aforementioned (4) was dissolved in dichloromethane (15 ml), and triethylamine (0.93 ml) and di-tert-butyl dicarbonate (1.5 g) were added thereto at 0°C. The reaction mixture was stirred at 0°C for 1 hr. The reaction mixture was concentrated under reduced pressure at 40°C. The obtained residue was purified by silica gel column chromatography (hexane/ethyl acetate =80/20 - 60/40) to give tert-butyl (6S)-6-phenyl-4,7-diazaspiro[2.5]octane-7-carboxylate (1.37 g) as a colorless oil. MS(ESI) m/z: 289.3[M+H]⁺
(6) tert-Butyl (6S)-6-phenyl-4,7-diazaspiro[2.5]octane-7-carboxylate (500 mg) obtained in the aforementioned (5) was dissolved in THF (5 ml), and methyl 4-oxobutanoate (300 mg), acetic acid (0.1 ml) and sodium triacetoxyborohydride (480 mg) were added thereto. The reaction mixture was stirred at room temperature for 2 hr. To the reaction mixture was added saturated aqueous sodium hydrogen carbonate solution, and the mixture was stirred for 10 min and extracted with ethyl acetate. The organic layer was washed with saturated brine, dried over anhydrous sodium sulfate, and concentrated under reduced pressure. The obtained residue was purified by silica gel column chromatography (hexane/ethyl acetate =80/20 - 50/50) to give tert-butyl (6S)-4-(4-methoxy-4-oxobutyl)-6-phenyl-4,7-diazaspiro[2.5]octane-7-carboxylate (638 mg; yield 95%) as a white solid.
   MS(ESI) m/z: 388.8[M+H]⁺
(7) tert-Butyl (6S)-4-(4-methoxy-4-oxobutyl)-6-phenyl-4,7-diazaspiro[2.5]octane-7-carboxylate (638 mg) obtained in the aforementioned (6) was dissolved in methanol (6 ml), and 4N hydrogen chloride-dioxane solution (4 mol/l; 6 ml) was added thereto. The reaction mixture was stirred at room temperature for 1 hr. The reaction mixture was concentrated under reduced pressure to give a crude product (533.5 mg; yield 99%) of the title compound as a white solid.

### Reference Example 109:

### Production of (2R,5S)-5-(4-chlorophenyl)-2-methyl-1-(4-nitrophenyl)sulfonylpiperazine

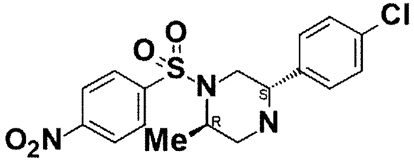

(1) To a solution of (S)-2-amino-2-(4-chlorophenyl)ethanol (1.00 g) in acetonitrile (18 ml) was added (2R)-2-methyl-1-[(4-nitrophenyl)sulfonyl]aziridine (1.41 g), and the mixture was stirred at 60°C for 3 hr. The reaction mixture was cooled to room temperature, and concentrated under reduced pressure. The residue was purified by NH silica gel chromatography (solvent: n-hexane/ethyl acetate =20/80 - 0/100) to give N-[(2R)-1-[[(1S)-1-(4-chlorophenyl)-2-hydroxyethyl]amino]propan-2-yl]-4-nitrobenzenesulfonamide (2.13 g; yield 88%) as a pale yellow viscous oil.
   MS(ESI) m/z: 414.1/416.1[M+H]⁺
(2) To a solution of N-[(2R)-1-[[(1S)-1-(4-chlorophenyl)-2-hydroxyethyl]amino]propan-2-yl]-4-nitrobenzenesulfonamide (1.81 g) obtained in the aforementioned (1) in THF (88 ml) were added triphenyl phosphine (2.29 g) and diisopropyl azodicarboxylate (40% toluene solution; about 1.9 mol/L) (3.5 ml), and the mixture was stirred at room temperature overnight. The reaction mixture was concentrated under reduced pressure, and the residue was purified by silica gel chromatography (solvent: n-hexane/ethyl acetate =65/35 - 0/100) to give the title compound (1.23 g; yield 71%) as a pale yellow solid.
   MS(ESI) m/z: 396.1/398.1[M+H]⁺

### Reference Examples 110 to 113:

The corresponding starting compounds were treated in the same manner as in Reference Example 109 to obtain crude products of the compounds described in the following Table 9.

**[Table 9]**

| **Ref. Ex.No.** | **structural formula** |
|---|---|
| 110 | 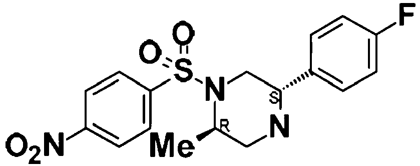 |
| 111 | 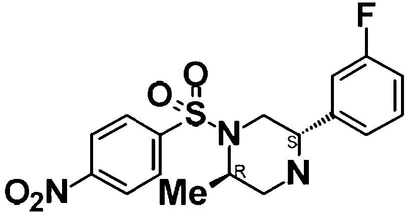 |
| 112 | 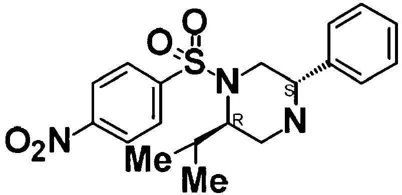 |
| 113 | 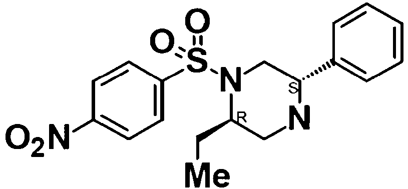 |

### Reference Example 114:

### Production of (3S,5S)-3-methyl-1-(4-nitrophenyl)sulfonyl-5-phenylpiperazine

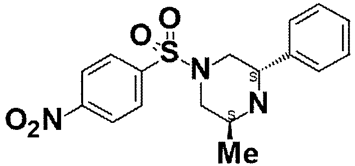

(1) To a solution of (2R)-1-(4-nitrophenyl)sulfonyl-2-phenylaziridine (3.5 g) in acetonitrile (35 ml) was added lithium perchlorate (371 mg). Successively, (2S)-2-aminopropan-1-ol (0.87 g) was added, and the mixture was washed with acetonitrile (10 ml). The reaction mixture was stirred at 60°C for 4 hr. The reaction mixture was cooled to room temperature, and concentrated under reduced pressure. The residue was purified by silica gel chromatography (solvent: chloroform/methanol=100/0 - 97/3) to give N-[(2S)-2-[[(1S)-2-hydroxy-1-methylethyl]amino]-2-phenylethyl]-4-nitrobenzenesulfonamide (2.49 g; yield 93%) as a pale yellow viscous oil.
   MS(ESI) m/z: 380.1[M+H]⁺
(2) To a solution of N-[(2S)-2-[[(1S)-2-hydroxy-1-methylethyl]amino]-2-phenylethyl]-4-nitrobenzenesulfonamide (1.97 g) obtained in the aforementioned (1) in THF (20 ml) were added triphenylphosphine (1.66 g), diisopropyl azodicarboxylate (40% toluene solution; about 1.9 mol/l) (1.2 ml), and the mixture was stirred at room temperature for 1 hr. The reaction mixture was concentrated under reduced pressure, and diisopropyl ether (10 ml) was added to the residue, and the mixture was heated in water bath at 50°C to give a solution. Seed crystals (about 2 mg) of triphenylphosphine oxide were added, and the mixture was stirred for 10 min at room temperature. The reaction suspension was filtered through Kiriyama funnel (φ40, NO5B) and washed with diisopropyl ether (15 ml). The filtrate was concentrated, and the residue was purified by silica gel chromatography (solvent: n-hexane/ethyl acetate =50/50 - 34/66) to give the title compound (399 mg; yield 23%) as a white solid.
   MS(ESI) m/z: 362.1[M+H]⁺

### Reference Example 115:

The corresponding starting compounds were treated in the same manner as in Reference Example 114 to obtain a crude product of the compound described in the following Table 10.

**[Table 10]**

| **Ref. Ex.No.** | **structural formula** |
|---|---|
| 115 | 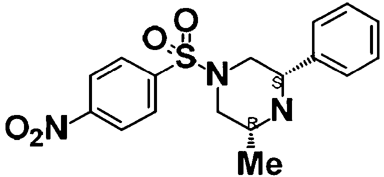 |

### Reference Example 116:

### Production of methyl 4-[(2R,5S)-5-(4-chlorophenyl)-2-methylpiperazin-1-yl]butanoate

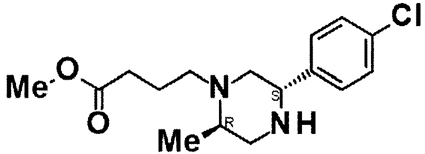

(1) (S)-2-Amino-2-(4-chlorophenyl)ethanol (2.8 g) was dissolved in THF (50 ml), and 2,4-dimethoxybenzaldehyde (2.85 g), acetic acid (0.94 ml) and sodium triacetoxyborohydride (4.5 g) were added thereto. The reaction mixture was stirred at room temperature for 2 hr. To the reaction mixture were added chloroform and water, and potassium carbonate was added thereto to set the pH to about 9, and the mixture was extracted with chloroform. The organic layer was washed with saturated brine, dried over anhydrous sodium sulfate, and concentrated under reduced pressure. The obtained residue was purified by silica gel column chromatography (hexane/ethyl acetate =70/30 - 40/60) to give (2S)-2-(4-chlorophenyl)-2-[(2,4-dimethoxyphenyl)methylamino]ethanol (4.37 g; yield 83%) as a white solid.
   MS(ESI) m/z: 322.1/324.1[M+H]⁺
(2) To a solution of (2S)-2-(4-chlorophenyl)-2-[(2,4-dimethoxyphenyl)methylamino]ethanol (4.27 g) obtained in the aforementioned (1) in acetonitrile (40 ml) was added (R)-2-methyl-1-[(4-nitrophenyl)sulfonyl]aziridine (3.21 g), and the mixture was stirred at 60°C for 7 hr. The reaction mixture was cooled to room temperature and concentrated under reduced pressure. The residue was purified by NH silica gel chromatography (solvent: n-hexane/ethyl acetate =50/50 - 0/100) to give N-[(2R)-1-[[(1S)-1-(4-chlorophenyl)-2-hydroxyethyl]-[(2,4-dimethoxyphenyl)methyl]amino]propan-2-yl]-4-nitrobenzenesulfonamide (7.00 g; yield 94%) as a pale yellow viscous oil.
   MS(ESI) m/z: 564.2/566.2[M+H]⁺
(3) To a solution of N-[(2R)-1-[[(1S)-1-(4-chlorophenyl)-2-hydroxyethyl]-[(2,4-dimethoxyphenyl)methyl]amino]propan-2-yl]-4-nitrobenzenesulfonamide (4.27 g) obtained in the aforementioned (2) in THF (40 ml) were added triphenyl phosphine (3.49 g), diisopropyl azodicarboxylate (40% toluene solution; about 1.9 mol/l) (6.1 ml), and the mixture was stirred at room temperature overnight. The reaction mixture was concentrated under reduced pressure, and the residue was purified by silica gel chromatography (solvent: n-hexane/ethyl acetate =80/20 - 60/40) to give (2S,5R)-2-(4-chlorophenyl)-1-[(2,4-dimethoxyphenyl)methyl]-5-methyl-4-(4-nitrophenyl)sulfonylpiperazine (4.00 g; yield 83%) as a pale yellow amorphous.
   MS(ESI) m/z: 546.2/548.2[M+H]⁺
(4) To a solution of (2S,5R)-2-(4-chlorophenyl)-1-[(2,4-dimethoxyphenyl)methyl]-5-methyl-4-(4-nitrophenyl)sulfonylpiperazine (4.00 g) obtained in the aforementioned (3) in methanol (73 mL) were added potassium carbonate (5.07 g) and thioglycolic acid (1.28 ml), and the mixture was stirred at 60°C for 2 hr. Water was added to the reaction mixture, and the mixture was extracted with chloroform. The organic layer was washed with saturated aqueous sodium hydrogen carbonate solution, dried over anhydrous sodium sulfate, and concentrated under reduced pressure. The residue was purified by silica gel chromatography (solvent: n-hexane/ethyl acetate =67/33 - 50/50) to give (2S,5R)-2-(4-chlorophenyl)-1-[(2,4-dimethoxyphenyl)methyl]-5-methylpiperazine (1.85 g; yield 70%) as a pale yellow solid.
   MS(ESI) m/z: 361.2/363.1[M+H]⁺
(5) (2S,5R)-2-(4-chlorophenyl)-1-[(2,4-dimethoxyphenyl)methyl]-5-methylpiperazine (350 mg) obtained in the aforementioned (4) was dissolved in DMF (2.4 ml), and methyl 4-bromobutanoate (0.18 ml), potassium carbonate (402 mg) and sodium iodide (145 mg) were added thereto. The reaction mixture was stirred at 80°C for 2 hr 30 min. To the reaction mixture were added ethyl acetate and water, and the mixture was extracted with ethyl acetate. The organic layer was washed with water and saturated brine, dried over anhydrous sodium sulfate, and concentrated under reduced pressure. The obtained residue was purified by silica gel column chromatography (hexane/ethyl acetate =75/25 - 40/60) to give methyl 4-[(2R,5S)-5-(4-chlorophenyl)-4-[(2,4-dimethoxyphenyl)methyl]-2-methylpiperazin-1-yl]butanoate (438 mg; yield 98%) as a colorless viscous oil.
   MS(ESI) m/z: 461.4/463.4[M+H]⁺
(6) Methyl 4-[(2R,5S)-5-(4-chlorophenyl)-4-[(2,4-dimethoxyphenyl)methyl]-2-methylpiperazin-1-yl]butanoate (430 mg) obtained in the aforementioned (5) was dissolved in trifluoroacetic acid (3.7 ml), and Milli-Q water (1.9 ml) was added thereto. The reaction mixture was stirred at 60°C for 2 hr. To the reaction mixture was added chloroform, and potassium carbonate was added to adjust to around pH 9. The mixture was extracted with chloroform, and the organic layer was washed with water and saturated brine, dried over anhydrous sodium sulfate, and concentrated under reduced pressure. The obtained residue was purified by silica gel column chromatography (hexane/ethyl acetate =75/25 - 60/40) to give the title compound (79.5 mg; yield 27%) as a colorless viscous oil. MS(ESI) m/z: 311.2/313.2[M+H]⁺

### Reference Examples 117 to 124:

The corresponding starting compounds were treated in the same manner as in Reference Example 116 to obtain crude products of the compounds described in the following Table 11-1 and Table 11-2.

**[Table 11-1]**

| **Ref. Ex.No.** | **structural formula** |
|---|---|
| 117 | 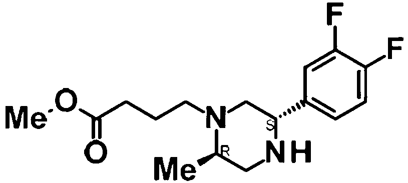 |
| 118 | 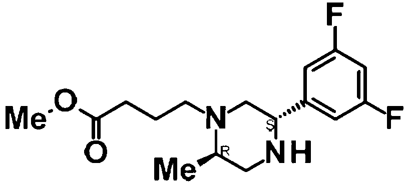 |
| 119 | 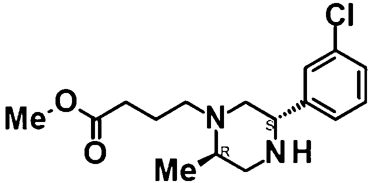 |
| 120 | 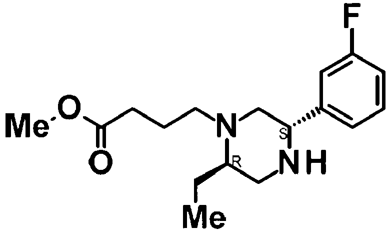 |

**[Table 11-2]**

| **Ref. Ex.No.** | **structural formula** |
|---|---|
| 121 | 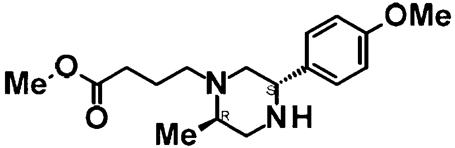 |
| 122 | 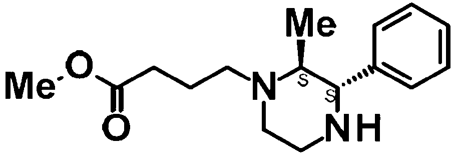 |
| 123 | 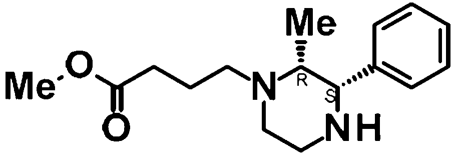 |
| 124 | 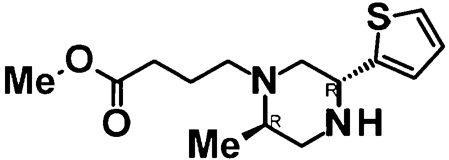 |

### Reference Example 125:

### Production of methyl 3-[(2R,5S)-5-(4-chlorophenyl)-2-methylpiperazin-1-yl]propanoate

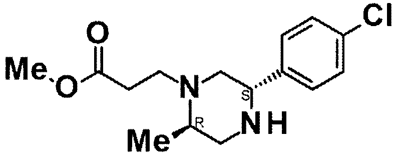

(1) (2S,5R)-2-(4-chlorophenyl)-1-[(2,4-dimethoxyphenyl)methyl]-5-methylpiperazine (350 mg) was dissolved in dichloromethane (5.0 ml), and methyl 3-oxopropanoate (300 mg) and sodium triacetoxyborohydride (1.03 g) were added thereto. The reaction mixture was stirred at room temperature for 3 hr. Methyl 3-oxopropanoate (150 mg) was added again, and the reaction mixture was stirred for 2 hr 30 min. To the reaction mixture were added chloroform and water, and the mixture was extracted with chloroform. The organic layer was washed with saturated aqueous sodium hydrogen carbonate solution and saturated brine, dried over anhydrous sodium sulfate, and concentrated under reduced pressure. The obtained residue was purified by silica gel column chromatography (hexane/ethyl acetate =85/15 - 70/40) to give methyl 3-[(2R,5S)-5-(4-chlorophenyl)-4-[(2,4-dimethoxyphenyl)methyl]-2-methylpiperazin-1-yl]propanoate (394.9 mg; yield 91%) as a yellow viscous oil.
   MS(ESI) m/z: 447.4/449.3[M+H]⁺
(2) Methyl 3-[(2R,5S)-5-(4-chlorophenyl)-4-[(2,4-dimethoxyphenyl)methyl]-2-methylpiperazin-1-yl]propanoate (430 mg) obtained in the aforementioned (1) was dissolved in trifluoroacetic acid (3.5 ml), and Milli-Q water (0.18 ml) was added thereto. The reaction mixture was stirred at 40°C for 1 hr. To the reaction mixture was added chloroform, and the mixture was neutralized with saturated aqueous sodium hydrogen carbonate solution, extracted with chloroform, and the organic layer was washed with water and saturated brine, dried over anhydrous sodium sulfate, and concentrated under reduced pressure. The obtained residue was purified by silica gel column chromatography (hexane/ethyl acetate =75/25 - 60/40) to give the title compound (217.4 mg; yield 84%) as a colorless viscous oil.
   MS(ESI) m/z: 297.2/299.2[M+H]⁺

### Reference Examples 126 to 128:

The corresponding starting compounds were treated in the same manner as in Reference Example 125 to obtain crude products of the compounds described in the following Table 12.

**[Table 12]**

| **Ref. Ex.No.** | **structural formula** |
|---|---|
| 126 | 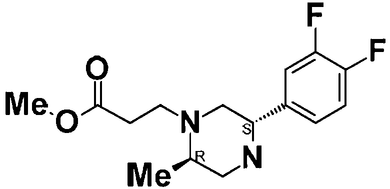 |
| 127 | 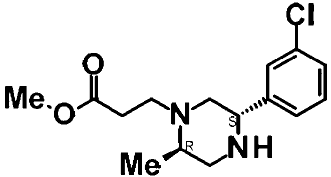 |
| 128 | 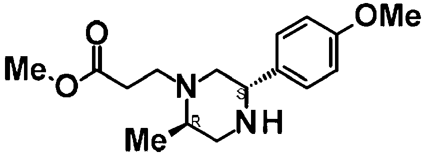 |

### Reference Example 129 - 135:

The corresponding starting compounds were treated in the same manner as in Reference Example 60 to obtain crude products of the compounds described in the following Table 13-1 and Table 13-2.

**[Table 13-1]**

| **Ref. Ex.No.** | **structural formula** | **instrument analysis data** |
|---|---|---|
| 129 | 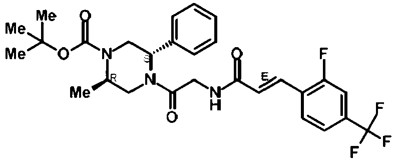 | MS (ESI) m/z: 550.3[M+H]+ |
| 130 | 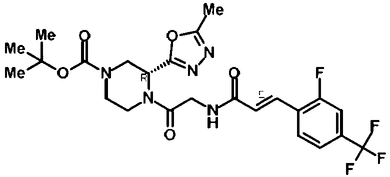 | MS(ESI) m/z: 542.2[M+H]+ |
| 131 | 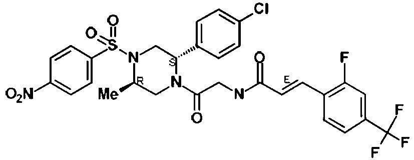 | MS(ESI) m/z: 669.2/671 .2[M+H]+ |
| 132 | 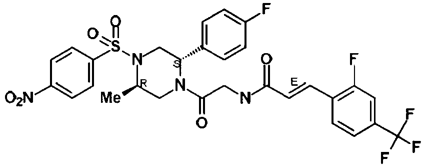 | MS(ESI) m/z: 653.2[M+H ]+ |
| 133 | 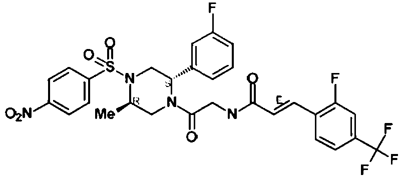 | MS(ESI) m/z: 653.2[M+H ]+ |
| 134 | 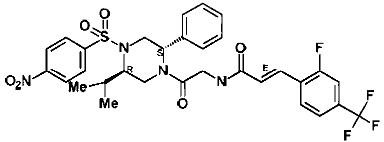 | MS(ESI) m/z: 663.3[M+H ]+ |

**[Table 13-2]**

| **Ref. Ex.No.** | **structural formula** | **instrument analysis data** |
|---|---|---|
| 135 | 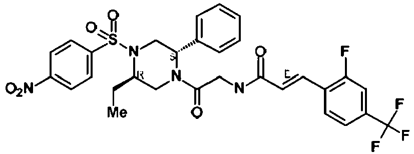 | MS(ESI) m/z: 649.2[M+H]+ |

### Reference Example 136:

### Production of methyl (2R)-2-(5-chloro-2-thienyl)-2-[(2,4-dimethoxyphenyl)methylaminoethanol

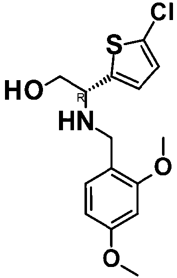

(1) (2R)-2-tert-butoxycarbonylamino)-2-(2-thienyl)acetic acid (1.0 g) was dissolved in methanol (10 ml) and toluene (40 ml), (trimethylsilyl)diazomethane (7.0 ml, 0.6 mol/l hexane solution) was slowly added thereto, and the mixture was stirred at room temperature for 2 hr. The reaction mixture was concentrated under reduced pressure, and the obtained residue was purified by silica gel column chromatography (hexane/ethyl acetate =90/10 - 50/50) to give methyl (2R)-2-tert-butoxycarbonylamino)-2-(2-thienyl)acetate (1.0 g; yield 97%) as a colorless viscous oil.
(2) To a solution of methyl (2R)-2-tert-butoxycarbonylamino)-2-(2-thienyl)acetate (400 mg) obtained in the aforementioned (1) in dichloromethane (7.5 ml) was added N-chlorosuccinimide (235 mg), and the mixture was stirred at room temperature for 2 hr. N-chlorosuccinimide (235 mg) was added again and the mixture was stirred at room temperature overnight. To the reaction mixture was added saturated aqueous sodium hydrogen carbonate solution, and the mixture was extracted with chloroform. The organic layer was concentrated under reduced pressure, and the residue was purified by NH silica gel chromatography (solvent: n-hexane/ethyl acetate =90/10 - 50/50) to give (2R)-2-tert-butoxycarbonylamino-2-(5-chloro-2-thienyl)acetic acid (235 mg; yield 52%) as a colorless viscous oil.
(3) To a mixture of ethanol (2.7 ml), lithium chloride (84 mg) and sodium borohydride (75 mg) was added a solution of (2R)-2-tert-butoxycarbonylamino)-2-(5-chloro-2-thienyl)acetic acid (232 mg) obtained in the aforementioned (2) in THF (2.7 ml), and the mixture was stirred at room temperature for 2 hr. The reaction mixture was cooled to 0°C, saturated aqueous ammonium chloride solution was added, and the mixture was extracted with chloroform. The organic layer was concentrated under reduced pressure, and the residue was purified by silica gel column chromatography (hexane/ethyl acetate =80/20 - 40/60) to give tert-butyl-N-[(1R)-1-(5-chloro-2-thienyl)-2-hydroxyethyl]carbamate (188 mg; yield 89%) as a colorless solid.
(4) To a solution of tert-butyl-N-[(1R)-1-(5-chloro-2-thienyl)-2-hydroxyethyl]carbamate (185 mg) obtained in the aforementioned (3) in dichloromethane (5 mL) was added 2,2,2-trifluoroacetic acid (0.5 ml), and the mixture was stirred at room temperature overnight. To the reaction mixture was added toluene, and the solvent was evaporated under reduced pressure. To the residue was added diethylether, and the precipitated solid was collected by filtration to give (2R)-2-amino-2-(5-chloro-2-thienyl)ethanol (194 mg; yield 99%) as a pale yellow solid.
(5) (2R)-2-Amino-2-(5-chloro-2-thienyl)ethanol (191 mg) obtained in the aforementioned (4) was dissolved in THF (4.0 ml), 2,4-dimethoxybenzaldehyde (108 mg) and diisopropylethylamine (0.11 ml) was added thereto, and the mixture was stirred for 1 min. To the reaction mixture were added acetic acid (0.1 ml) and sodium triacetoxyborohydride (180 mg), and the mixture was stirred at room temperature for 3 hr. To the reaction mixture were added saturated aqueous sodium hydrogen carbonate solution and ethyl acetate, and the mixture was extracted with ethyl acetate. The organic layer was concentrated under reduced pressure. The obtained residue was purified by NH silica gel column chromatography (hexane/ethyl acetate =90/10 - 34/66) to give the title compound (78 mg; yield 36%) as a colorless solid.
   MS(ESI) m/z: 328.2/330.2[M+H]⁺

### Reference Example 137:

### Production of (2S)-1-(4-nitrophenyl)sulfonyl -2-(trifluoromethyl)aziridine

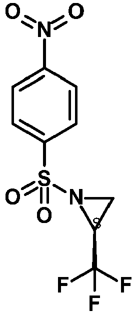

(1) (2S)-2-Amino-3,3,3-trifluoro-propan-1-ol hydrochloride (750 mg) was dissolved in pyridine (1.5 ml) and acetonitrile (10 ml), and the mixture was stirred for 30 min. To the reaction mixture was added 4-nitrobenzenesulfonyl chloride (2.3 g), and the mixture was stirred at room temperature for 1 hr. To the reaction mixture was added saturated aqueous citric acid solution, and the mixture was extracted with ethyl acetate. The organic layer was washed with saturated brine, dried, and concentrated under reduced pressure. The obtained residue was directly purified by silica gel column chromatography (hexane/ethyl acetate =100/0 - 70/30) to give 4-nitro-N-[(1S)-2,2,2-trifluoro-1-(hydroxymethyl)ethyl]benzenesulfonamide (843 mg; yield 59%) as a colorless solid.
(2) To a solution of 4-nitro-N-[(1S)-2,2,2-trifluoro-1-(hydroxymethyl)ethyl]benzenesulfonamide (843 mg) obtained in the aforementioned (1) in THF (6.0 ml) were added triphenylphosphine (774 mg), diisopropyl azodicarboxylate (1.9 mol/l, THF solution) (1.4 ml), and the mixture was stirred at room temperature for 1 hr. The reaction mixture was concentrated under reduced pressure, and the residue was purified by silica gel chromatography (solvent: n-hexane/ethyl acetate =100/0 - 70/30) to give the title compound (395 mg; yield 49%) as a colorless solid.

### Reference Example 138:

### Production of (2R)-1-(4-nitrophenyl)sulfonyl -2-(trideuteriomethyl)aziridine

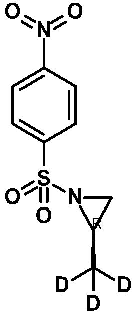

(1) D-Alanine-3,3,3-D3 (1.0 g) was dissolved in 1,4-dioxane (33.0 ml)), 1N aqueous sodium hydroxide solution (33 ml) and di-tert-butyl dicarbonate (2.8 g) were added thereto, and the mixture was stirred at room temperature for 24 hr. The reaction mixture was washed with diethylether, and the aqueous layer was extracted with ethyl acetate. The organic layer was dried·concentrated under reduced pressure to give (2R)-2-(tert-butoxycarbonylamino)-3,3,3-trideuterio-propanoic acid (1.9 g; yield 90%) as a colorless solid.
   MS(ESI) m/z: 191.2[M+H]⁺
(2) A solution of (2R)-2-(tert-butoxycarbonylamino)-3,3,3-trideuterio-propanoic acid (1.9 g) obtained in the aforementioned (1) in THF (6.6 ml) was cooled to 0°C, boranetetrahydrofuran complex (1.0 mol/l, THF solution) (20 ml) was added thereto, and the mixture was stirred at room temperature for 3 hr 30 min. To the reaction mixture was added water in water bath, ethyl acetate and saturated aqueous sodium hydrogen carbonate solution were added thereto, and the mixture was extracted with ethyl acetate. The organic layer was washed with saturated brine, concentrated under reduced pressure, and the residue was purified by silica gel chromatography (solvent: n-hexane/ethyl acetate =70/30 - 50/50) to give tert-butyl N-[(1R)-2,2,2-trideuterio-1-(hydroxymethyl)ethyl]carbamate (853 mg; yield 49%) as a colorless solid.
   MS(ESI) m/z: 179.2[M+H]⁺
(3) tert-Butyl N-[(1R)-2,2,2-trideuterio-1-(hydroxymethyl)ethyl]carbamate (853 mg) was dissolved in 1,4-dioxane (12 ml) and 4N hydrochloric acid (1,4-dioxane solution) (12 ml), and the mixture was stirred for 1 hr. Methanol (6 ml) was added thereto, and the mixture was further stirred at room temperature for 4 hr. The reaction mixture was concentrated under reduced pressure to give (2R)-2-amino-3,3,3-trideuterio-propan-1-ol hydrochloride (556 mg; yield 98%) as a colorless solid.
   MS(ESI) m/z: 79.0[M+H]⁺
(4) (2R)-2-Amino-3,3,3-trideuterio-propan-1-ol hydrochloride (552 mg) was dissolved in pyridine (1.2 ml) and acetonitrile (3.2 ml), triethylamine (1.0 ml) was added thereto under ice-cooling, and the mixture was stirred for 30 min. Successively, 4-nitrobenzenesulfonyl chloride (2.4 g) was added to the reaction mixture, and the mixture was stirred under ice-cooling for 2 hr. To the reaction mixture were added ethyl acetate (50 ml) and water (50 ml), and the mixture was washed with 1N aqueous citric acid solution (30 ml). To the organic layer was added water (50 ml), and then added isopropylamine (1.4 ml), and the mixture was stirred for 1 hr. To the reaction mixture was added 1N aqueous citric acid solution (30 ml), and the mixture was extracted with ethyl acetate. The organic layer was washed with 1N aqueous citric acid solution, dried and concentrated under reduced pressure to give the title compound (999 mg; yield 85%) as a pale yellow solid.

### Reference Example 139:

The corresponding starting compounds were treated in the same manner as in Reference Example 116 to obtain the compound described in the following Table 14.

**[Table 14]**

| Reference Example No. | structural formula |
|---|---|
| 139 | 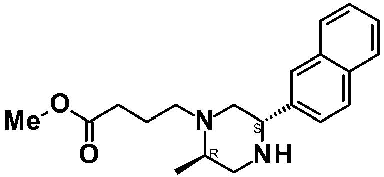 |

### Reference Examples 140 to 143:

The corresponding starting compounds were treated in the same manner as in Reference Example 125 to obtain crude products of the compounds described in the following Table 15.

**[Table 15]**

| Reference Example No. | structural formula |
|---|---|
| 140 | 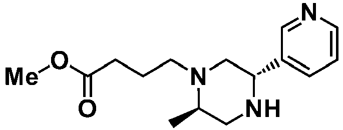 |
| 141 | 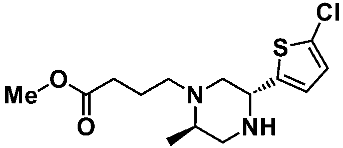 |
| 142 | 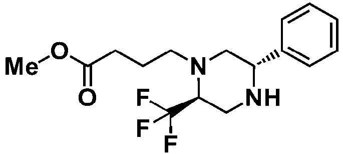 |
| 143 | 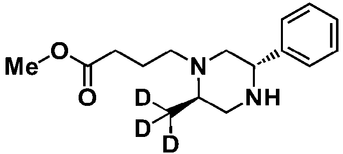 |

### Reference Example 144:

### Production of tert-butyl 3-(1,2-benzoxazol-3-yl)piperazine-1-carboxylate

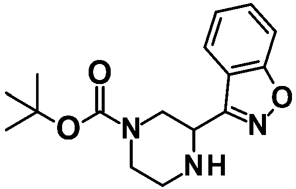

(1) Butylmagnesium chloride (530 ml:2 mol/l) was cooled to 0°C, isopropylamine (108 g) was added thereto, and the mixture was stirred at 50°C for 2 hr. After cooling to 0°C again, a solution of methyl benzo[D]isoxazole-3-carboxylic acid (31.3 g) and chloroacetic acid (51 g) in THF was added, and the mixture was warmed to room temperature and stirred overnight. 1N Hydrochloric acid and ethyl acetate were added thereto, the mixture was extracted with ethyl acetate, and the organic layer was dried and concentrated under reduced pressure. The residue was purified by silica gel column chromatography to give 1-(1,2-benzoxazol-3-yl)-2-chloro-ethanone (31.3 g; yield 91%).
(2) 1-(1,2-Benzoxazol-3-yl)-2-chloro-ethanone (500 mg) was dissolved in 1,4-dioxane (8.8 ml) and, after cooling to 0°C, a solution of ethylenediamine (0.85 ml) in 1,4-dioxane (1.8 ml) was added thereto, and the mixture was stirred at room temperature for 24 hr. The reaction mixture was concentrated under reduced pressure. To the residue were added methanol (10.7 ml) and water (0.5 ml), sodium borohydride (484 mg) was added thereto under ice-cooling, and the mixture was stirred at room temperature for 5 hr. To the reaction mixture was added 1N hydrochloric acid and, after stirring for 10 min, potassium carbonate was added thereto, and the mixture was extracted with chloroform. The organic layer was washed with water and saturated brine, dried and concentrated under reduced pressure. The residue was purified by NH silica gel column chromatography (methanol/ethyl acetate =0/100 - 5/95) to give 3-piperazin-2-yl-1,2-benzoxazole (209 mg; yield 40%) as a yellow oil.
   MS(ESI) m/z: 204.0[M+H]⁺
(3) To a solution of 3-piperazin-2-yl-1,2-benzoxazole (209 mg) obtained in the aforementioned (1) in dichloromethane (4.0 ml) were added triethylamine (0.42 ml) and di-tert-butyl dicarbonate (233 mg), and the mixture was stirred at room temperature for 18 hr. Water was added to the reaction mixture, and the mixture was extracted with chloroform. The organic layer was washed with water and saturated brine, and dried and concentrated under reduced pressure. The residue was purified by silica gel chromatography (solvent: n-hexane/ethyl acetate =70/30 - 50/50) to give the title compound (168.8 mg; yield 55%) as a colorless oil.
   MS(ESI) m/z: 304.2[M+H]⁺

### Reference Example 145:

The corresponding starting compounds were treated in the same manner as in Reference Example 116 to obtain the compound described in the following Table 16.

**[Table 16]**

| Reference Example No. | structural formula |
|---|---|
| 145 | 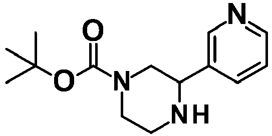 |

### Reference Example 146:

### Production of tert-butyl 3-(3-phenyl-1,2,4-oxadiazol-5-yl)piperazine-1-carboxylate

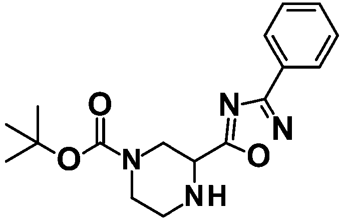

(1) O1-tert-Butyl O3-methyl piperazine-1,3-dicarboxylate (5.0 g) was dissolved in 1,4-dioxane (20.5 ml) and, after cooling to 0°C, 2N aqueous sodium hydroxide solution (22.5 ml) and di-tert-butyl dicarbonate (5.4 g) were added, and the mixture was stirred at room temperature for 20 hr. The reaction mixture was washed with diisopropylether and water. To the aqueous layer were added ethyl acetate and 1N hydrochloric acid, and the mixture was extracted with ethyl acetate. The organic layer was washed with water and saturated brine, dried and concentrated under reduced pressure to give 1,4-bis(tert-butoxycarbonyl)piperazine-2-carboxylic acid (4.4 g; yield 65%) as a yellow solid.
   MS(ESI) m/z: 329.4[M-H]⁻
(2) To a solution of 1,4-bis(tert-butoxycarbonyl)piperazine-2-carboxylic acid (2.5 g) obtained in the aforementioned (1) in DMF (30 ml) were added 1-[bis(dimethylamino)methylene]-1H-benzotriazolium 3-oxide tetrafluoroborate triethylamine (2.4 g), HOBt (205 mg), triethylamine (6.55 ml) and 1-hydroxybenzotriazole (1.1 g), and the mixture was stirred at room temperature for 2 hr. The reaction mixture was heated to 110°C and stirred for 4 hr. Water was added to the reaction mixture, and the precipitated solid was collected by filtration to give di-tert-butyl 2-(3-phenyl-1,2,4-oxadiazol-5-yl)piperazine-1,4-carboxylate (2.88 g; yield 88%) as a pale yellow solid.
   MS(ESI) m/z: 431.4[M+H]⁺
(3) To a solution of di-tert-butyl 2-(3-phenyl-1,2,4-oxadiazol-5-yl)piperazine-1,4-carboxylate (2.88 g) obtained in the aforementioned (2) in dichloromethane (13.4 ml) was added 2,2,2-trifluoroacetic acid (13.4 ml), and the mixture was stirred at room temperature for 2 hr. Chloroform was added to the reaction mixture, and the mixture was concentrated under reduced pressure. The precipitated solid was collected by filtration to give di-tert-butyl 3-phenyl-5-piperazin-2-yl-1,2,4-oxadiazole; 2,2,2-trifluoroacetate (2.78 g; yield 90%) as a pale yellow solid.
   MS(ESI) m/z: 231.2[M+H]⁺
(4) To a solution of di-tert-butyl 3-phenyl-5-piperazin-2-yl - 1,2,4-oxadiazole; 2,2,2-trifluoroacetate (2.78 g) obtained in the aforementioned (3) in 1,4-dioxane (24.0 ml) were added triethylamine (2.96 ml) and di-tert-butyl dicarbonate (1.3 g) under ice-cooling, and the mixture was stirred at the same temperature for 1 hr 30 min, warmed to room temperature and then stirred for 2 hr. To the reaction mixture were added ethyl acetate and water, and the mixture was extracted. The organic layer was washed successively with water and saturated brine, dried and concentrated under reduced pressure. The residue was purified by NH silica gel chromatography (solvent: n-hexane/ethyl acetate =65/35 - 50/50) to give to give the title compound (1.96 g; yield 98%) as a yellow oil.
   MS(ESI) m/z: 331.3[M+H]⁺

### Reference Example 147:

### Production of O1-tert-butyl O3-methyl (3R)-piperazine-1,3-dicarboxylate

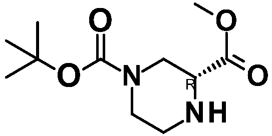

(1) (R)-1-((benzyloxy)carbonyl)-4-(tert-butoxycarbonyl)piperazine-2-carboxylic acid (5.0 g) was dissolved in DMF (45.7 ml), calcium carbonate (3.8 g) and methyl iodide (0.94 ml) were added thereto, and the mixture was stirred at room temperature for 1 hr. To the reaction mixture were added ethyl acetate and saturated aqueous ammonium chloride solution and the mixture was extracted. The organic layer was washed successively with water and saturated brine, dried and concentrated under reduced pressure to give O1-benzyl O4-tert-butyl O2-methyl (2R)-piperazine-1,2,4-tricarboxylate (5.6 g; yield 108%) as a pale yellow solid.
(2) To a solution of O1-benzyl O4-tert-butyl O2-methyl (2R)-piperazine-1,2,4-tricarboxylate (5.6 g) obtained in the aforementioned (1) in methanol (74 ml) was added 10% palladium catalyst (2.5 g), and the mixture was stirred at room temperature under hydrogen atmosphere for 4 hr. The reaction mixture was filtered through celite and washed with chloroform. The filtrate was concentrated under reduced pressure to give the title compound (3.3 g; yield 91%) as a yellow oil.

### Reference Examples 148 to 151:

The corresponding starting compounds were treated in the same manner as in Reference Example 60 to obtain the compounds described in the following Table 17.

**[Table 17]**

| **Ref. Ex. No.** | **structural formula** | **instrument analysis data** |
|---|---|---|
| 148 | 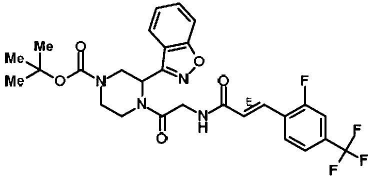 | MS(ESI) m/z: 575.4 [ M-H]- |
| 149 | 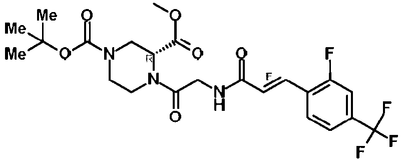 | MS(ESI) m/z: 462.2[M +H-tBu]+ |
| 150 | 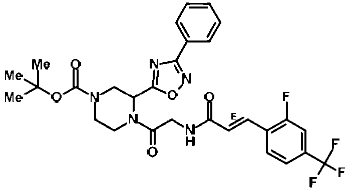 | MS(ESI) m/z: 602.4 [ M-H]- |
| 151 | 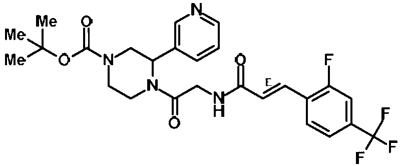 | MS(ESI) m/z: 537.3 [ M+H]+ |

### Reference Example 152:

### Production of tert-butyl (3R)-4-[2-[[(E)-3-[2-fluoro-4-(trifluoromethyl)phenyl]prop-2-enoyl]amino]acetyl]-3-(3-methyl-1,2,4-oxadiazol-5-yl)piperazine-1-carboxylate

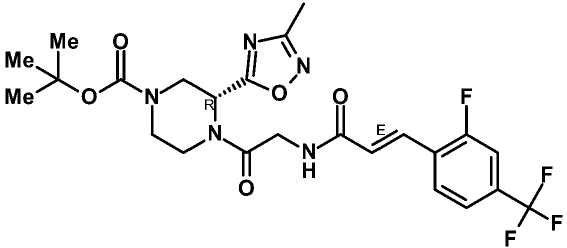

(1) O1-tert-Butyl O3-methyl (3R)-piperazine-1,3-dicarboxylate (581 mg) was dissolved in DMF (10 ml), diisopropylethylamine (1.0 ml), HATU (797 mg), and 2-[[2-[[(E)-3-[2-fluoromethyl-4-(trifluoromethyl)phenyl]prop-2-enoylamino acid were added thereto, and the mixture was stirred at room temperature for 1 hr. To the reaction mixture were added ethyl acetate and aqueous ammonium chloride solution, and the mixture was extracted with ethyl acetate. The organic layer was washed successively with water and saturated brine, dried and concentrated under reduced pressure to give O1-tert-butyl 03-methyl (3R)-4-[2-[[(E)-3-[2-fluoro-4-(trifluoromethyl)phenyl]prop-2-enoyl]amino]acetyl]piperazine-1, 3-carboxylate (970 mg; yield 94%) as a yellow oil.
   MS(ESI) m/z: 462.2[M-tBu+2H]⁺
(2) To a solution of O1-tert-butyl O3-methyl (3R)-4-[2-[[(E)-3-[2-fluoro-4-(trifluoromethyl)phenyl]prop-2-enoyl]amino]acetyl]piperazine-1,3-carboxylate (970 mg) obtained in the aforementioned (1) in methanol (16.0 ml) was added 1N aqueous sodium hydroxide solution (16 ml), and the mixture was stirred at room temperature for 2 hr. To the reaction mixture were added 1N hydrochloric acid (4.0 ml) and water (4.0 ml), and the precipitated solid was collected by filtration to give (2R)-4-tert-butoxycarbonyl-1-[2-[[(E)-3-[2-fluoro-4-(trifluoromethyl)phenyl]prop-2-enoyl]amino]acetyl]piperazine-2-carboxylic acid (910 mg; yield 94%) as a pale-yellow solid.
   MS(ESI) m/z: 502.3[M-H]⁻
(3) To a solution of (2R)-4-tert-butoxycarbonyl -1-[2-[[(E)-3-[2-fluoro-4-(trifluoromethyl)phenyl]prop-2-enoyl]amino]acetyl]piperazine-2-carboxylic acid (200 mg) obtained in the aforementioned (2) in DMF (2.5 ml) were added 1,1'-carbonyldiimidazole (97 mg) and acetamidoxime (30 mg), and the mixture was stirred at 120°C for 7 hr. To the reaction mixture were added saturated aqueous sodium hydrogen carbonate and chloroform, and the mixture was partitioned. The organic layer was concentrated under reduced pressure, and the residue was purified by reversed-phase HPLC (10 mmol/1 aqueous ammonium carbonate solution/acetonitrile =60/40 - 30/70) to give the title compound (31 mg; yield 14%) as a colorless viscous oil.
   MS(ESI) m/z: 542.4[M+H]⁺

### Example 1:

### Production of tert-butyl 2-[2-[2-[[(E)-3-(4-chlorophenyl)prop-2-enoyl]amino]acetyl]-3,4-dihydro-1H-isoquinolin-6-yl]acetate (compound (I-1))

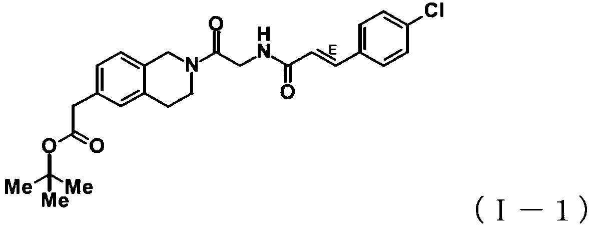

To a solution of tert-butyl 2-(1,2,3,4-tetrahydroisoquinolin-6-yl)acetate (30 mg) produced in Reference Example 8 in DMF (1 ml) were added 2-[[(E)-3-(4-chlorophenyl)prop-2-enoyl]amino]acetic acid (35 mg), N,N-diisopropylethylamine (0.042 ml) and HATU (65 mg) at room temperature, and the reaction mixture was stirred at room temperature for 3 hr. Water was added to the reaction mixture, and the aqueous layer was extracted with ethyl acetate. The organic layer was washed successively with 1N aqueous sodium hydroxide solution and 1N hydrochloric acid. The solvent was evaporated under reduced pressure, and the obtained residue was purified by column chromatography (solvent: n-hexane/ethyl acetate =100/0 - 20/80) to give the title compound (40.6 mg; yield 68.5%) as a colorless oil.
MS(ESI) m/z: 469.3/471.4[M+H]⁺

### Examples 2 to 82:

The corresponding starting compounds were treated in the same manner as in Example 1 to obtain the compounds described in the following Table 18-1 to Table 18-16.

**[Table 18-1]**

| **Example No. (compound No.)** | **structural formula** | **instrument analysis data** |
|---|---|---|
| 2 (I-2) | 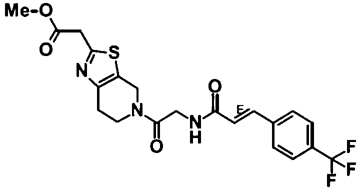 | MS(ESI) m/z:468.3 [M+H]+ |
| 3 (I-3) | 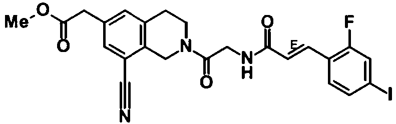 | MS (ESI) m/z:562.2 [M+H]+ |
| 4 (I-4) | 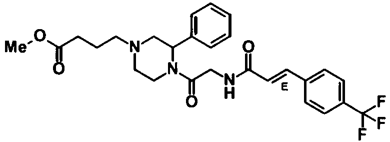 | MS(ESI) m/z:518.4 [M+H]+ |
| 5 (I-5) | 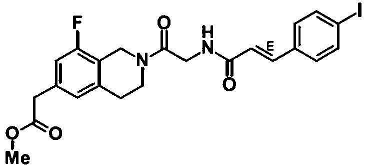 | MS (ESI) m/z : 537. 2 [M+H]+ |
| 6 (I-6) | 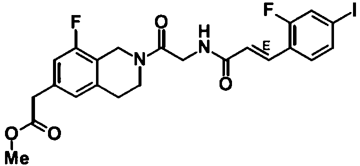 | MS(ESI) m/z:555. 2 [M+H]+ |

**[Table 18-2]**

| **Example No. (compound No.)** | **structural formula** | **instrument analysis data** |
|---|---|---|
| 7 (I-7) | 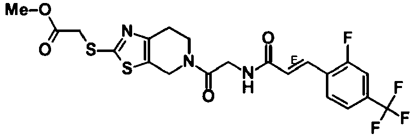 | MS(ESI) m/z:518.2 [M+H]+ |
| 8 (I-8) | 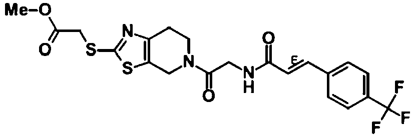 | MS(ESI) m/z:500.2 [M+H]+ |
| 9 (I-9) | 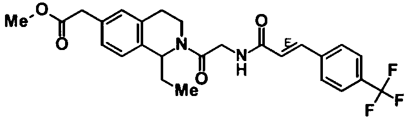 | MS(ESI) m/z:189.3 [M+H]+ |
| 10 (I-10) | 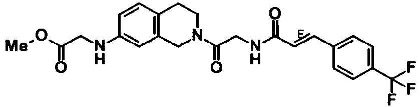 | MS(ESI) m/z:476,3 [M+H]+ |
| 11 (I-11) | 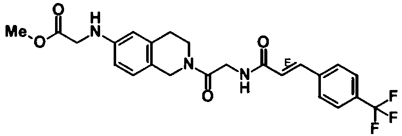 | MS(EST) m/z:476.1 [M+H]+ |

**[Table 18-3]**

| **Example No. (compound No.)** | **structural formula** | **instrument analysis data** |
|---|---|---|
| 12 (I-12) | 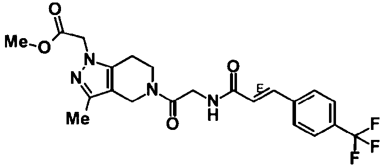 | MS(ESI) m/z:465.3 [M+H]+ |
| 13 (I-13) | 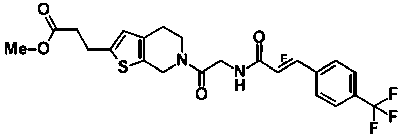 | MS(ESI) m/z:481.3 [M+H]+ |
| 14 (I-14) | 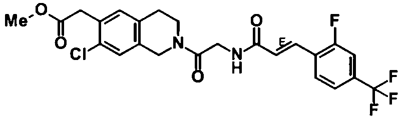 | MS(ESI) m/z:513.3/5 15. 2 [M+H]+ |
| 15 (I-15) | 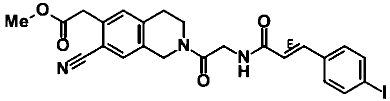 | MS(ESI) m/z:544.2 [M+H]+ |
| 16 (I-16) | 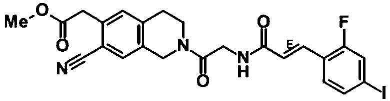 | MS(ESI) m/z:562.0 [M+H]+ |

**[Table 18-4]**

| **Example No. (compound No.)** | **structural formula** | **instrument analysis data** |
|---|---|---|
| 17 (I - 17) | 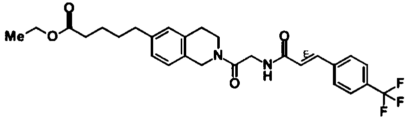 | MS(EST) m/z:517.4 [M+H]+ |
| 18 (I- 18) | 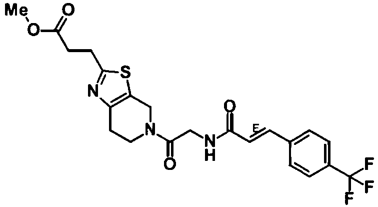 | MS(EST) m/z:480.3 [M-H]- |
| 19 (I-19) | 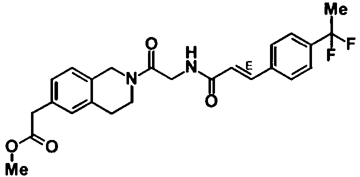 | MS(ESI) m/z:457.4 [M+H]+ |
| 20 (I-20) | 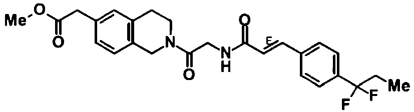 | MS(EST) m/z:471.4 [M+H]+ |
| 21 (I-21) | 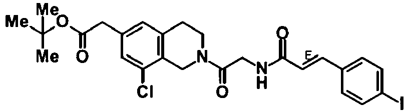 | MS(ESI) m/z:595.2/ 597.2[M+H]+ |

**[Table 18-5]**

| **Example No. (compound No.)** | **structural formula** | **instrument analysis data** |
|---|---|---|
| 22 (I-22) | 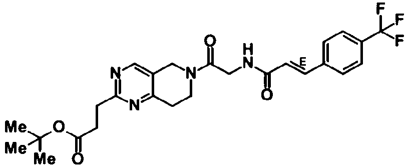 | ¹H-NMR (400MHz, CDCl₃) δ ppm:1.43 (9H, s), 2.77 (2 H, m), 2. 95-3. 06 (2H, m), 3.22 (2H, m), 3.90 (2H, m), 4.33 (2H, m), 4.71 (2 H, d), 6.61 (1H, d), 6.94 (1H, s), 6.87-7.02 (511, m), 8.45 (1H, s) |
| 23 (I- 23) | 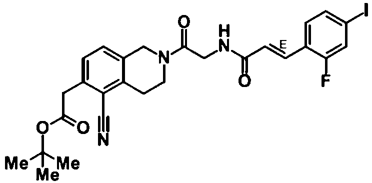 | MS(EST) m/z:604.3 [M+H]+ |
| 24 (I-24) | 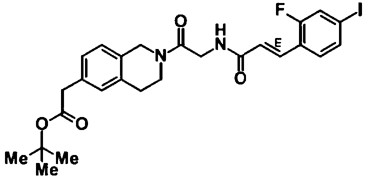 | MS(ESI) m/z:579.2 [M+H]+ |
| 25 (I-25) | 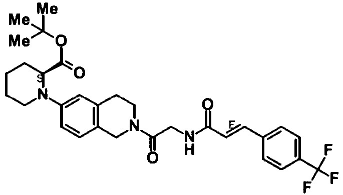 | MS(ESI) m/z:* 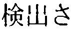 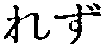 |
| 26 (I-26) | 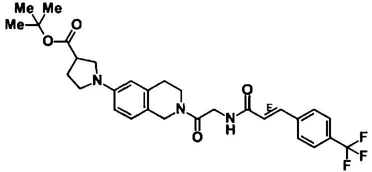 | MS(EST) m/z:558.1 [M+H]+ |

**[Table 18-6]**

| **Example No. (compound No.)** | **structural formula** | **instrument analysis data** |
|---|---|---|
| 27 (I -27) | 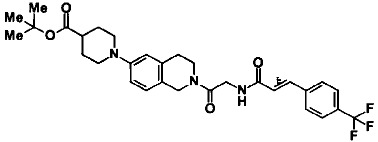 | MS(ESI) m/z:572. 4 [M+H]+ |
| 28 (I-28) | 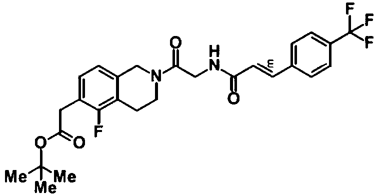 | MS(ESI) m/z:521.2 [M+II] + |
| 29 (I- 29) | 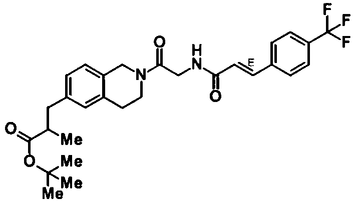 | MS (EST) m/z:529.5 [M-H]- |
| 30 (I-30) | 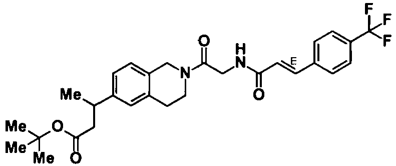 | MS (EST) m/z:529.5 [M-H]- |
| 31 (I-31) | 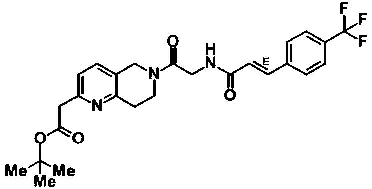 | MS (ESI) m/z:504.3 [M+H]+ |

**[Table 18-7]**

| **Example No. (compound No.)** | **structural formula** | **instrument analysis data** |
|---|---|---|
| 32 (I-32) | 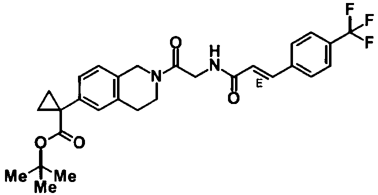 | MS(ESI) m/z:529.3 [M+H]+ |
| 33 (I - 33) | 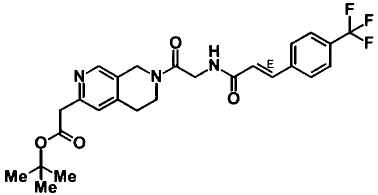 | MS(ESI) m/z:501.3 [M+H]+ |
| 34 (I-34) | 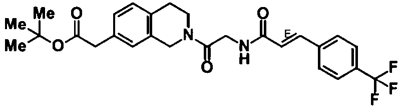 | MS(ESI) m/z:503.3 [M+H]+ |
| 35 (I- 35) | 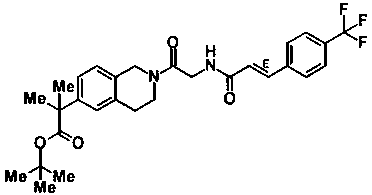 | MS(ESI) m/z:531.4 [M+H]+ |
| 36 (I-36) | 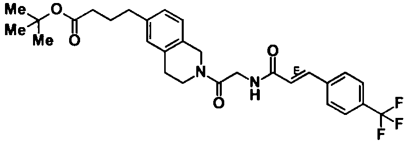 | MS(ESI) m/z:531.2 [M+H]+ |

**[Table 18-8]**

| **Example No. (compound No.)** | **structural formula** | **instrument analysis data** |
|---|---|---|
| 37 (I-37) | 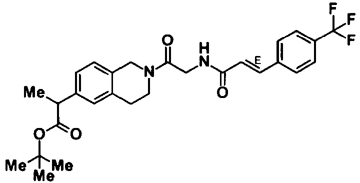 | MS(ESI) m/z:517.3 [M+H]+ |
| 38 (I-38) | 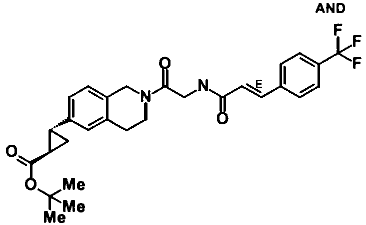 | MS(ESI) m/z:529.3 [M+H] + |
| 39 (I-39) | 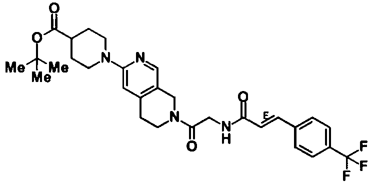 | MS (EST) m/z : 573. 4 [M+H]+ |
| 40 (I-40) | 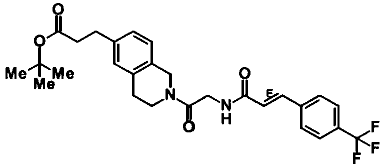 | MS(ESI) m/z:517.3 [M+H]+ |
| 41 (I-41) | 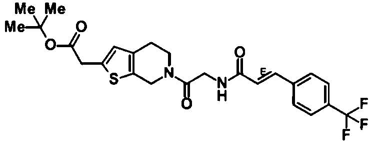 | MS(ESI) m/z:509.3 [M+H]+ |

**[Table 18-9]**

| **Example No. (compound No.)** | **structural formula** | **instrument analysis data** |
|---|---|---|
| 42 (I-42) | 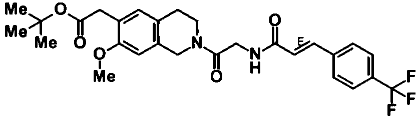 | MS(ESI) m/z:531.2 [M-H]- |
| 43 (I-43) | 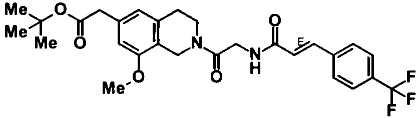 | MS (ESI) m/z:533.3 [M+H]+ |
| 44 (1-44) | 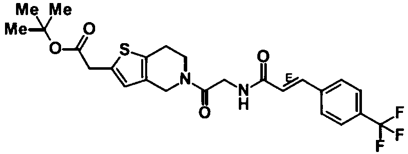 | MS(EST) m/z:507. 2 [M-H]- |
| 45 (I-45) | 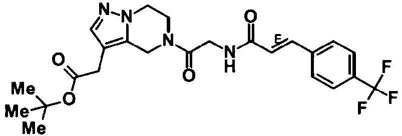 | MS(EST) m/z:191.2 [M-H]- |
| 46 (I-46) | 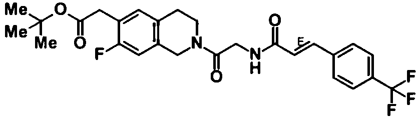 | MS(EST) m/z:521.3 [M+H]+ |

**[Table 18-10]**

| **Example No. (compound No.)** | **structural formula** | **instrument analysis data** |
|---|---|---|
| 47 (I-47) | 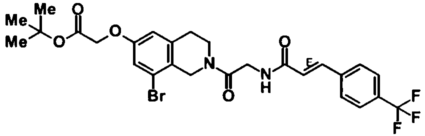 | MS (ESI) m/z: 595. 1/ 597. 1 [M-H]- |
| 48 (I-48) | 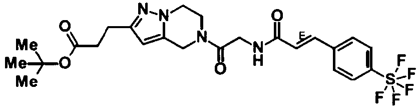 | MS(ESI) m/z:565.3 [M+II] + |
| 49 (I -49) | 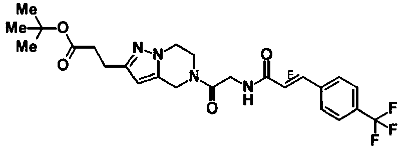 | MS(ESI) m/z:507.1 [M+H]+ |
| 50 (I-50) | 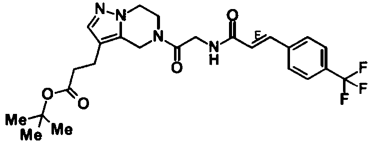 | MS(EST) m/z:505. 4 [M-H]- |
| 51 (I - 51) | 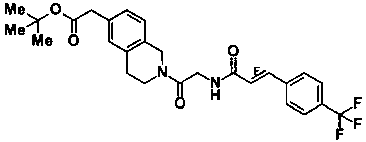 | MS(ESI) m/z:503.2 [M+H]+ |

**[Table 18-11]**

| **Example No. (compound No.)** | **structural formula** | **instrument analysis data** |
|---|---|---|
| 52 (I-52) | 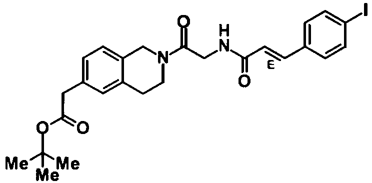 | MS(ESI) m/z:561.2 [M+H]+ |
| 53 (I-53) | 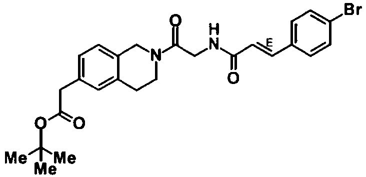 | MS(ESI) m/z: 513.3 /515. 3[M+H]+ |
| 54 (I-54) | 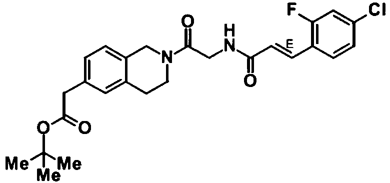 | MS(ESI) m/z:487.3/ 488. 3 [M+H]+ |
| 55 (I - 55) | 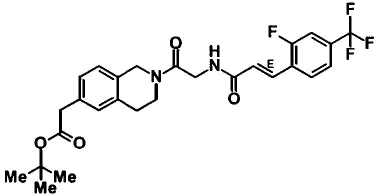 | MS(ESI)m/z:521.3 [M+H]+ |
| 56 (I - 56) | 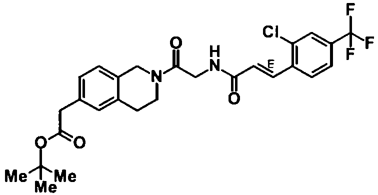 | MS(ESI) m/z:537.3/ 539. 3 [M+H] + |

**[Table 18-12]**

| **Example No. (compound No.)** | **structural formula** | **instrument analysis data** |
|---|---|---|
| 57 (I -57) | 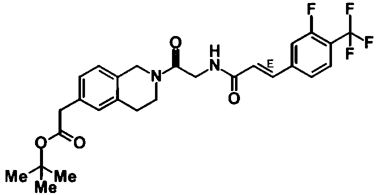 | MS(ESI) m/z:521.3 [M+H]+ |
| 58 (I-58) | 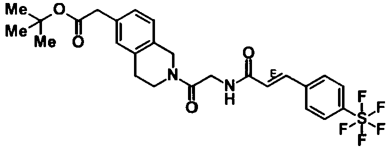 | MS(EST) m/z:561.1 [M+H]+ |
| 59 (I- 59) | 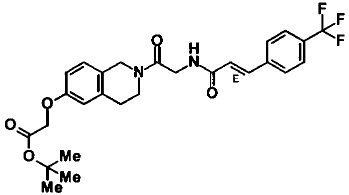 | MS(ESI) m/z:519.2 [M+H]+ |
| 60 (I -60) | 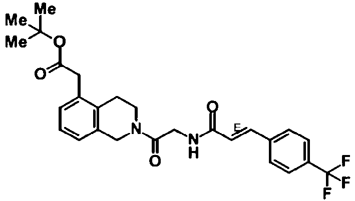 | MS(ESI) m/z:503.3 [M+H]+ |
| 61 (I-61) | 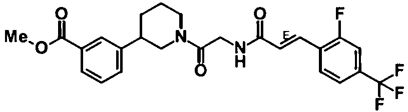 | MS(EST) m/z:493.2 [M+H]+ |

**[Table 18-13]**

| **Example No. (compound No.)** | **structural formula** | **instrument analysis data** |
|---|---|---|
| 62 (I-62) | 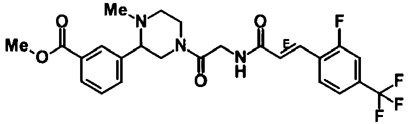 | MS(ESI) m/z:508.2 [M+H]+ |
| 63 (I-63) | 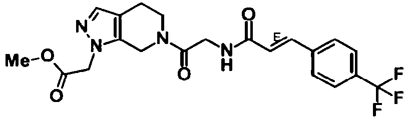 | MS(EST) m/z:451.3 [M+H]+ |
| 64 (I-64) | 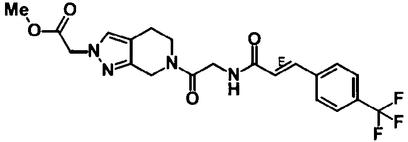 | MS (EST) m/z:451.3 [M+H]+ |
| 65 (I-65) | 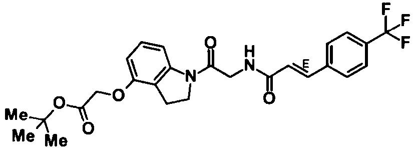 | MS (ESI) m/z:505.2 [M+H]+ |
| 66 (I-66) | 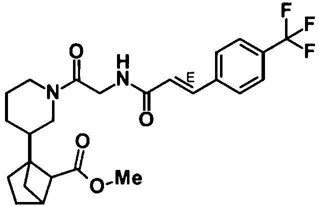 | MS(ESI) m/z:479.4 [M+H]+ |

**[Table 18-14]**

| **Example No. (compound No.)** | **structural formula** | **instrument analysis data** |
|---|---|---|
| 67 (I - 67) | 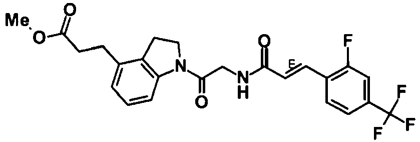 | MS(EST) m/z:479.2 [M+H]+ |
| 68 (I - 68) | 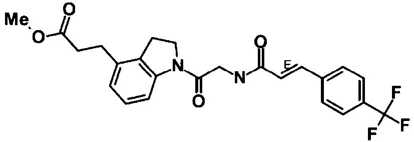 | MS(EST) m/z:161.2 [M+H]+ |
| 69 (I - 69) | 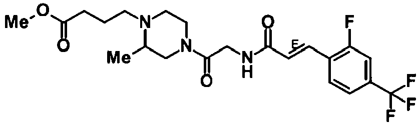 | MS(ESI) m/z:171.3 [M+H]+ |
| 70 (I - 70) | 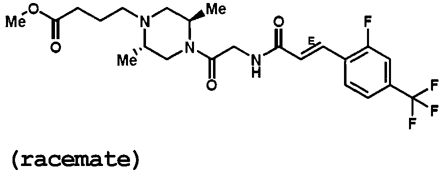 | MS(EST) m/z:488.1 [M+H]+ |
| 71 (I - 71) | 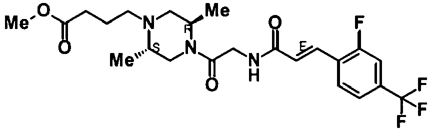 | MS(ESI) m/z: 488.3 [M+H]+ |

**[Table 18-15]**

| **Example No. (compound No.)** | **structural formula** | **instrument analysis data** |
|---|---|---|
| 72 (I - 72) | 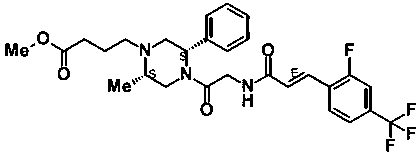 | MS(EST) m/z:550.1 [M+H]+ |
| 73 (I - 73) | 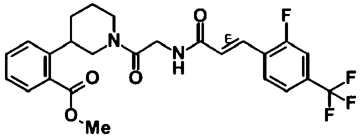 | MS (EST) m/z: 493. 1 [M+H]+ |
| 74 (I - 74) | 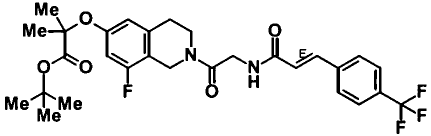 | MS (EST) m/z:565.4 [M+H]+ |
| 75 (I - 75) | 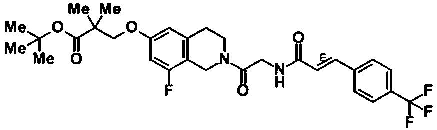 | MS (EST) m/z:579.1 [M+H]+ |
| 76 (I - 76) | 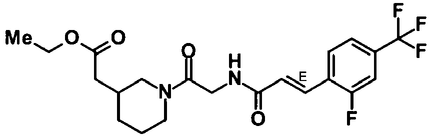 | MS(EST) m/z:415.2 [M+H]+ |

**[Table 18-16]**

| **Example No. (compound No.)** | **structural formula** | **instrument analysis data** |
|---|---|---|
| 77 (I - 77) | 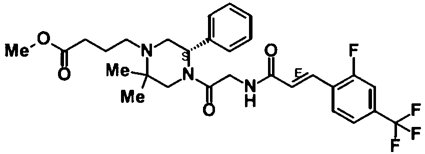 | MS(ESI) m/z:562.3 [M-H]- |
| 78 (I - 78) | 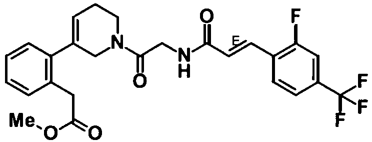 | MS(ESI) m/z:503.2 [M-H]- |
| 79 (I - 79) | 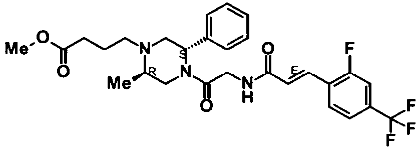 | MS (EST) m/z:550.0 [M+H]+ |
| 80 (I - 80) | 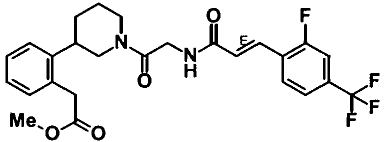 | MS(ESI) m/z:505.2 [M-H]- |
| 81 (I - 81) | 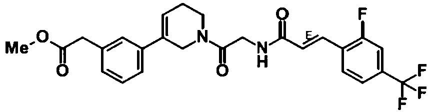 | MS(EST) m/z:505.2 [M+H] + |
| 82 (I - 82) | 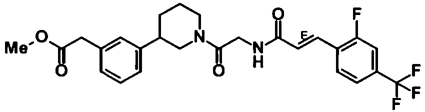 | MS (EST) m/z:505.2 [M-H]- |

### Example 83:

### Production of methyl 4-[4-[2-[[(E)-3-[2-fluoro-4-(trifluoromethyl)phenyl]prop-2-enoyl]amino]acetyl]-3-phenylpiperazin-1-yl]butanoate (compound (I-83))

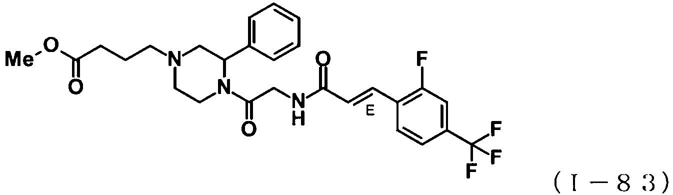

(1) To a solution of tert-butyl 4-[2-[[(E)-3-[2-fluoro-4-(trifluoromethyl)phenyl]prop-2-enoyl]amino]acetyl]-3-phenylpiperazine-1-carboxylate (318.9 mg) produced in Reference Example 98 in chloroform (2 ml) was added trifluoroacetic acid (0.91 ml), and the reaction mixture was stirred at room temperature for 17 hr. The reaction mixture was concentrated under reduced pressure, aqueous sodium carbonate solution was added thereto, and the mixture was extracted with chloroform. The organic layer was dried over sodium sulfate, concentrated under reduced pressure, and dried under vacuum to give (E)-3-[2-fluoro-4-(trifluoromethyl)phenyl]-N-[2-oxo-2-(2-phenylpiperazin-1-yl)ethyl]prop-2-enamide (238.6 mg; yield 92.0%) as a pale yellow amorphous.
   MS(ESI) m/z: 436.2[M+H]⁺
(2) To a solution of (E)-3-[2-fluoro-4-(trifluoromethyl)phenyl]-N-[2-oxo-2-(2-phenylpiperazin-1-yl)ethyl]prop-2-enamide (60 mg) obtained in the aforementioned (1) in DMF (0.35 ml) were added potassium carbonate (58 mg), sodium iodide (21 mg) and methyl 4-bromobutanoate (38 mg), and the mixture was stirred at 80°C for 4 hr. The reaction mixture was allowed to cool to room temperature, ethyl acetate was added, and the mixture was washed successively with water and saturated brine. The organic layer was dried over sodium sulfate, concentrated under reduced pressure, and the obtained residue was purified by silica gel column chromatography (solvent: n-hexane/ethyl acetate =75/25 - 50/50) to give the title compound (61.5 mg; yield 83.3%) as a white solid. MS(ESI) m/z: 536.3[M+H]⁺

### Examples 84 to 104:

The corresponding starting compounds were treated in the same manner as in Example 83 to obtain the compounds described in the following Table 19-1 to Table 19-4.

**[Table 19-1]**

| **Example No. (compound No.)** | **structural formula** | **instrument analysis data** |
|---|---|---|
| 84 (I - 84 ) | 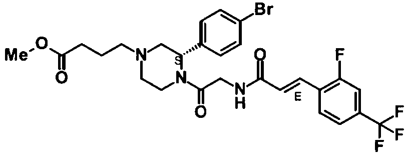 | MS(ESI) m/z:611.2/ 616.2 [M+H]+ |
| 85 (I - 85) | 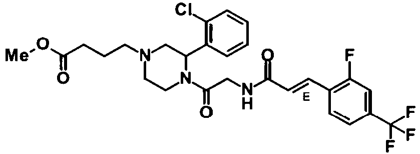 | MS (EST) m/z:570.2/ 572.1 [M+H]+ |
| 86 (I - 86) | 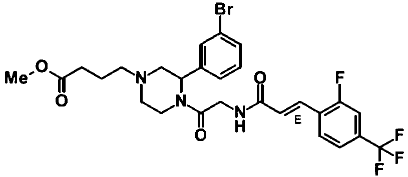 | MS(EST) m/z:614.2/ 616.1 [M+H]+ |
| 87 (I - 87) | 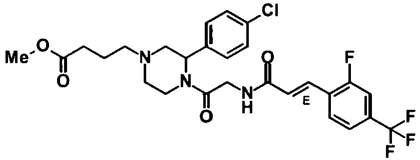 | MS (EST) m/z:568. 3/ 570.3 [M-H]- |
| 88 (I - 88) | 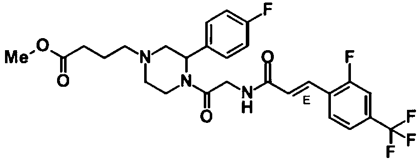 | MS (EST) m/z:552.3 [M-H]- |

**[Table 19-2]**

| **Example No. (compound No.)** | **structural formula** | **instrument analysis data** |
|---|---|---|
| 89 (I - 89) | 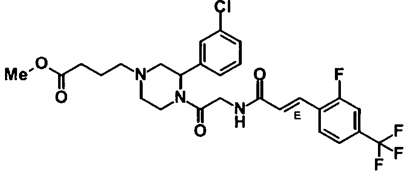 | MS(EST) m/z:568. 2/5 70. 2 [M-H]- |
| 90 (I - 90) | 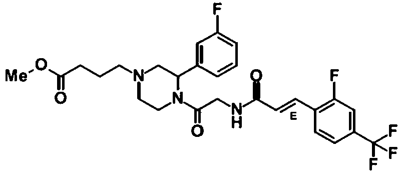 | MS (EST) m/z:552.3 [M-H]- |
| 91 (I - 91) | 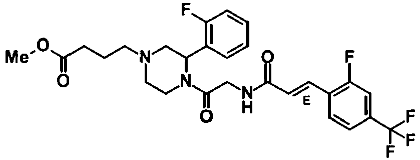 | MS (EST) m/z:552.3 [M-H]- |
| 92 (I - 92) | 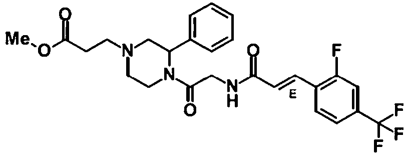 | MS (ESI) m/z:522.2 [M+H]+ |
| 93 (I - 93) | 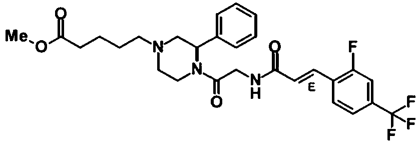 | MS (EST) m/z:550.2 [M+H] + |

**[Table 19-3]**

| **Example No. (compound No.)** | **structural formula** | **instrument analysis data** |
|---|---|---|
| 94 (I - 94) | 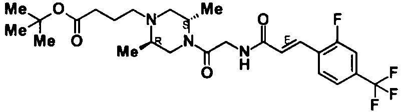 | MS(ESI) m/z:528.3 [M-H]- |
| 95 (I - 95) | 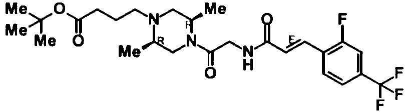 | MS(ESI) m/z:528.3 [M-H]- |
| 96 (I - 96) | 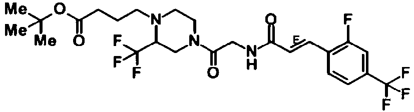 | MS(EST) m/z: * 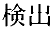 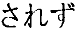 |
| 97 (I - 97) | 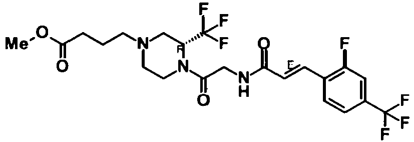 | MS(EST) m/z:526.2 [M-H]- |
| 98 (I - 98) | 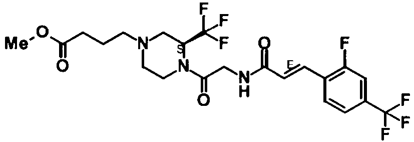 | MS(EST) m/z:526.3 [M-H]- |

**[Table 19-4]**

| **Example No. (compound No.)** | **structural formula** | **instrument analysis data** |
|---|---|---|
| 99 (I - 99) | 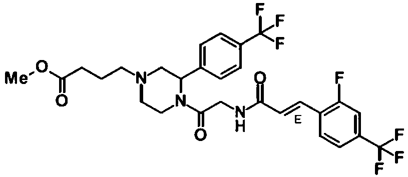 | MS(ESI) m/z:604.2 [M+H]+ |
| 100 (I - 100) | 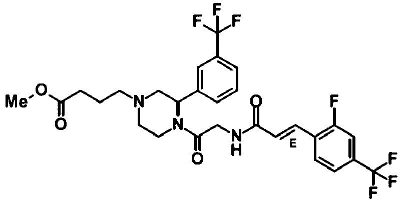 | MS (ESI) m/z:604.2 [M+H]+ |
| 101 (I - 101) | 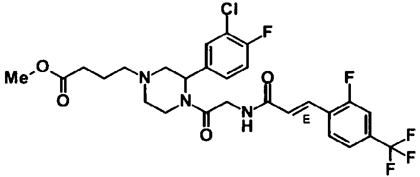 | MS(ESI) m/z:588.2/ 590.2 [M+H]+ |
| 102 (I - 102) | 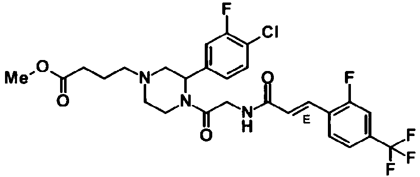 | MS (EST) m/z:588.2/ 590.1 [M+H]+ |
| 103 (I - 103) | 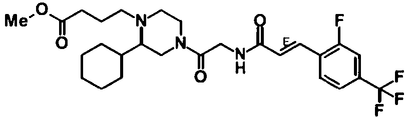 | MS(ESI) m/z:540.3 [M-H]- |

### Example 104:

### Production of methyl 4-[[3-(4-chlorophenyl)-4-[2-[[(E)-3-[2-fluoro-4-(trifluoromethyl)phenyl]prop-2-enoyl]amino]acetyl]piperazin-1-yl]methyl]benzoate (compound (I-104))

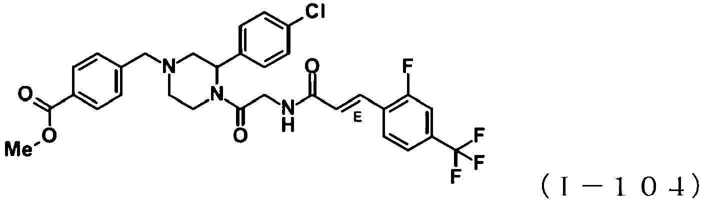

(1) To a solution of tert-butyl 3-(4-chlorophenyl)-4-[2-[[(E)-3-[2-fluoro-4-(trifluoromethyl)phenyl]prop-2-enoyl]amino]acetyl]piperazine-1-carboxylate (812.1 mg) produced in Reference Example 99 in chloroform (4.8 ml) was added trifluoroacetic acid (2.2 ml), and the reaction mixture was stirred at room temperature for 19.5 hr. The reaction mixture was concentrated under reduced pressure, aqueous sodium carbonate solution was added, and the mixture was extracted with chloroform. The organic layer was dried over sodium sulfate, concentrated under reduced pressure, and dried under vacuum to give (E)-N-[2-[2-(4-chlorophenyl)piperazin-1-yl]-2-oxoethyl]-3-[2-fluoro-4-(trifluoromethyl)phenyl]prop-2-enamide (651.6 mg; yield 97.3%) as a pale yellow amorphous.
   MS(ESI) m/z: 470.2,472.2[M+H]⁺
(2) To a solution of (E)-N-[2-[2-(4-chlorophenyl)piperazin-1-yl]-2-oxoethyl]-3-[2-fluoro-4-(trifluoromethyl)phenyl]prop-2-enamide (50 mg) obtained in the aforementioned (1) in dichloromethane (0.53 ml) were added methyl 4-formylbenzoate (26.2 mg) and sodium triacetoxyborohydride (67.6 mg), and the reaction mixture was stirred at room temperature for 14 hr. Water was added to the reaction mixture, and the mixture was extracted with chloroform. The organic layer was washed with saturated brine, dried over sodium sulfate, and concentrated under reduced pressure. The obtained residue was purified by silica gel column chromatography (solvent: n-hexane/ethyl acetate =75/25 - 50/50) to give the title compound (51.0 mg; yield 77.6%) as a white amorphous.
   MS(ESI) m/z: 618.2/620.2[M+H]⁺

### Examples 105 to 108:

The corresponding starting compounds were treated in the same manner as in Example 104 to obtain the compounds described in the following Table 20.

**[Table 20]**

| **Example No. (compound No.)** | **structural formula** | **instrument analysis data** |
|---|---|---|
| 105 (I - 105) | 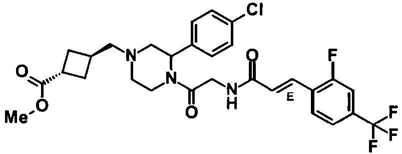 | MS (EST) m/z:596.3/ 598.2 [M+H]+ |
| 106 (I - 106) | 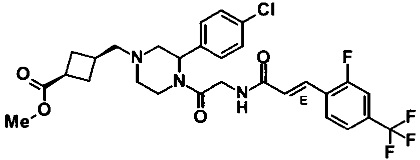 | MS (EST) m/z:596.3/ 598.2 [M+H]+ |
| 107 (I - 107) | 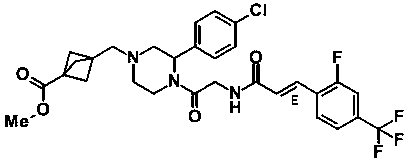 | MS(ESI) m/z:608.3/ 610.2 [M+H]+ |
| 108 (I - 108) | 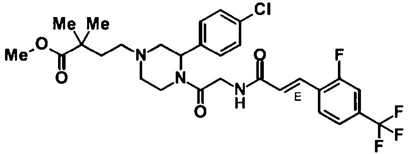 | MS(ESI) m/z:596.4/ 598.3 [M-H]- |

### Example 109:

### Production of tert-butyl 2-[3-[2-[[(E)-3-[4-(trifluoromethyl)phenyl]prop-2-enoyl]amino]acetyl]-1,2,4,5-tetrahydro-3-benzazepin-7-yl]acetate (compound (I-109))

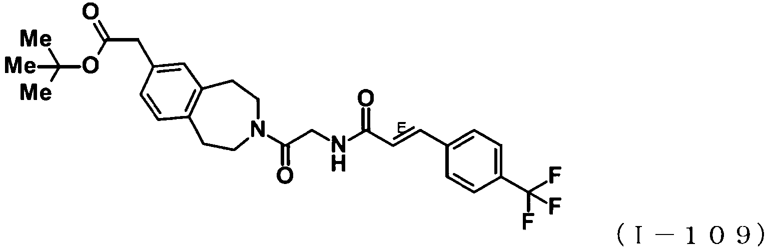

(E)-N-[2-(7-bromo-1,2,4,5-tetrahydro-3-benzazepin-3-yl)-2-oxoethyl]-3-[4-(trifluoromethyl)phenyl]prop-2-enamide (160 mg) produced in Reference Example 60, (1-tert-butoxy vinyloxy)tert-butyldimethylsilane (230 mg), potassium carbonate (92 mg) and bis(tri-tert-butylphosphine)palladium(0) (51 mg) were added to DMF (0.66 ml), and the mixture was stirred by a microwave reactor for 1 hr while heating to 115°C. Water was added to the reaction mixture, and the mixture was extracted with ethyl acetate. The organic layer was washed with saturated brine, dried over anhydrous magnesium sulfate, and concentrated under reduced pressure. The residue was purified by reversed-phase HPLC (10 mmol/l aqueous ammonium carbonate solution/acetonitrile =40/60 - 10/90) to give the title compound (23 mg; yield 13.4%) as a pale yellow solid.
MS(ESI) m/z: 517.1[M+H]⁺

### Examples 110 to 126:

The corresponding starting compounds were treated in the same manner as in Example 109 to obtain the compounds described in the following Table 21-1 to Table 21-4.

**[Table 21-1]**

| **Example No. (compound No.)** | **structural formula** | **instrument analysis data** |
|---|---|---|
| 110 (I - 110) | 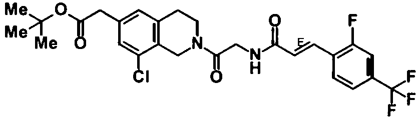 | MS(ESI) m/z:555.3 /557.3 [M+H]+ |
| 111 (I - 111) | 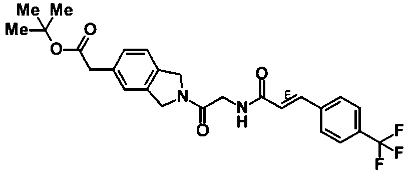 | MS (ESI) m/z:489.2 [M+H]+ |
| 112 (I - 112) | 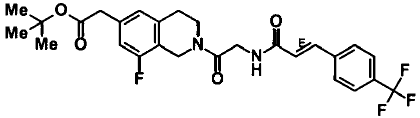 | MS(ESI) m/z:521.3 [M+H]+ |
| 113 (I - 113) | 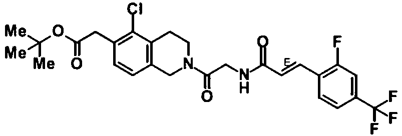 | MS(ESI) m/z:555.4/ 557.4 [M+H]+ |
| 114 (I - 114) | 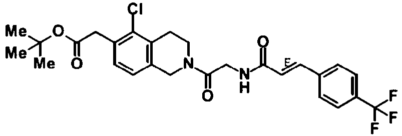 | MS(ESI) m/z:537.3/ 539.2 [M+H]+ |

**[Table 21-2]**

| **Example No. (compound No.)** | **structural formula** | **instrument analysis data** |
|---|---|---|
| 115 (I - 115) | 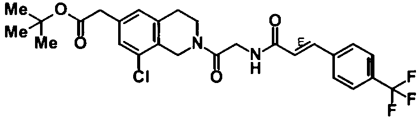 | MS (ESI) m/z:537.3 /539.3 [M+H] + |
| 116 (I - 116) | 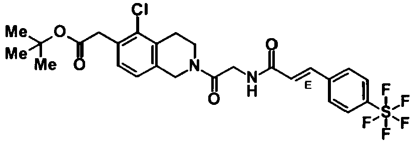 | MS(ESI) m/z:595.3/ 597.3 [M+H]+ |
| 117 (I - 117) | 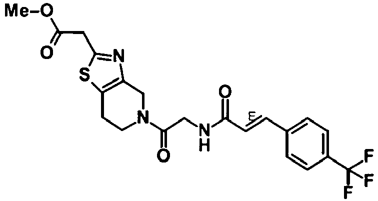 | MS(ESI) m/z:468.2 [M+H]+ |
| 118 (I - 118) | 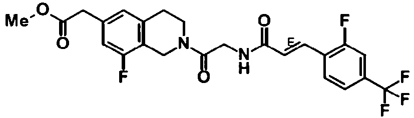 | MS (ESI) m/z:497.3 [M+H]+ |
| 119 (I - 119) | 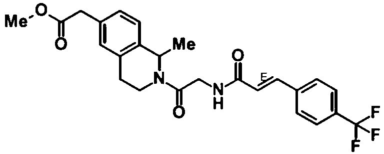 | MS(ESI) m/z:475.2 [M+H]+ |

**[Table 21-3]**

| **Example No. (compound No.)** | **structural formula** | **instrument analysis data** |
|---|---|---|
| 120 (I - 120) | 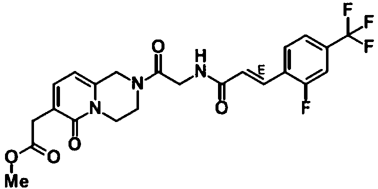 | MS(EST) m/z:496.3 [M+H]+ |
| 121 (I - 121) | 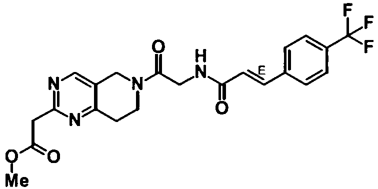 | MS(EST) m/z:463. 3 [M+H]+ |
| 122 (I - 122) | 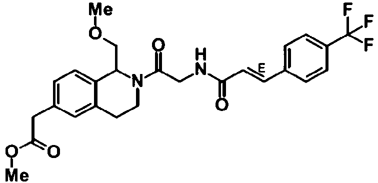 | MS(ESI) m/z:505.3 [M+H]+ |
| 123 (I - 123) | 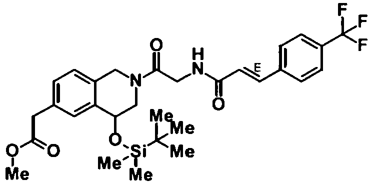 | MS(ESI) m/z:591.4 [M+H]+ |
| 124 (I - 124) | 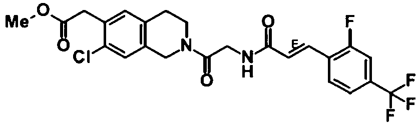 | MS(ESI) m/z:513.3/ 515.2 [M+H]+ |

**[Table 21-4]**

| **Example No. (compound No.)** | **structural formula** | **instrument analysis data** |
|---|---|---|
| 125 (I -125) | 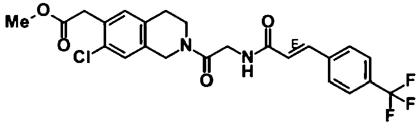 | MS (EST) m/z:495.3/ 497.3 [M+H]+ |
| 126 (I - 126) | 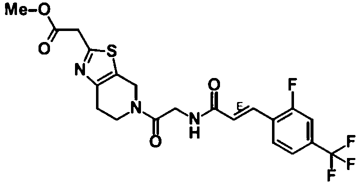 | MS(ESI) m/z: 486.1 [M+H]+ |

### Example 127:

### Production of tert-butyl 2-[8-cyano-2-[2-[[(E)-3-[2-fluoro-4-(trifluoromethyl)phenyl]prop-2-enoyl]amino]acetyl]-3,4-dihydro-1H-isoquinolin-6-yl]acetate (compound (I-127))

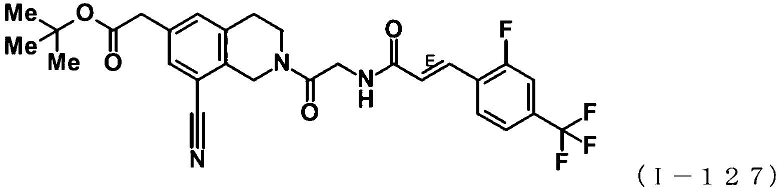

To a solution of tert-butyl 2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetate (38 mg) in water (1.5 ml) and dioxane (5 ml) were added potassium hexacyanoferrate (II) trihydrate (19 mg), chloro(2-dicyclohexylphosphino-2',4',6'-triisopropyl-1,1'-biphenyl)[2-(2'-amino-1,1'-biphenyl)]palladium(II) (6.5 mg) and potassium acetate (7 mg), and the mixture was stirred at an outer temperature of 100°C for 2 hr. The reaction mixture was allowed to cool, saturated aqueous ammonium chloride solution was added thereto, and the mixture was extracted with ethyl acetate, dried over sodium sulfate, and evaporated under reduced pressure. The residue was purified by reversed-phase HPLC (10 mmol/l aqueous ammonium carbonate solution/acetonitrile =80/20 - 50/50) to give the title compound (25 mg; yield 67%) as a colorless solid.
MS(ESI) m/z: 546.3[M+H]⁺

### Examples 128 to 133:

The corresponding starting compounds were treated in the same manner as in Example 127 to obtain the compounds described in the following Table 22.

**[Table 22]**

| **Example No. (compound No.)** | **structural formula** | **instrument analysis data** |
|---|---|---|
| 128 (I - 128) | 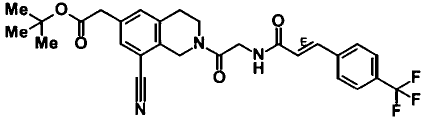 | MS (EST) m/z:528.3 [M+H]+ |
| 129 (I - 129) | 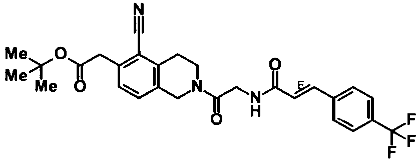 | MS(EST) m/z :528.3 [M+H]+ |
| 130 (I - 130) | 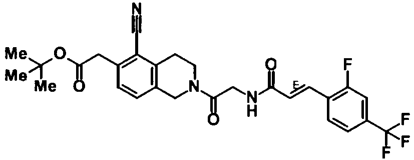 | MS(EST) m/z : 546. 2 [M+H]+ |
| 131 (I - 131) | 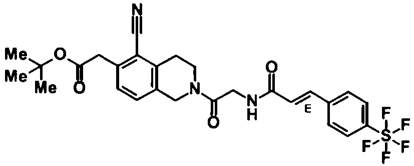 | MS (ESI) m/z:586.3 [M+H]+ |
| 132 (I - 132) | 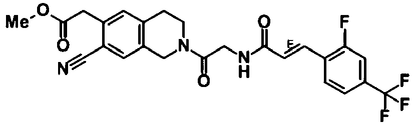 | MS (EST) m/z:504.3 [M+H]+ |
| 133 (I - 133) | 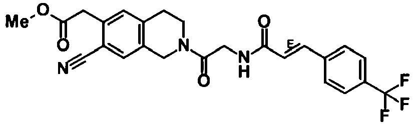 | MS(ESI) m/z:486.3 [M+H]+ |

### Example 134:

### Production of tert-butyl 2-[8-methyl-2-[2-[[(E)-3-[4-(trifluoromethyl)phenyl]prop-2-enoyl]amino]acetyl]-3,4-dihydro-1H-isoquinolin-6-yl]acetate (compound (I-134))

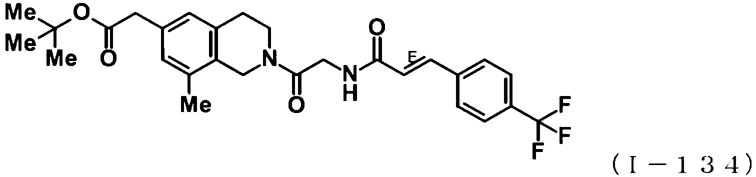

To a solution of tert-butyl 2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetate (38 mg) in tetrahydrofuran (5 ml) were added bis(trimethylaluminum)-1,4-diazabicyclo[2,2,2]octane adduct (43 mg) and chloro(2-dicyclohexylphosphino-2',4',6'-triisopropyl-1,1'-biphenyl)[2-(2'-amino-1,1'-biphenyl)]palladium(II) (9.5 mg), and the mixture was stirred at an outer temperature of 100°C for 1 hr. The reaction mixture was allowed to cool, 1N hydrochloric acid was added thereto, and the mixture was extracted with ethyl acetate. The organic layer was dried over sodium sulfate, and concentrated under reduced pressure. The residue was purified by reversed-phase HPLC (10 mmol/l aqueous ammonium carbonate solution/acetonitrile =80/20 - 50/50) to give the title compound (23 mg; yield 82%) as a pale yellow solid.
MS(ESI) m/z: 517.4[M+H]⁺

### Example 135:

The corresponding starting compounds were treated in the same manner as in Example 134 to obtain the compound described in the following Table 23.

**[Table 23]**

| **Example No. (compound No.)** | **structural formula** | **instrument analysis data** |
|---|---|---|
| 135 (I - 135) | 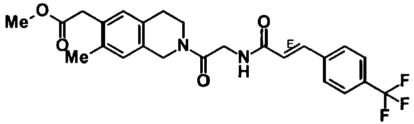 | MS(ESI) m/z:475.2 [M+H] + |

### Example 136:

### Production of methyl 4-[3-(3-cyclopropylphenyl)-4-[2-[[(E)-3-[2-fluoro-4-(trifluoromethyl)phenyl]prop-2-enoyl]amino]acetyl]piperazin-1-yl]butanoate (compound (I-136))

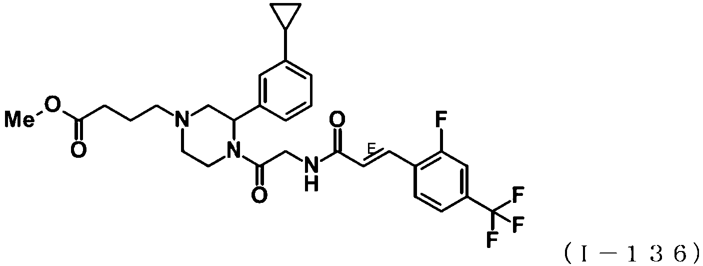

To a solution of methyl 4-[3-(3-bromophenyl)-4-[2-[[(E)-3-[2-fluoro-4-(trifluoromethyl)phenyl]prop-2-enoyl]amino]acetyl]piperazin-1-yl]butanoate (38 mg) produced in Example 86 in DMF (0.47 ml) were added 2-cyclopropyl-4,4,5,5-tetramethyl-1,3,2-dioxaborolane (29.2 mg), dichloro bis(diphenylphosphino)ferrocene palladium(II) dichloromethane adduct (7.7 mg) and potassium carbonate (48 mg), and the mixture was stirred under nitrogen atmosphere at 80°C for 6 hr 40 min, and thereafter stirred for 19 hr 50 min while allowing to cool to room temperature. 2-Cyclopropyl-4,4,5,5-tetramethyl-1,3,2-dioxaborolane (31 mg), dichloro bis(diphenylphosphino)ferrocene palladium(II) dichloromethane adduct (8.1 mg) and potassium carbonate (24 mg) were added to the reaction mixture at room temperature, and the mixture was stirred under nitrogen atmosphere at 85°C for 5 hr 30 min, and thereafter stirred for 16 hr while allowing to cool to room temperature. 2-Cyclopropyl-4,4,5,5-tetramethyl-1,3,2-dioxaborolane (31 mg), dichloro bis(diphenylphosphino)ferrocene palladium(II) dichloromethane adduct (8.1 mg) and potassium carbonate (24 mg) were added to the reaction mixture at room temperature, and the reaction mixture was stirred under nitrogen atmosphere at 85°C for 8 hr. The reaction mixture was cooled to room temperature, water was added, and the mixture was extracted with ethyl acetate. The organic layer was washed successively with water and saturated brine, dried over sodium sulfate, and concentrated under reduced pressure. The residue was purified by column chromatography (solvent: n-hexane/ethyl acetate =50/50 - 0/100) to give the title compound (13.7 mg; yield 20.6%) as a colorless amorphous.
MS(ESI) m/z: 576.2[M+H]⁺

### Example 137:

The corresponding starting compounds were treated in the same manner as in Example 136 to obtain the compound described in the following Table 24.

**[Table 24]**

| **Example No. (compound No.)** | **structural formula** | **instrument analysis data** |
|---|---|---|
| 137 (I - 137) | 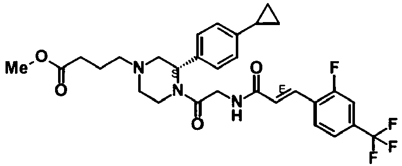 | MS(ESI) m/z:576.3 [M+H] + |

### Example 138:

### Production of tert-butyl 3-[3-bromo-5-[2-[[(E)-3-[4-(pentafluoro-λ6-sulfanyl)phenyl]prop-2-enoyl]amino]acetyl]-6,7-dihydro-4H-pyrazolo[1,5-a]pyrazin-2-yl]propanoate (compound (I-138))

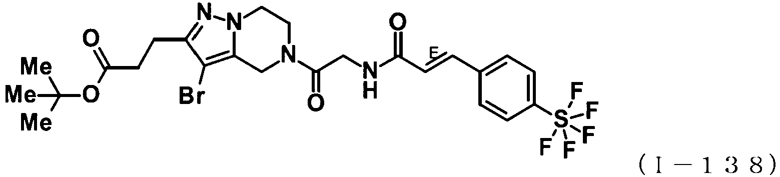

To a solution of tert-butyl 3-[5-[2-[[(E)-3-[4-(pentafluoro-λ6-sulfanyl)phenyl]prop-2-enoyl]amino]acetyl]-6,7-dihydro-4H-pyrazolo[1,5a]pyrazin-2-yl]propanoate (104 mg) in chloroform (1.2 ml) was added N-bromosuccinimide (45 mg), and the mixture was stirred at an outer temperature of 60°C for 1 hr. The reaction mixture was allowed to cool, aqueous sodium thiosulfate solution and aqueous potassium carbonate solution were added thereto, and the mixture was extracted with chloroform. The organic layer was dried over sodium sulfate and concentrated under reduced pressure to give a crude product of the title compound as a yellow oil.

### Example 139:

The corresponding starting compounds were treated in the same manner as in Reference Example 138 to obtain a crude product of the compound described in the following Table 25.

**[Table 25]**

| **Example No. (compound No.)** | **structural formula** |
|---|---|
| 139 (I - 139) | 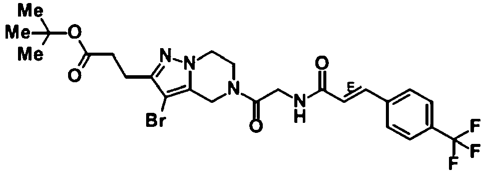 |

### Example 140:

### Production of 2-[3-[2-[[(E)-3-[4-(trifluoromethyl)phenyl]prop-2-enoyl]amino]acetyl]-1,2,4,5-tetrahydro-3-benzazepin-7-yl]acetic acid (compound (I-140))

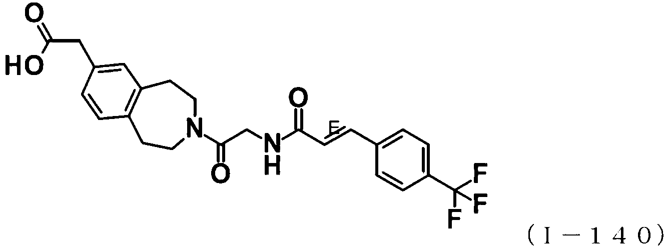

To a solution of tert-butyl 3-[3-[2-[[(E)-3-[4-(trifluoromethyl)phenyl]prop-2-enoyl]amino]acetyl]-1,2,4,5-tetrahydro-3-benzazepin-7-yl]acetate (21 mg) produced in Example 109 in dichloromethane (1 ml) was added trifluoroacetic acid (0.1 ml) at room temperature, and the mixture was stirred overnight. The reaction mixture was concentrated under reduced pressure, toluene was added, and the solvent was evaporated under reduced pressure. The obtained residue was purified by reversed-phase HPLC (10 mmol/l aqueous ammonium carbonate solution/acetonitrile =80/20 - 50/50) to give the title compound (16 mg; yield 85%) as a pale yellow solid.
MS(ESI) m/z: 461.4[M+H]⁺

### Examples 141 to 199:

The corresponding starting compounds were treated in the same manner as in Example 140 to obtain the compounds described in the following Table 26-1 to Table 26-12.

**[Table 26-1]**

| **Example No. (compound No.)** | **structural formula** | **instrument analysis data** |
|---|---|---|
| 141 (I - 141) | 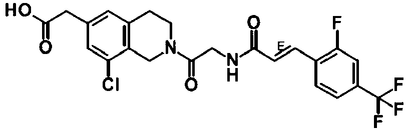 | MS(ESI) m/z:499.2/ 501.2 [M+H]+ |
| 142 (I - 142) | 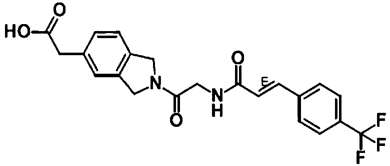 | MS(ESI) m/z:433.2 [M+H]+ |
| 143 (I - 143) | 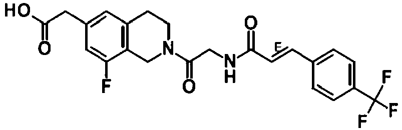 | MS (ESI) m/z:465.3 [M+H]+ |
| 144 (I - 144) | 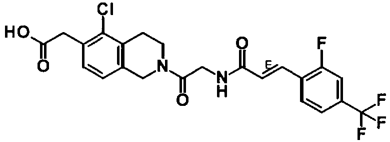 | MS(ESI) m/z:499.2/ 501.2 [M+H]+ |
| 145 (I - 145) | 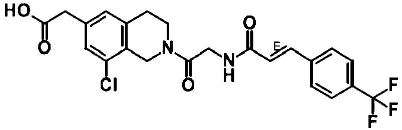 | MS(ESI) m/z:481.2/ 483. 2[M+H]+ |

**[Table 26-2]**

| **Example No. (compound No.)** | **structural formula** | **instrument analysis data** |
|---|---|---|
| 146 (I - 146) | 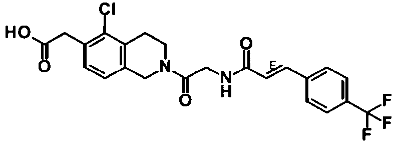 | MS(ESI) m/z:481.3/ 483.3 [M+H]+ |
| 147 (I - 147) | 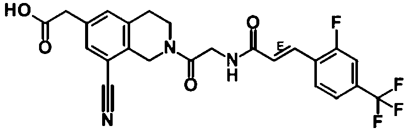 | MS(ESI) m/z:490.3 [M+H]+ |
| 148 (I - 148) | 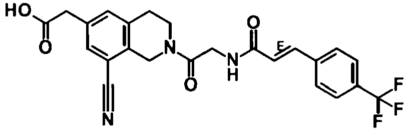 | MS(ESI) m/z:472.3 [M+H]+ |
| 149 (I - 149) | 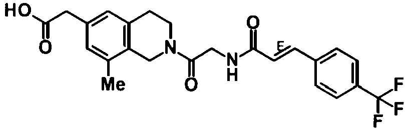 | MS(EST) m/z:461.3 [M+H]+ |
| 150 (I - 150) | 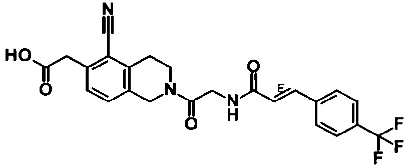 | MS(ESI) m/z:472.3 [M+H]+ |

**[Table 26-3]**

| **Example No. (compound No.)** | **structural formula** | **instrument analysis data** |
|---|---|---|
| 151 (I - 151) | 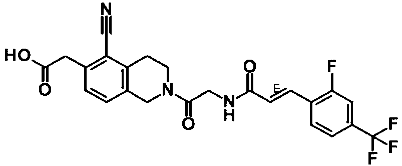 | MS(EST) m/z:490.3 [M+H]+ |
| 152 (I - 152) | 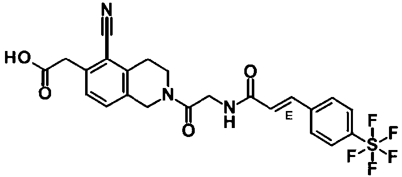 | MS (EST) m/z:530. 2 [M+H]+ |
| 153 (I - 153) | 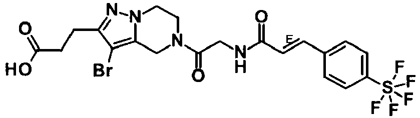 | MS(ESI) m/z:585.2/ 587.2 [M-H]- |
| 154 (I - 154) | 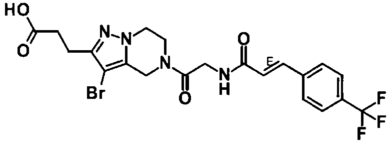 | MS(ESI) m/z:527.2/ 529.2 [M-H]- |
| 155 (I - 155) | 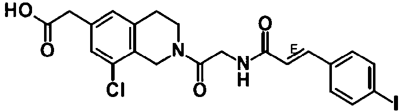 | MS (EST) m/z:539.2/ 541. 2[M+H]+ |

**[Table 26-4]**

| **Example No. (compound No.)** | **structural formula** | **instrument analysis data** |
|---|---|---|
| 156 (I - 156) | 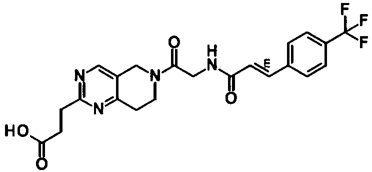 | MS(EST) m/z:463.3 [M+H]+ |
| 157 (I - 157) | 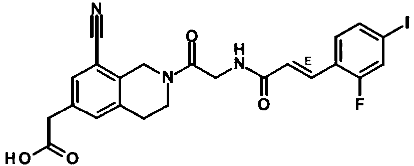 | MS (EST) m/z:548.2 [M+H]+ |
| 158 (I - 158) | 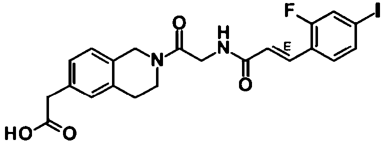 | MS(ESI) m/z:523.2 [M+H]+ |
| 159 (I - 159) | 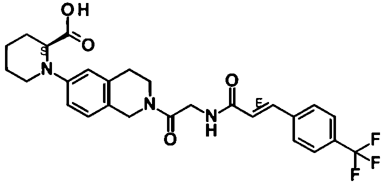 | MS (ESI) m/z:516.3 [M+H]+ |
| 160 (I - 160) | 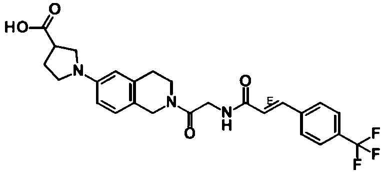 | MS(EST) m/z:502.3 [M+H]+ |

**[Table 26-5]**

| **Example No. (compound No.)** | **structural formula** | **instrument analysis data** |
|---|---|---|
| 161 (I - 161) | 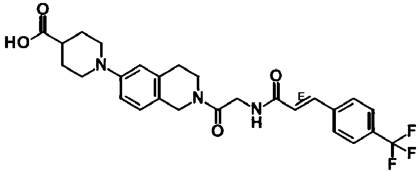 | MS(ESI) m/z:516.3 [M+H]+ |
| 162 (I - 162) | 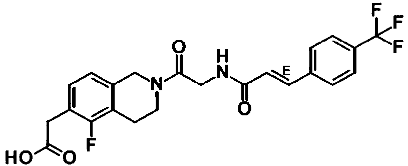 | MS (EST) m/z:465.2 [M+H]+ |
| 163 (I - 163) | 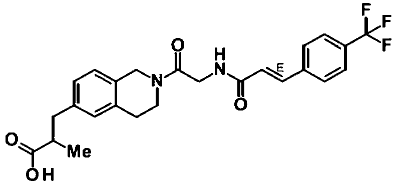 | MS (ESI) m/z:475.3 [M+H] + |
| 164 (1 - 164) | 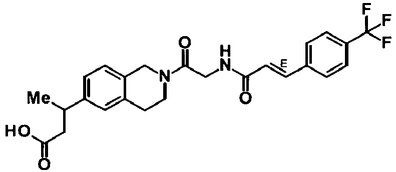 | MS(ESI) m/z:475.3 [M+H] + |
| 165 (I - 165) | 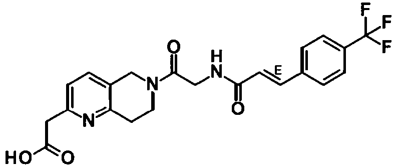 | MS(EST) m/z:448.3 [M+H]+ |

**[Table 26-6]**

| **Example No. (compound No.)** | **structural formula** | **instrument analysis data** |
|---|---|---|
| 166 (I - 166) | 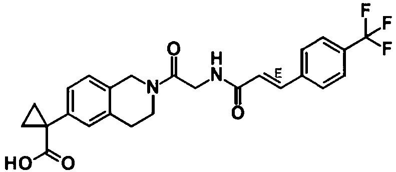 | MS(EST) m/z:473.3 [M+H]+ |
| 167 (I - 167) | 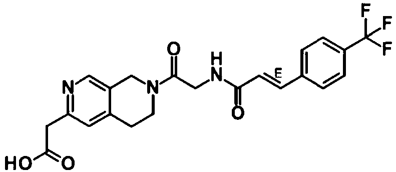 | MS(ESI) m/z:448.3 [M+H]+ |
| 168 (I - 168) | 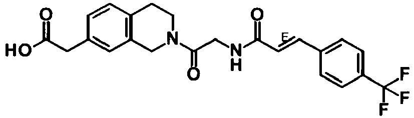 | MS(ESI) m/z:447.3 [M+H]+ |
| 169 (I - 169) | 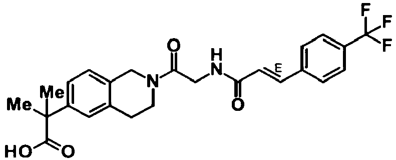 | MS(EST) m/z:475.3 [M+H]+ |
| 170 (I - 170) | 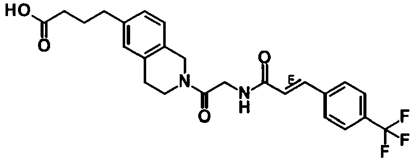 | MS(ESI) m/z:475.3 [M+H]+ |

**[Table 26-7]**

| **Example No. (compound No.)** | **structural formula** | **instrument analysis data** |
|---|---|---|
| 171 (I - 171) | 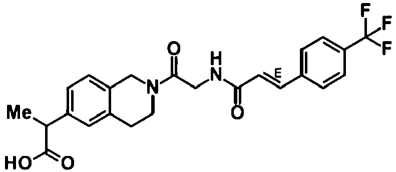 | MS(EST) m/z:461.3 [M+H]+ |
| 172 (I - 172) | 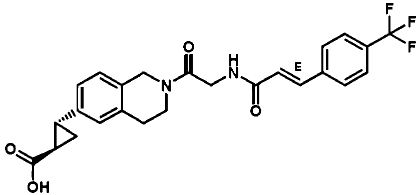 | MS(EST) m/z:473.3 [M+H]+ |
| 173 (I - 173) | 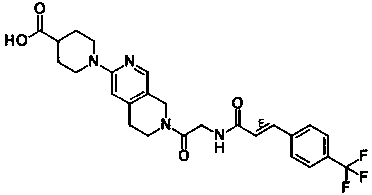 | MS (EST) m/z:517.3 [M+H]+ |
| 174 (I - 174) | 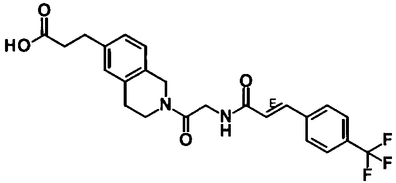 | MS(ESI) m/z:461.2 [M+H]+ |
| 175 (I - 175) | 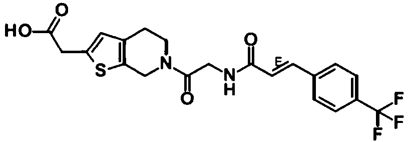 | MS(ESI) m/z:453.3 [M+H]+ |

**[Table 26-8]**

| **Example No. (compound No.)** | **structural formula** | **instrument analysis data** |
|---|---|---|
| 176 (I - 176) | 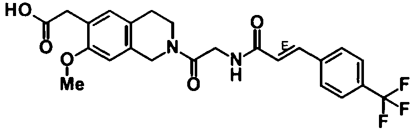 | MS(ESI) m/z:475. 1 [M-111- |
| 177 (I - 177) | 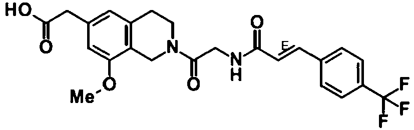 | MS(EST) m/z:477.3 [M+H] + |
| 178 (I - 178) | 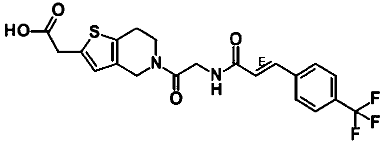 | MS(EST) m/z:451. 1 [M-H]- |
| 179 (I - 179) | 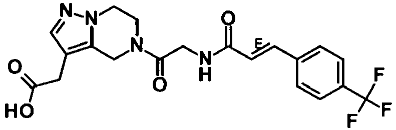 | MS(ESI) m/z:435.0 [M-H]- |
| 180 (I - 180) | 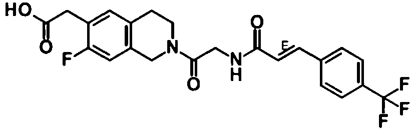 | MS(ESI) m/z:465.3 [M+H]+ |

**[Table 26-9]**

| **Example No. (compound No.)** | **structural formula** | **instrument analysis data** |
|---|---|---|
| 181 (I - 181) | 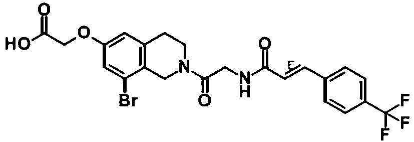 | MS(ESI) m/z:539.0/ 541.0 [M-H]- |
| 182 (I - 182) | 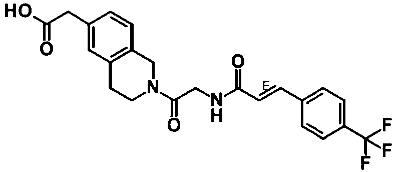 | MS(ESI) m/z:447.3 [M+H]+ |
| 183 (I - 183) | 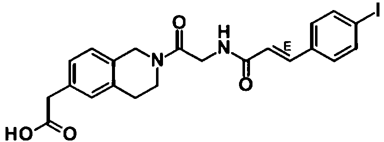 | MS(ESI) m/z:505.2 [M+H]+ |
| 184 (I - 184) | 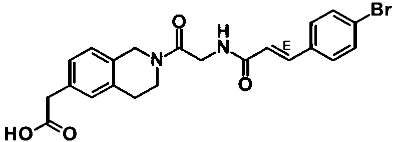 | MS(EST) m/z:457.2/ 459. 2[M+H]+ |
| 185 (I - 185) | 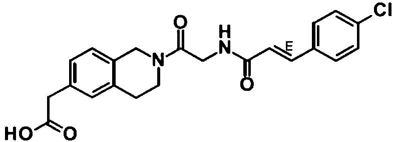 | MS(ESI) m/z:413.2/ 415.2 [M+H]+ |

**[Table 26-10]**

| **Example No. (compound No.)** | **structural formula** | **instrument analysis data** |
|---|---|---|
| 186 (I - 186) | 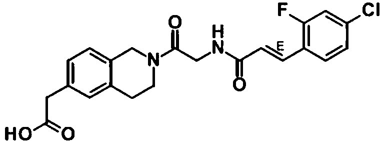 | MS(EST) m/z:431.3/ 432.3 [M+H]+ |
| 187 (I - 187) | 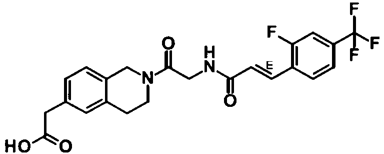 | MS(EST) m/z:465.3 [M+H]+ |
| 188 (I - 188) | 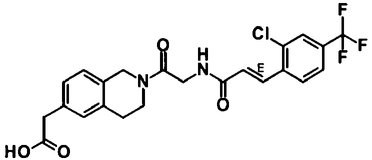 | MS(ESI) m/z:481.3/ 483.3[M+H]+ |
| 189 (I - 189) | 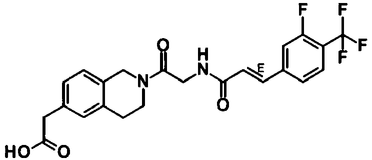 | MS(ESI) m/z:465.3 [M+H]+ |
| 190 (I -190) | 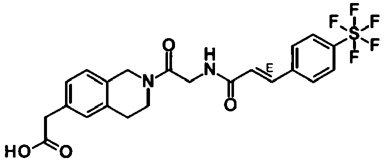 | MS(ESI) m/z:505.3 [M+H]+ |

**[Table 26-11]**

| **Example No. (compound No.)** | **structural formula** | **instrument analysis data** |
|---|---|---|
| 191 (I - 191) | 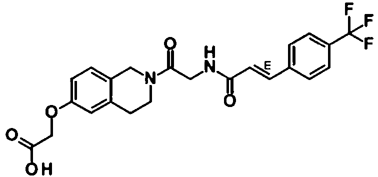 | MS(ESI) m/z:463. 1 [M+H]+ |
| 192 (I - 192) | 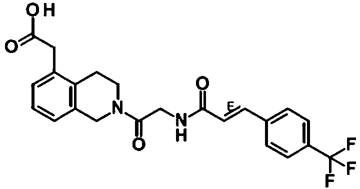 | MS (EST) m/z:445.3 [M-H]- |
| 193 (I - 193) | 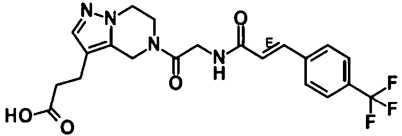 | MS(EST) m/z:419.3 [M-H]- |
| 194 (I - 194) | 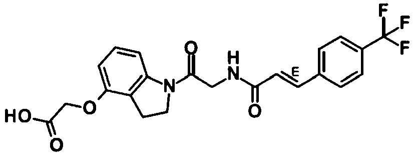 | MS(ESI) m/z: 449.1 [M+H]+ |
| 195 (I - 195) | 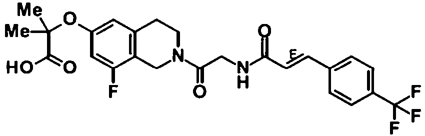 | MS(ESI) m/z:509.3 [M+H]+ |

**[Table 26-12]**

| **Example No. (compound No.)** | **structural formula** | **instrument analysis data** |
|---|---|---|
| 196 (I- 196) | 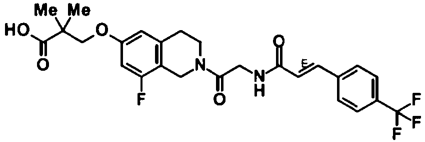 | MS (EST) m/z:523.3 [M+H]+ |
| 197 (I - 197) | 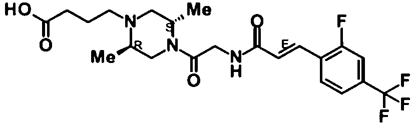 | MS(ESI) m/z:474.4 [M+H]+ |
| 198 (I - 198) | 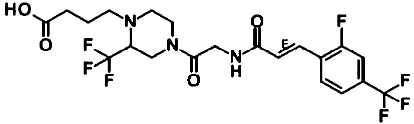 | MS(ESI) m/z:514.3 [M+H]+ |
| 199 (I - 199) | 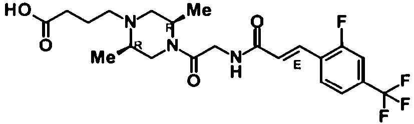 | MS(ESI) m/z:474.3 [M+H]+ |

### Example 200:

### Production of 3-[5-[2-[[(E)-3-[4-(trifluoromethyl)phenyl]prop-2-enoyl]amino]acetyl]-6,7-dihydro-4H-[1,3]thiazolo[5,4-c]pyridin-2-yl]propanoic acid (compound (I-200))

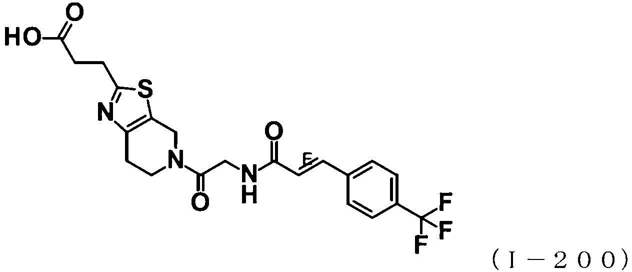

To a solution of methyl 3-[5-[2-[[(E)-3-[4-(trifluoromethyl)phenyl]prop-2-enoyl]amino]acetyl]-6,7-dihydro-4H-thiazolo[5,4-c]pyridin-2-yl]propanoate (30 mg) in dichloroethane (2 ml) was added trimethyltin hydroxide (33.9 mg), and the mixture was stirred at an outer temperature of 80°C for 20 hr. To the reaction mixture was added trimethyltin hydroxide (21.9 mg), and the mixture was further stirred at an outer temperature of 80°C for 4 hr. The reaction mixture was allowed to cool, concentrated under reduced pressure, and the obtained residue was purified by reversed-phase HPLC (10 mmol/l aqueous ammonium carbonate solution/acetonitrile =80/20 - 50/50) to give the title compound (19.3 mg; yield 66%) as a colorless solid.
MS(ESI) m/z: 468.3[M+H]⁺

### Examples 201 to 272:

The corresponding starting compounds were treated in the same manner as in Example 200 to obtain the compounds described in the following Table 27-1 to Table 27-14.

**[Table 27-1]**

| **Example No. (compound No.)** | **structural formula** | **instrument analysis data** |
|---|---|---|
| 201 (I - 201) | 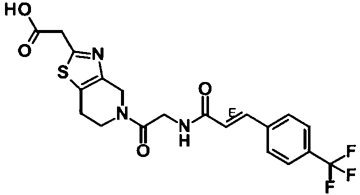 | MS(EST) m/z:454.2 [M+H]+ |
| 202 (I - 202) | 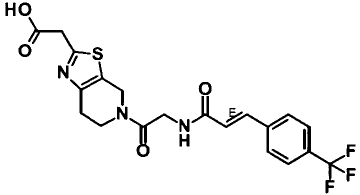 | MS(ESI) m/z:454.3 [M+H]+ |
| 203 (I - 203) | 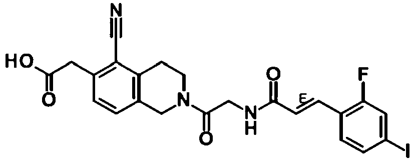 | MS (ESI) m/z:548.2 [M+H]+ |
| 204 (I - 204 ) | 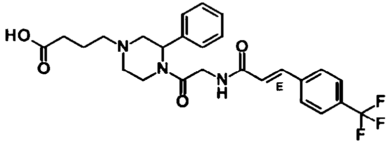 | MS (EST) m/z:504.4 [M+H]+ |
| 205 (I - 205) | 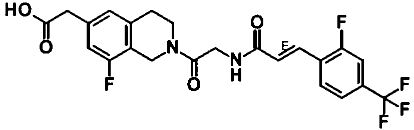 | MS(ESI) m/z:483.3 [M+H]+ |

**[Table 27-2]**

| **Example No. (compound No.)** | **structural formula** | **instrument analysis data** |
|---|---|---|
| 206 (I - 206) | 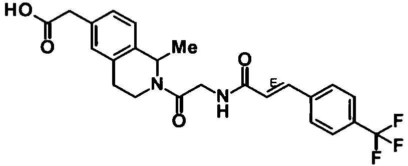 | MS(EST) m/z:161.3 [M+H]+ |
| 207 (I - 207) | 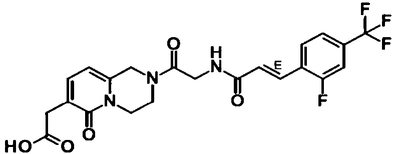 | MS(ESI) m/z:482.3 [M+H]+ |
| 208 (I - 208) | 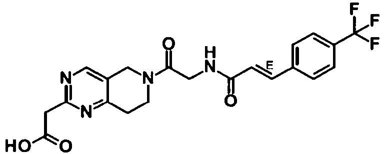 | MS(EST) m/z:449.3 [M+H]+ |
| 209 (I - 209) | 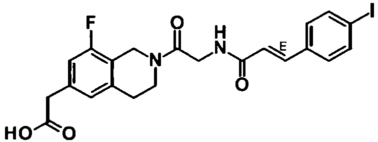 | MS(ESI) m/z:523.2 [M+H] + |
| 210 (I - 210) | 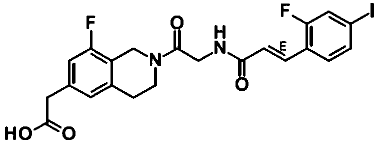 | MS(ESI) m/z:541.2 [M+H]+ |

**[Table 27-3]**

| **Example No. (compound No.)** | **structural formula** | **instrument analysis data** |
|---|---|---|
| 211 (I - 211) | 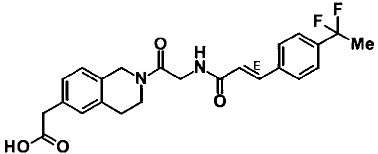 | MS(ESI) m/z:443.3 [M+H] + |
| 212 (I - 212) | 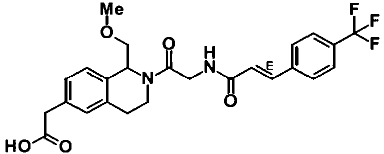 | MS(ESI) m/z:491.3 [M+H]+ |
| 213 (I - 213) | 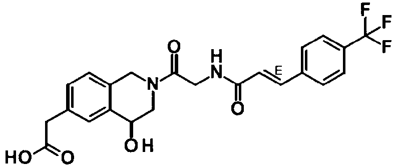 | MS(ESI) m/z:463.3 [M+H]+ |
| 214 (I - 214) | 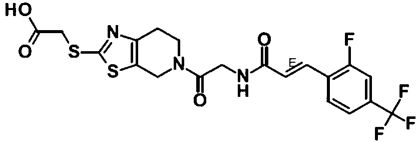 | MS (ESI) m/z:502.3 [M-H]- |
| 215 (I - 215) | 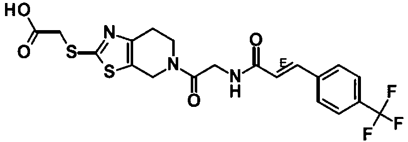 | MS (EST) m/z:484.3 [M-H]- |

**[Table 27-4]**

| **Example No. (compound No.)** | **structural formula** | **instrument analysis data** |
|---|---|---|
| 216 (I - 216) | 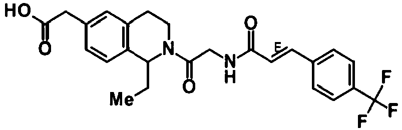 | MS(EST) m/z:473.3 [M-H]- |
| 217 (I - 217) | 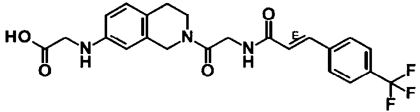 | MS(ESI) m/z:460.2 [M-H]- |
| 218 (I - 218) | 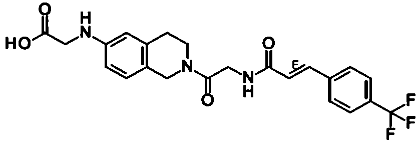 | MS (EST) m/z:462. 1 [M+H]+ |
| 219 (I - 219) | 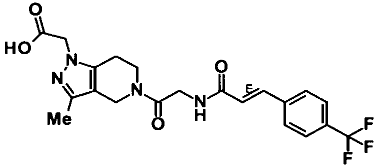 | MS (EST) m/z:451.3 [M+H] + |
| 220 (I - 220) | 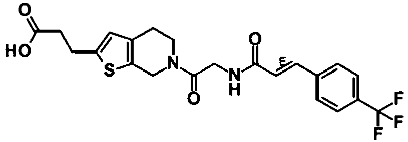 | MS(ESI) m/z:465.2 [M-H]- |

**[Table 27-5]**

| **Example No. (compound No.)** | **structural formula** | **instrument analysis data** |
|---|---|---|
| 221 (I - 221) | 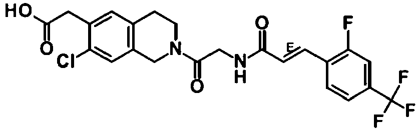 | MS(ESI) m/z:499.0/ 501.0 [M+H]+ |
| 222 (I - 222) | 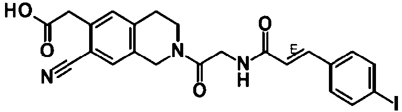 | MS(ESI) m/z:529.9 [M+H]+ |
| 223 (I - 223) | 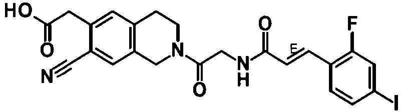 | MS(ESI) m/z:547.9 [M+H]+ |
| 224 (I - 224) | 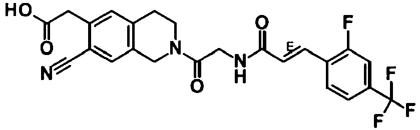 | MS (ESI) m/z:490.0 [M+H]+ |
| 225 (I - 225) | 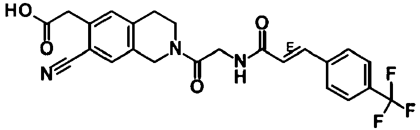 | MS (ESI) m/z:472.1 [M+H]+ |

**[Table 27-6]**

| **Example No. (compound No.)** | **structural formula** | **instrument analysis data** |
|---|---|---|
| 226 (I - 226) | 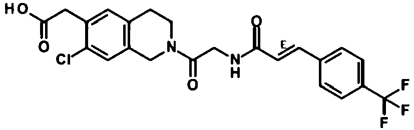 | MS(ESI) m/z:481.0/ 483. 0 [M+H]+ |
| 227 (I - 227) | 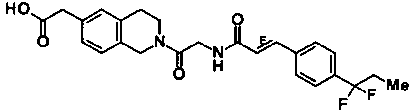 | MS(ESI) m/z:457.3 [M+H]+ |
| 228 (I - 228) | 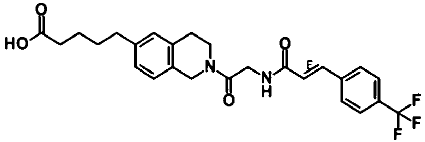 | MS(ESI) m/z:489.0 [M+H]+ |
| 229 (I - 229) | 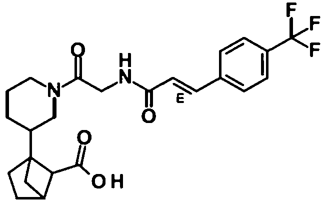 | MS(ESI) m/z:465.3 [M+H]+ |
| 230 (I - 230) | 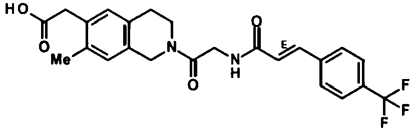 | MS(EST) m/z:461.0 [M+H]+ |
| 231 (I - 231) | 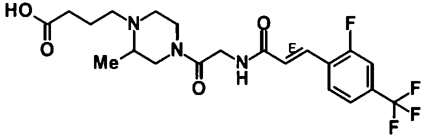 | MS (ESI) m/z:460.3 [M+H]+ |

**[Table 27-7]**

| **Example No. (compound No.)** | **structural formula** | **instrument analysis data** |
|---|---|---|
| 232 (I - 232) | 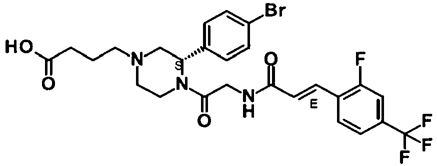 | MS(ESI) m/z:598.2/ 600. 2 [M-H]- |
| 233 (I - 233) | 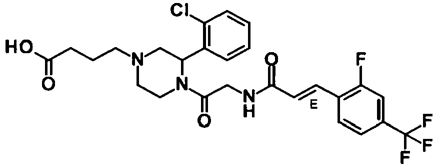 | MS(ESI) m/z:556.2/ 558.1 [M+H]+ |
| 234 (I - 234) | 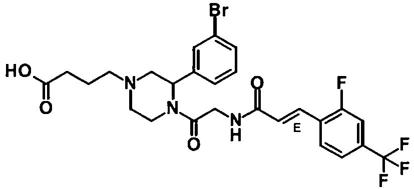 | MS (EST) m/z:600.1/ 602.1 [M+H]+ |
| 235 (I - 235) | 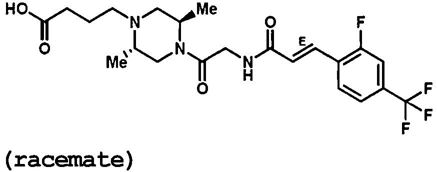 | MS(ESI) m/z:474.3 [M+H]+ |
| 236 (I - 236) | 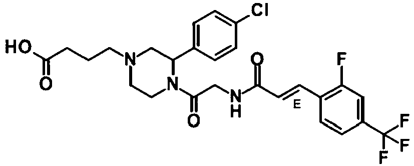 | MS (EST) m/z:554.2/ 556.2 [M-H]- |

**[Table 27-8]**

| **Example No. (compound No.)** | **structural formula** | **instrument analysis data** |
|---|---|---|
| 237 (I - 237) | 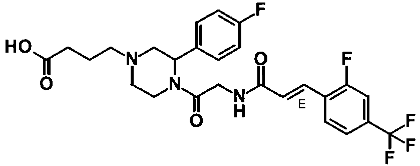 | MS (EST) m/z:538.3 [M-H]- |
| 238 (I - 238) | 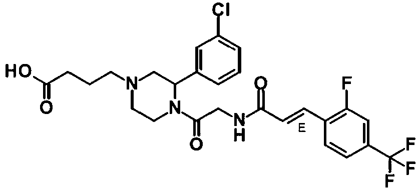 | MS (EST) m/z:554.2/ 556.2 [M-H]- |
| 239 (I - 239) | 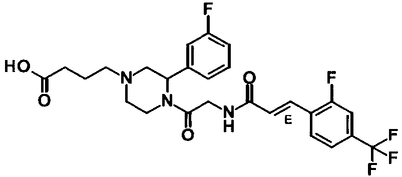 | MS (EST) m/z:538.3 [M-H]- |
| 240 (I - 240) | 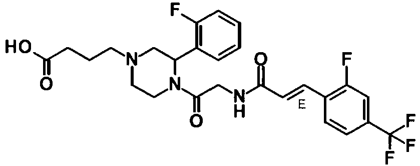 | MS (ESI) m/z:538.3 [M-H]- |
| 241 (I - 241) | 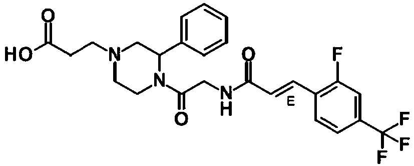 | MS (EST) m/z:506.3 [M-H]- |

**[Table 27-9]**

| **Example No** . **(compound No.)** | **structural formula** | **instrument analysis data** |
|---|---|---|
| 242 (I - 242) | 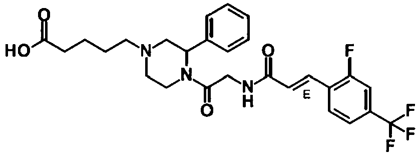 | MS (ESI) m/z:534.3 [M-H]- |
| 243 (I - 243) | 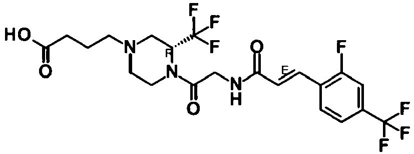 | MS (ESI) m/z:514.3 [M+H]+ |
| 244 (I - 244) | 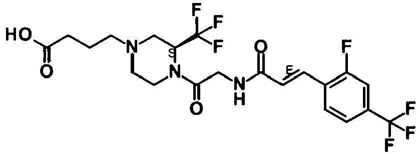 | MS(ESI) m/z:514.3 [M+H]+ |
| 245 (I - 245) | 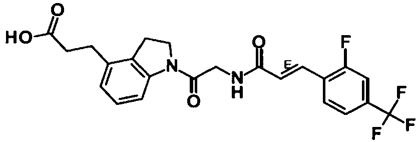 | MS(ESI) m/z:465.2 [M+H]+ |
| 246 (I - 246) | 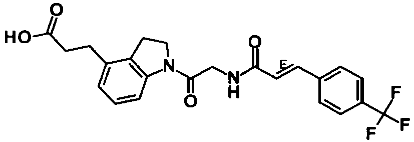 | MS(ESI) m/z:417.0 [M+H]+ |

**[Table 27-10]**

| **Example No. (compound No.)** | **structural formula** | **instrument analysis data** |
|---|---|---|
| 247 (I - 247) | 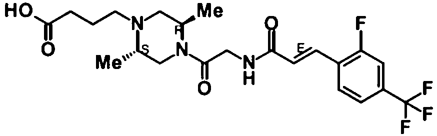 | MS(ESI) m/z:474.2 [M+H]+ |
| 248 (I - 248) | 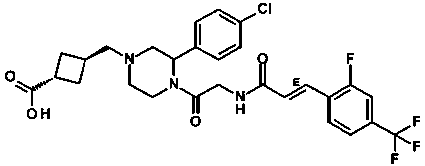 | MS(ESI) m/z:580.2 /582. 2 [M-H]- |
| 249 (I - 249) | 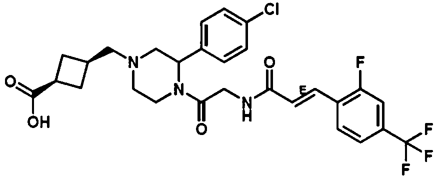 | MS (ESI) m/z:580. 3 /582. 2 [M-H]- |
| 250 (I - 250) | 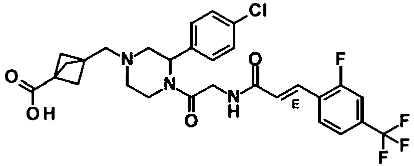 | MS (EST) m/z:592.3 /594.3 [M-H]- |
| 251 (I - 251) | 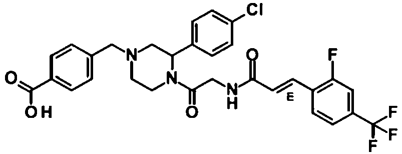 | MS(ESI) m/z:602.2 /604.2 [M-H]- |

**[Table 27-11]**

| **Example No. (compound No.)** | **structural formula** | **instrument analysis data** |
|---|---|---|
| 252 (I - 252) | 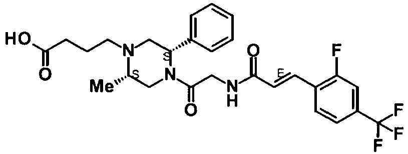 | MS(EST) m/z : 536. 2 [M+H] + |
| 253 (I - 253) | 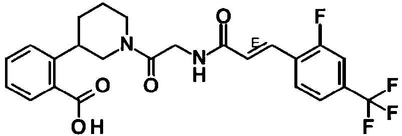 | MS(ESI) m/z:479. 4 [M+H]+ |
| 254 (I - 254) | 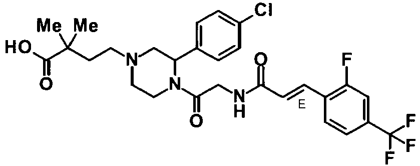 | MS (ESI) m/z : 582. 3/ 584.3 [M-H]- |
| 255 (I - 255) | 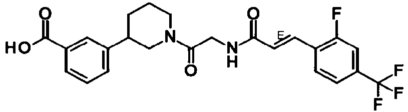 | MS (EST) m/z:477.3 [M-H]- |
| 256 (I - 256) | 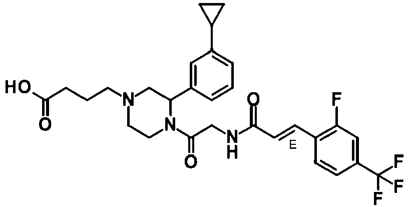 | MS(ESI) m/z:560. 3 [M-H]- |

**[Table 27-12]**

| **Example No. (compound No.)** | **structural formula** | **instrument analysis data** |
|---|---|---|
| 257 (I - 257) | 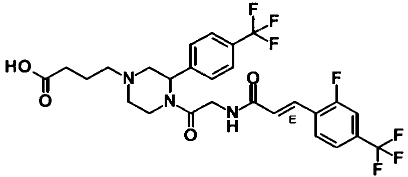 | MS(EST) m/z:588. 3 [M-H]- |
| 258 (I - 258) | 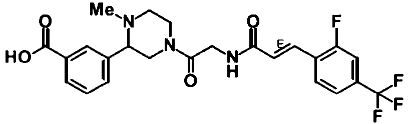 | MS(EST) m/z:494. 2 [M+H]+ |
| 259 (I - 259) | 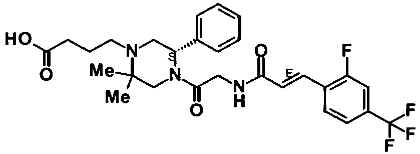 | MS(ESI) m/z:548.2 [M-H]- |
| 260 (I - 260) | 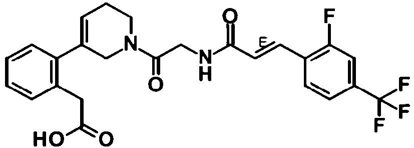 | MS(ESI) m/z:491.2 [M+H]+ |
| 261 (I - 261) | 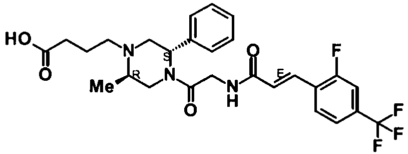 | MS(ESI) m/z:534.2 [M-H]- |

**[Table 27-13]**

| **Example No. (compound No.)** | **structural formula** | **instrument analysis data** |
|---|---|---|
| 262 (I - 262) | 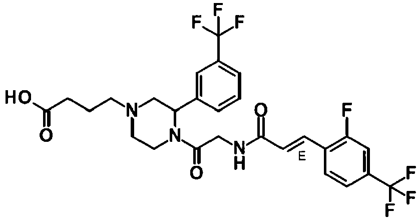 | MS (EST) m/z:588.2 [M-H]- |
| 263 (I - 263) | 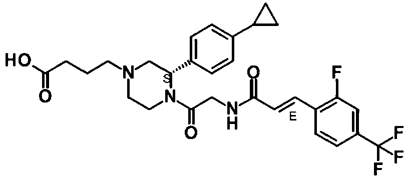 | MS (ESI) m/z:560.3 [M-H]- |
| 264 (I - 264) | 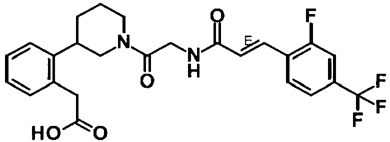 | MS(ESI) m/z:493.2 [M+H]+ |
| 265 (I - 265) | 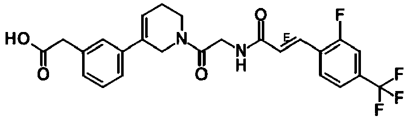 | MS(ESI) m/z:491. 1 [M+H]+ |
| 266 (I - 266) | 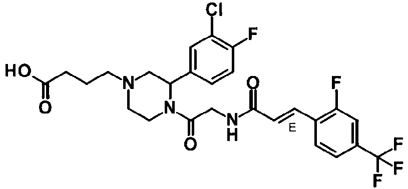 | MS (ESI) m/z:572.2/ 574.2 [M-H]- |

**[Table 27-14]**

| **Example No. (compound No.)** | **structural formula** | **instrument analysis data** |
|---|---|---|
| 267 (I - 267) | 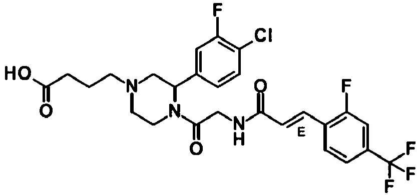 | MS(ESI) m/z:572.2/ 574.2 [M-H]- |
| 268 (I - 268) | 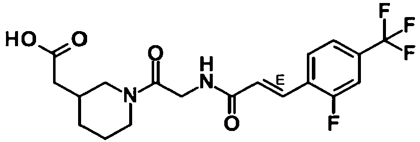 | MS(ESI) m/z:417.2 [M+H]+ |
| 269 (I - 269) | 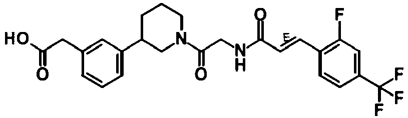 | MS(ESI) m/z: 493. 2 [M+H]+ |
| 270 (I - 270) | 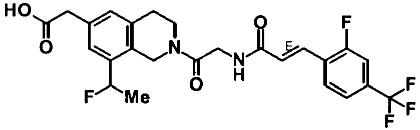 | MS(EST) m/z:511.1 [M+H]+ |
| 271 (I - 270) | 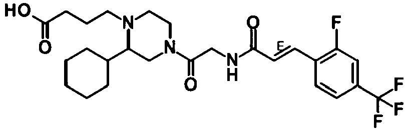 | MS(ESI) m/z:528.2 [M+H]+ |
| 272 (I - 272) | 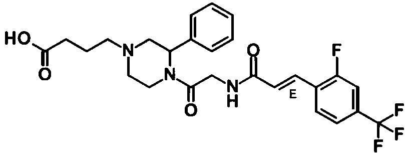 | MS(ESI) m/z: 520.3 [M-H]- |

### Example 273:

### Production of (E)-N-[2-[6-(2-amino-2-oxoethyl)-3,4-dihydro-1H-isoquinolin-2-yl]-2-oxoethyl]-3-[4-(trifluoromethyl)phenyl]prop-2-enamide (compound (I-273))

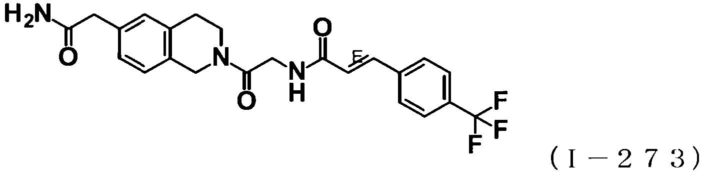

To a solution of 2-[2-[2-[[(E)-3-[4-(trifluoromethyl)phenyl]prop-2-enoyl]amino]acetyl]-3,4-dihydro-1H-isoquinolin-6-yl]acetic acid (90 mg) in dichloromethane (1 ml) were added methanesulfonamide (104 mg), N,N-dimethylpyridine-4-amine (25 mg) and 3-(ethyliminomethylenamino)-N,N-dimethyl-propan-1-amine hydrochloride (47 mg), and the mixture was stirred at room temperature overnight. To the reaction mixture were added methanol and saturated aqueous ammonium chloride solution, and the mixture was concentrated under reduced pressure. The obtained residue was purified by reversed-phase HPLC (10 mmol/l aqueous ammonium carbonate solution/acetonitrile =70/30 - 40/60) to give the title compound (21 mg; yield 23%) as a colorless solid.
MS(ESI) m/z: 446.3[M+H]⁺

### Examples 274 to 284:

The corresponding starting compounds were treated in the same manner as in Example 273 to obtain the compounds described in the following Table 28-1 to Table 28-3.

**[Table 28-1]**

| **Example No. (compound No.)** | **structural formula** | **instrument analysis data** |
|---|---|---|
| 274 (I-274) | 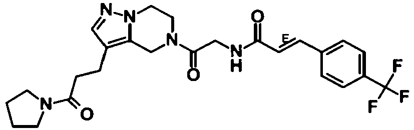 | MS(ESI) m/z:504.4 [M+H]+ |
| 275 (I-275) | 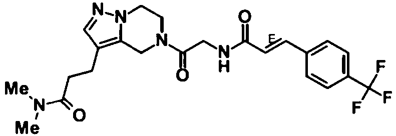 | MS (ESI) m/z:178.1 [M+H]+ |
| 276 (I-276) | 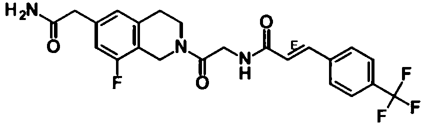 | MS(ESI) m/z:464.2 [M+H]+ |
| 277 (I-277) | 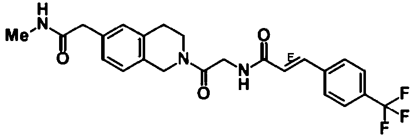 | MS(EST) m/z:460.2 [M+H]+ |
| 278 (I-278) | 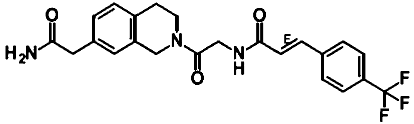 | MS(ESI) m/z:446.3 [M+H]+ |

**[Table 28-2]**

| **Example No. (compound No.)** | **structural formula** | **instrument analysis data** |
|---|---|---|
| 279 (I-279) | 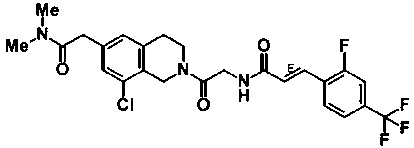 | MS (EST) m/z:526. 2/ 528.2[M+H]+ |
| 280 (I-280) | 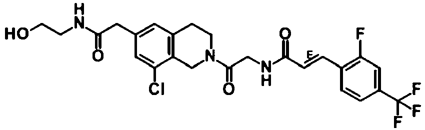 | MS (ESI) m/z:542.2/ 544.2[M+H]+ |
| 281 (I-281) | 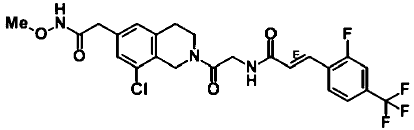 | MS (EST) m/z :528.1/ 530. 1 [M+H]+ |
| 282 (I-282) | 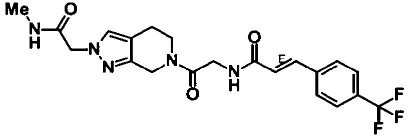 | MS (EST) m/z:450.2 [M+H]+ |
| 283 (I-283) | 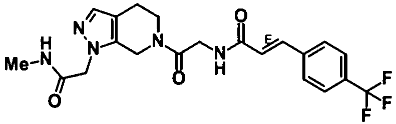 | MS (ESI) m/z:450.2 [M+H] + |

**[Table 28-3]**

| **Example No. (compound No.)** | **structural formula** | **instrument analysis data** |
|---|---|---|
| 284 (I-284) | 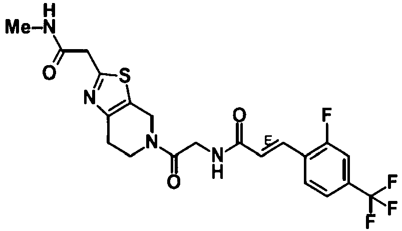 | MS(ESI) m/z:485.2 [M+H]+ |

### Example 285:

### Production of methyl 2-[8-(1-fluoroethyl)-2-[2-[[(E)-3-[2-fluoro-4-(trifluoromethyl)phenyl]prop-2-enoyl]amino]acetyl]-3,4-dihydro-1H-isoquinolin-6-yl]acetate (compound (I-285))

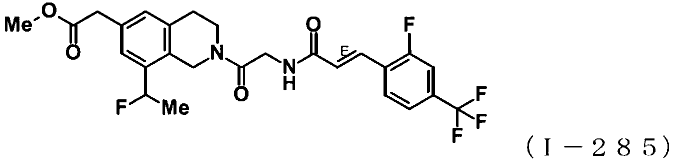

(1) (E)-N-[2-(6-bromo-8-chloro-3,4-dihydro-1H-isoquinolin-2-yl)-2-oxoethyl]-3-[2-fluoro-4-(trifluoromethyl)phenyl]prop-2-enamide (380 mg) produced in Reference Example 65 was dissolved in DMF (1.5 ml), tert-butyl(1-methoxyvinyloxy)dimethylsilane (413 mg), potassium carbonate (202 mg) and bis(tri-tert-butylphosphine)palladium(0) (112 mg) were added, and the reaction mixture was stirred under microwave irradiation at 120°C for 1 hr. The reaction mixture was allowed to cool to room temperature, saturated aqueous ammonium chloride solution was added, and the mixture was extracted with ethyl acetate. The organic layer was washed with saturated brine, dried over anhydrous magnesium sulfate, and concentrated under reduced pressure, and the residue was purified by reversed-phase HPLC (10 mmol/1 aqueous ammonium carbonate solution/acetonitrile =50/50 - 20/80) to give methyl 2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetate (112 mg; yield 30%) as a yellow amorphous.
   MS(ESI) m/z: 513.2/515.1[M+H]⁺
(2) To a solution of methyl 2-[8-chloro-2-[2-[[(E)-3-[2-fluoro-4-(trifluoromethyl)phenyllprop-2-enoyl]amino]acetyl]-3,4-dihydro-1H-isoquinolin-6-yl]acetate (109 mg) obtained in the aforementioned (1) in toluene (1 ml) were added chloro(2-dicyclohexylphosphino-2',4',6'-triisopropyl-1,1'-biphenyl)(2'-amino-1,1'-biphenyl-2-yl)palladium(II) (17 mg) and tributyl (1-ethoxyvinyl)tin (92 mg), and the reaction mixture was stirred under nitrogen atmosphere at 140°C for 2 hr. The reaction mixture was cooled to room temperature, hydrochloric acid (1 mol/l, 2 ml) and THF (2 ml) were added thereto, and the mixture was stirred at room temperature for 1 hr. To the reaction mixture was added aqueous sodium hydroxide solution (1 mol/l, 2 ml), and the mixture was extracted with ethyl acetate. The organic layer was concentrated under reduced pressure, and the residue was purified by reversed-phase HPLC (10 mmol/l aqueous ammonium carbonate solution/acetonitrile =60/40 - 30/70) to give methyl 2-[8-acetyl-2-[2-[[(E)-3-[2-fluoro-4-(trifluoromethyl)phenyl]prop-2-enoyl]amino]acetyl]-3,4-dihydro-1H-isoquinolin-6-yl]acetate (72 mg; yield 65%) as a pale yellow amorphous.
   MS(ESI) m/z: 521.2[M+H]⁺
(3) Methyl 2-[8-acetyl-2-[2-[[(E)-3-[2-fluoro-4-(trifluoromethyl)phenyl]prop-2-enoyl]amino]acetyl]-3,4-dihydro-1H-isoquinolin-6-yl]acetate (72 mg) obtained in the aforementioned (2) was dissolved in methanol (1 ml), and sodium borohydride (11 mg) was added under ice-cooling. The reaction mixture was warmed to room temperature and stirred for 1 hr. To the reaction mixture was added saturated aqueous ammonium chloride solution, and the mixture was extracted with chloroform. The organic layer was concentrated under reduced pressure, and the residue was purified by reversed-phase HPLC (10 mmol/1 aqueous ammonium carbonate solution/acetonitrile =50/50 - 20/80) to give methyl 2-[2-[2-[[(E)-3-[2-fluoro-4-(trifluoromethyl)phenyl]prop-2-enoyl]amino]acetyl]-8-(1-hydroxyethyl)-3,4-dihydro-1H-isoquinolin-6-yl]acetate (47 mg; yield 65%) as a colorless amorphous.
   MS(ESI) m/z: 523.2[M+H]⁺
(4) Methyl 2-[2-[2-[[(E)-3-[2-fluoro-4-(trifluoromethyl)phenyl]prop-2-enoyl]amino]acetyl]-8-(1-hydroxyethyl)-3,4-dihydro-1H-isoquinolin-6-yl]acetate (44 mg) obtained in the aforementioned (3) was dissolved in dichloromethane (1 ml), and bis(2-methoxyethyl)aminotrifluorosulfur (0.023 ml) was added dropwise thereto under nitrogen atmosphere. The reaction mixture was stirred at room temperature for 2 hr, saturated aqueous sodium hydrogen carbonate solution was added, and the mixture was extracted with chloroform. The organic layer was dried over sodium sulfate and concentrated under reduced pressure, and the residue was purified by reversed-phase HPLC (10 mmol/l aqueous ammonium carbonate solution/acetonitrile =50/50 - 20/80) to give the title compound (37 mg; yield 83.8%) as a colorless amorphous. MS(ESI) m/z: 525.3[M+H]⁺

### Examples 286, 287:

### Production of both enantiomers of 2-[2-[2-[[(E)-3-[4-(trifluoromethyl)phenyl]prop-2-enoyl]amino]acetyl]-3,4-dihydro-1H-isoquinolin-6-yl]propanoic acid (compound (I-286), compound (I-287))

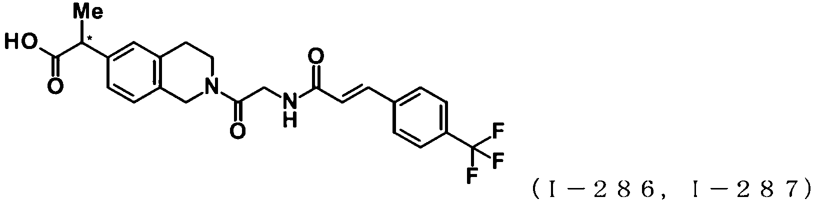

The racemate of 2-[2-[2-[[(E)-3-[4-(trifluoromethyl)phenyl]prop-2-enoylamino]acetyl]-3,4-dihydro-1H-isoquinolin-6-yl]propanoic acid (compound (I-171)) (62 mg) produced in Example 171 was optically resolved by chiral HPLC to give two enantiomers of the title compound as shown in the following Table 29.

**[Table 29]**

| Example No. (compound No.) | instrument analysis data | analysis conditions |
|---|---|---|
| 286 (I-286) | MS(ESI) m/z: 461.3[M+H]⁺ | column: CHIRALPAK IG-3 (4.6×150mm) |
| | | mobile phase: methanol/acetonitrile/acetic acid (80/20/0.1) |
| | | flow rate: 0.5ml/min |
| | | temperature: 25°C |
| | | analysis channel: PDA269.0nm |
| | | retention time (min):11.998 |
| 287 (enantiomer of compound (I-286)) (I-287) | MS(ESI) m/z: 461.3[M+H]⁺ | column: CHIRALPAK IG-3 (4.6×150mm) |
| | | mobile phase: methanol/acetonitrile/acetic acid (80/20/0.1) |
| | | flow rate: 0.5ml/min |
| | | temperature: 25°C |
| | | analysis channel: PDA269.0nm |
| | | retention time (min):14.969 |

### Examples 288,289

### Production of both enantiomers of (1R*,2R*)-2-[2-[2-[[(E)-3-[4-(trifluoromethyl)phenyl]prop-2-enoyl]amino]acetyl]-3,4-dihydro-1H-isoquinolin-6-yl]cyclopropane-1-carboxylic acid (compound (I-288), compound (I-289))

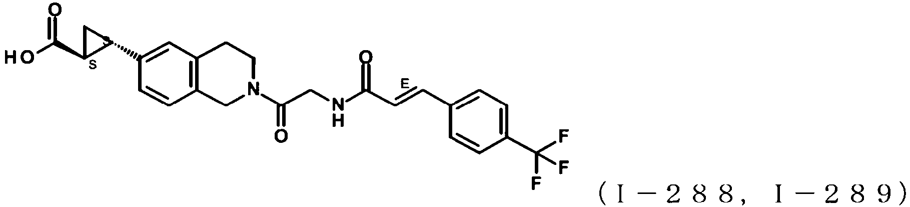

The racemate of (1R*,2R*)-2-[2-[2-[[(E)-3-[4-(trifluoromethyl)phenyl]prop-2-enoylamino]acetyl]-3,4-dihydro-1H-isoquinolin-6-yl]cyclopropanoic acid (compound (I-172)) (58 mg) produced in Example 172 was optically resolved by chiral HPLC to give two enantiomers of the title compound as shown in the following Table 30.

**[Table 30]**

| Example No. (compound No.) | instrument analysis data | analysis conditions |
|---|---|---|
| 288 (I-288) | MS(ESI) m/z: 473.3[M+H]⁺ | column: CHIRALPAK IE-3 (4.6×150mm) |
| | | mobile phase: hexane/ethanol/acetic acid (15/85/0.1) |
| | | flow rate: 0.5ml/min |
| | | temperature: 25°C |
| | | analysis channel: PDA269.0nm |
| | | retention time (min):10.088 |
| 289 (enantiomer of compound (I-288)) (I-289) | MS(ESI) m/z: 473.3[M+H]⁺ | column: CHIRALPAK IE-3 (4.6×150mm) |
| | | mobile phase: hexane/ethanol/acetic acid (15/85/0.1) |
| | | flow rate: 0.5ml/min |
| | | temperature: 25°C |
| | | analysis channel: PDA269.0nm |
| | | retention time (min):12.411 |

### Examples 290, 291:

### Production of both enantiomers of 4-[4-[2-[[(E)-3-[2-fluoro-4-(trifluoromethyl)phenyl]prop-2-enoyl]amino]acetyl]-3-phenylpiperazin-1-yl]butanoic acid (compound (I-290), compound (I-291))

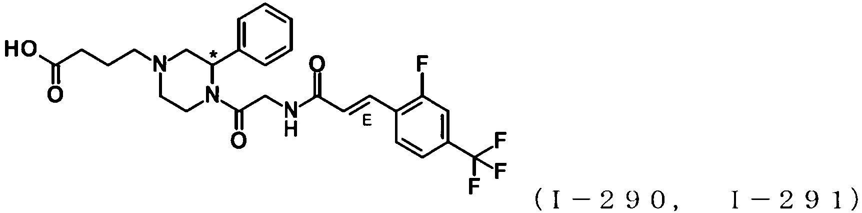

The racemate of 4-[4-[2-[[(E)-3-[2-fluoro-4-(trifluoromethyl)phenyl]prop-2-enoyl]amino]acetyl]-3-phenylpiperazin-1-yl]butanoic acid (compound (I-272)) (43.3 mg) produced in Example 272 was optically resolved by chiral HPLC to give two enantiomers of the title compound as shown in the following Table 31.

**[Table 31]**

| Example No. (compound No.) | instrument analysis data | analysis conditions |
|---|---|---|
| 290 (I-290) | MS(ESI) m/z: 520.3 [M-H]- | column: CHIRALPAK ID-3 (4.6×150mm) |
| | | mobile phase: 2-propanol/ethanol/acetic acid (30/70/0.1) |
| | | flow rate: 0.5ml/min |
| | | temperature: 25°C |
| | | analysis channel: PDA265.0nm |
| | | retention time (min):7.716 |
| 291 (enantiomer of compound (I-290)) (I-291) | MS (ESI) m/z: 520.3 [M-H]- | column: CHIRALPAK ID-3 (4.6×150mm) |
| | | mobile phase: 2-propanol/ethanol/acetic acid (30/70/0.1) |
| | | flow rate: 0.5ml/min |
| | | temperature: 25°C |
| | | analysis channel: PDA265.0nm |
| | | retention time (min):10.486 |

### Examples 292, 293:

### Production of both enantiomers of 4-[3-(3-bromophenyl)-4-[2-[[(E)-3-[2-fluoro-4-(trifluoromethyl)phenyl]prop-2-enoyl]amino]acetyl]piperazin-1-yl]butanoic acid (compound (I-292), compound (I-293))

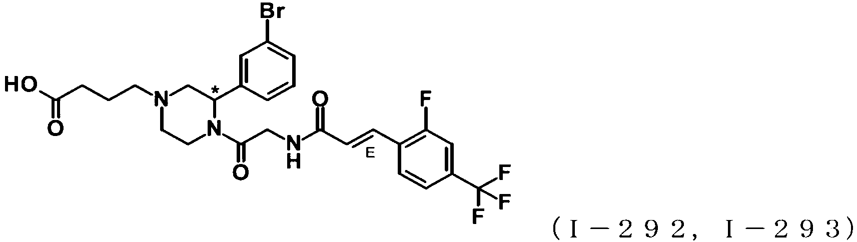

The racemate of 4-[3-(3-bromophenyl)-4-[2-[[(E)-3-[2-fluoro-4-(trifluoromethyl)phenyl]prop-2-enoyl]amino]acetyl]piperazin-1-yl]butanoic acid (compound (I-234)) (21.7 mg) produced in Example 234 was optically resolved by chiral HPLC to give two enantiomers of the title compound as shown in the following Table 32.

**[Table 32]**

| Example No. (compound No.) | instrument analysis data | analysis conditions |
|---|---|---|
| 292 (I-292) | MS(ESI) m/z: 598.2/600.2 [M-H]- | column: CHIRALPAK IBN-3 (4.6×150mm) |
| | | mobile phase: hexane/ethanol/acetic acid (40/60/0.1) |
| | | flow rate: 0.5ml/min |
| | | temperature: 25°C |
| | | analysis channel: PDA267.0nm |
| | | retention time (min):10.665 |
| 293 (enantiomer of compound (I-292)) (I-293) | MS(ESI) m/z: 598.2/600.2 [M-H]- | column: CHIRALPAK IBN-3 (4.6×150mm) |
| | | mobile phase: hexane/ethanol/acetic acid (40/60/0.1) |
| | | flow rate: 0.5ml/min |
| | | temperature: 25°C |
| | | analysis channel: PDA267.0nm |
| | | retention time (min):13.932 |

### Examples 294, 295

### Production of both enantiomers of 4-[3-(4-chlorophenyl)-4-[2-[[(E)-3-[2-fluoro-4-(trifluoromethyl)phenyl]prop-2-enoyl]amino]acetyl]piperazin-1-yl]butanoic acid (compound (I-294), compound (I-295))

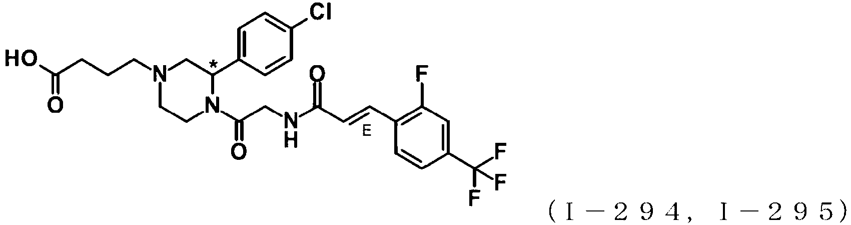

The racemate of 4-[3-(4-chlorophenyl)-4-[2-[[(E)-3-[2-fluoro-4-(trifluoromethyl)phenyl]prop-2-enoyl]amino]acetyl]piperazin-1-yl]butanoic acid (compound (I-236)) (39.9 mg) produced in Example 236 was optically resolved by chiral HPLC to give two enantiomers of the title compound as shown in the following Table 33.

**[Table 33]**

| Example No. (compound No.) | instrument analysis data | analysis conditions |
|---|---|---|
| 294 (I-294) | MS(ESI) m/z: 554.2/556.2 [M-H]- | column: CHIRALPAK IBN-3 (4.6×150mm) |
| | | mobile phase: hexane/ethanol/acetic acid (40/60/0.1) |
| | | flow rate: 0.5ml/min |
| | | temperature: 25°C |
| | | analysis channel: PDA267.0nm |
| | | retention time (min):9.639 |
| 295 (enantiomer of compound (1-294)) (I-295) | MS(ESI) m/z: 554.2/556.2 [M-H]- | column: CHIRALPAK IBN-3 (4.6×150mm) |
| | | mobile phase: hexane/ethanol/acetic acid (40/60/0.1) |
| | | flow rate: 0.5ml/min |
| | | temperature: 25°C |
| | | analysis channel: PDA267.0nm |
| | | retention time (min):12.196 |

### Examples 296, 297

### Production of both enantiomers of 3-[[3-(4-chlorophenyl)-4-[2-[[(E)-3-[2-fluoro-4-(trifluoromethyl)phenyl]prop-2-enoyl]amino]acetyl]piperazin-1-yl]methyl]cyclobutane-1-carboxylic acid (compound (I-296), compound (I-297))

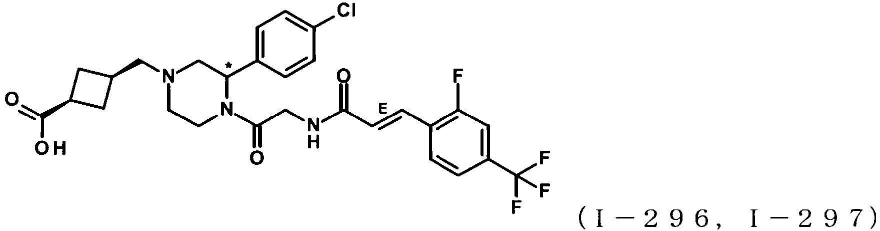

The racemate of 3-[[3-(4-chlorophenyl)-4-[2-[[(E)-3-[2-fluoro-4-(trifluoromethyl)phenyl]prop-2-enoyl]amino]acetyl]piperazin-1-yl]methyl]cyclobutane-1-carboxylic acid (compound (I-249)) (29 mg) produced in Example 249 was optically resolved by chiral HPLC to give two enantiomers of the title compound as shown in the following Table 34.

**[Table 34]**

| Example No. (compound No.) | instrument analysis data | analysis conditions |
|---|---|---|
| 296 (I-296) | MS(ESI) m/z: 580.2/582.3 [M-H]- | column: CHIRALPAK IBN-3 (4.6×150mm) |
| | | mobile phase: hexane/ethanol/acetic acid (40/60/0.1) |
| | | flow rate: 0.5ml/min |
| | | temperature: 25°C |
| | | analysis channel: PDA267.0nm |
| | | retention time (min):9.749 |
| 297 (enantiomer of compound (I-296)) (I-297) | MS(ESI) m/z: 580.2/582.2 [M-H]- | column: CHIRALPAK IBN-3 (4.6×150mm) |
| | | mobile phase: hexane/ethanol/acetic acid (40/60/0.1) |
| | | flow rate: 0.5ml/min |
| | | temperature: 25°C |
| | | analysis channel: PDA267.0nm |
| | | retention time (min):12.633 |

### Examples 298 to 323:

The corresponding starting compounds were treated in the same manner as in Example 1 to obtain the compounds described in the following Table 35-1 to Table 35-5.

**[Table 35-1]**

| **Example No. (compound No.)** | **structural formula** | **instrument analysis data** |
|---|---|---|
| 298 (I - 298) | 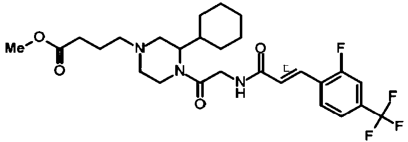 | MS(ESI) m/z: 542. 1 [M+H ]+ |
| 299 (I - 299) | 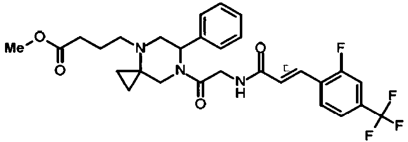 | MS(ESI) m/z: 562.2[M+H ]+ |
| 300 (I - 300) | 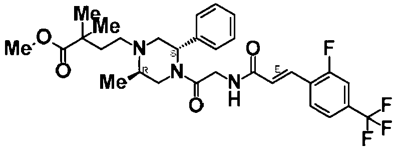 | MS(ESI) m/z: 578.3[M+H ]+ |
| 301 (I - 301) | 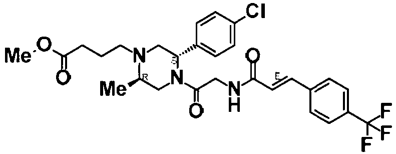 | MS(ESI) m/z: 566.3/568 .3 [M+H]+ |
| 302 (I - 302) | 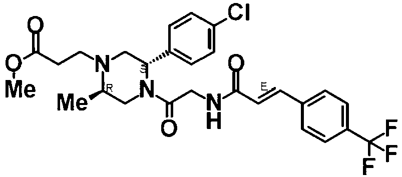 | MS(ESI) m/z: 552.3/554 .3 [M+H]+ |

**[Table 35-2]**

| **Example No. (compound No.)** | **structural formula** | **instrument analysis data** |
|---|---|---|
| 303 (I - 303) | 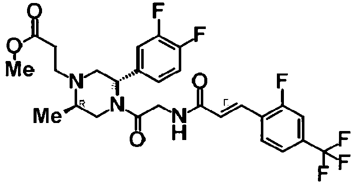 | MS(ESI) m/z: 572.3[M+H ]+ |
| 304 (I - 304) | 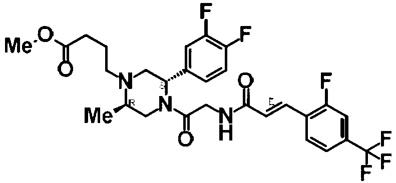 | MS(ESI) m/z: 586.3[M+H ]+ |
| 305 (I - 305) | 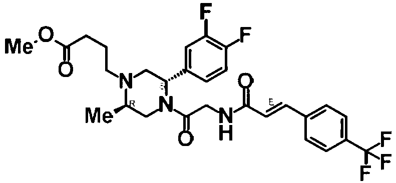 | MS(ESI) m/z: 568.3[M+H ]+ |
| 306 (I - 306) | 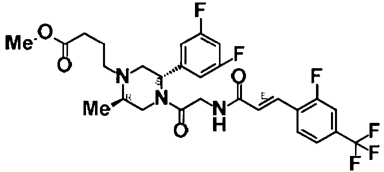 | MS(ESI) m/z: 584.3 [M-H]- |
| 307 (I - 307) | 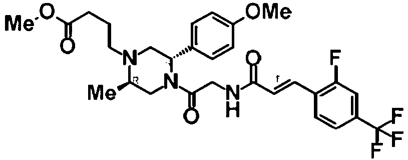 | MS(ESI) m/z: 580.2[M+H ]+ |

**[Table 35-3]**

| **Example No. (compound No.)** | **structural formula** | **instrument analysis data** |
|---|---|---|
| 308 (I - 308) | 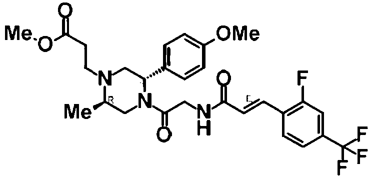 | MS(ESI) m/z: 566.5[M+H ]+ |
| 309 (I - 309) | 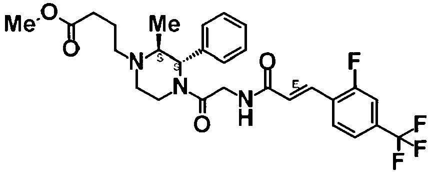 | MS(ESI) m/z: 550.3[M+H ]+ |
| 310 (I - 310) | 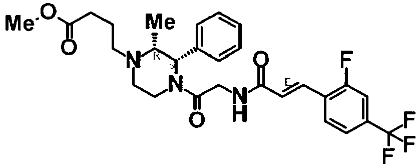 | MS(ESI) m/z: 550.0[M+H ]+ |
| 311 (I - 311) | 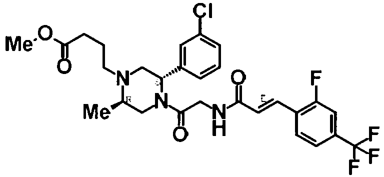 | MS(ESI) m/z: 584.3/586 .2 [M+H]+ |
| 312 (I - 312) | 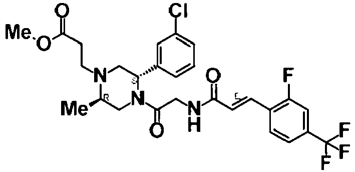 | MS (ESI) m/z: 570.3/572 .2 [M+H]+ |
| 313 (I - 313) | 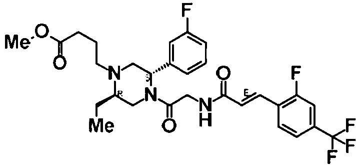 | MS(ESI) m/z: 582.3 [M+ H]+ |

**[Table 35-4]**

| **Example No. (compound No.)** | **structural formula** | **instrument analysis data** |
|---|---|---|
| 314 (I - 314) | 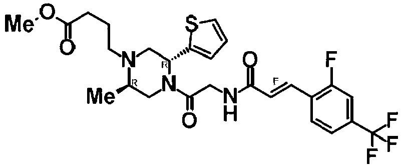 | MS(ESI) m/z: 556. 5[M+H ]+ |
| 315 (I - 315) | 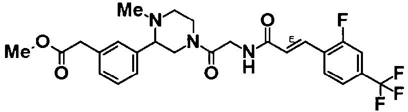 | MS(ESI) m/z: 522. 2[M+H ]+ |
| 316 (I - 316) | 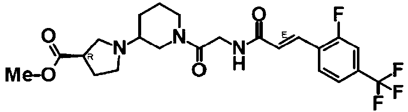 | MS(ESI) m/z: 486. 3[M+H ]+ |
| 317 (I - 317) | 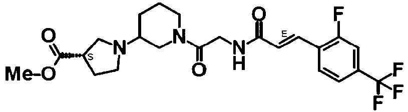 | MS(ESI) m/z: 486. 3[M+H ]+ |

**[Table 35-5]**

| **Example No. (compound No.)** | **structural formula** | **instrument analysis data** |
|---|---|---|
| 318 (I - 318) | 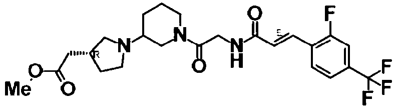 | MS(ESI) m/z: 500. 2[M+H ]+ |
| 319 (I - 319) | 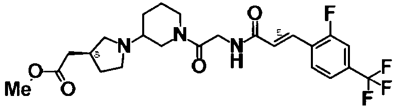 | MS(ESI) m/z: 500.2[M+H ]+ |

### Examples 320 to 323:

The corresponding starting compounds were treated in the same manner as in Example 83 to obtain the compounds described in the following Table 36.

In Example 320, progress was made up to hydrolysis under the reaction conditions of Example 83 and the corresponding carboxylic acid was obtained.

**[Table 36]**

| **Example No. (compound No.)** | **structural formula** | **instrument analysis data** |
|---|---|---|
| 320 (I - 320) | 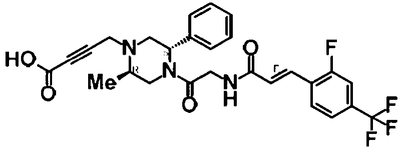 | MS(ESI) m/z: 532.2[M+H ]+ |
| 321 (I - 321) | 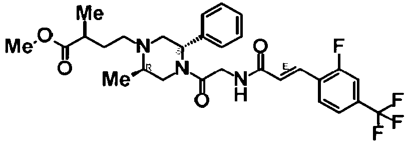 | MS(ESI) m/z: 564.3[M+H ]+ |
| 322 (I - 322) | 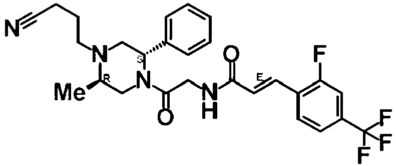 | MS(ESI) m/z: 517.2[M+H ]+ |
| 323 (I - 323) | 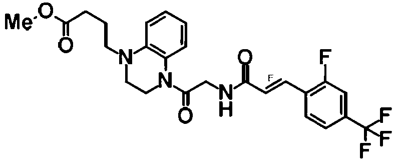 | MS(ESI) m/z: 508.2[M+H ]+ |

### Example 324:

### Production of methyl 4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoate (compound (I-324))

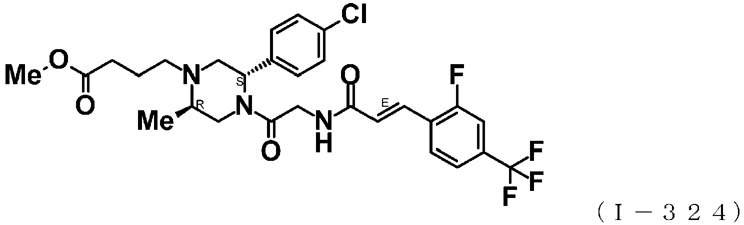

(1) To a solution of (2R,5S)-5-(4-chlorophenyl)-2-methyl-1-(4-nitrophenyl)sulfonyl piperazine (300 mg) produced in Reference Example 109 in DMF (3.1 ml) were added 2-[[(E)-3-[2-fluoro-4-(trifluoromethyl)phenyl]prop-2-enoyl]amino]acetic acid (232 mg), HATU (318 mg) and N,N-diisopropylethylamine (0.33 ml), and the mixture was stirred at room temperature overnight. To the reaction mixture were added 2-[[(E)-3-[2-fluoro-4-(trifluoromethyl)phenyl]prop-2-enoyl]amino]acetic acid (45 mg) and HATU (86 mg), and the mixture was stirred again at room temperature overnight. Water was added to the reaction mixture and the mixture was extracted with ethyl acetate. The organic layer was washed with saturated brine, dried over anhydrous sodium sulfate, and concentrated under reduced pressure. The residue was purified by NH silica gel chromatography (solvent: n-hexane/ethyl acetate =67/33 - 50/50), silica gel chromatography (solvent: n-hexane/ethyl acetate =50/50 - 0/100) to give (E)-N-[2-[(2S,5R)-2-(4-chlorophenyl)-5-methyl-4-(4-nitrophenyl)sulfonylpiperazin-1-yl]-2-oxoethyl]-3-[2-fluoro-4-(trifluoromethyl)phenyl]prop-2-enamide (339 mg; yield 67%) as a yellow viscous oil.
   MS(ESI) m/z: 669.2/671.2[M+H]⁺
(2) To a solution of (E)-N-[2-[(2S,5R)-2-(4-chlorophenyl)-5-methyl-4-(4-nitrophenyl)sulfonylpiperazin-1-yl]-2-oxoethyl]-3-[2-fluoro-4-(trifluoromethyl)phenyl]prop-2-enamide (320 mg) obtained in the aforementioned (1) in methanol (3.2 ml) were added potassium carbonate (185 mg) and 1-dodecanethiol (0.287 ml), and the mixture was stirred at room temperature for 1 hr. Water was added to the reaction mixture, and the mixture was extracted with chloroform. The organic layer was washed with saturated brine, dried over anhydrous sodium sulfate, and concentrated under reduced pressure. The residue was purified by NH silica gel chromatography (solvent: n-hexane/ethyl acetate =25/75 - 0/100) to give (E)-N-[2-[(2S,5R)-2-(4-chlorophenyl)-5-methylpiperazin-1-yl]-2-oxoethyl]-3-[2-fluoro-4-(trifluoromethyl)phenyl]prop-2-enamide (123 mg; yield 53%) as a pale yellow viscous oil.
   MS(ESI) m/z: 484.2/486.2[M+H]⁺
(3) To a solution of (E)-N-[2-[(2S,5R)-2-(4-chlorophenyl)-5-methylpiperazin-1-yl]-2-oxoethyl]-3-[2-fluoro-4-(trifluoromethyl)phenyl]prop-2-enamide (120 mg) obtained in the aforementioned (2) in DMF (0.62 ml) were added potassium carbonate (103 mg), sodium iodide (38 mg) and methyl 4-bromobutyrate (48 µL), and the mixture was stirred at 85°C overnight. The reaction mixture was cooled to room temperature, water was added, and the mixture was extracted with ethyl acetate. The organic layer was washed with saturated brine, dried over anhydrous sodium sulfate, and concentrated under reduced pressure. The residue was purified by silica gel chromatography (solvent: n-hexane/ethyl acetate =75/25 - 40/60) to give the title compound (110 mg; yield 76%) as a colorless viscous oil.
   MS(ESI) m/z: 584.2/586.2[M+H]⁺

### Examples 325 to 328:

The corresponding starting compounds were treated in the same manner as in Example 324 to obtain the compounds described in the following Table 37.

**[Table 37]**

| **Example No. (compound No.)** | **structural formula** | **instrument analysis data** |
|---|---|---|
| 325 (I - 325) | 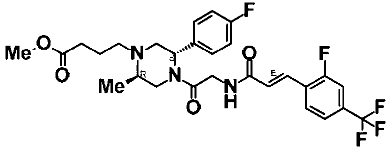 | MS(ESI) m/z: 568.2[M+ H]+ |
| 326 (I - 326) | 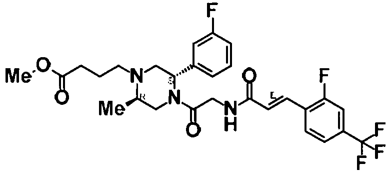 | MS(ESI) m/z: 568.2[M+ H]+ |
| 327 (I - 327) | 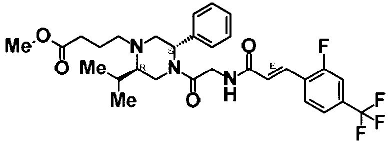 | MS(ESI) m/z: 578.3[M+ H]+ |
| 328 (I - 328) | 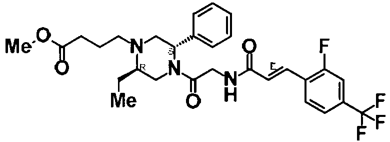 | MS(ESI) m/z: 564.3[M+ H]+ |

### Examples 329 to 330:

The corresponding starting compounds were treated in the same manner as in Example 104 to obtain the compounds described in the following Table 38.

**[Table 38]**

| **Example No. (compound No.)** | **structural formula** | **instrument analysis data** |
|---|---|---|
| 329 (I - 329) | 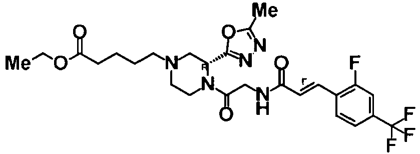 | MS(ESI) m/z: 570.4[M +H]+ |
| 330 (I - 330) | 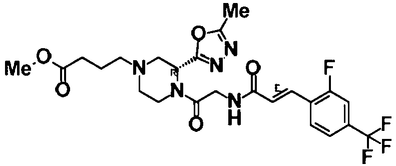 | MS(ESI) m/z: 542.6[M +H]+ |

### Example 331:

### Production of methyl 4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoate (compound (I-331))

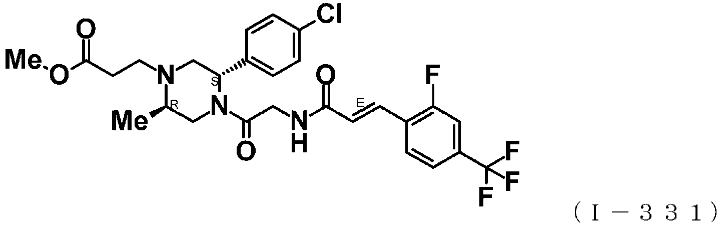

To a solution of (E)-N-[2-[(2S,5R)-2-(4-chlorophenyl)-5-methylpiperazin-1-yl]-2-oxoethyl]-3-[2-fluoro-4-(trifluoromethyl)phenyl]prop-2-enamide (160 mg) in dichloromethane (1.6 ml) were added methyl 3-oxopropanoate (101 mg) and sodium triacetoxyborohydride (1.03 g), and the mixture was stirred at room temperature overnight. Water was added to the reaction mixture and the mixture was extracted with ethyl acetate. The organic layer was washed with water and saturated brine, dried over anhydrous sodium sulfate, and concentrated under reduced pressure. The residue was purified by silica gel chromatography (solvent: n-hexane/ethyl acetate =60/40 - 0/100) to give the title compound (141 mg; yield 75%) as a colorless viscous oil.
MS(ESI) m/z: 570.2/572.1[M+H]⁺

### Examples 332 to 333:

The corresponding starting compounds were treated in the same manner as in Example 331 to obtain the compounds described in the following Table 39.

**[Table 39]**

| **Example No. (compound No.)** | **structural formula** | **instrument analysis data** |
|---|---|---|
| 332 (I - 332) | 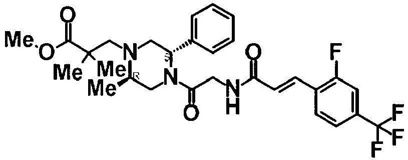 | MS(ESI) m/z: 564.3 [ M+H]+ |
| 333 (I - 333) | 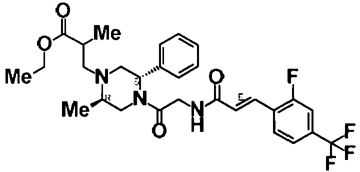 | MS(ESI) m/z: 564.0[M +H]+ |

### Examples 334 to 335:

The corresponding starting compounds were treated in the same manner as in Example 109 to obtain the compounds described in the following Table 40.

**[Table 40]**

| **Example No. (compound No.)** | **structural formula** | **instrument analysis data** |
|---|---|---|
| 334 (I-334) | 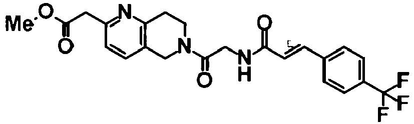 | MS(ESI) m/z: 462.1 [ M+H]+ |
| 335 (I-335) | 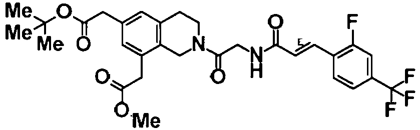 | MS(ESI) m/z: 593.3 [ M+H]+ |

### Example 336:

The corresponding starting compounds were treated in the same manner as in Example 140 to obtain the compound described in the following Table 41.

**[Table 41]**

| **Example No. (compound No.)** | **structural formula** | **instrument analysis data** |
|---|---|---|
| 336 (I-336) | 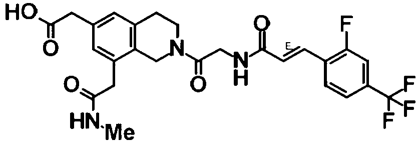 | MS (ESI) m/z: 536. 1[M +H] + |

### Examples 337 to 372:

The corresponding starting compounds were treated in the same manner as in Example 200 to obtain the compounds described in the following Table 42-1 to Table 42-8.

**[Table 42-1]**

| **Example No.** | **structural formula** | **instrument analysis data** |
|---|---|---|
| 337 (I-337) | 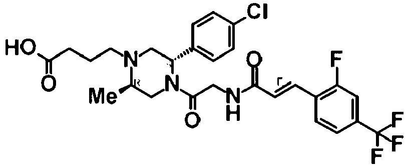 | MS(ESI) m/z: 568.3/57 0.2 [M-H]- |
| 338 (I-338) | 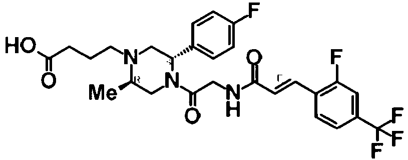 | MS (ESI) m/z: 552.3 [M-H]- |
| 339 (I-339) | 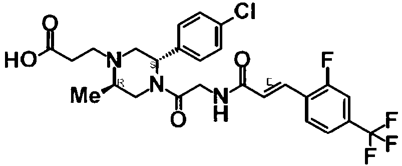 | MS(ESI) m/z: 554.2/55 6.2 [M-H]- |
| 340 (I-340) | 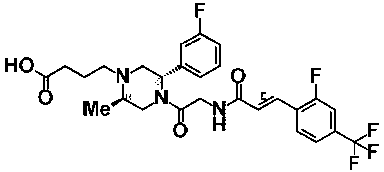 | MS(ESI) m/z: 552.3 [M-H]- |
| 341 (I-341) | 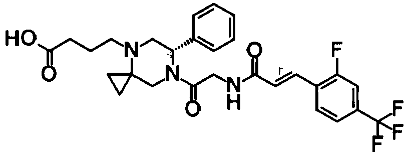 | MS(ESI) m/z: 548.2 [M+ H]+ |

**[Table 42-2]**

| **Example No.** | **structural formula** | **instrument analysis data** |
|---|---|---|
| 3 4 2 (I-34 ) | 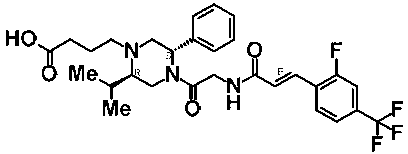 | MS(ESI) m/z: 562.3 [M-H]- |
| 343 (I-343) | 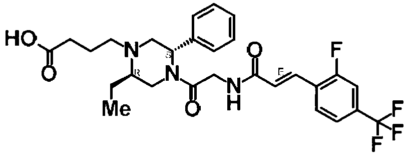 | MS(ESI) m/z: 548.2 [M-H]- |
| 344 (I-344) | 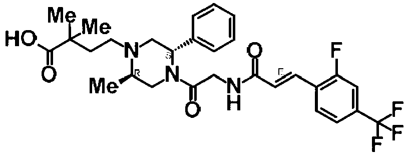 | MS(ESI) m/z: 564.3[M+ H]+ |
| 345 (I-345) | 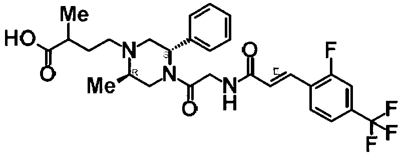 | MS(ESI) m/z: 550.5[M+ H]+ |
| 346 (I-346) | 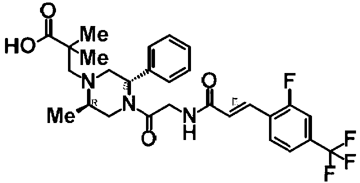 | MS(ESI) m/z: 548.3 [M-H]- |

**[Table 42-3]**

| **Example No.** | **structural formula** | **instrument analysis data** |
|---|---|---|
| 347 (I-347) | 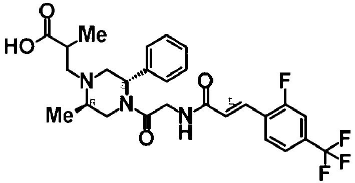 | MS(ESI) m/z: 536.3[M+ H]+ |
| 348 (I-348) | 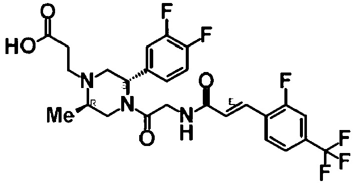 | MS(ESI) m/z: 556.3 [M-H]- |
| 349 (I-349) | 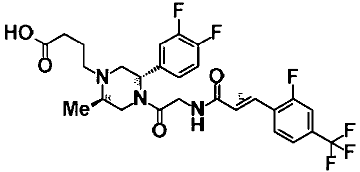 | MS(ESI) m/z: 570.3 [M-H]- |
| 350 (I-350) | 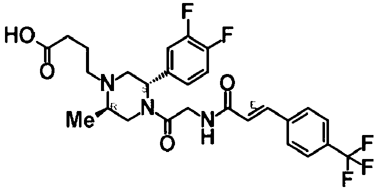 | MS(ESI) m/z: 554.2 [M-H]- |

**[Table 42-4]**

| **Example No.** | **structural formula** | **instrument analysis data** |
|---|---|---|
| 351 (I-351) | 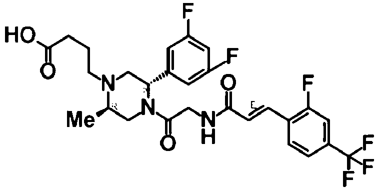 | MS(ESI) m/z: 570.3 [M-H]- |
| 352 (I-352) | 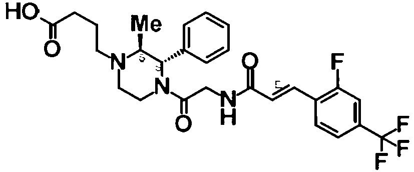 | MS (ESI) m/z: 536.3 [M+ H]+ |
| 353 (I-353) | 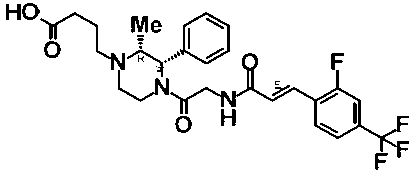 | MS(ESI) m/z: 536.3[M+ H]+ |
| 354 (I-354) | 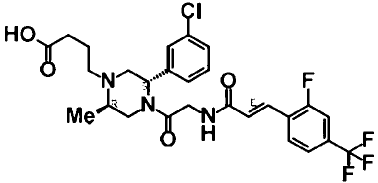 | MS(ESI) m/z: 568.3/57 0.2 [M-H]- |

**[Table 42-5]**

| **Example No.** | **structural formula** | **instrument analysis data** |
|---|---|---|
| 355 (I-355) | 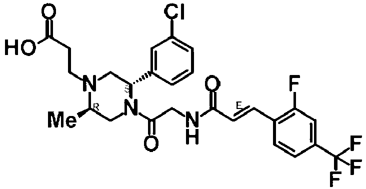 | MS(ESI) m/z: 554.2/55 6.3 [M-H]- |
| 356 (I-356) | 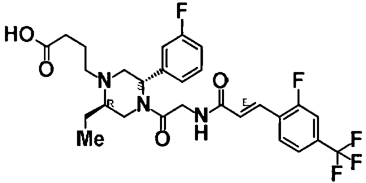 | MS(ESI) m/z: 566.4 [M-H]- |
| 357 (I-357) | 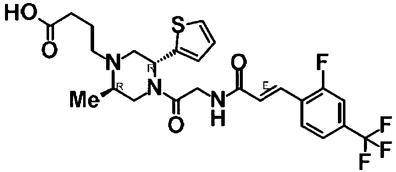 | MS (ESI) m/z: 542.2[M+ H]+ |
| 358 (I-358) | 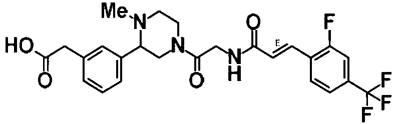 | MS(ESI) m/z: 508.2 [M+ H]+ |

**[Table 42-6]**

| **Example No.** | **structural formula** | **instrument analysis data** |
|---|---|---|
| 359 (I-359) | 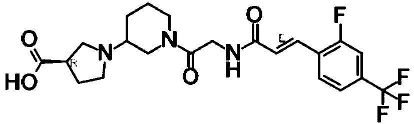 | MS(ESI) m/z: 472.2[M+ H]+ |
| 360 (I-360) | 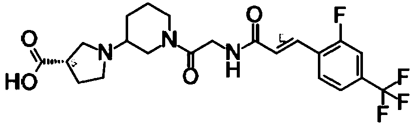 | MS(ESI) m/z: 472.2[M+ H]+ |
| 361 (I-361) | 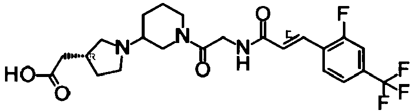 | MS(ESI) m/z: 486. 2[M+ H]+ |
| 362 (I-362) | 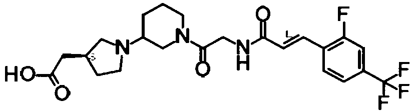 | MS(ESI) m/z: 486. 2[M+ H]+ |
| 363 (I-363) | 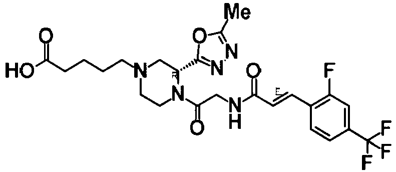 | MS(ESI) m/z: 540.2[M-H]- |
| 364 (I-364) | 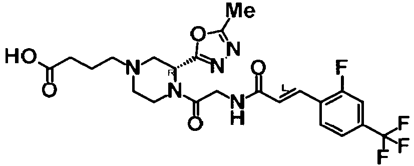 | MS(ESI) m/z: 526.2[M-H]- |

**[Table 42-7]**

| **Example No.** | **structural formula** | **instrument analysis data** |
|---|---|---|
| 365 (I-365) | 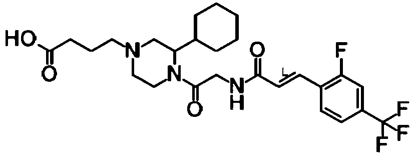 | MS(ESI) m/z: 528.3 [M+ H]+ |
| 366 (I-366) | 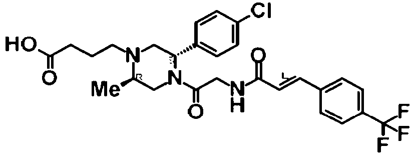 | MS(ESI) m/z: 550.2/55 2.2 [M-H]- |
| 3 6 7 (I-367) | 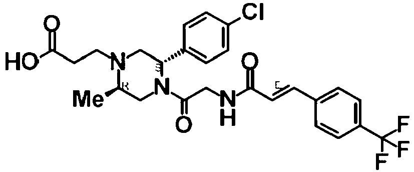 | MS(ESI) m/z: 536.2/53 8.2 [M-H]- |
| 368 (I-368) | 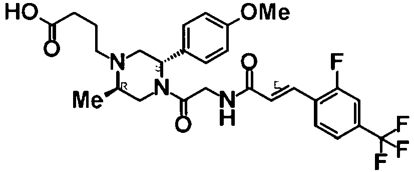 | MS(ESI) m/z:564.4[M-H] - |

**[Table 42-8]**

| **Example No.** | **structural formula** | **instrument analysis data** |
|---|---|---|
| 369 (I-369) | 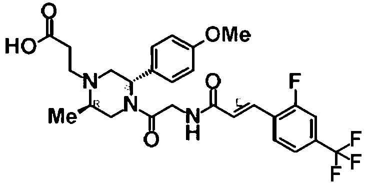 | MS(ESI) m/z: 552.4 [M-H]- |
| 370 (I-370) | 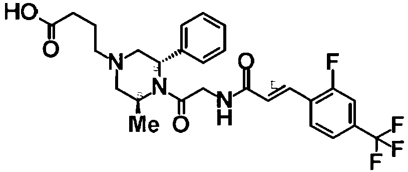 | MS(ESI) m/z: 536.2[M+ H]+ |
| 371 (I-371) | 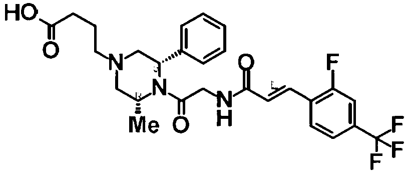 | MS (ESI) m/z: 536. 2[M+ H]+ |
| 372 (I-372) | 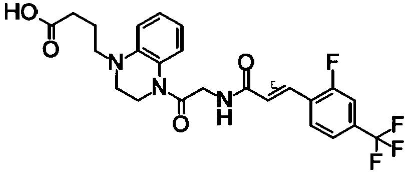 | MS(ESI) m/z: 492.2 [M+ H]+ |

### Examples 373 to 374:

The corresponding starting compounds were treated in the same manner as in Example 273 to obtain the compounds described in the following Table 43.

**[Table 43]**

| **Example No. (compound No.)** | **structural formula** | **instrument analysis data** |
|---|---|---|
| 373 (I-373) | 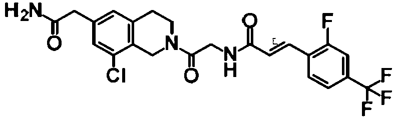 | MS(ESI) m/z: 498.1/5 00.1[M+H]+ |
| 374 (I-374) | 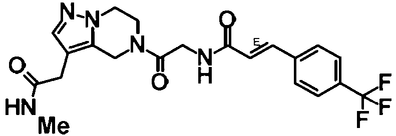 | MS(ESI) m/z: 450.2 [M+H]+ |

### Example 375:

### Production of tert-butyl 2-[2-[2-[[(E)-3-[2-fluoro-4-(trifluoromethyl)phenyl]prop-2-enoyl]amino]acetyl]-3-[2-(methylamino)-2-oxoethyl]-3,4-dihydro-1H-isoquinolin-6-yl]acetate (compound (I-375))

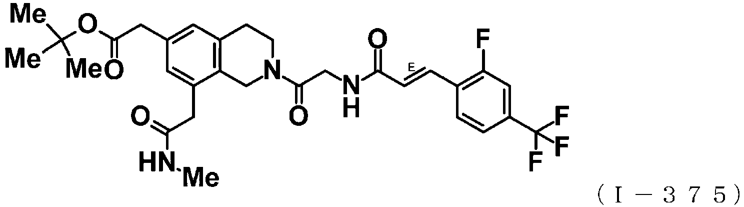

tert-Butyl 2-[2-[2-[[(E)-3-[2-fluoro-4-(trifluoromethyl)phenyl]prop-2-enoyl]amino]acetyl]-8-(methoxycarbonylmethyl)-3,4-dihydro-1H-isoquinolin-6-yl]acetate (40 mg) produced in Example 335 was added to 9.8 mol/l methylamine/methanol solution (5 ml), and the mixture was stirred at room temperature for 2 hr. The reaction mixture was evaporated under reduced pressure, and the obtained residue was purified by reversed-phase HPLC (10 mmol/1 aqueous ammonium carbonate solution/acetonitrile =50/50 - 20/80) to give the title compound (30 mg; yield 75%) as a colorless solid.
MS(ESI) m/z: 592.4[M+H]

### Examples 376 to 377:

The corresponding starting compounds were treated in the same manner as in Example 375 to obtain the compounds described in the following Table 44.

**[Table 44]**

| **Example No. (compound No.)** | **structural formula** | **instrument analysis data** |
|---|---|---|
| 376 (I-376) | 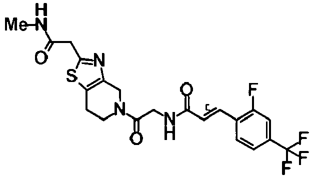 | MS (ESI) m/z: 485.1[M +H]+ |
| 377 (I-377) | 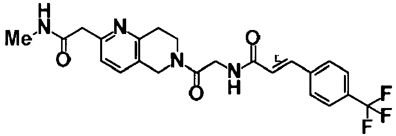 | MS(ESI) m/z: 461.2[M +H]+ |

### Example 378:

### Production of (E)-N-[2-[8-chloro-6-(1,2,3λ4,4-tetraazacyclopenta-2,4-dien-5-yl methyl) -3,4-dihydro-1H-isoquinolin-2-yl]-2-oxoethyl]-3-[2-fluoro-4-(trifluoromethyl)phenyl]prop-2-enamide (compound (I-378))

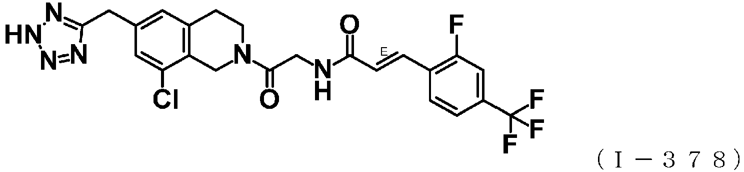

(1) (E)-N-[2-[6-(2-amino-2-oxoethyl)-8-chloro-3,4-dihydro-1H-isoquinolin-2-yl]-2-oxoethyl]-3-[2-fluoro-4-(trifluoromethyl)phenyl]prop-2-enamide (77 mg) produced in Example 373, 1-chloromethyl-4-fluoro-1,4-diazabicyclo[2.2.2]octane bis(tetrafluoroborate) (33 mg), and palladium acetate (10.5 mg) were added to acetonitrile (8 ml), and the mixture was stirred at room temperature overnight. To the reaction mixture was added chloroform, and the reaction mixture was evaporated under reduced pressure. The obtained residue was purified by reversed-phase HPLC (10 mmol/l aqueous ammonium carbonate solution/acetonitrile =60/40 - 30/70) to give (E)-N-[2-[8-chloro-6-(cyanomethyl)-3,4-dihydro-1H-isoquinolin-2-yl]-2-oxoethyl]-3-[2-fluoro-4-(trifluoromethyl)phenyl]prop-2-enamide (16 mg; yield 22%) as a pale yellow amorphous.
   MS(ESI) m/z: 480.1/482.1[M+H]⁺
(2) (E)-N-[2-[8-chloro-6-(cyanomethyl)-3,4-dihydro-1H-isoquinolin-2-yl]-2-oxoethyl]-3-[2-fluoro-4-(trifluoromethyl)phenyl]prop-2-enamide (14 mg) obtained in the aforementioned (1), azidotrimethylsilane (0.011 ml), and dibutyltin oxide (2.1 mg) were successively added to toluene (1 ml), and the mixture was stirred at an outer temperature of 100°C for 4 hr stirred. To the reaction solution were added azidotrimethylsilane (0.023 ml) and dibutyltin oxide (2 mg), and the mixture was stirred at an outer temperature of 100°C for 2 hr. To the reaction solution were further added azidotrimethylsilane (0.023 ml) and dibutyltin oxide (2 mg), and the mixture was stirred at an outer temperature of 100°C for 3 hr. The reaction mixture was evaporated under reduced pressure, methanol was added, and the solvent was evaporated under reduced pressure. The obtained residue was purified by reversed-phase HPLC (10 mmoL/l aqueous ammonium carbonate solution/acetonitrile =70/30 - 40/60) to give the title compound (10 mg; yield 66%) as a pale yellow amorphous.
   MS(ESI) m/z: 523.1/525.2[M+H]⁺

### Example 379:

### Production of (E)-3-[2-fluoro-4-(trifluoromethyl)phenyl]-N-[2-[(2S,5R)-4-[3-(1,2,3λ4,4-tetraazacyclopenta-2,4-dien-5-yl)propyl]-5-methyl-2-phenylpiperazin-1-yl]-2-oxoethyl]prop-2-enamide (compound (I-379))

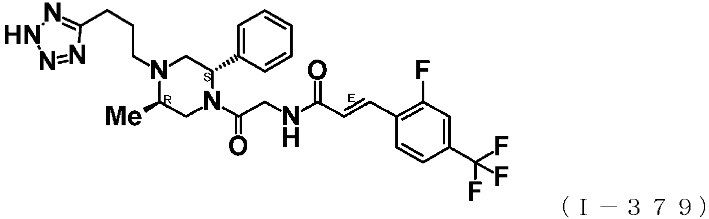

(1) (E)-N-[2-[(5R)-4-(3-cyanopropyl)-5-methyl-2-phenylpiperazin-1-yl]-2-oxoethyl]-3-[2-fluoro-4-(trifluoromethyl)phenyl]prop-2-enamide (77 mg) produced in Example 322, azidotrimethylsilane (0.045 ml), and dibutyl tin oxide (8 mg) were successively added to toluene (1 ml), and the mixture was stirred at an outer temperature of 100°C for 4 hr. To the reaction solution was added azidotrimethylsilane (0.045 ml), and the mixture was stirred at an outer temperature of 100°C for 1 hr. To the reaction mixture was added saturated aqueous ammonium chloride solution and the mixture was extracted with chloroform. The organic layers were combined and evaporated under reduced pressure, and the obtained residue was purified by reversed-phase HPLC (10 mmol/l aqueous ammonium carbonate solution/acetonitrile =70/30 - 40/60) to give the title compound (35 mg; yield 60%) as a pale yellow amorphous. MS(ESI) m/z: 560.3[M+H]⁺

### Example 380:

### Production of methyl 4-[(3S,5S)-4-[2-[[(E)-3-[2-fluoro-4-(trifluoromethyl)phenyl]prop-2-enoyl]amino]acetyl]-3-methyl-5-phenylpiperazin-1-yl]butanoate (compound (I-380))

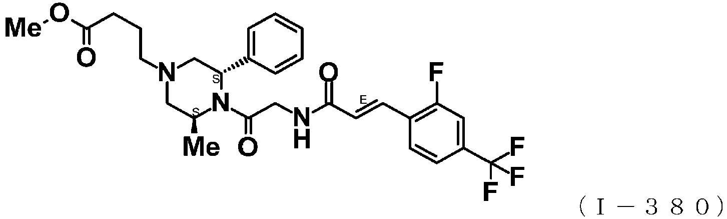

(1) DMF (0.08 ml) was added to a solution of N-benzyloxycarbonylglycine (1.08 g) in THF. To the reaction mixture was gradually added oxalyl chloride (0.48 ml), and the mixture was stirred at room temperature for 15 min to prepare an acid chloride solution. To a solution of (3S,5S)-3-methyl-1-(4-nitrophenyl)sulfonyl-5-phenylpiperazine (200 mg) produced in Reference Example 114 in THF were added pyridine (0.2 ml) and the prepared acid chloride solution (1.27 ml) mentioned above, and the mixture was stirred under nitrogen atmosphere at room temperature for 1 hr. To the reaction mixture were added ethyl acetate and water, and the organic layer was washed successively with 1N hydrochloric acid and saturated aqueous sodium hydrogen carbonate solution, and concentrated under reduced pressure. The residue was purified by column chromatography (solvent: n-hexane/ethyl acetate =60/40 - 40/60 - 30/70) to give benzyl N-[2-[(2S,6S)-2-methyl-4-(4-nitrophenyl)sulfonyl-6-phenylpiperazin-1-yl]-2-oxoethyl]carbamate (299 mg; yield 92%) as a colorless amorphous. MS(ESI) m/z: 553.3[M+H]⁺
(2) To a solution of benzyl N-[2-[(2S,6S)-2-methyl-4-(4-nitrophenyl)sulfonyl -6-phenylpiperazin-1-yl]-2-oxoethyl]carbamate (299 mg) obtained in the aforementioned (1) in methanol (3 ml) were added potassium carbonate (383 mg) and thioglycolic acid (0.094 ml), and the mixture was stirred at 60°C for 30 min. The reaction mixture was allowed to cool and concentrated under reduced pressure. To the residue were added ethyl acetate and water, and the mixture was partitioned. The aqueous layer was extracted again with chloroform. The organic layers were combined, dried over anhydrous sodium sulfate, and concentrated under reduced pressure. To the residue was added DMF (0.62 ml), and potassium carbonate (95 mg), sodium iodide (86 mg) and 4-bromobutyric acid methyl (97 µL) were added thereto, and the mixture was stirred at 60°C for 2 hr. The reaction mixture was cooled to room temperature, ethyl acetate and brine were added thereto, and the mixture was partitioned. The organic layer was concentrated under reduced pressure. The residue was purified by silica gel chromatography (solvent: n-hexane/ethyl acetate =55/45 - 30/70) to give methyl 4-[(3S,5S)-3-methyl-5-phenyl-4-[2-(benzyloxyamino)acetyl]piperazin-1-yl]butanoate (170.6 mg; yield 60%) as a colorless viscous oil. MS(ESI) m/z: 468.3[M+H]⁺
(3) To a solution of methyl 4-[(3S,5S)-3-methyl-5-phenyl-4-[2-(benzyloxyamino)acetyl]piperazin-1-yl]butanoate (170 mg) obtained in the aforementioned (2) in methanol (2 ml) was added palladium-carbon (23.8 mg), and the mixture was stirred under hydrogen atmosphere at room temperature for 1 hr. To the reaction mixture was added palladium-carbon (23.8 mg) again, and the mixture was stirred under hydrogen atmosphere at room temperature for 1 hr. The reaction mixture was filtered through celite, and the filtrate was concentrated. To the residue were added 2-[[(E)-3-[2-fluoro-4-(trifluoromethyl)phenyl]prop-2-enoyl]amino]acetic acid (78 mg), HOBt (52 mg) and WSC hydrochloride (75 mg), and the mixture was stirred at room temperature for 1 hr. To the reaction mixture was added brine, and the mixture was partitioned. The organic layer was washed with brine, and concentrated under reduced pressure. The residue was purified twice by silica gel chromatography (first time: acidic silica, solvent: n-hexane/ethyl acetate =50/50 - 30/70 - 0/100, second time: NH silica, solvent: n-hexane/ethyl acetate =60/40 - 40/60) to give the title compound (135.5 mg; yield 81%) as a pale yellow amorphous.
   MS(ESI) m/z: 536.2[M+H]⁺

### Example 381:

The corresponding starting compounds were treated in the same manner as in Example 380 to obtain the compound described in the following Table 45.

**[Table 45]**

| **Example No. (compound No.)** | **structural formula** | **instrument analysis data** |
|---|---|---|
| 381 (I-381) | 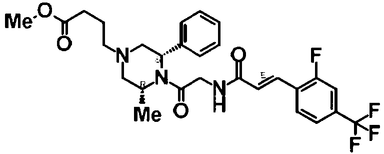 | MS(ESI) m/z: 550.3[M +H]+ |

### Example 382:

### Production of 3-[(2R,5S)-5-(4-cyclopropylphenyl)-4-[2-[[(E)-3-[2-fluoro-4-(trifluoromethyl)phenyl]prop-2-enoyl]amino]acetyl]-2-methylpiperazin-1-yl]propanoic acid (compound (I-382))

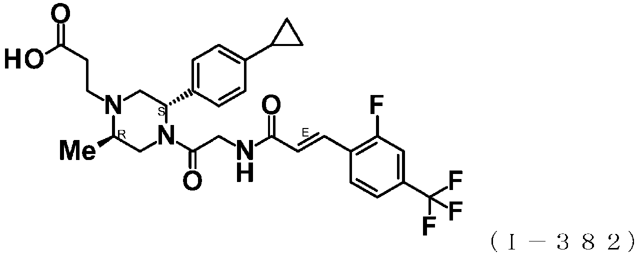

To a solution of 4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid (40 mg) produced in Example 339 in 1,4-dioxane (0.36 ml) were added chloro(2-dicyclohexylphosphino-2',6'-dimethoxy-1,1'-biphenyl) [2-(2'-amino-1,1'-biphenyl)]palladium(II) (2.6 mg), tripotassium phosphate (46 mg), water (0.145 ml) and cyclopropylboronic acid (12.4 mg), and the mixture was stirred under nitrogen atmosphere at 120°C for 2 hr. To the reaction mixture were added chloro(2-dicyclohexylphosphino-2',6'-dimethoxy-1,1'-biphenyl)[2-(2'-amino-1,1'-biphenyl)]palladium(II) (2.6 mg), tripotassium phosphate (46 mg), and cyclopropylboronic acid (12.4 mg) and the mixture was stirred at 125°C for 6 hr. Furthermore, chloro(2-dicyclohexylphosphino-2',6'-dimethoxy-1,1'-biphenyl)[2-(2'-amino-1,1'-biphenyl)]palladium(II) (2.6 mg), tripotassium phosphate (46 mg), and cyclopropylboronic acid (12.4 mg) were added thereto, and the mixture was stirred at 125°C for 6 hr. The reaction mixture was cooled to room temperature, water and chloroform were added, and the mixture was filtered through celite. The filtrate was adjusted to pH 7 with 0.5 mol/l hydrochloric acid, and extracted with chloroform. The organic layer was washed with saturated brine, dried over sodium sulfate, and concentrated under reduced pressure. The residue was purified by column chromatography (solvent :chloroform/methanol=99/1 - 90/10) to give the title compound (14.5 mg; yield 36%) as a colorless amorphous.
MS(ESI) m/z: 560.3[M-H]⁻

### Examples 383 to 385:

The corresponding starting compounds were treated in the same manner as in Example 382 to obtain the compounds described in the following Table 46.

**[Table 46]**

| **Example No. (compound No.)** | **structural formula** | **instrument analysis data** |
|---|---|---|
| 383 (I-383) | 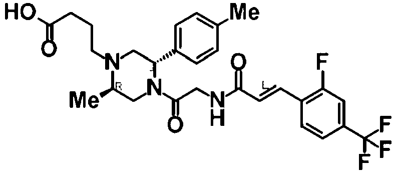 | MS(ESI) m/z: 548.3 [ M-H]- |
| 384 (I-384) | 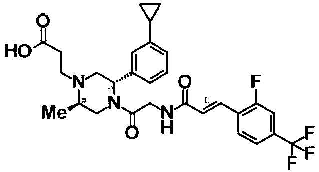 | MS (ESI) m/z: 560.3 [ M-H]- |
| 385 (I-385) | 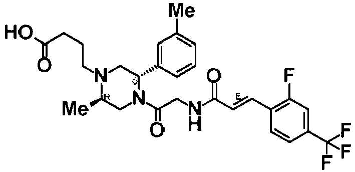 | MS(ESI) m/z: 548.3 [ M-H]- |

### Examples 386 to 390:

The corresponding starting compounds were treated in the same manner as in Example 1 to obtain the compounds described in the following Table 47.

**[Table 47]**

| **Example No. (compound No.)** | **structural formula** | **instrument analysis data** |
|---|---|---|
| 386 (I-386) | 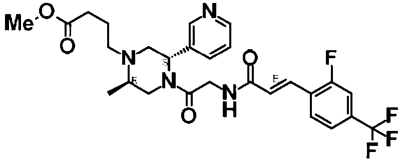 | MS(ESI) m/z: 551.4 [ M+H] + |
| 387 (I-387) | 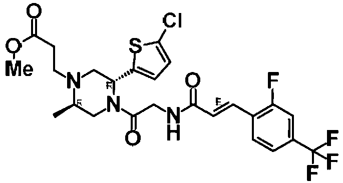 | MS(ESI) m/z: 576.3/5 78.3 [M+H]+ |
| 388 (I-388) | 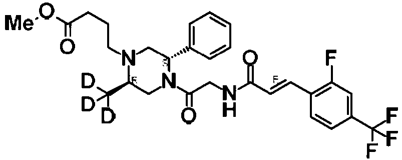 | MS(ESI) m/z: 553.1 [ M+H]+ |
| 389 (I- 389) | 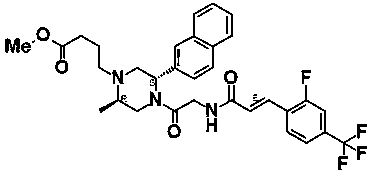 | MS (ESI) m/z: 600.3[M +H] + |
| 390 (I- 390) | 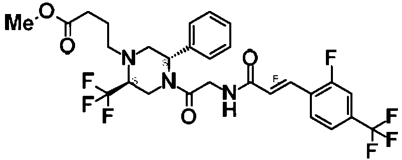 | MS(ESI) m/z: 604.3 [ M+H]+ |

### Examples 391 to 392:

The corresponding starting compounds were treated in the same manner as in Example 83 to obtain the compounds described in the following Table 48.

**[Table 48]**

| **Example No. (compound No.)** | **structural formula** | **instrument analysis data** |
|---|---|---|
| 391 (I-391) | 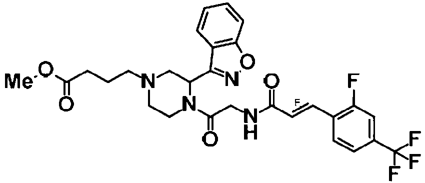 | MS(ESI) m/z: 577.3 [M+H]+ |
| 392 (I- 392) | 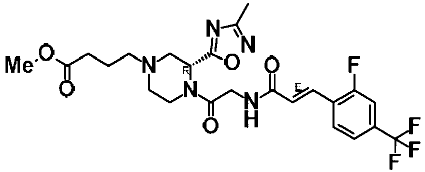 | MS(ESI) m/z: 542.3 [ M+H] + |

### Examples 393 to 394:

The corresponding starting compounds were treated in the same manner as in Example 104 to obtain the compounds described in the following Table 49.

**[Table 49]**

| **Example No. (compound No.)** | **structural formula** | **instrument analysis data** |
|---|---|---|
| 393 (I-393) | 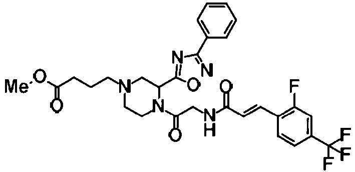 | MS(ESI) m/z: 604.3 [M+H]+ |
| 394 (I-394) | 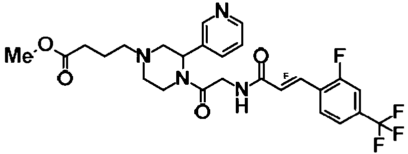 | MS (ESI) m/z: 537. 3 [M+H]+ |

### Examples 395 to 403:

The corresponding starting compounds were treated in the same manner as in Example 200 to obtain the compounds described in the following Table 50-1 to Table 50-2.

**[Table 50-1]**

| **Example No.** (compound **No.)** | **structural formula** | **instrument** analysis data |
|---|---|---|
| 395 (I-395) | 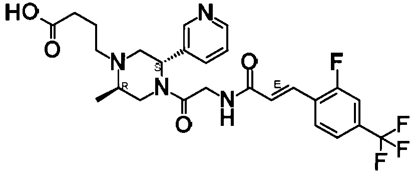 | MS(ESI) m/z: 537.3 [M+H]+ |
| 396 (I-396) | 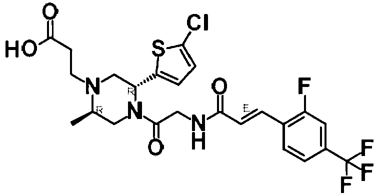 | MS(ESI) m/z: 562.2/5 64. 2 [M+H]+ |
| 397 (I-397) | 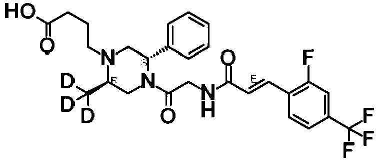 | MS(ESI) m/z: 539.4 [M-H]- |
| 398 (I-398) | 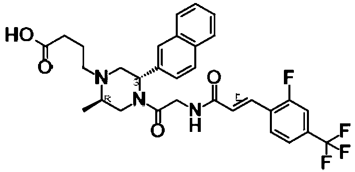 | MS(ESI) m/z: 584.3 [M-H]- |
| 399 (I-399) | 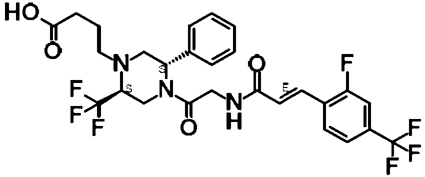 | MS(ESI) m/z: 590. 3 [M+H]+ |

**[Table 50-2]**

| **Example No. (compound No.)** | **structural formula** | **instrument analysis data** |
|---|---|---|
| 400 (I-400) | 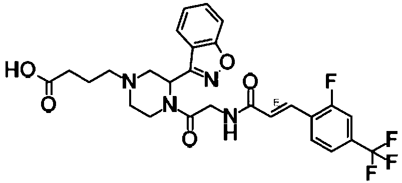 | MS(ESI) m/z: 561.3 [M-H]- |
| 401 (I-401) | 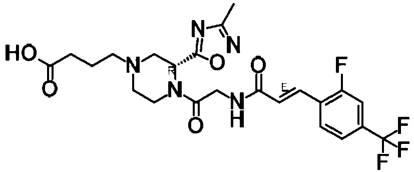 | MS(ESI) m/z: 528.3 [M+H]+ |
| 402 (I-402) | 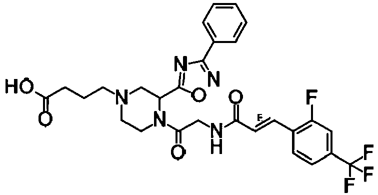 | MS (ESI) m/z: 590.2 [M+H]+ |
| 403 (I-403) | 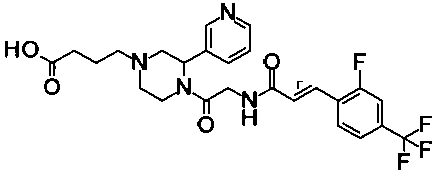 | MS(ESI) m/z: 523. 2 [M+H]+ |

### Experimental Example 1:

hB0AT1 inhibitory test

### <Experimental method>

A test compound dissolved in dimethyl sulfoxide (DMSO) was added to a 96 well plate for solid phase radioactivity measurement containing a scintillator at the bottom of the well (final concentration of DMSO 0.5%). DMSO alone as a control and DMSO containing N-(4-bromophenyl)-3,5-dichloro-2-hydroxybenzamide (final concentration 10 µM) for the measurement of B0AT1 non-specific uptake were similarly added at 0.5 µL per well. Human B0AT1 stable expression CHO cells suspended in a buffer (buffer containing 96 mM sodium chloride, 2 mM potassium chloride, 1.8 mM calcium chloride, 1 mM magnesium chloride, 0.01% bovine serum albumin, and 10 mM 2-[4-(2-hydroxyethyl)-1-piperazinyl]ethanesulfonic acid) were added by 90 µL to 75000 cells/well. After standing at ordinary temperature for 30 min or longer, the cells were subjected to a phenylalanine uptake experiment. A buffer containing L-phenylalanine and [3,4,5-3H]-L-phenylalanine was added by 10 µL per well (final concentration of phenylalanine 0.25 mM), and the radioactivity of the plate bottom surface at ordinary temperature was measured with a scintillation counter over time, and the radioactivity value after 100 to 200 min was analyzed. A value obtained by subtracting the B0AT1 non-specific uptake from the control uptake was taken as 100%, and the concentration necessary for each test compound to achieve 50% inhibition (IC₅₀ value) was determined by nonlinear regression using a logistic model.

The results are shown in the following Table 51-1 to Table 51-8.

**[Table 51-1]**

| **compound No .** | hB0AT1 IC₅₀ [nM] |
|---|---|
| I-4 | 50 |
| I-11 | 81 |
| 1-12 | 89 |
| I-13 | 123 |
| I-26 | 332 |
| I-40 | 996 |
| 1-42 | 225 |
| 1-43 | 709 |
| I-49 | 134 |
| I-61 | 1673 |
| 1-62 | 463 |
| I-118 | 42 |
| I-140 | 436 |
| I-141 | 34 |
| I-142 | 241 |
| I-143 | 151 |
| I-144 | 65 |
| I-145 | 86 |
| I-146 | 67 |
| I-147 | 100 |
| I-148 | 207 |
| I-149 | 132 |
| I-150 | 182 |
| I-151 | 57 |
| I-152 | 96 |
| I-153 | 146 |
| I-154 | 95 |
| I-155 | 84 |
| I-156 | 251 |
| I-157 | 102 |
| I-158 | 83 |

**[Table 51-2]**

| **compound No.** | hB0AT1 IC₅₀[nM] |
|---|---|
| I-159 | 171 |
| I-160 | 156 |
| I-161 | 259 |
| I-162 | 141 |
| I-163 | 223 |
| I-164 | 386 |
| I-165 | 60 |
| I-166 | 192 |
| I-167 | 106 |
| I-168 | 447 |
| I-169 | 381 |
| I-170 | 240 |
| I-171 | 91 |
| I-172 | 155 |
| I-173 | 477 |
| I-174 | 149 |
| I-175 | 111 |
| I-176 | 243 |
| I-177 | 171 |
| I-178 | 92 |
| I-179 | 872 |
| I-180 | 122 |
| I-181 | 138 |
| I-182 | 85 |
| I-183 | 46 |
| I-184 | 127 |
| I-185 | 168 |
| I-186 | 76 |
| I-187 | 59 |
| I-188 | 115 |
| I-189 | 424 |

**[Table 51-3]**

| **compound No.** | hB0AT1 IC₅₀[nM] |
|---|---|
| I-190 | 102 |
| I-191 | 104 |
| I-192 | 397 |
| I-193 | 459 |
| I-191 | 74 |
| I-195 | 186 |
| I-196 | 151 |
| I-197 | 2397 |
| I-199 | 370 |
| I-200 | 192 |
| I-201 | 54 |
| I-202 | 119 |
| I-203 | 56 |
| I-201 | 246 |
| I-205 | 99 |
| I-206 | 279 |
| I-207 | 579 |
| I-208 | 224 |
| I-209 | 70 |
| I-210 | 59 |
| I-211 | 263 |
| I-212 | 196 |
| I-213 | 156 |
| I-211 | 91 |
| I-215 | 165 |
| I-216 | 358 |
| I-217 | 202 |
| I-218 | 71 |
| I-219 | 256 |
| I-220 | 100 |

**[Table 51-4]**

| **compound No.** | hB0AT1 TC₅₀[nM] |
|---|---|
| I-221 | 71 |
| I-222 | 107 |
| I-223 | 86 |
| I-224 | 110 |
| I-225 | 295 |
| I-226 | 101 |
| I-227 | 667 |
| I-228 | 462 |
| I-229 | 152 |
| I-230 | 124 |
| I-231 | 234 |
| I-232 | 30 |
| I-233 | 609 |
| I-234 | 50 |
| I-235 | 289 |
| I-236 | 94 |
| I-237 | 199 |
| I-238 | 97 |
| I-239 | 302 |
| I-240 | 197 |
| I-241 | 161 |
| I-242 | 158 |
| I-243 | 3703 |
| I-244 | 613 |
| I-245 | 45 |
| I-246 | 44 |
| I-247 | 2479 |
| I-248 | 147 |
| I-249 | 96 |
| I-250 | 506 |
| I-251 | 366 |

**[Table 51-5]**

| **compound No.** | hB0AT1 IC₅₀[nM] |
|---|---|
| I-251 | 97 |
| I-255 | 766 |
| I-256 | 18 |
| I-257 | 108 |
| I-258 | 1327 |
| I-259 | 140 |
| I-260 | 638 |
| I-261 | 30 |
| I-262 | 125 |
| I-263 | 18 |
| I-273 | 22 |
| I-274 | 34 |
| I-275 | 78 |
| I-276 | 17 |
| I-277 | 30 |
| I-278 | 40 |
| I-279 | 21 |
| I-280 | 19 |
| I-281 | 7 |
| I-282 | 700 |
| I-283 | 256 |
| I-281 | 25 |
| I-286 | 303 |
| I-287 | 178 |
| I-288 | 152 |
| I-289 | 87 |
| I-290 | 119 |
| I-292 | 1203 |
| I-293 | 39 |
| I-291 | 266 |
| I-295 | 51 |
| I-296 | 747 |
| I-297 | 37 |

**[Table 51-6]**

| **compound No.** | hB0AT1 IC50[nM] |
|---|---|
| I-320 | 352 |
| I-336 | 661 |
| I-337 | 31 |
| I-338 | 54 |
| I-339 | 33 |
| I-340 | 62 |
| I-341 | 1682 |
| I-342 | 338 |
| I-343 | 91 |
| I-341 | 122 |
| I-345 | 1776 |
| I-346 | 1043 |
| I-347 | 2687 |
| I-348 | 45 |
| I-349 | 93 |
| I-350 | 184 |
| I-351 | 75 |
| I-352 | 3262 |
| I-353 | 400 |
| I-354 | 74 |
| I-355 | 42 |
| I-356 | 97 |
| I-357 | 107 |
| I-358 | 223 |
| I-359 | 1111 |
| I-360 | 2862 |
| I-361 | 879 |
| I-362 | 1405 |
| I-363 | 1074 |
| I-364 | 1141 |
| I-365 | 69 |
| I-366 | 43 |

**[Table 51-7]**

| **compound No.** | hB0AT1 IC₅₀[nM] |
|---|---|
| I-367 | 36 |
| I-368 | 145 |
| I-369 | 164 |
| I-370 | 143 |
| I-371 | 3384 |
| I-372 | 2005 |
| I-374 | 54 |
| I-376 | 77 |
| I-377 | 48 |
| I-378 | 232 |
| I-379 | 2524 |
| I-382 | 63 |
| I-383 | 64 |
| I-384 | 31 |
| I-385 | 70 |

**[Table 51-8]**

| **compound No.** | hB0AT1 IC₅₀[nM] |
|---|---|
| I-395 | 636 |
| I-396 | 71 |
| I-397 | 75 |
| I-398 | 42 |
| I-399 | 2886 |
| I-400 | 292 |
| I-401 | 2897 |
| I-402 | 235 |
| I-403 | 1721 |

### Experimental Example 2:

### Evaluation of hERG (human Ether-a-go-go Related Gene) channel inhibitory activity

### <Experiment method>

The hERG channel inhibitory activity of the compound of the present invention was measured using CHO cells (B' SYS GmbH) forcibly expressing hERG channels involved in human rapidly active delayed rectifier potassium current (IKr) by the whole-cell patch clamp method using an auto-patch clamp system (SyncroPatch384PE).

### (Solution preparation)

The extracellular and intracellular solutions used for the measurement were prepared as follows.
Extracellular fluid: 140 mM NaCl, 4 mM KCl, 1 mM MgCl₂, 2 mM CaCl₂, 10 mM HEPES, 5 mM D-Glucose (adjusted to pH 7.4 with NaOH), MilliQ water, sterilized filter (0.1 µm)
Intracellular solution: 120 mM KF, 20 mM KCl, 10 mM EGTA, 10 mM HEPES (adjusted to pH 7.2 with KOH), MilliQ water, filter sterilization (0.1 µm)
Test substance solution: The 10 mM DMSO solution was stepwise diluted with the outside solution/DMSO-containing outside solution to prepare the test compound solutions at each concentration required for IC₅₀ calculation (final DMSO concentration 0.3-0.4%), and applied at room temperature.
(Current value measurement and data analysis)

Cell suspension, extracellular fluid, intracellular fluid, and measurement plate (NPC-384 plate) were placed in the auto-patch clamp system, and hERG current measurements were performed using the whole-cell patch clamp method. The voltage protocol was set to a holding potential of -80 mV, with a depolarizing pulse preceded by -90 mV (100 ms) and followed by -80 mV (100 ms).

The depolarizing pulse was +40 ms (500 ms), and then the hERG peak current value obtained during the ramp phase was measured when the voltage was lowered to -80 mV over 100 ms. The protocol was applied at 5-second intervals for 5 minutes for each concentration.

SyncroPatch software (manufactured by Nanion) was used for data analysis. Four concentrations were applied incrementally for each test substance, and the average hERG Peak current value obtained from the last three stimuli of each applied concentration was used as the evaluation data. The IC₅₀ value was calculated from the inhibition rate of the current at each concentration of each test substance relative to the preapplication value using Hill's equation (Hill equation) using the relevant software.

The same experiments were also performed using the example compounds described in WO 2023/145804 as comparative example compounds 1-4.

The results are shown in the following Table 52-1 to Table 52-4.

**[Table 52-1]**

| | **structural formula** | **bERG inhibition IC₅₀ [µm]** |
|---|---|---|
| **Comparative Example compound 1** | 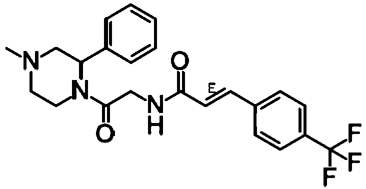 | 2.09 |
| **Comparative Example compound 2** | 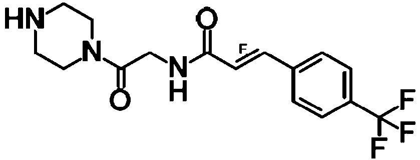 | 8.72 |
| **Comparative Example compound 3** | 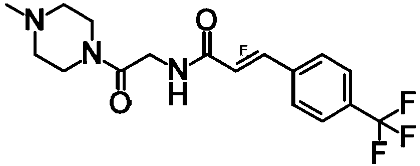 | 3.63 |
| **Comparative Example compound 4** | 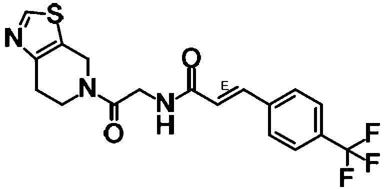 | 5.96 |

**[Table 52-2]**

| **compound No.** | hERG IC₅₀[µM] |
|---|---|
| I-141 | >30 |
| I-143 | >30 |
| I-145 | >30 |
| I-146 | 29. 89 |
| I-147 | >30 |
| I-148 | >30 |
| I-149 | >30 |
| I-150 | 24.91 |
| I-151 | >30 |
| I-153 | >30 |
| I-154 | 22. 09 |
| I-157 | >30 |
| I-158 | >30 |
| I-159 | 28.91 |
| I-162 | >30 |
| I-166 | >30 |
| I-171 | >30 |
| I-172 | >30 |
| I-173 | >30 |
| I-174 | >30 |
| I-176 | >30 |
| I-178 | >30 |
| I-180 | >30 |
| I-182 | >30 |
| I-183 | >30 |
| I-184 | >30 |
| I-185 | >30 |
| I-186 | >30 |
| I-187 | >30 |
| I-190 | >30 |
| I-191 | >30 |
| I-194 | >30 |
| I-195 | >30 |
| I-196 | >30 |
| I-197 | >30 |
| I-198 | >30 |
| I-199 | >30 |
| I-201 | >30 |

**[Table 52-3]**

| **compound No.** | hERG IC₅₀[µM] |
|---|---|
| I-202 | >30 |
| I-204 | >30 |
| I-205 | >30 |
| I-209 | >30 |
| I-210 | >30 |
| I-214 | >30 |
| I-215 | >30 |
| I-223 | 28.46 |
| I-224 | >30 |
| I-225 | 26. 21 |
| I-226 | >30 |
| I-231 | >30 |
| I-232 | 19. 28 |
| I-233 | >30 |
| I-234 | >30 |
| I-235 | >30 |
| I-236 | >30 |
| I-237 | >30 |
| I-238 | >30 |
| I-239 | >30 |
| I-240 | >30 |
| I-241 | >30 |
| I-242 | >30 |
| I-243 | >30 |
| I-244 | >30 |
| I-248 | >30 |
| I-250 | >30 |
| I-252 | >30 |
| I-253 | 26. 23 |
| I-255 | >30 |
| I-257 | 29. 56 |
| I-261 | >30 |
| I-267 | >30 |
| I-271 | 16. 39 |
| I-286 | >30 |
| I-287 | >30 |
| I-288 | >30 |
| I-289 | >30 |
| I-290 | >30 |

**[Table 52-4]**

| **compound No.** | hERG IC₅₀[µM] |
|---|---|
| I-337 | 15. 47 |
| I-338 | >30 |
| I-339 | >30 |
| I-340 | >30 |
| I-341 | >30 |
| I-342 | >30 |
| I-343 | >30 |
| I-348 | >30 |
| I-349 | >30 |
| I-350 | >30 |
| I-351 | >30 |
| I-353 | >30 |
| I-354 | 22. 02 |
| I-355 | >30 |
| I-356 | >30 |
| I-363 | >30 |
| I-364 | >30 |
| I-367 | 23. 89 |
| I-368 | >30 |
| I-369 | >30 |
| I-383 | 15. 25 |
| I-384 | 15. 82 |
| I-385 | 20. 96 |
| I-395 | >30 |
| I-396 | >30 |
| I-397 | >30 |
| I-398 | 25.63 |
| I-401 | >30 |

The efficacy of the B0AT1 inhibitor found in the present invention in vivo can be measured, for example, as follows.

### Experimental Example 3:

### Mouse urinary phenylalanine excretion evaluation test

### (1) Preparation of compound and phenylalanine administration solution

The test compound is weighed, and 200 µL of 0.5% carboxymethylcellulose sodium salt (Sigma-Aldrich), a saline solution (for intraperitoneal administration), or water (for oral administration) is added. Using a mixer mill (Retsch), the mixture is pulverized, suspended, and adjusted to the optimum concentration. Thereafter, sonication is performed for 10 min using an ultrasonic disintegrator (Nihon seiki). An administration solution is prepared on the day of test compound administration. L-phenylalanine (Sigma-Aldrich) is dissolved in saline (Otsuka Fresh Food Injection, Otsuka Pharmaceutical Factory, Inc.) by sonication and adjusted to 25 mg/ml. An administration solution is prepared on the day of phenylalanine administration.

### (2) Administration of compound and collection of urine sample

Male C57BL/6J (Charles River Japan, Inc.) weighing 20 g-30 g (8-11 weeks old) are used as test animals. Until 2 days prior to the test, they are group-reared in PC cages. Two days prior to the test, the general conditions of the test animals are confirmed to be normal, and the body weight is measured. The test animals are kept singly in stainless steel metabolic cages for mouse (Shinano Seisakusho), and fed ad libitum with tap water and feed. In the morning of the day of test compound administration, the body weight of the animals is measured, and assigned to groups using the grouping program GPR3 ver.3.0.2 so that the body weight on the test day and the body weight change from 2 days before the test are homogeneous among the groups by a simulation method. The test compounds are administered intraperitoneally or orally, and the test animals are housed in clean metabolic cages. Thirty minutes after administration of the test compound, phenylalanine is intraperitoneally administered at 500 mg/20 ml/kg. Urine is collected 4 hr, 6 hr, or 24 hr after administration of the test compound, and the urine weight is measured. When urine is collected 4 hr and 6 hr after administration, the mouse is restrained and the urine in the bladder is urinated. Contaminants and urine are separated by centrifugation, and the supernatant is collected. Using urinalysis paper Lifestix (Siemens Healthcare Diagnostics), glucose, occult blood, and leukocyte are measured. The collected urine is stored at -20°C or below and used for phenylalanine concentration measurement.

### (3) Measurement of urinary phenylalanine by LC-MS/MS

To a urine sample (10 µL) is added 190 µL of IS solution (d5-phenylalanine dissolved in acetonitrile/methanol=7/3 at a concentration of 1 µg/mL) and, after mixing, centrifuged supernatant (3 µL) is analyzed by LC-MS/MS. As a calibration standard, 10 µl of a standard solution containing an IS solution and 180 µl of an IS solution are added to 10 µl of physiological saline, mixed, and centrifuged. The supernatant (3 µl) is analyzed by LC-MS/MS under the following conditions.

### <measurement conditions>

MS: Quattro Premier XE
LC: ACQUITY UPLC
column: ACQUITY UPLC HSS C18 Column, 100Å, 1.8 µm, 2.1 mm × 100 mm
mobile phase A: 0.025% aqueous heptafluorobutyric acid solution mobile phase B: acetonitrile
gradient (B%): 2-2-98-98-2 (0.00-0.50-3.50-4.25-5.00 min) column temperature: 50°C
flow rate: 0.40 mL/min

As shown in Table 51-1 to Table 51-8, it was confirmed that compound (I) of the present invention or a salt thereof has superior inhibitory activity against B0AT1.

Also, as shown in Table 52-1 to Table 52-4, all of the compound (I) of the present invention or a salt thereof showed reduced hERG inhibitory activity as compared with Comparative Example compounds 1 to 4, and were confirmed to have superior safety.

From the above results, it is expected that compound (I) of the present invention or a salt thereof exhibits prophylactic and/or therapeutic effects on the symptoms of amino acid metabolism disorders such as phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, isovaleric acidemia, and the like.

### [Industrial Applicability]

The compound (I) of the present invention or a pharmaceutically acceptable salt thereof has a superior inhibitory activity against B0AT1 and also has superior safety (that is, toxicity is low). Therefore, a pharmaceutical composition containing the compound can be used for the treatment and/or prevention of diseases whose symptoms can be relieved by a B0AT1 inhibitory action, specifically, for example, amino acid metabolism disorders such as phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, isovaleric acidemia, and the like. Since these amino acid metabolism disorders are designated intractable diseases that require long-term medical treatments including a very strict dietary therapy (amino acid-restricted diet) for life, the compound (I) of the present invention or a pharmaceutically acceptable salt thereof can provide a safe and effective prophylactic and/or therapeutic drug.

This application is based on a patent application No. 2023-062369 filed in Japan (filing date: April 6, 2023) and a patent application No. 2023-123729 filed in Japan (filing date: July 28, 2023), the contents of which are incorporated in full herein.

## Claims

1. A compound represented by the formula (I): 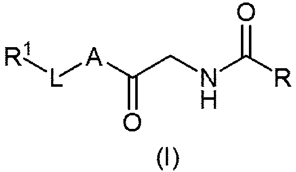 wherein
R is an optionally substituted C₆₋₁₀ aryl-ethenyl group;
R¹ is a carboxy group, or a group biologically equivalent to a carboxy group;
L is a divalent organic group comprising a group selected from the group consisting of a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, each of which is optionally further substituted; and
A is a divalent nitrogen-containing heterocyclic group optionally further substituted,
or a pharmaceutically acceptable salt thereof.

2. The compound according to claim 1 or a pharmaceutically acceptable salt thereof, wherein, in the formula (I),
R is a 2-phenylethenyl group optionally substituted by 1 to 3 substituents selected from the group consisting of a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, and a pentafluorosulfanyl group;
R¹ is a carboxy group, or a group biologically equivalent to a carboxy group;
L is a C₁₋₆ alkylene group, a C₆₋₁₀ arylene group, a C₃₋₁₄ cycloalkylene group, a C₂₋₆ alkenylene group, a C₂₋₆ alkynylene group, or a 3- to 14-membered divalent non-aromatic heterocyclic group, each of which optionally has, in the inside or at the end, one or more divalent groups selected from the group consisting of -NR²- (wherein R² is a hydrogen atom or a C₁₋₆ alkyl group), -O-, -S-, -C(=O)-, a C₁₋₆ alkylene group, a C₆₋₁₀ arylene group, a C₃₋₁₄ cycloalkylene group, a C₂₋₆ alkenylene group, a C₂₋₆ alkynylene group, and a 3- to 14-membered divalent non-aromatic heterocyclic group, and each of which is optionally further substituted by 1 to 3 substituents selected from substituent group a; and
A is a divalent nitrogen-containing non-aromatic heterocyclic group represented by the following formula: 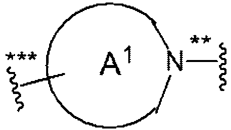
wherein ** is a bonding site with a carbonyl group, *** is a bonding site with group L, and ring A¹ is a 3- to 10-membered monocycle, a 8- to 14-membered fused cycle, a 6- to 16-membered spiro cycle, or a 6- to 14-membered bridged cycle, and optionally has, as a ring-constituting atom besides a nitrogen atom and a carbon atom, hetero atom(s) selected from the group consisting of a nitrogen atom, an oxygen atom, and a sulfur atom, which is optionally further substituted by 1 to 3 substituents selected from substituent group a
(Substituent group a):
a halogen atom;
a hydroxy group;
a cyano group;
an oxo group;
a C₁₋₆ alkyl group optionally substituted by substituent(s) selected from the group consisting of a halogen atom, a hydroxy group, a cyano group, a C₁₋₆ alkoxy group, a C₆₋₁₄ aryl group, a carboxy group, a C₁₋₆ alkoxy-carbonyl group, a carbamoyl group optionally substituted by 1 or 2 C₁₋₆ alkyl groups, and a C₆₋₁₄ aryloxy group;
a C₁₋₆ alkoxy group optionally substituted by halogen atom(s);
a C₁₋₆ alkylsulfonyl group optionally substituted by halogen atom(s);
a C₁₋₆ alkyl-carbonyl group;
a C₁₋₆ alkoxy-carbonyl group;
a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
a di-C₁₋₆ alkylamino group;
a tri-substituted silyl group;
a tri-substituted silyl oxy group;
a C₆₋₁₄ hydrocarbocyclic group optionally substituted by 1 to 3 substituents selected from substituent group b;
a 5- to 14-membered aromatic heterocyclic group optionally substituted by 1 to 3 substituents selected from substituent group b; and
a 3- to 14-membered non-aromatic heterocyclic group optionally substituted by 1 to 3 substituents selected from substituent group b
(Substituent group b):
a halogen atom;
a hydroxy group;
a cyano group;
an oxo group;
a carboxy group;
a C₁₋₆ alkyl group optionally substituted by substituent(s) selected from the group consisting of a halogen atom, a hydroxy group, a cyano group, and a C₁₋₆ alkoxy group;
a C₃₋₈ cycloalkyl group optionally substituted by substituent(s) selected from the group consisting of a halogen atom, a hydroxy group, a cyano group, and a C₁₋₆ alkoxy group;
a C₆₋₁₀ aryl group optionally substituted by substituent(s) selected from the group consisting of a halogen atom, a hydroxy group, a cyano group, a C₁₋₆ alkyl group, and a C₁₋₆ alkoxy group;
a 5- to 14-membered aromatic heterocyclic group optionally substituted by substituent(s) selected from the group consisting of a halogen atom, a hydroxy group, a cyano group, a C₁₋₆ alkyl group, and a C₁₋₆ alkoxy group;
a 3- to 14-membered non-aromatic heterocyclic group optionally substituted by substituent(s) selected from the group consisting of a halogen atom, a hydroxy group, a cyano group, a C₁₋₆ alkyl group, and a C₁₋₆ alkoxy group;
a C₁₋₆ alkoxy group optionally substituted by substituent(s) selected from the group consisting of a halogen atom, a hydroxy group, a cyano group, and a C₁₋₆ alkoxy group;
a C₁₋₆ alkylsulfanyl group optionally substituted by substituent(s) selected from the group consisting of a halogen atom, a hydroxy group, a cyano group, and a C₁₋₆ alkoxy group;
a C₃₋₈ cycloalkyloxy group optionally substituted by substituent(s) selected from the group consisting of a halogen atom, a hydroxy group, a cyano group, and a C₁₋₆ alkoxy group;
a C₁₋₆ alkylsulfonyl group optionally substituted by halogen atom(s);
a C₁₋₆ alkyl-carbonyl group optionally substituted by halogen atom(s);
a C₁₋₆ alkoxy-carbonyl group;
a carbamoyl group optionally substituted by 1 or 2 C₁₋₆ alkyl groups optionally substituted by substituent(s) selected from the group consisting of a halogen atom, a hydroxy group, a di-C₁₋₆ alkylamino group, and a C₁₋₆ alkoxy group;
a sulfamoyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from the group consisting of a halogen atom, a hydroxy group, a cyano group, and a C₁₋₆ alkoxy group; and
a pentafluorosulfanyl group.

3. The compound according to claim 1 or a pharmaceutically acceptable salt thereof, wherein, in the formula (I),
R is a 2-(E)-phenylethenyl group optionally substituted by 1 to 3 substituents selected from the group consisting of a halogen atom, a C₁₋₆ alkyl group optionally substituted by halogen atom(s), and a pentafluorosulfanyl group;
R¹ is a carboxy group or a group biologically equivalent to a carboxy group;
L is a C₁₋₆ alkylene group optionally having, in the inside or at the end, one or more divalent groups selected from the group consisting of
-NR²- (wherein R² is a hydrogen atom or a C₁₋₆ alkyl group), -O-, -S-, -C(=O)-, a C₆₋₁₀ arylene group, a C₃₋₁₀ cycloalkylene group, a C₂₋₆ alkynylene group, and a 3- to 8-membered divalent non-aromatic heterocyclic group, and optionally further substituted by 1 to 3 substituents selected from the substituent group a, a C₆₋₁₀ arylene group optionally further substituted by 1 to 3 substituents selected from the substituent group a,
a C₃₋₁₀ cycloalkylene group optionally further substituted by 1 to 3 substituents selected from the substituent group a,
a C₂₋₄ alkynylene group optionally further substituted by 1 to 3 substituents selected from the substituent group a, or
a 3- to 8-membered divalent non-aromatic heterocyclic group optionally further substituted by 1 to 3 substituents selected from the substituent group a; and
A is a divalent nitrogen-containing non-aromatic heterocyclic group represented by the following formula: 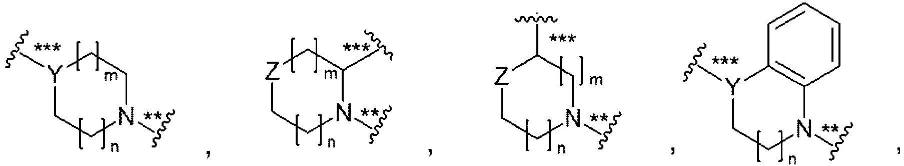 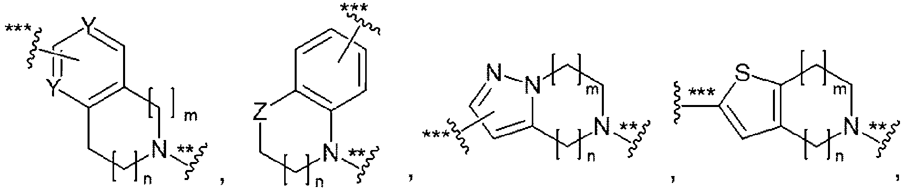 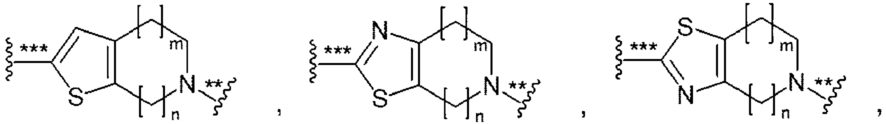 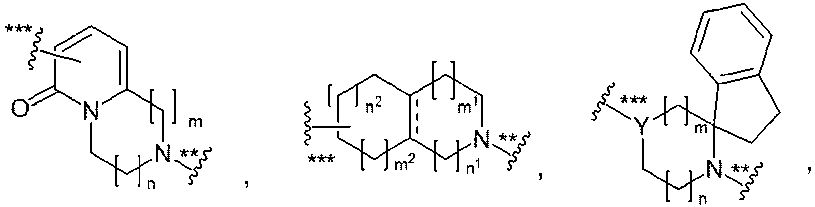 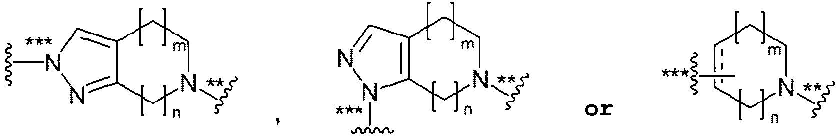
wherein a wavy line is a bonding site of the group represented by the chemical formula,
a bond represented by
is a single bond or a double bond, each Y is independently a nitrogen atom or CR³ (wherein R³ is a hydrogen atom or a C₁₋₆ alkyl group), Z is an oxygen atom, a sulfur atom, NR⁴ or CR⁵R⁶ (wherein R⁴, R⁵ and R⁶ are each independently a hydrogen atom or a C₁₋₆ alkyl group), n is an integer of 0 to 2, m is an integer of 0 to 2, n¹ and n² are each independently an integer of 0 to 2, m¹ and m² are each independently an integer of 0 to 2, ** is a bonding site with a carbonyl group, and *** is a bonding site with group L,
which non-aromatic heterocyclic group is optionally further substituted by 1 to 3 substituents selected from the substituent group a.

4. The compound according to claim 3 or a pharmaceutically acceptable salt thereof, wherein, in the formula (I),
L is a C₁₋₆ alkylene group optionally having, in the inside or at the end, one or more divalent groups selected from the group consisting of
-NR²- (wherein R² is a hydrogen atom or a C₁₋₆ alkyl group), -O-, -S-, a C₃₋₁₀ cycloalkylene group, and a 3- to 8-membered divalent non-aromatic heterocyclic group, and optionally further substituted by 1 to 3 substituents selected from the substituent group a,
a C₃₋₁₀ cycloalkylene group optionally further substituted by 1 to 3 substituents selected from the substituent group a, or a 3- to 8-membered divalent non-aromatic heterocyclic group optionally further substituted by 1 to 3 substituents selected from the substituent group a.

5. The compound according to claim 3 or a pharmaceutically acceptable salt thereof, wherein, in the formula (I),
L is a C₁₋₆ alkylene group optionally further substituted by 1 to 3 substituents selected from the substituent group a.

6. The compound according to claim 5 or a pharmaceutically acceptable salt thereof, wherein, in the formula (I),
A is a divalent nitrogen-containing non-aromatic heterocyclic group represented by the following formula: 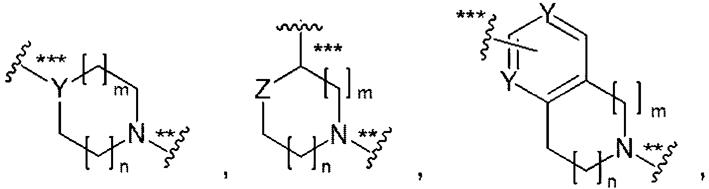 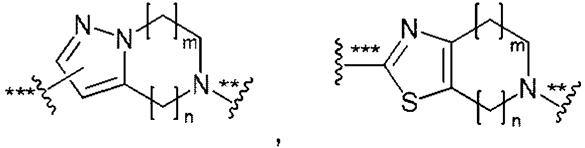 or 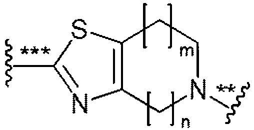
(wherein each symbol is as defined above), and optionally further substituted by 1 to 3 substituents selected from the substituent group a.

7. The compound according to claim 5 or a pharmaceutically acceptable salt thereof, wherein, in the formula (I),
A is a divalent nitrogen-containing non-aromatic heterocyclic group represented by the following formula: 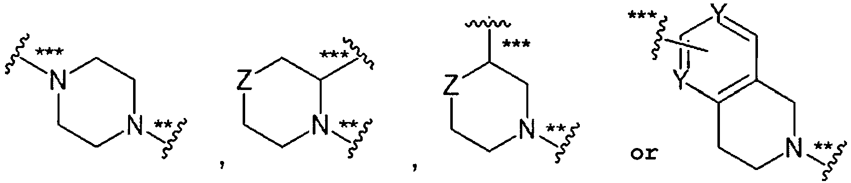
(wherein each symbol is as defined above, provided that when Z is CR⁵R⁶, the optionally further substituted substituent is selected from the group consisting of, among the substituents in the substituent group a, a C₆₋₁₄ hydrocarbocyclic group optionally substituted by 1 to 3 substituents selected from substituent group b; a 5- to 14-membered aromatic heterocyclic group optionally substituted by 1 to 3 substituents selected from substituent group b; and a 3- to 14-membered non-aromatic heterocyclic group optionally substituted by 1 to 3 substituents selected from the substituent group b), and optionally further substituted by 1 to 3 substituents selected from the substituent group a.

8. The compound according to claim 5 or a pharmaceutically acceptable salt thereof, wherein, in the formula (I),
A is a divalent nitrogen-containing non-aromatic heterocyclic group represented by the following formula: 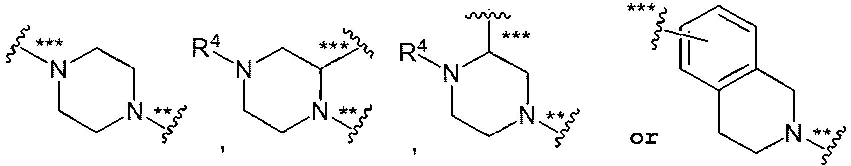
(wherein each symbol is as defined above), and optionally further substituted by 1 to 3 substituents selected from the substituent group a.

9. The compound according to claim 5 or a pharmaceutically acceptable salt thereof, wherein, in the formula (I),
A is a divalent nitrogen-containing non-aromatic heterocyclic group represented by the following formula: 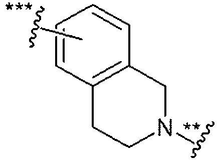
(wherein each symbol is as defined above), and optionally further substituted by 1 to 3 substituents selected from the substituent group a.

10. The compound according to claim 5 or a pharmaceutically acceptable salt thereof, wherein, in the formula (I),
A is a divalent nitrogen-containing non-aromatic heterocyclic group represented by the following formula: 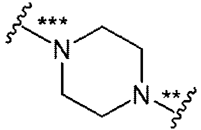
(wherein each symbol is as defined above), and optionally further substituted by 1 to 3 substituents selected from the substituent group a.

11. The compound according to any one of claims 1 to 10, or a pharmaceutically acceptable salt thereof, wherein, in the formula (I),
R¹ is a carboxy group, an optionally substituted C₁₋₆ alkoxy-carbonyl group, or an optionally substituted carbamoyl group.

12. A compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
3-[2-[2-[[(E)-3-[4-(trifluoromethyl)phenyl]prop-2-enoyl]amino]acetyl]-3,4-dihydro-1H-isoquinolin-6-yl]propanoic acid;
N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
1-[7-[2-[[(E)-3-[4-(trifluoromethyl)phenyl]prop-2-enoyl]amino]acetyl]-6,8-dihydro-5H-2,7-naphthyridin-3-yl]piperidine-4-carboxylic acid;
3-[3-bromo-5-[2-[[(E)-3-[4-(trifluoromethyl)phenyl]prop-2-enoyl]amino]acetyl]-6,7-dihydro-4H-pyrazolo[1,5-a]pyrazin-2-yl]propanoic acid;
2-[[2-[2-[[(E)-3-[4-(trifluoromethyl)phenyl]prop-2-enoyl]amino]acetyl]-3,4-dihydro-1H-isoquinolin-6-yl]oxy]acetic acid;
2-[2-[2-[[(E)-3-[4-(pentafluoro-A6-sulfanyl)phenyl]prop-2-enoyl]amino]acetyl]-3,4-dihydro-1H-isoquinolin-6-yl]acetic acid;
(1R*,2R*)-2-[2-[2-[[(E)-3-[4-(trifluoromethyl)phenyl]prop-2-enoyl]amino]acetyl]-3,4-dihydro-1H-isoquinolin-6-yl]cyclopropane-1-carboxylic acid and both enantiomers thereof;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[7-[2-[[(E)-3-[4-(trifluoromethyl)phenyl]prop-2-enoyl]amino]acetyl]-6,8-dihydro-5H-2,7-naphthyridin-3-yl]acetic acid;
3-[3-bromo-5-[2-[[(E)-3-[4-(pentafluoro-λ6-sulfanyl)phenyl]prop-2-enoyl]amino]acetyl]-6,7-dihydro-4H-pyrazolo[1,5-a]pyrazin-2-yl]propanoic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[[2-[2-[[(E)-3-[4-(trifluoromethyl)phenyl]prop-2-enoyl]amino]acetyl]-3,4-dihydro-1H-isoquinolin-6-yl]amino]acetic acid;
2-[2-[2-[[(E)-3-(4-iodophenyl)prop-2-encyl]amino]acetyl]-3,4-dihydro-1H-isoquinolin-6-yl]acetic acid;
2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-cyano-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-(2-fluoro-4-iodophenyl)prop-2-enoyl]amino]acetyl]-3,4-dihydro-1H-isoquinolin-6-yl]acetic acid;
2-[8-fluoro-2-[2-[[(E)-3-(4-iodophenyl)prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[[5-[2-[[(E)-3-[4-(trifluoromethyl)phenyl]prop-2-enoyl]amino]acetyl]-6,7-dihydro-4H-[1,3]thiazolo[5,4-c]pyridin-2-yl]sulfanyl]acetic acid;
2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-cyano-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
(E)-N-[2-[6-(2-amino-2-oxoethyl)-3,4-dihydro-1H-isoquinolin-2-yl]-2-oxoethyl]-3-[4-(trifluoromethyl)phenyl]prop-2-enamide;
2-[5-[2-[[(E)-3-[4-(trifluoromethyl)phenyl]prop-2-enoyl]amino]acetyl]-6,7-dihydro-4H-[1,3]thiazolo[4,5-c]pyridin-2-yl]acetic acid;
(E)-N-[2-[6-[2-(methylamino)-2-oxoethyl]-3,4-dihydro-1H-isoquinolin-2-yl]-2-oxoethyl]-3-[4-(trifluoromethyl)phenyl]prop-2-enamide;
4-[4-[2-[[(E)-3-[2-fluoro-4-(trifluoromethyl)phenyl]prop-2-enoyl]amino]acetyl]-3-phenylpiperazin-1-yl]butanoic acid and both enantiomers thereof;
2-[[1-[2-[[(E)-3-[4-(trifluoromethyl)phenyl]prop-2-enoyl]amino]acetyl]-2,3-dihydroindol-4-yl]oxy]acetic acid;
1-[1-[2-[[(E)-3-[4-(trifluoromethyl)phenyl]prop-2-enoyl]amino]acetyl]piperidin-3-yl]bicyclo[2.1.1]hexane-5-carboxylic acid;
2-[[8-fluoro-2-[2-[[(E)-3-[4-(trifluoromethyl)phenylJprop-2-enoyl]amino]acetyl]-3,4-dihydro-1H-isoquinolin-6-yl]oxy]-2-methylpropanoic acid;
3-[[8-fluoro-2-[2-[[(E)-3-[4-(trifluoromethyl)phenyl]prop-2-enoyl]amino]acetyl]-3,4-dihydro-1H-isoquinolin-6-yl]oxy]-2,2-dimethylpropanoic acid;
4-[4-[2-[[(E)-3-[2-fluoro-4-(trifluoromethyl)phenyl]prop-2-enoyl]amino]acetyl]-2-methylpiperazin-1-yl]butanoic acid;
4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
3-[[3-(4-chlorophenyl)-4-[2-[[(E)-3-[2-fluoro-4-(trifluoromethyl)phenyl]prop-2-enoyl]amino]acetyl]piperazin-1-yl]methyl]cyclobutane-1-carboxylic acid and both enantiomers thereof;
4-[3-(4-chlorophenyl)-4-[2-[[(E)-3-[2-fluoro-4-(trifluoromethyl)phenyl]prop-2-enoyl]amino]acetyl]piperazin-1-yl]-2,2-dimethylbutanoic acid;
4-[3-(3-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid and both enantiomers thereof;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[3-(4-chloro-3-fluorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
4-[(2R,5S)-5-(4-chlorophenyl)-4-[2-[[(E)-3-[4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid or a pharmaceutically acceptable salt thereof.

13. A compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
3-[2-[2-[[(E)-3-[4-(trifluoromethyl)phenyl]prop-2-enoyl]amino]acetyl]-3,4-dihydro-1H-isoquinolin-6-yl]propanoic acid;
N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
(1R*,2R*)-2-[2-[2-[[(E)-3-[4-(trifluoromethyl)phenyl]prop-2-enoyl]amino]acetyl]-3,4-dihydro-1H-isoquinolin-6-yl]cyclopropane-1-carboxylic acid and both enantiomers thereof;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[2-[2-[[(E)-3-(4-iodophenyl)prop-2-enoyl]amino]acetyl]-3,4-dihydro-1H-isoquinolin-6-yl]acetic acid;
2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-cyano-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-(2-fluoro-4-iodophenyl)prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[5-[2-[[(E)-3-[4-(trifluoromethyl)phenyl]prop-2-enoyl]amino]acetyl]-6,7-dihydro-4H-[1,3]thiazolo[4,5-c]pyridin-2-yl]acetic acid;
4-[(3S)-4-[2-[[(E)-3-[2-fluoro-4-(trifluoromethyl)phenyl]prop-2-enoyl]amino]acetyl]-3-phenylpiperazin-1-yl]butanoic acid;
1-[1-[2-[[(E)-3-[4-(trifluoromethyl)phenyl]prop-2-enoyl]amino]acetyl]piperidin-3-yl]bicyclo[2.1.1]hexane-5-carboxylic acid;
4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
3-[[3-(4-chlorophenyl)-4-[2-[[(E)-3-[2-fluoro-4-(trifluoromethyl)phenyl]prop-2-enoyl]amino]acetyl]piperazin-1-yl]methyl]cyclobutane-1-carboxylic acid and both enantiomers thereof;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid and both enantiomers thereof;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[3-(4-chloro-3-fluorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
4-1(2R,55)-5-(4-chlorophenyl)-4-[2-[[(E)-3-[4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid,
or a pharmaceutically acceptable salt thereof.

14. A compound selected from the group consisting of 2-[7-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[2-[2-[[(E)-3-(4-iodophenyl)prop-2-enoyl]amino]acetyl]-3,4-dihydro-1H-isoquinolin-6-yl]acetic acid;
2-[5-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-fluoro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[7-chloro-2-[2-[[(E)-3-[4-(trifluoromethyl)phenyl]prop-2-enoyl]amino]acetyl]-3,4-dihydro-1H-isoquinolin-6-yl]acetic acid;
2-[5-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
4-[(3S)-4-[2-[[(E)-3-[2-fluoro-4-(trifluoromethyl)phenyl]prop-2-enoyl]amino]acetyl]-3-phenylpiperazin-1-yl]butanoic acid;
1-[1-[2-[[(E)-3-[4-(trifluoromethyl)phenyl]prop-2-enoyl]amino]acetyl]piperidin-3-yl]bicyclo[2.1.1]hexane-5-carboxylic acid;
4-[(3S)-3-(4-bromophenyl)-4-[2-[[(E)-3-[2-fluoro-4-(trifluoromethyl)phenyl]prop-2-enoyl]amino]acetyl]piperazin-1-yl]butanoic acid;
4-[3-(3-bromophenyl)-4-[2-[[(E)-3-[2-fluoro-4-(trifluoromethyl)phenyl]prop-2-enoyl]amino]acetyl]piperazin-1-yl]butanoic acid;
4-[3-(4-chlorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-5-(3,4-difluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(3,4-difluorophenyl)-2-methyl-4-[2-[[(E)-3-[4-(trifluoromethyl)phenyl]prop-2-enoyl]amino]acetyl]piperazin-1-yl]butanoic acid;
4-[(2R,5S)-5-(4-chlorophenyl)-4-[2-[[(E)-3-[4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
4-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-5-(4-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]-2,2-dimethylbutanoic acid,
or a pharmaceutically acceptable salt thereof.

15. A compound selected from the group consisting of 2-[2-[2-[[(E)-3-[4-(trifluoromethyl)phenyl]prop-2-enoyl]amino]acetyl]-3,4-dihydro-1H-isoquinolin-6-yl]acetic acid;
3-[2-[2-[[(E)-3-[4-(trifluoromethyl)phenyl]prop-2-enoyl]amino]acetyl]-3,4-dihydro-1H-isoquinolin-6-yl]propanoic acid;
N,N-dimethyl-3-[5-[2-[[(E)-3-[4-(trifluoromethyl)phenyl]prop-2-enoyl]amino]acetyl]-6,7-dihydro-4H-pyrazolo[1,5-a]pyrazin-3-yl]propanamide;
2-[2-[2-[[(E)-3-[4-(pentafluoro-λ6-sulfanyl)phenyl]prop-2-enoyl]amino]acetyl]-3,4-dihydro-1H-isoquinolin-6-yl]acetic acid;
2-[2-[2-[[(E)-3-[4-(trifluoromethyl)phenyl]prop-2-enoyl]amino]acetyl]-3,4-dihydro-1H-isoquinolin-6-yl]propanoic acid;
2-[8-fluoro-2-[2-[[(E)-3-[4-(trifluoromethyl)phenyl]prop-2-enoyl]amino]acetyl]-3,4-dihydro-1H-isoquinolin-6-yl]acetic acid;
2-[2-[2-[[(E)-3-[2-fluoro-4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[4-(trifluoromethyl)phenyl]prop-2-enoyl]amino]acetyl]-3,4-dihydro-1H-isoquinolin-6-yl]acetic acid;
2-[8-chloro-2-[2-[[(E)-3-[2-fluoro-4-(trifluoromethyl)phenyl]prop-2-enoyl]amino]acetyl]-3,4-dihydro-1H-isoquinolin-6-yl]acetic acid;
4-[(3R)-3-(4-chlorophenyl)-4-[2-[[(E)-3-[2-fluoro-4-(trifluoromethyl)phenyl]prop-2-enoyl]amino]acetyl]piperazin-1-yl]butanoic acid;
4-[(2R,5S)-4-[2-[[(E)-3-[2-fluoro-4-(trifluoromethyl)phenyl]prop-2-enoyl]amino]acetyl]-2-methyl-5-phenylpiperazin-1-yl]butanoic acid;
3-[(2R,5S)-5-(4-chlorophenyl)-4-[2-[[(E)-3-[4-(trifluoromethyl)phenyl]prop-2-enoyl]amino]acetyl]-2-methylpiperazin-1-yl]propanoic acid;
3-[(2R,5S)-5-(4-chlorophenyl)-4-[2-[[(E)-3-[2-fluoro-4-(trifluoromethyl)phenyl]prop-2-enoyl]amino]acetyl]-2-methylpiperazin-1-yl]propanoic acid;
4-[(2R,5S)-5-(3-fluorophenyl)-4-[2-[[(E)-3-[2-fluoro-4-(trifluoromethyl)phenyl]prop-2-enoyl]amino]acetyl]-2-methylpiperazin-1-yl]butanoic acid;
4-[(2R,5S)-2-ethyl-4-[2-[[(E)-3-[2-fluoro-4-(trifluoromethyl)phenyl]prop-2-enoyl]amino]acetyl]-5-phenylpiperazin-1-yl]butanoic acid; and
4-[(3S)-3-(4-cyclopropylphenyl)-4-[2-[[(E)-3-[2-fluoro-4-(trifluoromethyl)phenyl]prop-2-enoyl]amino]acetyl]piperazin-1-yl]butanoic acid,
or a pharmaceutically acceptable salt thereof.

16. A B0AT1 inhibitor comprising a compound according to any one of claims 1 to 15 or a pharmaceutically acceptable salt thereof.

17. A pharmaceutical composition comprising a compound according to any one of claims 1 to 15 or a pharmaceutically acceptable salt thereof, and a pharmaceutically acceptable carrier.

18. The pharmaceutical composition according to claim 17, for the prophylaxis and/or treatment of a disease whose symptoms can be alleviated by the B0AT1 inhibitory action.

19. The pharmaceutical composition according to claim 18, wherein the disease whose symptoms can be alleviated by the B0AT1 inhibitory action is an amino acid metabolism disorder.

20. The B0AT1 inhibitor according to claim 19, wherein the amino acid metabolism disorder is phenylketonuria, urea cycle disorder, hypertyrosinemia (types 1-3), hypermethioninemia, maple syrup urine disease, homocystinuria, nonketotic hyperglycinemia, propionic acidemia, methylmalonic acidemia, or isovaleric acidemia.

21. The pharmaceutical composition according to claim 19, wherein the amino acid metabolism disorder is phenylketonuria.
